# EUROPEAN PATENT APPLICATION

(11) **EP 2 669 285 A1**
(43) Date of publication of application: **04.12.2013**
(21) Application number: 12739093.8
(22) Date of filing: 25.01.2012
(51) Int. Cl.: C07D 471/04, A61K 31/437, A61K 31/444, A61K 31/5377, A61P 13/02, A61P 43/00

(54) **PYRAZOLOPYRIDINE DERIVATIVE OR PHARMACOLOGICALLY ACCEPTABLE SALT THEREOF**

(30) Priority: 26.01.2011 JP 2011013783
(71) Applicant: Kyorin Pharmaceutical Co., Ltd., Tokyo 101-8311 (JP); Kissei Pharmaceutical Co., Ltd., Matsumoto-shi Nagano 399-8710 (JP)
(72) Inventor: SETO, Shigeki, Shimotsuga-gun Tochigi 329-0114 (JP); UMEI, Kentaro, Shimotsuga-gun Tochigi 329-0114 (JP); NISHIGAYA, Yosuke, Shimotsuga-gun Tochigi 329-0114 (JP); TANIOKA, Asao, Shimotsuga-gun Tochigi 329-0114 (JP); KONDO, Tatsuhiro, Joetsu-shi Niigata 942-0145 (JP); KONDO, Atsushi, Azumino-shi Nagano 399-8304 (JP); TATANI, Kazuya, Azumino-shi Nagano 399-8304 (JP); KAWAMURA, Naohiro, Azumino-shi Nagano 399-8304 (JP)
(74) Representative: Boult Wade Tennant
(86) International application number: PCT/JP2012/051518
(87) International publication number: WO 2012/102297

(57) **Abstract**

A pyrazolopyridine derivative represented by the following formula (I) or a pharmacologically acceptable salt thereof exhibits a strong EP₁ receptor antagonistic effect. Thus, the derivative or the pharmacologically acceptable salt is useful as a therapeutic agent for lower urinary tract symptoms (LUTS), particularly, overactive bladder syndrome (OABs), or a prophylactic agent therefor and furthermore, is also useful in the treatment, prevention, or suppression of various pathological conditions in which the EP₁ receptor is involved, such as inflammatory disease, pain disease, osteoporosis, and cancer. 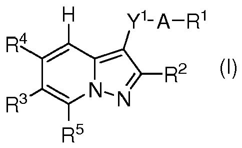 [A is a benzene ring or the like, Y¹ is C₁₋₆ alkylene, R¹ is -C(=O)-OZ¹ or the like, Z¹ is H or the like, R² is a branched C₃₋₆ alkyl group or the like, R³ is H or the like, R⁴ is a hydrogen atom or the like, and R⁵ is a hydrogen atom or the like].

## Description

### Technical Field

The present invention relates to a pyrazolopyridine derivative having an EP₁ receptor antagonistic effect that is useful as a pharmaceutical product, or a pharmacologically acceptable salt thereof, a pharmaceutical composition containing it, and pharmaceutical use thereof.

### Background Art

With the development of an aging society/stressful society, the number of patients with lower urinary tract dysfunction (LUTD) is increasing. LUTD is a generic name for urine collection disorder and dysuria, and symptoms developed from LUTD are lower urinary tract symptoms (LUTS). There is overactive bladder syndrome (OABs) as one of LUTS. In general, OABs is also called overactive bladder (OAB). In any case, it is a disease defined as a "symptom or syndrome that has urgency to urinate as an essential requirement and is usually accompanied by urinary frequency and nocturia. Urge incontinence is not essential". These symptoms that accompany OABs interfere with the entirety of life such as jobs, daily life, and mental activity and reduce the quality of life (QOL). Currently, anticholinergic agents are first-line agents as therapeutic agents for OABs. However, the anticholinergic agents need to be used with due consideration given to antimuscarinic effects such as dry mouth or residual urine and moreover, are not always effective for all patients (see, for example, Non Patent Literature 1). Under such circumstances, the development of therapeutic agents with a mechanism different from the anticholinergic agents has been demanded (see, for example, Non Patent Literature 1).

In recent years, the role of the urothelium in LUTS, particularly, OABs, has received attention. As for LUTS, it has also become clear that various chemical transmitters are released in urothelial cells and cause bladder reflex via receptors of bladder sensory nerve terminals. Among others, prostaglandin E₂ (PGE₂), one of the chemical transmitters, increases bladder reflex by binding to prostaglandin E receptor 1 (EP₁ receptor) in afferent nerves (particularly, C fibers) in the urothelium. Moreover, PGE₂ contracts the bladder by binding to the EP₁ receptor present in bladder smooth muscles. In fact, it has been reported that EP₁ receptor antagonists suppress both of increase in bladder reflex and increase in afferent nervous activity by PGE₂ (see, for example, Non Patent Literature 2 and Non Patent Literature 3). From these, it is suggested that PGE₂ is involved in the contraction of bladder smooth muscles and increase in bladder sensory nerves via the EP₁ receptor. Furthermore, it has also been reported that EP₁ receptor antagonists increase a bladder capacity without increasing the amount of residual urine (see, for example, Non Patent Literature 4).

Four subtypes, EP₁ as well as EP₂, EP₃, and EP₄, are present as receptors of PGE₂. The EP₁ receptor is present in the lung, skeletal muscles, and renal collecting tubules and the like, in addition to the bladder and the urothelium (see, for example, Non Patent Literature 2). Hence, it is expected that a therapeutic agent for the desired disease can be developed by changing the selectivity against a PGE₂ receptor subtype or the target organ or target tissue of the agent.

Currently, a compound represented by chemical structural formula (A): 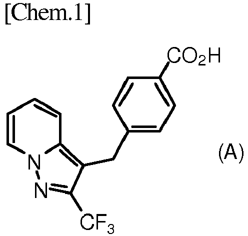 is disclosed as a compound having a tumor necrosis factor-converting enzyme inhibitor in Non Patent Literature 5. However, this compound differs in chemical structural formula at the position of a substituent from the compound of the present invention. Moreover, there is neither description nor suggestion about an EP₁ receptor antagonistic effect. Incidentally, it is needless to say that such a compound is not included in claims of the present application.

### Citation List

### Non Patent Literature

Non Patent Literature 1: Narihito Seki, "FOLIA PHARMACOLOGICA JAPONICA", 2007, vol. 129, p. 368-373
Non Patent Literature 2: Xiaojun Wang, et al., "Biomedical Research", 2008, vol. 29, p. 105-111
Non Patent Literature 3: Masahito Kawatani, "PAIN RESEARCH", 2004, vol. 19, p. 185-190
Non Patent Literature 4: Masanobu Maekawa, "Journal of The Japan Neurogenic Bladder Society", 2008, vol.19, p.169
Non Patent Literature 5: Zhonghui Lu, et al., "Bioorganic & Medicinal Chemistry Letters", 2008, vol.18, p.1958-1962

### Summary of Invention

### Technical Problem

A compound that has an excellent EP₁ receptor antagonistic effect as prophylactic and therapeutic agents for various pathological conditions described above and can serve as a pharmaceutical product sufficiently satisfactory also in terms of less side effects and the like, has not yet been found.

An object of the present invention is to provide a compound having an EP₁ receptor antagonistic effect or a pharmacologically acceptable salt thereof, a pharmaceutical composition containing it, and pharmaceutical use thereof.

### Solution to Problem

The present inventors have conducted diligent studies and consequently completed the present invention by finding that a pyrazolopyridine derivative represented by the following formula (I) (hereinafter, also referred to as compound (I)) or a pharmacologically acceptable salt thereof has a strong EP₁ receptor antagonistic effect.

Specifically, the present invention is as follows:

[1] A pyrazolopyridine derivative represented by the following formula (I) or a pharmacologically acceptable salt thereof: 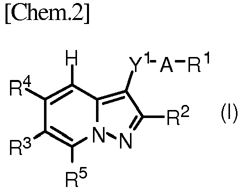 wherein A is a group selected from the group consisting of the following a) to h): 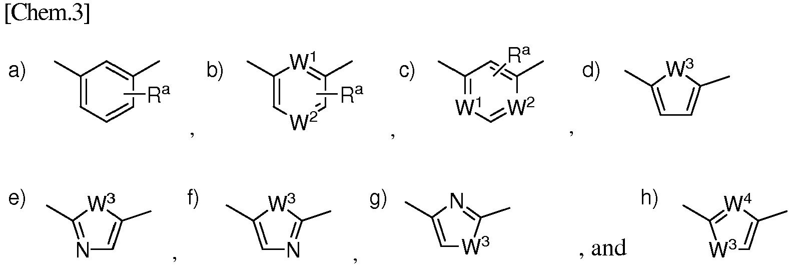 or a group i) constituted together with R¹: 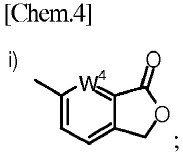 R^{a} is a group selected from the group consisting of the following j) to q):
j) a hydrogen atom,
k) a halogen atom,
l) a hydroxy group,
m) a cyano group,
n) a nitro group,
o) an amino group unsubstituted or substituted by one or two C₁₋₆ alkyl groups,
p) a C₁₋₆ alkyl group unsubstituted or substituted by one or two groups independently selected from the group consisting of a halogen atom, a C₁₋₆ alkoxy group, a C₁₋₆ alkylsulfanyl group, a C₁₋₆ alkylsulfinyl group, a C₁₋₆ alkylsulfonyl group, a hydroxy group, and a cyano group, and
q) a C₁₋₆ alkoxy group unsubstituted or substituted by one or two groups independently selected from the group consisting of a halogen atom, a C₁₋₆ alkoxy group, a C₁₋₆ alkylsulfanyl group, a C₁₋₆ alkylsulfonyl group, a hydroxy group, and a cyano group;
   one of W¹ and W² is a nitrogen atom, and the other is -CH= or a nitrogen atom;
   W³ is an oxygen atom or a sulfur atom;
   W⁴ is -CH= or a nitrogen atom;
   Y¹ is C₁₋₆ alkylene;
   R¹ is a group selected from the group consisting of the following r) to x):
r) -C(=O)-OZ¹,
s) -C(=O)-NHSO₂Z²,
t) -C(=O)-NHOH,
u) -C(=O)-NHCN,
v) -NH-C(=O)-Z³,
w) an acidic 5-membered heterocyclic group, and
x) a 6-membered ring group substituted by a phenolic hydroxy group;
   Z¹ is a hydrogen atom, a C₁₋₆ alkyl group, or a C₇₋₁₀ aralkyl group;
   Z² and Z³ are independently a group selected from the group consisting of the following aa) to ee):
aa) a C₁₋₆ alkyl group,
bb) a halo-C₁₋₆ alkyl group,
cc) a C₃₋₆ cycloalkyl group,
dd) an aryl group unsubstituted or in which a ring is substituted by one to five groups independently selected from the group consisting of a halogen atom, a C₁₋₆ alkyl group, a halo-C₁₋₆ alkyl group, and a C₁₋₆ alkoxy group, and
ee) a heterocyclic group;
R² is a group selected from the group consisting of the following A) to H):
A) a branched C₃₋₆ alkyl group,
B) a C₃₋₆ cycloalkyl group unsubstituted or in which a ring is substituted by one C₁₋₆ alkyl group,
C) a phenyl group unsubstituted or in which a ring is substituted by one to five groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, and a cyano group,
D) a 6-membered aromatic heterocyclic group unsubstituted or in which a ring is substituted by one to four groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, and a cyano group,
E) a 5-membered aromatic heterocyclic group unsubstituted or in which a ring is substituted by one to four groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, and a cyano group,
F) an amino group substituted by one or two C₁₋₆ alkyl groups,
G) a 4- to 8-membered cyclic amino group unsubstituted or in which a ring is substituted by one to three groups independently selected from the group consisting of a C₁₋₆ alkyl group and a halogen atom, and
H) a C₁₋₆ alkoxy group;
R³ is a group selected from the group consisting of the following I) to W):
I) a hydrogen atom,
J) a halogen atom,
K) a hydroxy group,
L) a cyano group,
M) a nitro group,
N) a C₃₋₆ cycloalkyl group,
O) a C₂₋₆ alkenyl group,
P) a C₁₋₇ alkanoyl group,
Q) a C₁₋₆ alkylsulfanyl group,
R) a C₁₋₆ alkylsulfinyl group,
S) a C₁₋₆ alkylsulfonyl group,
T) an amino group unsubstituted or substituted by one or two C₁₋₆ alkyl groups,
U) a C₁₋₆ alkyl group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, a benzoyloxy group, and a cyano group,
V) a C₁₋₆ alkoxy group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, and a cyano group, and
W) a phenyl group unsubstituted or in which a ring is substituted by one to five groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, and a cyano group; and
R⁴ and R⁵ each independently represent a hydrogen atom, a halogen atom, a C₁₋₆ alkyl group, or a C₁₋₆ alkoxy group.

[2] The pyrazolopyridine derivative or the pharmacologically acceptable salt thereof according to [1], wherein in the above formula (I), A is a group selected from the group consisting of the following a), b), d), and h): 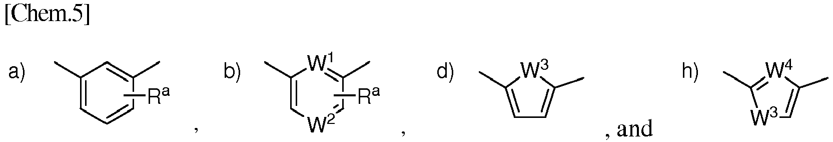 wherein R^{a}, W¹, W², W³, and W⁴ have the same meanings as above.

[3] The pyrazolopyridine derivative or the pharmacologically acceptable salt thereof according to [2], wherein in the above formula (I), A is a group selected from the group consisting of the following a), b1), and d1): [Chem.6]

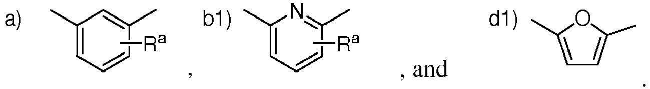

[4] The pyrazolopyridine derivative or the pharmacologically acceptable salt thereof according to [3], wherein in the above formula (I), Y¹ is methylene.

[5] The pyrazolopyridine derivative or the pharmacologically acceptable salt thereof according to [4], wherein in the above formula (I), R⁴ and R⁵ are each independently a hydrogen atom, a halogen atom, or a C₁₋₆ alkoxy group.

[6] The pyrazolopyridine derivative or the pharmacologically acceptable salt thereof according to [5], wherein in the above formula (I), Z¹ is a hydrogen atom or a C₁₋₆ alkyl group, and Z² is a C₁₋₆ alkyl group.

[7] The pyrazolopyridine derivative or the pharmacologically acceptable salt thereof according to [6], wherein in the above formula (I), R² is a group selected from the group consisting of the following A, B), C1), D1), E1), and G):
A) a branched C₃₋₆ alkyl group,
B) a C₃₋₆ cycloalkyl group unsubstituted or in which a ring is substituted by one C₁₋₆ alkyl group,
C1) a phenyl group unsubstituted or in which a ring is substituted by one to five groups independently selected from the group consisting of a halogen atom, a C₁₋₆ alkyl group, a halo-C₁₋₆ alkyl group, a C₁₋₆ alkoxy group, a halo-C₁₋₆ alkoxy group, and a cyano group, D1) a 6-membered aromatic heterocyclic group,
E1) a 5-membered aromatic heterocyclic group, and
G) a 4- to 8-membered cyclic amino group unsubstituted or in which a ring is substituted by one to three groups independently selected from the group consisting of a C₁₋₆ alkyl group and a halogen atom.

[8] The pyrazolopyridine derivative or the pharmacologically acceptable salt thereof according to [7], wherein in the above formula (I), R² is a group selected from the group consisting of the following A), B), C1), D2), E2), and G1):
A) a branched C₃₋₆ alkyl group,
B) a C₃₋₆ cycloalkyl group unsubstituted or in which a ring is substituted by one C₁₋₆ alkyl group,
C1) a phenyl group unsubstituted or in which a ring is substituted by one to five groups independently selected from the group consisting of a halogen atom, a C₁₋₆ alkyl group, a halo-C₁₋₆ alkyl group, a C₁₋₆ alkoxy group, a halo-C₁₋₆ alkoxy group, and a cyano group,
D2) a pyridyl group,
E2) a thienyl group, and
G1) a morpholinyl group.

[9] The pyrazolopyridine derivative or the pharmacologically acceptable salt thereof according to [7], wherein in the above formula (I), R³ is a group selected from the group consisting of the following I) to L), N), O) to Q), and T) to W):
I) a hydrogen atom,
J) a halogen atom,
K) a hydroxy group,
L) a cyano group,
N) a C₃₋₆ cycloalkyl group,
O) a C₂₋₆ alkenyl group,
P) a C₁₋₇ alkanoyl group,
Q) a C₁₋₆ alkylsulfanyl group,
T) an amino group unsubstituted or substituted by one or two C₁₋₆ alkyl groups,
U) a C₁₋₆ alkyl group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, a benzoyloxy group, and a cyano group,
V) a C₁₋₆ alkoxy group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, and a cyano group, and
W) a phenyl group unsubstituted or in which a ring is substituted by one to five groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, and a cyano group.

[10] The pyrazolopyridine derivative or the pharmacologically acceptable salt thereof according to [9], wherein in the above formula (I), R³ is a hydrogen atom, a halogen atom, a cyano group, a C₃₋₆ cycloalkyl group, a C₂₋₆ alkenyl group, a C₁₋₇ alkanoyl group, an amino group, a methylamino group, a halo-C₁₋₆ alkyl group, a C₁₋₆ alkylsulfanyl-C₁₋₆ alkyl group, a C₁₋₆ alkylsulfonyl-C₁₋₆ alkyl group, a hydroxy-C₁₋₆ alkyl group, a benzoyloxy-C₁₋₆ alkyl group, a C₁₋₆ alkyl group, a C₁₋₆ alkoxy group, or a phenyl group.

[11] The pyrazolopyridine derivative or the pharmacologically acceptable salt thereof according to [9], wherein in the above formula (I), R^{a} is a group selected from the group consisting of the following j), k), o), p1), and q1):
j) a hydrogen atom,
k) a halogen atom,
o) an amino group unsubstituted or substituted by one or two C₁₋₆ alkyl groups,
p1) a C₁₋₆ alkyl group unsubstituted or substituted by one or two groups independently selected from the group consisting of a C₁₋₆ alkoxy group, a C₁₋₆ alkylsulfonyl group, and a hydroxy group, and
q1) a C₁₋₆ alkoxy group unsubstituted or substituted by one or two groups independently selected from the group consisting of a C₁₋₆ alkoxy group, a C₁₋₆ alkylsulfonyl group, and a hydroxy group.

[12] The pyrazolopyridine derivative or the pharmacologically acceptable salt thereof according to [11], wherein in the above formula (I), R^{a} is a hydrogen atom, a halogen atom, a C₁₋₆ alkyl group, a hydroxy-C₁₋₆ alkyl group, or a C₁₋₆ alkoxy group.

[13] The pyrazolopyridine derivative or the pharmacologically acceptable salt thereof according to [1], wherein the compound represented by the above formula (I) is
3-[(6-chloro-2-phenylpyrazolo[1,5-a]pyridin-3-yl)methyl]benzoic acid,
6-[(6-chloro-2-phenylpyrazolo[1,5-a]pyridin-3-yl)methyl]pyridine-2-carboxylic acid,
3-[(6-methoxy-2-phenylpyrazolo[1,5-a]pyridin-3-yl)methyl]benzoic acid,
6-[(6-methoxy-2-phenylpyrazolo[1,5-a]pyridin-3-yl)methyl]pyridine-2-carboxylic acid,
6-[(2-phenyl-6-vinylpyrazolo[1,5-a]pyridin-3-yl)methyl]pyridine-2-carboxylic acid,
6-[(2-*tert*-butyl-6-methoxypyrazolo[1,5-a]pyridin-3-yl)methyl]pyridine-2-carboxylic acid,
6- [[2-phenyl-6-(trifluoromethyl)pylazolo[1,5-a]pyridin-3-yl]methyl]pyridine-2-carboxylic acid,
6-[(6-methyl-2-phenylpyrazolo[1,5-a]pyridin-3-yl)methyl]pyridine-2-carboxylic acid,
6-[[2-(3-fluorophenyl)-6-methoxypyrazolo[1,5-a]pyridin-3-yl]methyl]pyridine-2-carboxylic acid,
6-[[6-chloro-2-(3-fluorophenyl)pylazolo[1,5-a]pyridin-3-yl]methyl]pyridine-2-carboxylic acid,
6-methoxy-2-phenyl-3-[[6-(1*H*-tetrazol-5-yl)pyridin-2-yl]methyl]pylazolo[1,5-a]pyridine,
6-chloro-2-phenyl-3-[[6-(1*H*-tetrazol-5-yl)pyridin-2-yl]methyl]pyrazolo[1,5-a]pyridine,
5-[(6-methoxy-2-phenylpyrazolo[1,5-a]pyridin-3-yl)methyl]furan-2-carboxylic acid,
6-[(6-bromo-2-phenylpyrazolo[1,5-a]pyridin-3-yl)methyl]pyridine-2-carboxylic acid,
6-[(2-cyclopentyl-6-methoxypyrazolo[1,5-a]pyridin-3-yl)methyl]pyridine-2-carboxylic acid,
6-[[2-(4-fluorophenyl)-6-methoxypylazolo[1,5-a]pyridin-3-yl]methyl]pyridine-2-carboxylic acid,
6-[[6-methoxy-2-(thiophen-3-yl)pyrazolo[1,5-a]pyridin-3-yl]methyl]pyridine-2-carboxylic acid,
2-[(6-chloro-2-phenylpyrazolo[1,5-a]pyridin-3-yl)methyl]thiazole-4-carboxylic acid,
6-[[6-(methylthio)-2-phenylpylazolo[1,5-a]pyridin-3-yl]methyl]pyridine-2-carboxylic acid,
6-[(2-*tert*-butyl-6-chloropyrazolo[1,5-a]pyridin-3-yl)methyl]pyridine-2-carboxylic acid,
6-[[6-chloro-2-(piperidin-1-yl)pyrazolo[1,5-a]pyridin-3-yl]methyl]pyridine-2-carboxylic acid,
6-[[6-chloro-2-(1-methylcyclopropyl)pyrazolo[1,5-a]pyridin-3-yl]methyl]pyridine-2-carboxy lic acid,
6-[(6-chloro-2-phenylpyrazolo[1,5-a]pyridin-3-yl)methyl]-*N-*(methylsulfonyl)pyridin-2-carb oxamide,
3-[6-[(6-chloro-2-phenylpyrazolo[1,5-a]pyridin-3-yl)methyl]pyridin-2-yl]-1,2,4-oxadiazol-5( 4*H*)-one, or
3-[6-[(6-chloro-2-phenylpyrazolo[1,5-a]pyridin-3-yl)methyl]pyridin-2-yl]-1,2,4-oxadiazol-5( 4*H*)-thione.

[14] A pharmaceutical composition comprising the pyrazolopyridine derivative or the pharmacologically acceptable salt thereof according to any one of [1] to [13] as an active ingredient.

[15] An EP₁ receptor antagonist comprising the pyrazolopyridine derivative or the pharmacologically acceptable salt thereof according to any one of [1] to [13] as an active ingredient.

[16] A therapeutic or prophylactic agent for lower urinary tract symptoms comprising the pyrazolopyridine derivative or the pharmacologically acceptable salt thereof according to any one of [1] to [13] as an active ingredient.

[17] A method for treating or preventing lower urinary tract symptoms, comprising administering a pharmaceutical composition comprising the pyrazolopyridine derivative or the pharmacologically acceptable salt thereof according to any one of [1] to [13] as an active ingredient to a patient.

[18] Use of the pyrazolopyridine derivative or the pharmacologically acceptable salt thereof according to any one of [1] to [13] for the manufacture of a pharmaceutical composition for prevention or treatment of lower urinary tract symptoms.
[19] The pyrazolopyridine derivative or the pharmacologically acceptable salt thereof according to any one of [1] to [13] for use in prevention or treatment of lower urinary tract symptoms.

### Advantageous Effects of Invention

The compound (I) of the present invention or a pharmacologically acceptable salt thereof exhibited a strong EP₁ receptor antagonistic effect, for example, in an EP₁ receptor antagonistic effect confirmation test. Furthermore, the compound of the present invention and the salt thereof are safe as toxicity is also low. Accordingly, the compound (I) of the present invention or the pharmacologically acceptable salt thereof is useful as a therapeutic agent for lower urinary tract symptoms (LUTS), particularly, overactive bladder syndrome (OABs), and the like, or a prophylactic agent therefor on the basis of the EP₁ receptor antagonistic effect confirmation test.

Furthermore, the compound (I) of the present invention or the pharmacologically acceptable salt thereof has high usefulness for the treatment, prevention, or suppression of various pathological conditions in which the EP₁ receptor is involved (lower urinary tract symptoms (LUTS), inflammatory disease, pain disease, osteoporosis, cancer, and the like).

### Description of Embodiments

Hereinafter, the present invention will be described in detail.

"C₁₋₆ alkylene" means a divalent linear or branched saturated hydrocarbon chain with a carbon number of 1 to 6. Examples thereof include -CH₂-, -(CH₂)₂-, -CH(CH₃)-, -(CH₂)₃-, -CH(CH₃)CH₂-, -CH₂CH(CH₃)-, -CH(CH₂CH₃)-, -C(CH₃)₂-, -(CH₂)₄-, -CH(CH₃)-(CH₂)₂-, -(CH₂)₂-CH(CH₃)-, -CH(CH₂CH₃)-CH₂-, -C(CH₃)₂CH₂-, -CH₂-C(CH₃)₂-, -CH(CH₃)-CH(CH₃)-, -(CH₂)₅-, -CH(CH₃)-(CH₂)₃-, -C(CH₃)₂CH₂CH₂-, -(CH₂)₆-, -C(CH₃)₂-(CH₂)₃-, and the like. Examples of one that is preferred include -CH₂-. Incidentally, in the present specification, -CH₂- is also referred to as methylene.

A "C₁₋₆ alkyl group" means a linear or branched alkyl group with a carbon number of 1 to 6. Examples thereof include a methyl group, an ethyl group, a propyl group, an isopropyl group, a butyl group, an isobutyl group, a *sec*-butyl group, a *tert*-butyl group, a pentyl group, an isopentyl group, a neopentyl group, a 1-methylbutyl group, a 2-methylbutyl group, a 1,2-dimethylpropyl group, a hexyl group, an isohexyl groupand the like.

Examples of a "halogen atom" include a fluorine atom, a chlorine atom, a bromine atom, and an iodine atom.

A "C₁₋₆ alkoxy group" means a linear or branched alkoxy group with a carbon number of 1 to 6. Examples thereof include a methoxy group, an ethoxy group, a propoxy group, an isopropoxy group, an isobutoxy group, a butoxy group, a *sec*-butoxy group, a *tert*-butoxy group, a pentyloxy group, a hexyloxy group, and the like. Examples of one that is preferred include a methoxy group and an ethoxy group.

A "halo-C₁₋₆ alkoxy group" means a C₁₋₆ alkoxy group substituted by 1 to 5 halogen atoms of the same type or different types. Examples thereof include a monofluoromethoxy group, a difluoromethoxy group, a trifluoromethoxy group, a 2-fluoroethoxy group, a 2-chloroethoxy group, a 2,2-difluoroethoxy group, a 1,1-difluoroethoxy group, a 1,2-difluoroethoxy group, a 2,2,2-trifluoroethoxy group, a 1,1,2,2,2-pentafluoroethoxy group, a 2,2,2-trichloroethoxy group, a 3-fluoropropoxy group, a 2-fluoropropoxy group, a 1-fluoropropoxy group, a 3,3-difluoropropoxy group, a 2,2-difluoropropoxy group, a 1,1-difluoropropoxy group, a 4-fluorobutoxy group, a 5-fluoropentyloxy group, a 6-fluorohexyloxy group, and the like. Examples of one that is preferred include a monofluoromethoxy group, a difluoromethoxy group, and a trifluoromethoxy group.

A "C₁₋₇ alkanoyl group" means an acyl group derived from a linear or branched aliphatic carboxylic acid having 1 to 7 carbon atoms, and examples thereof include a formyl group, an acetyl group, a propanoyl group, a butanoyl group, a pentanoyl group, a hexanoyl group, and the like.

A "C₁₋₆ alkylsulfanyl group" means a group represented by (C₁₋₆ alkyl)-S-. Examples thereof include a methylsulfanyl group, an ethylsulfanyl group, a propylsulfanyl group, an isopropylsulfanyl group, a butylsulfanyl group, an isobutylsulfanyl group, a *sec*-butylsulfanyl group, a pentylsulfanyl group, a hexylsulfanyl group, and the like.

A "C₁₋₆ alkylsulfinyl group" means a group represented by (C₁₋₆ alkyl)-S(=O)-. Examples thereof include a methylsulfinyl group, an ethylsulfinyl group, a propylsulfinyl group, an isopropylsulfinyl group, a butylsulfinyl group, an isobutylsulfinyl group, a *sec*-butylsulfinyl group, a pentylsulfinyl group, a hexylsulfinyl group, and the like.

A "C₁₋₆ alkylsulfonyl group" means a group represented by (C₁₋₆ alkyl)-SO₂-. Examples thereof include a methylsulfonyl group, an ethylsulfonyl group, a propylsulfonyl group, an isopropylsulfonyl group, a butylsulfonyl group, an isobutylsulfonyl group, a *sec*-butylsulfonyl group, a pentylsulfonyl group, a hexylsulfonyl group, and the like.

A "C₇₋₁₀ aralkyl group" means an alkyl group with a carbon number of 1 to 4 substituted by a phenyl group. Examples thereof include a benzyl group, a phenethyl group, a 1-phenylethyl group, a 3-phenylpropyl group, a 4-phenylbutyl group, and the like.

A "halo-C₁₋₆ alkyl group" means a C₁₋₆ alkyl group substituted by 1 to 5 halogen atoms of the same type or different types. Examples thereof include a fluoromethyl group, a difluoromethyl group, a trifluoromethyl group, a 2-fluoroethyl group, a 2-chloroethyl group, a 2,2-difluoroethyl group, a 1,1-difluoroethyl group, a 1,2-difluoroethyl group, a 2,2,2-trifluoroethyl group, a 1,1,2,2,2-pentafluoroethyl group, a 2,2,2-trichloroethyl group, a 3-fluoropropyl group, a 2-fluoropropyl group, a 1-fluoropropyl group, a 3,3-difluoropropyl group, a 2,2-difluoropropyl group, a 1,1-difluoropropyl group, a 4-fluorobutyl group, a 5-fluoropentyl group, a 6-fluorohexyl group, 2,2,2-trifluoro-1-trifluoromethylethyl, and the like. Examples of one that is preferred include a fluoromethyl group, a difluoromethyl group, and a trifluoromethyl group.

A "C₃₋₆ cycloalkyl group" means a monocyclic saturated alicyclic hydrocarbon group with a carbon number of 3 to 6. Examples thereof can include a cyclopropyl group, a cyclobutyl group, a cyclopentyl group, a cyclohexyl group, and the like.

A "C₃₋₆ cycloalkyl group in which a ring is substituted by one C₁₋₆ alkyl group" refers to the above-described C₃₋₆ cycloalkyl group in which a ring is substituted by the above-described C₁₋₆ alkyl group. Examples thereof include a 1-methylcyclopropyl group, a 1-ethylcyclopropyl group, a 1-methylcyclobutyl group, a 2-methylcyclobutyl group, a 1-methylcyclopentyl group, a 2-methylcyclopentyl group, a 1-methylcyclohexyl group, a 2-methylcyclohexyl group, and the like. One that is preferred is a 1-methylcyclopropyl group and a 1-ethylcyclopropyl group, and one that is more preferred is a 1-methylcyclopropyl group.

An "aryl group" means an aromatic hydrocarbon group with a carbon number of 6 to 14, such as a phenyl group, an indenyl group, a naphthyl group, a phenanthrenyl group, and an anthracenyl group. One that is preferred is a "C₆₋₁₀ aryl group", which means an aromatic hydrocarbon group with a carbon number of 6 to 10, and examples thereof include a phenyl group, an indenyl group, and a naphthyl group.

A "heterocyclic group" refers to a 5- to 7-membered heterocyclic group containing 1 to 4 atoms selected from among a sulfur atom, an oxygen atom, and a nitrogen atom, and examples thereof can include aromatic heterocyclic groups such as a furyl group, a thienyl group, a pyrrolyl group, an azepinyl group, a pyrazolyl group, an imidazolyl group, an oxazolyl group, an isoxazolyl group, a thiazolyl group, an isothiazolyl group, a 1,2,3-oxadiazolyl group, a triazolyl group, a tetrazolyl group, a thiadiazolyl group, a pyranyl group, a pyridyl group, a pyridazinyl group, a pyrimidinyl group, and a pyrazinyl group, and saturated heterocyclic groups such as a morpholinyl group, a thiomorpholinyl group, a pyrrolidinyl group, a pyrrolinyl group, an imidazolidinyl group, an imidazolinyl group, a pyrazolidinyl group, a pyrazolinyl group, a piperidyl group, a piperazinyl group, and the like. Incidentally, the "heterocyclic group" may be condensed with another cyclic group, and examples thereof can include an isobenzofuranyl group, a benzoxazolyl group, a benzisoxazolyl group, a benzothiazolyl group, a benzisothiazolyl group, a chromenyl group, a chromanonyl group, a xanthenyl group, a phenoxazinyl group, an indolizinyl group, an isoindolizinyl group, an indolyl group, an indazolyl group, a purinyl group, a quinolizinyl group, an isoquinolyl group, a quinolyl group, a phthalazinyl group, a naphthyridinyl group, a quinoxalinyl group, a quinazolinyl group, a carbazolyl group, a carbolinyl group, an acridinyl group, an isoindolinyl group, and the like.

A "branched C₃₋₆ alkyl group" means a branched alkyl group with a carbon number of 3 to 6. Examples thereof include an isopropyl group, an isobutyl group, a *sec*-butyl group, a *tert*-butyl group, an isopentyl group, a neopentyl group, a *tert*-pentyl group, a 1-methylbutyl group, a 2-methylbutyl group, a 1,2-dimethylpropyl group, a 1-ethylpropyl group, an isohexyl group, and the like. Examples of one that is preferred include an isopropyl group, an isobutyl group, a *sec*-butyl group, a *tert*-butyl group, and a 1-ethylpropyl group.

A "hydroxy-C₁₋₆ alkyl group" means a C₁₋₆ alkyl group substituted by a hydroxy group. Examples thereof include a hydroxymethyl group, a 1-hydroxyethyl group, a 1-hydroxy-1,1-dimethylmethyl group, a 2-hydroxyethyl group, a 2-hydroxy-2-methylpropyl group, a 3-hydroxypropyl group, and the like.

A "5-membered aromatic heterocyclic group" means a 5-membered aromatic ring group containing 1 to 4 heteroatoms selected from an oxygen atom, a nitrogen atom, and a sulfur atom in the ring. Examples thereof include a furyl group, a pyrrolyl group, a thienyl group, an imidazolyl group, a pyrazolyl group, a 1,2,4-triazolyl group, an isothiazolyl group, an isoxazolyl group, an oxazolyl group, a thiazolyl group, a 1,3,4-oxadiazolyl group, a 1,2,4-oxadiazolyl group, and the like.

A "6-membered aromatic heterocyclic group" means a 6-membered aromatic ring group containing 1 to 4 nitrogen atoms in the ring. Examples thereof include a pyridyl group, a pyrimidinyl group, a pyrazinyl group, a pyridazinyl group, and the like.

Examples of a "4- to 8-membered cyclic amino group unsubstituted or in which a ring is substituted by one to three groups independently selected from the group consisting of a C₁₋₆ alkyl group and a halogen atom" include a group selected from the group shown below:

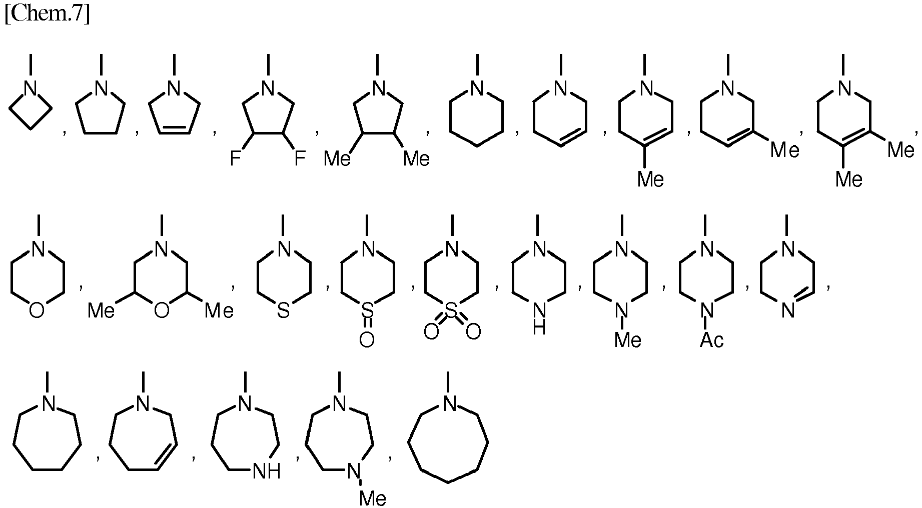

An "acidic 5-membered heterocyclic group" means a 5-membered ring containing a nitrogen atom bonded to an acidic proton in the ring, or a 5-membered nitrogen-containing heterocyclic ring having a phenolic hydroxy group. Examples thereof include groups of the group consisting of formulae: 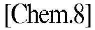 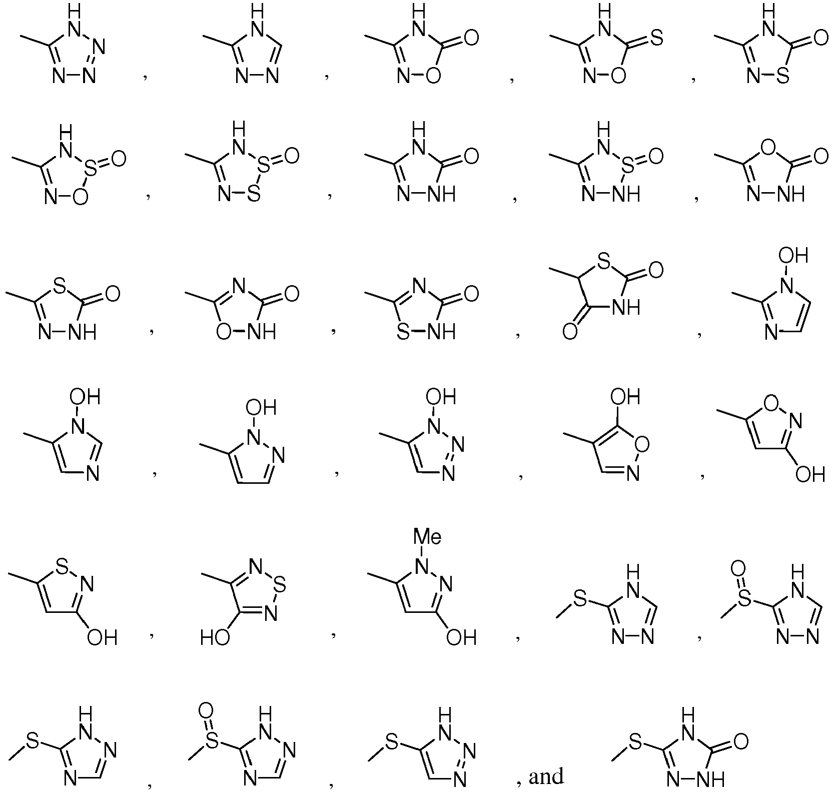 One that is preferred is a group selected from the group consisting of formulae: 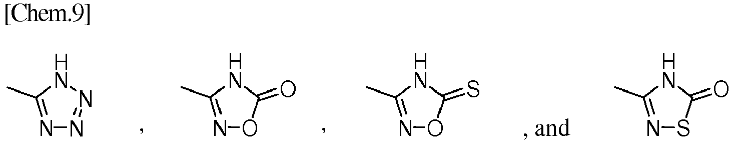 One that is more preferred is a 5-tetrazolyl group represented by a formula:

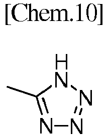

A "6-membered aromatic ring group substituted by a phenolic hydroxy group" means a 6-membered heterocyclic group or aromatic ring group having a phenolic hydroxy group. It is, for example, groups represented by formulae:

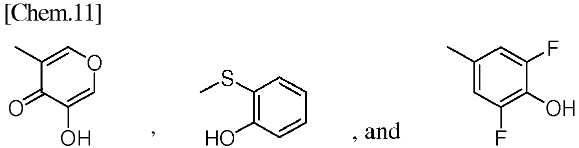

A "C₂₋₆ alkenyl group" means a linear or branched unsaturated hydrocarbon group with a carbon number of 2 to 6 having at least one double bond. Examples thereof include a vinyl group, a 2-propenyl group, a 1-propenyl group, a 3-propenyl group, a 1-buten-1-yl group, a 1-buten-2-yl group, a 1-buten-3-yl group, a 1-buten-4-yl group, a 2-buten-1-yl group, a 2-buten-2-yl group, a 1-penten-1-yl group, a 1-penten-2-yl group, a 1-penten-3-yl group, a 2-penten-1-yl group, a 2-penten-2-yl group, a 2-penten-3-yl group, a 1-hexen-1-yl group, a 1-hexen-2-yl group, a 1-hexen-3-yl group, a 2-methyl-1-propen-1-yl group, and the like.

Preferable substituents for the compound (I) of the present invention or a pharmacologically acceptable salt thereof are as follows:

A is preferably a benzene ring, a pyridine ring, a furan ring, a thiazole ring, a benzene ring or a pyridine ring in which a ring is substituted by halogen atoms, a benzene ring or a pyridine ring in which a ring is substituted by C₁₋₆ alkyl groups, a benzene ring or a pyridine ring in which a ring is substituted by hydroxy-C₁₋₆ alkyl groups, or a benzene ring or a pyridine ring in which a ring is substituted by C₁₋₆ alkoxy groups, more preferably a benzene ring, a pyridine ring, a pyridine ring in which a ring is substituted by halogen atoms, a pyridine ring in which a ring is substituted by C₁₋₆ alkyl groups, a pyridine ring in which a ring is substituted by hydroxy-C₁₋₆ alkyl groups, or a pyridine ring in which a ring is substituted by C₁₋₆ alkoxy groups, particularly preferably a benzene ring, a pyridine ring, a pyridine ring substituted by a chlorine atom, a pyridine ring substituted by a methyl group, a pyridine ring substituted by a hydroxymethyl group, or a pyridine ring substituted by a methoxy group.

Y¹ is preferably methylene.

R¹ is preferably -C(=O)-OZ¹, -C(=O)-NHSO₂Z², or an acidic 5-membered heterocyclic group, more preferably -C(=O)-OH, -C(=O)-O-C₁₋₆ alkyl group, -C(=O)-NHSO₂-C₁₋₆ alkyl group, or a tetrazolyl group, particularly preferably -C(=O)-OH, -C(=O)-OMe, -C(=O)-NHSO₂Me, or a 5-tetrazolyl group.

R² is preferably a branched C₃₋₆ alkyl group, a C₃₋₆ cycloalkyl group unsubstituted or in which a ring is substituted by one C₁₋₆ alkyl group, a phenyl group, a phenyl group in which a ring is substituted by halogen atoms, a phenyl group in which a ring is substituted by C₁₋₆ alkyl groups, a phenyl group in which a ring is substituted by halo-C₁₋₆ alkyl groups, a phenyl group in which a ring is substituted by C₁₋₆ alkoxy groups, a 6-membered aromatic heterocyclic group, a 5-membered aromatic heterocyclic group, or a 4- to 8-membered cyclic amino group, more preferably a branched C₃₋₆ alkyl group, a C₃₋₆ cycloalkyl group unsubstituted or in which a ring is substituted by one C₁₋₆ alkyl group, a phenyl group, a phenyl group in which a ring is substituted by halogen atoms, a phenyl group in which a ring is substituted by C₁₋₆ alkyl groups, a phenyl group in which a ring is substituted by halo-C₁₋₆ alkyl groups, a phenyl group in which a ring is substituted by C₁₋₆ alkoxy groups, a pyridyl group, a thienyl group, or a morpholinyl group, particularly preferably an isobutyl group, a *tert*-butyl group, a cyclopropyl group, a cyclopentyl group, a cyclohexyl group, a 1-methylcyclopropyl group, a phenyl group, a 4-fluorophenyl group, a 4-chlorophenyl group, a 4-methylphenyl group, a 4-trifluoromethylphenyl group, a 4-methoxyphenyl group, a 3-pyridyl group, a 3-thienyl group, or a 4-morpholinyl group.

R³ is preferably a hydrogen atom, a halogen atom, a cyano group, a C₃₋₆ cycloalkyl group, a C₂₋₆ alkenyl group, a C₁₋₇ alkanoyl group, an amino group, an amino group substituted by C₁₋₆ alkyl groups, a C₁₋₆ alkyl group, a halo-C₁₋₆ alkyl group, a C₁₋₆ alkylsulfanyl-C₁₋₆ alkyl group, a C₁₋₆ alkylsulfonyl-C₁₋₆ alkyl group, a hydroxy-C₁₋₆ alkyl group, a benzoyloxy-C₁₋₆ alkyl group, a C₁₋₆ alkoxy group, or a phenyl group, more preferably a hydrogen atom, a fluorine atom, a chlorine atom, a bromine atom, a cyano group, a cyclopropyl group, a vinyl group, a 2-propenyl group, an acetyl group, an amino group, a methylamino group, a methyl group, a trifluoromethyl group, a 2-bromoethyl group, a 2-(methylsulfanyl)ethyl group, a 2-(methylsulfonyl)ethyl group, a hydroxyethyl group, a methoxy group, or a phenyl group.

R⁴ is preferably a hydrogen atom, a halogen atom, or a C₁₋₆ alkoxy group, more preferably a hydrogen atom or a methoxy group.

R⁵ is preferably a hydrogen atom, a halogen atom, or a C₁₋₆ alkoxy group, more preferably a hydrogen atom or a methoxy group.

6-[(2-phenylpyrazolo[1,5-a]pyridin-3-yl)methyl]pyridine-2-carboxylic acid,
3-[(6-chloro-2-phenylpyrazolo[1,5-a]pyridin-3-yl)methyl]benzoic acid,
6-[(6-chloro-2-phenylpyrazolo[1,5-a]pyridin-3-yl)methyl]pyridine-2-carboxylic acid,
3-[(6-methoxy-2-phenylpyrazolo[1,5-a]pyridin-3-yl)methyl]benzoic acid,
6-[(6-methoxy-2-phenylpyrazolo[1,5-a]pyridin-3-yl)methyl]pyridine-2-carboxylic acid,
5-[(6-methoxy-2-phenylpyrazolo[1,5-a]pyridin-3-yl)methyl]furan-2-carboxylic acid,
3-[(2-phenylpyrazolo[1,5-a]pyridin-3-yl)methyl]benzoic acid,
*N*-methanesulfonyl-6-[(6-methoxy-2-phenylpylazolo[1,5-a]pyridin-3-yl)methyl]pyridine-2-c arboxamide,
6-methoxy-2-phenyl-3-[[6-(1*H*-tetrazol-5-yl)pyridin-2-yl]methyl]pylazolo[1,5-a]pyridine,
6-[[2-phenyl-6-(propen-2-yl)pyrazolo[1,5-a]pyridin-3-yl]methyl]pyridine-2-carboxylic acid,
6-[(2-phenyl-6-vinylpyrazolo[1,5-a]pyridin-3-yl)methyl]pyridine-2-carboxylic acid,
6-[(6-cyano-2-phenylpyrazolo[1,5-a]pyridin-3-yl)methyl]pyridine-2-carboxylic acid,
6-[(6-acetyl-2-phenylpyrazolo[1,5-a]pyridin-3-yl)methyl]pyridine-2-carboxylic acid,
6-[(6-bromo-2-phenylpyrazolo[1,5-a]pyridin-3-yl)methyl]pyridine-2-carboxylic acid,
6-[(2-*tert*-butyl -6-methoxypyrazolo[1,5-a]pyridin-3-yl)methyl]pyridine-2-carboxylic acid,
6-[(2-isobutyl-6-methoxypyrazolo[1,5-a]pyridin-3-yl)methyl]pyridine-2-carboxylic acid,
6-[(2-cyclopropyl-6-methoxypyrazolo[1,5-a]pyridin-3-yl)methyl]pyridine-2-carboxylic acid,
6-[(2-cyclopentyl-6-methoxypyrazolo[1,5-a]pyridin-3-yl)methyl]pyridine-2-carboxylic acid,
6-[(2-cyclohexyl-6-methoxypyrazolo[1,5-a]pyridin-3-yl)methyl]pyridine-2-carboxylic acid,
6-[(6-fluoro-2-phenylpyrazolo[1,5-a]pyridin-3-yl)methyl]pyridine-2-carboxylic acid,
6-[(6-methyl-2-phenylpyrazolo[1,5-a]pyridin-3-yl)methyl]pyridine-2-carboxylic acid,
6-[[2-phenyl-6-(trifluoromethyl)pyrazolo[1,5-a]pyridin-3-yl]methyl]pyridine-2-carboxylic acid,
6-[(6-cyclopropyl-2-phenylpyrazolo[1,5-a]pyridin-3-yl)methyl]pyridine-2-carboxylic acid, 6-[[2-(4-fluorophenyl)-6-methoxypylazolo[1,5-a]pyridin-3-yl]methyl]pyridine-2-carboxylic acid,
6-[[6-methoxy-2-(4-methylphenyl)pylazolo[1,5-a]pyridin-3-yl]methyl]pyridine-2-carboxylic acid,
6-[[6-methoxy-2-(4-methoxyphenyl)pylazolo[1,5-a]pyridin-3-yl]methyl]pyridine-2-carboxyl ic acid,
6-[[2-(4-chlorophenyl)-6-methoxypylazolo[1,5-a]pyridin-3-yl]methyl]pyridine-2-carboxylic acid,
6-[[6-methoxy-2-(pyridin-3-yl)pylazolo[1,5-a]pyridin-3-yl]methyl]pyridine-2-carboxylic acid,
6-[[6-methoxy-2-[2-(trifluoromethyl)phenyl]pyrazolo[1,5-a]pyridin-3-yl]methyl]pyridine-2-carboxylic acid,
6-[[6-methoxy-2-[3-(trifluoromethyl)phenyl]pyrazolo[1,5-a]pyridin-3-yl]methyl]pyridine-2-carboxylic acid,
6-[[6-methoxy-2-[4-(trifluoromethyl)phenyl]pyrazolo[1,5-a]pyridin-3-yl]methyl]pyridine-2-carboxylic acid,
6-[[6-methoxy-2-(thiophen-3-yl)pyrazolo[1,5-a]pyridin-3-yl]methyl]pyridine-2-carboxylic acid,
6-[[6-chloro-2-(morpholin-4-yl)pylazolo[1,5-a]pyridin-3-yl]methyl]pyridine-2-carboxylic acid,
4-methoxy-6-[(6-methoxy-2-phenylpyrazolo[1,5-a]pyridin-3-yl)methyl]pyridine-2-carboxyli c acid,
4-chloro-6-[(6-methoxy-2-phenylpyrazolo[1,5-a]pyridin-3-yl)methyl]pyridine-2-carboxylic acid,
2-(hydroxymethyl)-5-[(6-methoxy-2-phenylpyrazolo[1,5-a]pyridin-3-yl)methyl]benzoic acid,
5-[(6-methoxy-2-phenylpyrazolo[1,5-a]pyridin-3-yl)methyl]-2-methylbenzoic acid,
6-[[6-(2-hydroxyethyl)-2-phenylpyrazolo[1,5-a]pyridin-3-yl]methyl]pyridine-2-carboxylic acid,
6-[[6-[2-(methylsulfanyl)ethyl]-2-phenylpyrazolo[1,5-a]pyridin-3-yl]methyl]pyridine-2-carb oxylic acid,
6-[[6-[2-(methylsulfonyl)ethyl]-2-phenylpylazolo[1,5-a]pyridin-3-yl]methyl]pyridine-2-carb oxylic acid, 6-[[2-(morpholin-4-yl)pyrazolo[1,5-a]pyridin-3-yl]methyl]pyridine-2-carboxylic acid,
6-[[6-methyl-2-(morpholin-4-yl)pylazolo[1,5-a]pyridin-3-yl]methyl]pyridine-2-carboxylic acid,
6-[[6-bromo-2-(morpholin-4-yl)pyrazolo[1,5-a]pyridin-3-yl]methyl]pyridine-2-carboxylic acid,
6-[[6-iodo-2-(morpholin-4-yl)pylazolo[1,5-a]pyridin-3-yl]methyl]pyridine-2-carboxylic acid,
6-[[2-(morpholin-4-yl)-6-(trifluoromethyl)pyrazolo[1,5-a]pyridin-3-yl]methyl]pyridine-2-car boxylic acid,
6-[[6-methoxy-2-(morpholin-4-yl)pylazolo[1,5-a]pyridin-3-yl]methyl]pyridine-2-carboxylic acid,
2-[(6-chloro-2-phenylpyrazolo[1,5-a]pyridin-3-yl)methyl]thiazole-4-carboxylic acid,
6-[(7-chloro-2-phenylpyrazolo[1,5-a]pyridine)methyl]pyridine-2-carboxylic acid,
3-[(6-methoxy-2-phenylpyrazolo[1,5-a]pyridin-3-yl)methyl]-5-methylbenzoic acid,
3-(hydroxymethyl)-5-[(6-methoxy-2-phenylpyrazolo[1,5-a]pyridin-3-yl)methyl]benzoic acid,
3-[(6-methoxy-2-phenylpyrzolo[1,5-a]pyridin-3-yl)methyl]-5-[(methylthio)methyl]benzoic acid,
3-[(6-methoxy-2-phenylpylazolo[1,5-a]pyridin-3-yl)methyl]-5-[(methylslfinyl)methyl]benzo ic acid,
3-[(6-methoxy-2-phenylpyrazolo[1,5-a]pyridin-3-yl)methyl]-5-[(methylsulfonyl)methyl]ben zoic acid, 3-amino-5-[(6-methoxy-2-phenylpyrazolo[1,5-a]pyridin-3-yl)methyl]benzoic acid,
6-[(2,6-diphenylpyrazolo[1,5-a]pyridin-3-yl)methyl]pyridine-2-carboxylic acid,
6-[[2-phenyl-6-(propen-2-yl)pyrazolo[1,5-a]pyridin-3-yl]methyl]pyridine-2-carboxylic acid,
6-[[6-(methylamino)-2-phenylpyrazolo[1,5-a]pyridin-3-yl]methyl]pyridine-2-carboxylic acid,
6-[[6-[2-(methylthio)ethyl]-2-phenylpyrazolo[1,5-a]pyridin-3-yl]methyl]pyridine-2-carboxyl ic acid,
6-[[6-(methylthio)-2-phenylpyrazolo[1,5-a]pyridin-3-yl]methyl]pyridine-2-carboxylic acid,
6-[[6-(methylsulfinyl)-2-phenylpylazolo[1,5-a]pyridin-3-yl]methyl]pyridine-2-carboxylic acid,
6-[[6-(methylsulfonyl)-2-phenylpyrazolo[1,5-a]pyridin-3-yl]methyl]pyridine-2-carboxylic acid,
6-[[6-chloro-2-cyclopropylpyrazolo[1,5-a]pyridin-3-yl]methyl]pyridine-2-carboxylic acid,
6-[(6-chloro-2-cyclopentylpyrazolo[1,5-a]pyridin-3-yl)methyl]pyridine-2-carboxylic acid,
6-[(6-chloro-2-cyclohexylpyrazolo[1,5-a]pyridin-3-yl)methyl]pyridine-2-carboxylic acid,
6-[(2-*tert*-butyl-6-chloropyrazolo[1,5-a]pyridin-3-yl)methyl]pyridine-2-carboxylic acid,
6-[[6-chloro-2-(2-methylpropyl)pyrazolo[1,5-a]pyridin-3-yl]methyl]pyridine-2-carboxylic acid,
6-[[6-chloro-2-(pyrrolidin-1-yl)pyrazolo[1,5-a]pyridin-3-yl]methyl]pyridine-2-carboxylic acid,
6-[[6-chloro-2-(piperidin-1-yl)pyrazolo[1,5-a]pyridin-3-yl]methyl]pyridine-2-carboxylic acid,
6-[[6-chloro-2-(hexahydro-1*H*-azepin-1-yl)pyrazolo[1,5-a]pyridin-3-yl]methyl]pyridine-2-ca rboxylic acid,
6-[[6-chloro-2-(dimethylamino)pyrazolo[1,5-a]pyridin-3-yl]methyl]pyridine-2-carboxylic acid,
6-[[6-chloro-2-(thiomorpholin-4-yl)pyrazolo[1,5-a]pyridin-3-yl]methyl]pyridine-2-carboxyli c acid,
6-[[6-chloro-2-(thiophen-3-yl)pyrazolo[1,5-a]pyridin-3-yl]methyl]pyridine-2-carboxylic acid,
6-[[6-chloro-2-(thiophen-2-yl)pyrazolo[1,5-a]pyridin-3-yl]methyl]pyridine-2-carboxylic acid,
6-[[6-chloro-2-(furan-2-yl)pyrazolo[1,5-a]pyridin-3-yl]methyl]pyridine-2-carboxylic acid,
6-[[6-chloro-2-(furan-3-yl)pyrazolo[1,5-a]pyridin-3-yl]methyl]pyridine-2-carboxylic acid,
6-[(6-chloro-2-methoxypyrazolo[1,5-a]pyridin-3-yl)methyl]pyridine-2-carboxylic acid,
6-[[6-chloro-2-(2-propyloxy)pyrazolo[1,5-a]pyridin-3-yl]methyl]pyridine-2-carboxylic acid,
6-[[6-chloro-2-(1-methylcylopropyl)pylazolo[1,5-a]pyridin-3-yl]methyl]pyridine-2-carboxy lic acid,
6-[[6-chloro-2-(4-fluorophenyl)pylazolo[1,5-a]pyridin-3-yl]methyl]pyridine-2-carboxylic acid,
6-[[6-chloro-2-(3-fluorophenyl)pylazolo[1,5-a]pyridin-3-yl]methyl]pyridine-2-carboxylic acid,
6-[[6-chloro-2-(2-fluorophenyl)pylazolo[1,5-a]pyridin-3-yl]methyl]pyridine-2-carboxylic acid,
6-[[6-chloro-2-(4-methoxyphenyl)pylazolo[1,5-a]pyridin-3-yl]methyl]pyridine-2-carboxylic acid,
6-[[6-chloro-2-(3-methoxyphenyl)pylazolo[1,5-a]pyridin-3-yl]methyl]pyridine-2-carboxylic acid,
6-[[6-chloro-2-(2-methoxyphenyl)pylazolo[1,5-a]pyridin-3-yl]methyl]pyridine-2-carboxylic acid,
6-[[6-chloro-2-(4-methylphenyl)pylazolo[1,5-a]pyridin-3-yl]methyl]pyridine-2-carboxylic acid,
6-[[6-chloro-2-(3-methylphenyl)pylazolo[1,5-a]pyridin-3-yl]methyl]pyridine-2-carboxylic acid,
6-[[6-chloro-2-(2-methylphenyl)pylazolo[1,5-a]pyridin-3-yl]methyl]pyridine-2-carboxylic acid,
6-[[6-chloro-2-[4-(trifluoromethyl)phenyl]pyrazolo[1,5-a]pyridin-3-yl]methyl]pyridine-2-car boxylic acid,
6-[[6-chloro-2-[3-(trifluoromethyl)phenyl]pyrazolo[1,5-a]pyridin-3-yl]methyl]pyridine-2-car boxylic acid,
6-[[6-chloro-2-[2-(trifluoromethyl)phenyl]pyrazolo[1,5-a]pyridin-3-yl]methyl]pyridine-2-car boxylic acid,
6-[[6-chloro-2-(4-chlorophenyl)pyrazolo[1,5-a]pyridin-3-yl]methyl]pyridine-2-carboxylic acid,
6-[[6-chloro-2-(3-chlorophenyl)pyrazolo[1,5-a]pyridin-3-yl]methyl]pyridine-2-carboxylic acid,
6-[[6-chloro-2-(2-chlorophenyl)pyrazolo[1,5-a]pyridin-3-yl]methyl]pyridine-2-carboxylic acid,
6-[[2-(2-fluorophenyl)-6-methoxypylazolo[1,5-a]pyridin-3-yl]methyl]pyridine-2-carboxylic acid,
6-[[2-(3-fluorophenyl)-6-methoxypyrazolo[1,5-a]pyridin-3-yl]methyl]pyridine-2-carboxylic acid,
4-(3-hydroxypropyl)-6-[(6-methoxy-2-phenylpyrazolo[1,5-a]pyridin-3-yl)methyl]pyridine-2 -carboxylic acid,
6-[(6-methoxy-2-phenylpyrazolo[1,5-a]pyridin-3-yl)methyl]-4-[3-(methylsulfonyl)propyl]py ridine-2-carboxylic acid,
6-[(6-methoxy-2-phenylpyrazolo[1,5-a]pyridin-3-yl)methyl]pyrazine-2-carboxylic acid,
6-[(6-chloro-2-phenylpylazolo[1,5-a]pyridin-3-yl)methyl]-*N*-(methylsulfonyl)pyridine-2-car boxamide,
3-[6-[(6-chloro-2-phenylpyrazolo[1,5-a]pyridin-3-yl)methyl]pyridin-2-yl]-1,2,4-oxadiazol-5( 4*H*)-one,
3-[6-[(6-chloro-2-phenylpyrazolo[1,5-a]pyridin-3-yl)methyl]pyridin-2-yl]-1,2,4-oxadiazol-5( 4*H*)-thione,
3-[6-[(6-chloro-2-phenylpyrazolo[1,5-a]pyridin-3-yl)methyl]pyridin-2-yl]-1,2,4-thiadiazol-5( 4*H*)-one,
6-chloro-2-phenyl-3-[[6-(1*H*-tetrazol-5-yl)pyridin-2-yl]methyl]pyrazolo[1 5-a]pyridine,
2-[6-[(6-chloro-2-phenylpyrazolo[1,5-a]pyridin-3-yl)methyl]-2-pyridylthio]phenol,
4-[6-[(6-chloro-2-phenylpylazolo[1,5-a]pyridin-3-yl)methyl]-2-pyridyl]-2,6-difluorophenol.

Moreover, another aspect of the present invention is as follows:
[P1] A pyrazolopyridine derivative represented by the following formula (I) or a pharmacologically acceptable salt thereof: 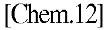 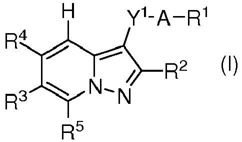 [wherein A is a group selected from the group consisting of the following a) to h): 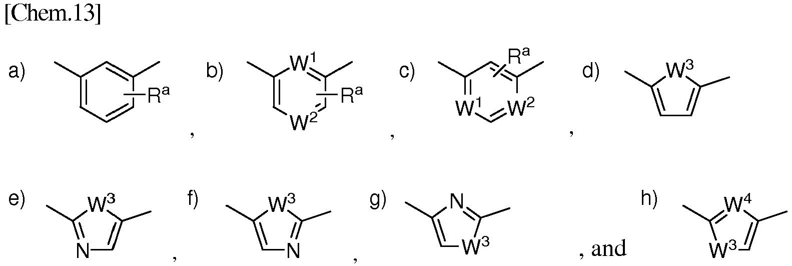 or a group i) constituted together with R¹: 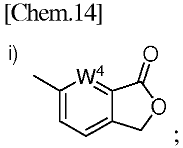
R^{a} is a group selected from the group consisting of the following j) to p):
j) a hydrogen atom,
k) a halogen atom,
1) a hydroxy group,
m) a cyano group,
n) an amino group unsubstituted or substituted by one or two C₁₋₆ alkyl groups,
o) a C₁₋₆ alkyl group unsubstituted or substituted by one or two groups independently selected from the group consisting of a halogen atom, a C₁₋₆ alkoxy group, a C₁₋₆ alkylsulfanyl group, a C₁₋₆ alkylsulfonyl group, a hydroxy group, and a cyano group, and
p) a C₁₋₆ alkoxy group unsubstituted or substituted by one or two groups independently selected from the group consisting of a halogen atom, a C₁₋₆ alkoxy group, a C₁₋₆ alkylsulfanyl group, a C₁₋₆ alkylsulfonyl group, a hydroxy group, and a cyano group;
   one of W¹ and W² is a nitrogen atom, and the other is -CH= or a nitrogen atom;
   W³ is an oxygen atom or a sulfur atom;
   W⁴ is -CH= or a nitrogen atom;
   Y¹ is C₁₋₆ alkylene;
R¹ is a group selected from the group consisting of the following q) to w):
q) -C(=O)-OZ¹,
r) -C(=O)-NHSO₂Z²,
s) -C(=O)-NHOH,
t) -C(=O)-NHCN,
u) -NH-C(=O)-Z³,
v) an acidic 5-membered heterocyclic group, and
w) a 6-membered ring group substituted by a phenolic hydroxy group;
Z¹ is a hydrogen atom, a C₁₋₆ alkyl group, or a C₇₋₁₀ aralkyl group;
Z² and Z³ are independently a group selected from the group consisting of the following aa) to ee):
aa) a C₁₋₆ alkyl group,
bb) a halo-C₁₋₆ alkyl group,
cc) a C₃₋₆ cycloalkyl group,
dd) an aryl group unsubstituted or in which a ring is substituted by one to five groups independently selected from the group consisting of a halogen atom, a C₁₋₆ alkyl group, a halo-C₁₋₆ alkyl group, and a C₁₋₆ alkoxy group, and
ee) a heterocyclic group;
R² is a group selected from the group consisting of the following A) to H):
A) a branched C₃₋₆ alkyl group,
B) a C₃₋₆ cycloalkyl group,
C) a phenyl group unsubstituted or in which a ring is substituted by one to five groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, and a cyano group,
D) a 6-membered aromatic heterocyclic group unsubstituted or in which a ring is substituted by one to four groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, and a cyano group,
E) a 5-membered aromatic heterocyclic group unsubstituted or in which a ring is substituted by one to four groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, and a cyano group,
F) an amino group substituted by one or two C₁₋₆ alkyl groups,
G) a 4- to 8-membered cyclic amino group unsubstituted or in which a ring is substituted by one to three groups independently selected from the group consisting of a C₁₋₆ alkyl group and a halogen atom, and
H) a C₁₋₆ alkoxy group;
R³ is a group selected from the group consisting of the following I) to W):
I) a hydrogen atom,
J) a halogen atom,
K) a hydroxy group,
L) a cyano group,
M) a nitro group,
N) a C₃₋₆ cycloalkyl group,
O) a C₂₋₆ alkenyl group,
P) a C₁₋₇ alkanoyl group,
Q) a C₁₋₆ alkylsulfanyl group,
R) a C₁₋₆ alkylsulfinyl group,
S) a C₁₋₆ alkylsulfonyl group,
T) an amino group unsubstituted or substituted by one or two C₁₋₆ alkyl groups,
U) a C₁₋₆ alkyl group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, a benzoyloxy group, and a cyano group,
V) a C₁₋₆ alkoxy group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, and a cyano group, and
W) a phenyl group unsubstituted or in which a ring is substituted by one to five groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, and a cyano group; and
R⁴ and R⁵ each independently represent a hydrogen atom, a halogen atom, a C₁₋₆ alkyl group, or a C₁₋₆ alkoxy group].

[P2] The pyrazolopyridine derivative or the pharmacologically acceptable salt thereof according to [P1], wherein in the above formula (I), A is a group selected from the group consisting of the following a) to d): 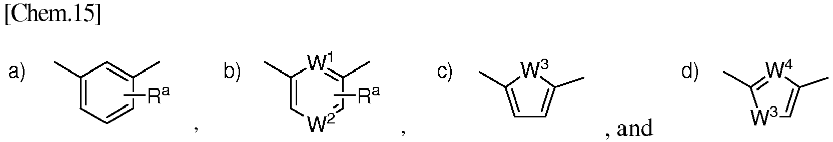 [R^{a}, W¹, W², W³, and W⁴ have the same meanings as above].

[P3] The pyrazolopyridine derivative or the pharmacologically acceptable salt thereof according to [P2], wherein in the above formula (I), A is a group selected from the group consisting of the following a) to c): 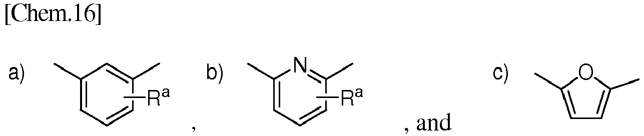 [P4] The pyrazolopyridine derivative or the pharmacologically acceptable salt thereof according to [P3], wherein in the above formula (I), Y¹ is methylene.

[P5] The pyrazolopyridine derivative or the pharmacologically acceptable salt thereof according to [P4], wherein in the above formula (I), R⁴ and R⁵ are each independently a hydrogen atom, a halogen atom, or a C₁₋₆ alkoxy group.

[P6] The pyrazolopyridine derivative or the pharmacologically acceptable salt thereof according to [P5], wherein in the above formula (I), Z¹ is a hydrogen atom or a C₁₋₆ alkyl group, and Z² is a C₁₋₆ alkyl group.

[P7] The pyrazolopyridine derivative or the pharmacologically acceptable salt thereof according to [P6], wherein in the above formula (I), R² is a group selected from the group consisting of the following a) to f):
a) a branched C₃₋₆ alkyl group,
b) a C₃₋₆ cycloalkyl group,
c) a phenyl group unsubstituted or in which a ring is substituted by one to five groups independently selected from the group consisting of a halogen atom, a C₁₋₆ alkyl group, a halo-C₁₋₆ alkyl group, a C₁₋₆ alkoxy group, a halo-C₁₋₆ alkoxy group, and a cyano group,
d) a 6-membered aromatic heterocyclic group,
e) a 5-membered aromatic heterocyclic group, and
f) a 4- to 8-membered cyclic amino group unsubstituted or in which a ring is substituted by one to three groups independently selected from C₁₋₆ alkyl groups and halogen atoms.

[P8] The pyrazolopyridine derivative or the pharmacologically acceptable salt thereof according to [P7], wherein in the above formula (I), R² is a group selected from the group consisting of the following a) to f):
a) a branched C₃₋₆ alkyl group,
b) a C₃₋₆ cycloalkyl group,
c) a phenyl group unsubstituted or in which a ring is substituted by one to five groups independently selected from the group consisting of a halogen atom, a C₁₋₆ alkyl group, a halo-C₁₋₆ alkyl group, a C₁₋₆ alkoxy group, a halo-C₁₋₆ alkoxy group, and a cyano group,
d) a pyridyl group,
e) a thienyl group, and
f) a morpholinyl group.

[P9] The pyrazolopyridine derivative or the pharmacologically acceptable salt thereof according to [P7], wherein in the above formula (I), R³ is a group selected from the group consisting of the following a) to 1):
a) a hydrogen atom,
b) a halogen atom,
c) a hydroxy group,
d) a cyano group,
e) a C₃₋₆ cycloalkyl group,
f) a C₂₋₆ alkenyl group,
g) a C₁₋₇ alkanoyl group,
h) a C₁₋₆ alkylsulfanyl group,
i) an amino group unsubstituted or substituted by one or two C₁₋₆ alkyl groups,
j) a C₁₋₆ alkyl group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, a benzoyloxy group, and a cyano group,
k) a C₁₋₆ alkoxy group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, and a cyano group, and
l) a phenyl group unsubstituted or in which a ring is substituted by one to five groups independently selected from the group consisting of a halogen atom, a C₁₋₆ alkyl group, a halo-C₁₋₆ alkyl group, a hydroxy C₁₋₆ alkyl group, a C₁₋₆ alkoxy group, a halo-C₁₋₆ alkoxy group, and a cyano group.
[P10] The pyrazolopyridine derivative or the pharmacologically acceptable salt thereof according to [P9], wherein in the above formula (I), R³ is a hydrogen atom, a halogen atom, a cyano group, a C₃₋₆ cycloalkyl group, a C₂₋₆ alkenyl group, a C₁₋₇ alkanoyl group, an amino group, a methylamino group, a halo-C₁₋₆ alkyl group, a C₁₋₆ alkylsulfanyl-C₁₋₆ alkyl group, a C₁₋₆ alkylsulfonyl-C₁₋₆ alkyl group, a hydroxy-C₁₋₆ alkyl group, a benzoyloxy-C₁₋₆ alkyl group, a C₁₋₆ alkyl group, a C₁₋₆ alkoxy group, or a phenyl group.

[P11] The pyrazolopyridine derivative or the pharmacologically acceptable salt thereof according to [P9], wherein in the above formula (I), R^{a} is a group selected from the group consisting of the following a) to e):
a) a hydrogen atom,
b) a halogen atom,
c) an amino group unsubstituted or substituted by one or two C₁₋₆ alkyl groups,
d) a C₁₋₆ alkyl group unsubstituted or substituted by one or two groups independently selected from the group consisting of a C₁₋₆ alkoxy group, a C₁₋₆ alkylsulfonyl group, and a hydroxy group, and
e) a C₁₋₆ alkoxy group unsubstituted or substituted by one or two groups independently selected from the group consisting of a C₁₋₆ alkoxy group, a C₁₋₆ alkylsulfonyl group, and a hydroxy group.

[P12] The pyrazolopyridine derivative or the pharmacologically acceptable salt thereof according to [P11], wherein in the above formula (I), R^{a} is a hydrogen atom, a halogen atom, a C₁₋₆ alkyl group, a hydroxy-C₁₋₆ alkyl group, or a C₁₋₆ alkoxy group.

[P13] The pyrazolopyridine derivative or the pharmacologically acceptable salt thereof according to [P1], wherein the above formula (I) is
3-[(6-chloro-2-phenylpyrazolo[1,5-a]pyridin-3-yl)methyl]benzoic acid,
6-[(6-chloro-2-phenylpyrazolo[1,5-a]pyridin-3-yl)methyl]pyridine-2-carboxylic acid,
3-[(6-methoxy-2-phenylpyrazolo[1,5-a]pyridin-3-yl)methyl]benzoic acid,
6-[(6-methoxy-2-phenylpyrazolo[1,5-a]pyridin-3-yl)methyl]pyridine-2-carboxylic acid,
6-[(2-phenyl-6-vinylpyrazolo[1,5-a]pyridin-3-yl)methyl]pyridine-2-carboxylic acid,
6-[(2-tert-butyl-6-methoxypyrazolo[1,5-a]pyridin-3-yl)methyl]pyridine-2-carboxylic acid,
6-[[2-phenyl-6-(trifluoromethyl)pyrazolo[1,5-a]pyridin-3-yl]methyl]pyridine-2-carboxylic acid, or
6-[(6-methyl-2-phenylpyrazolo[1,5-a]pyridin-3-yl)methyl]pyridine-2-carboxylic acid.

[P14] A pharmaceutical composition containing the pyrazolopyridine derivative or the pharmacologically acceptable salt thereof according to any one of [P1] to [P13] as an active ingredient.

[P15] An EP₁ receptor antagonist containing the pyrazolopyridine derivative or the pharmacologically acceptable salt thereof according to any one of [P1] to [P13] as an active ingredient.

[P16] A therapeutic or prophylactic agent for lower urinary tract symptoms containing the pyrazolopyridine derivative or the pharmacologically acceptable salt thereof according to any one of [P1] to [P13] as an active ingredient.

[P17] A method for treating or preventing lower urinary tract symptoms, comprising administering a pharmaceutical composition comprising the pyrazolopyridine derivative or the pharmacologically acceptable salt thereof according to any one of [P1] to [P13] as an active ingredient to a patient.

[P18] Use of the pyrazolopyridine derivative or the pharmacologically acceptable salt thereof according to any one of [P1] to [P13] for producing a pharmaceutical composition for prevention or treatment of lower urinary tract symptoms.

Preferable substituents for the pyrazolopyridine derivative or the pharmacologically acceptable salt thereof according to [P1] are as follows:

A is preferably a benzene ring, a pyridine ring, a benzene ring or a pyridine ring in which a ring is substituted by halogen atoms, a benzene ring or a pyridine ring in which a ring is substituted by C₁₋₆ alkyl groups, a benzene ring or a pyridine ring in which a ring is substituted by hydroxy-C₁₋₆ alkyl groups, a benzene ring or a pyridine ring in which a ring is substituted by C₁₋₆ alkoxy groups, or a furan ring, more preferably a benzene ring, a pyridine ring, a pyridine ring in which a ring is substituted by halogen atoms, a pyridine ring in which a ring is substituted by C₁₋₆ alkyl groups, a pyridine ring in which a ring is substituted by hydroxy-C₁₋₆ alkyl groups, or a pyridine ring in which a ring is substituted by C₁₋₆ alkoxy groups, particularly preferably a benzene ring, a pyridine ring, a pyridine ring substituted by a chlorine atom, a pyridine ring substituted by a methyl group, a pyridine ring substituted by a hydroxymethyl group, or a pyridine ring substituted by a methoxy group.

Y¹ is preferably methylene.

R¹ is preferably -C(=O)-OZ¹, -C(=O)-NHSO₂Z², or an acidic 5-membered heterocyclic group, more preferably -C(=O)-OH, -C(=O)-O-C₁₋₆ alkyl group, -C(=O)-NHSO₂-C₁₋₆ alkyl group, or a tetrazolyl group, particularly preferably -C(=O)-OH, -C(=O)-OMe, -C(=O)-NHSO₂Me, or a 5-tetrazolyl group.

R² is preferably a branched C₃₋₆ alkyl group, a C₃₋₆ cycloalkyl group, a phenyl group, a phenyl group in which a ring is substituted by halogen atoms, a phenyl group in which a ring is substituted by C₁₋₆ alkyl groups, a phenyl group in which a ring is substituted by halo-C₁₋₆ alkyl groups, a phenyl group in which a ring is substituted by C₁₋₆ alkoxy groups, a 6-membered aromatic heterocyclic group, a 5-membered aromatic heterocyclic group, or a 4- to 8-membered cyclic amino group, more preferably a branched C₃₋₆ alkyl group, a C₃₋₆ cycloalkyl group, a phenyl group, a phenyl group in which a ring is substituted by halogen atoms, a phenyl group in which a ring is substituted by C₁₋₆ alkyl groups, a phenyl group in which a ring is substituted by halo-C₁₋₆ alkyl groups, a phenyl group in which a ring is substituted by C₁₋₆ alkoxy groups, a pyridyl group, a thienyl group, or a morpholinyl group, particularly preferably an isobutyl group, a *tert*-butyl group, a cyclopropyl group, a cyclopentyl group, a cyclohexyl group, a phenyl group, a 4-fluorophenyl group, a 4-chlorophenyl group, a 4-methylphenyl group, a trifluoromethylphenyl group, a 4-methoxyphenyl group, a 3-pyridyl group, a 3-thienyl group, or a 4-morpholinyl group.

R³ is preferably a hydrogen atom, a halogen atom, a cyano group, a C₃₋₆ cycloalkyl group, a C₂₋₆ alkenyl group, a C₁₋₇ alkanoyl group, an amino group, an amino group substituted by C₁₋₆ alkyl groups, a C₁₋₆ alkyl group, a halo-C₁₋₆ alkyl group, a C₁₋₆ alkylsulfanyl-C₁₋₆ alkyl group, a C₁₋₆ alkylsulfonyl-C₁₋₆ alkyl group, a hydroxy-C₁₋₆ alkyl group, a benzoyloxy-C₁₋₆ alkyl group, a C₁₋₆ alkoxy group, or a phenyl group, more preferably a hydrogen atom, a fluorine atom, a chlorine atom, a bromine atom, a cyano group, a cyclopropyl group, a vinyl group, a 2-propenyl group, an acetyl group, an amino group, a methylamino group, a methyl group, a trifluoromethyl group, a 2-bromoethyl group, a 2-(methylsulfanyl)ethyl group, a 2-(methylsulfonyl)ethyl group, a hydroxyethyl group, a methoxy group, or a phenyl group.

R⁴ is preferably a hydrogen atom, a halogen atom, or a C₁₋₆ alkoxy group, more preferably a hydrogen atom or a methoxy group.

R⁵ is preferably a hydrogen atom, a halogen atom, or a C₁₋₆ alkoxy group, more preferably a hydrogen atom or a methoxy group.

The pharmacologically acceptable salt means a salt with a pharmaceutically acceptable non-toxic base or acid (for example, an inorganic or organic base and an inorganic or organic acid). Examples of a salt derived from the pharmaceutically acceptable non-toxic base can include salts with inorganic bases such as aluminum, ammonium, calcium, copper, ferrous iron, ferric iron, lithium, magnesium, manganese, manganous acid, potassium, sodium, and the like (examples of one that is particularly preferred include ammonium, calcium, manganese, potassium, and sodium salts), and salts with organic bases such as primary, secondary, and tertiary amines, substituted amine (for example, naturally occurring substituted amine), cyclic amine, and basic ion-exchange resins, and the like (for example, arginine, betaine, caffeine, choline, *N*,*N*'-dibenzylethylenediamine, diethylamine, 2-diethylaminoethanol, 2-dimethylaminoethanol, ethanolamine, ethylenediamine, *N*-ethylmorpholine, *N*-ethylpiperidine, glucamine, glucosamine, histidine, hydrabamine, isopropylamine, lysine, methylglucamine, morpholine, piperazine, piperidine, polyamine resin, procaine, purine, theobromine, triethylamine, trimethylamine, tripropylamine, tromethamine, and the like). Examples of a salt derived from the pharmaceutically acceptable non-toxic acid can include salts with inorganic acids such as hydrochloric acid, hydrobromic acid, sulfuric acid, nitric acid, and the like, and organic acids such as acetic acid, maleic acid, fumaric acid, succinic acid, lactic acid, malic acid, tartaric acid, citric acid, methanesulfonic acid, *p*-toluenesulfonic acid, salicylic acid, stearic acid, palmitic acid, and the like.

Furthermore, the compound (I) of the present invention or the pharmacologically acceptable salt thereof may be present as a hydrate or a solvate. All arbitrary hydrates and solvates formed by the pyrazolopyridine derivative represented by the above formula (I) or the salt thereof, including the preferable compounds specifically described above, are encompassed by the scope of the present invention. Examples of a solvent capable of forming the solvate include methanol, ethanol, 2-propanol, acetone, ethyl acetate, dichloromethane, diisopropyl ether, and the like.

In addition to racemates, optically active forms, stereoisomers, or rotational isomers are also included in the compound (I) of the present invention or the pharmacologically acceptable salt thereof. Moreover, prototropic tautomers are also included in the compound (I) of the present invention or the pharmacologically acceptable salt thereof.

The "EP₁ receptor antagonistic effect" as referred to in the present invention means an effect of inhibiting the binding of prostaglandin E₂ (PGE₂) to prostaglandin E receptor 1 (EP₁ receptor).

The EP₁ receptor antagonistic effect decreases the amount of intracellular calcium influx and reduces or suppresses an intracellular calcium concentration. As a result, the EP₁ receptor antagonistic effect exhibits effects such as smooth muscle-relaxing and sensory nerve stimulation-suppressing effects. Particularly, the EP₁ receptor antagonistic effect is useful as a therapeutic agent or a prophylactic agent for symptoms such as LUTS, particularly, OABs, by acting on the bladder, the urothelium, or the like.

Moreover, the EP₁ receptor antagonistic effect can be evaluated on the basis of an efficacy of inhibiting intracellular calcium influx by the stimulating effect of PGE₂ on the EP₁ receptor. This efficacy can be evaluated by an in vitro test or an in vivo test that follows "Pharmacological Test Examples" described in Japanese Patent Application Laid-Open No. 2008-214224.

The compound (I) of the present invention can be produced by various synthesis methods. Next, typical methods for producing the compound (I) of the present invention will be described.

(Method A) 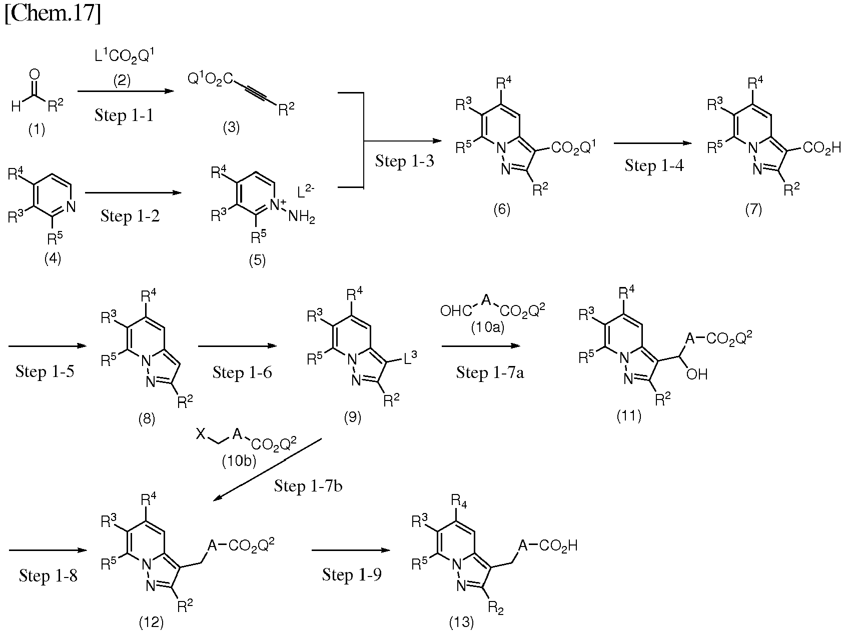 (wherein R², R³, R⁴, R⁵, and A have the same meanings as above; L¹ represents a chlorine atom, a bromine atom, an iodine atom, a methanesulfonyloxy group, or the like; L² represents an iodine atom, a mesitylsulfonyloxy group, a methanesulfonyloxy group, a trifluoromethanesulfonyloxy group, an acetyloxy group, or the like; L³ represents a bromine atom, an iodine atom, or the like; Q¹ and Q² each represent a C₁₋₆ alkyl group or a C₇₋₁₀ aralkyl group; and X represents a chlorine atom, a bromine atom, an iodine atom, a methanesulfonyloxy group, or the like).

### Step 1-1

The present step is a step of reacting compound (1) with compound (2) to produce compound (3). The compound (3) can be produced, for example, by reacting the compound (1) with carbon tetrabromide and triphenylphosphine in solvent A, treating the obtained bromo compound with a base such as *n*-butyllithium in solvent B to perform lithiation, and subsequently reacting it with the compound (2). Examples of the solvent A used can include dichloromethane, 1,2-dichloroethane, benzene, toluene, tetrahydrofuran, mixed solvents of them, and the like. Moreover, examples of the solvent B can include tetrahydrofuran, diethyl ether, 1,2-dimethoxyethane, 1,4-dioxane, mixed solvents of them, and the like. The reaction temperature can usually be carried out at -78°C to a solvent reflux temperature, and it is preferable to perform the reaction at -20 to 20°C. The reaction time differs depending on starting materials used, solvents, reaction temperature, and the like, but is usually 30 minutes to 3 days. Moreover, the compound (1) used in the present step can employ a commercially available product and alternatively, can also be produced according to methods described in literatures or methods equivalent thereto.

### Step 1-2

The present step is a step of *N*-aminating compound (4) to produce compound (5). The compound (5) can be produced, for example, by reacting the compound (4) with ethyl *O*-mesitylsulfonylacetohydroxamate or the like in the presence of an acid such as perchloric acid in a solvent. In this case, the compound (5) can be obtained as mesitylsulfonate. Examples of the solvent can include 1,4-dioxane, tetrahydrofuran, diethyl ether, 1,2-dimethoxyethane, mixed solvents of them, and the like. The reaction temperature can usually be carried out at -78°C to a solvent reflux temperature, and it is preferable to perform the reaction at -20 to 20°C. The reaction time differs depending on starting materials used, solvents, reaction temperature, and the like, but is usually 30 minutes to 3 days. Moreover, the compound (4) used in the present step can employ a commercially available product and alternatively, can also be produced according to methods described in literatures or methods equivalent thereto.

### Step 1-3

The present step is a step of reacting the compound (3) with the compound (5) to produce compound (6). The compound (6) can be produced, for example, by reacting the compound (3) with the compound (5) in the presence of a base in a solvent. Examples of the solvent can include methanol, ethanol, 2-propanol, *N*,*N*-dimethylformamide, 1,4-dioxane, tetrahydrofuran, mixed solvents of them, and the like. The reaction temperature can usually be carried out at -78°C to a solvent reflux temperature, and it is preferable to perform the reaction at -20 to 20°C. Potassium carbonate, sodium carbonate, or the like can be used as the base used. The reaction time differs depending on starting materials used, solvents, reaction temperature, and the like, but is usually 30 minutes to 3 days. Moreover, the compound (3) and the compound (5) used in the present step can employ commercially available products and alternatively, can also be produced according to methods described in literatures or methods equivalent thereto.

### Step 1-4

The present step is a step of hydrolyzing the ester moiety of the compound (6) to thereby produce compound (7). The compound (7) can be produced, for example, by reacting the compound (6) in the presence of a base in a solvent. Examples of the solvent can include tetrahydrofuran, 1,4-dioxane, diethyl ether, methanol, ethanol, propanol, 2-propanol, butanol, water, and the like. The reaction temperature can usually be carried out at 0°C to a solvent reflux temperature. Examples of the base used can include alkali metal salts such as potassium hydroxide, sodium hydroxide, and the like. The reaction time differs depending on starting materials used, solvents, reaction temperature, and the like, but is usually 30 minutes to 3 days.

### Step 1-5

The present step is a step of decarboxylating the compound (7) to thereby produce compound (8). The compound (8) can be produced, for example, by reacting the compound (7) at a high temperature in a solvent. Examples of the solvent can include 1,2-dichlorobenzene, diphenyl ether, xylene, toluene, and the like. The reaction temperature can usually be carried out at 50°C to a solvent reflux temperature, and it is preferable to perform the reaction at 100 to 180°C. The reaction time differs depending on starting materials used, solvents, reaction temperature, and the like, but is usually 30 minutes to 3 days.

### Step 1-6

The present step is a step of introducing a leaving group such as a bromine atom represented by the formula L³ to the compound (8) to produce compound (9). The compound (9) can be produced, for example, by performing bromination using bromine, *N*-bromosuccinimide, or the like in a solvent. Examples of the solvent used can include acetonitrile, carbon tetrachloride, chloroform, dichloromethane, tetrahydrofuran, diethyl ether, 1,2-dimethoxyethane, 1,4-dioxane, mixed solvents of them, and the like. The reaction temperature can usually be carried out at -78°C to a solvent reflux temperature, and it is preferable to perform the reaction at -78°C to 20°C. The reaction time differs depending on starting materials used, solvents, reaction temperature, and the like, but is usually 30 minutes to 3 days.

Moreover, for the production of compound (9) in which R⁵ is a hydrogen atom, as appropriate, the bromination reaction can be performed after replacing the hydrogen atom of R⁵ in the compound (8) by a protective group such as a trimethylsilyl group in order to allow lithiation reaction in Step 1-7 mentioned later to smoothly progress. As a method for introducing the trimethylsilyl group, for example, the compound (8) can be lithiated using lithium salt or the like in a solvent and then reacted with trimethylsilyl chloride or the like to thereby perform the production. Examples of the solvent used can include tetrahydrofuran, diethyl ether, 1,2-dimethoxyethane, 1,4-dioxane, mixed solvents of them, and the like. Examples of the lithium salt used can include lithium diisopropylamide, *n*-butyllithium, *sec*-butyllithium, and the like. The reaction temperature can usually be carried out at -78°C to 20°C, and it is preferable to perform the reaction at -78°C to -50°C. The reaction time differs depending on starting materials used, solvents, reaction temperature, and the like, but is usually 30 minutes to 3 days.

### Step 1-7a

The present step is a step of reacting the compound (9) with compound (10a) to produce compound (11). The compound (11) can be produced, for example, by lithiating the compound (9) using lithium salt or the like in a solvent and then reacting it with the compound (10a). Examples of the solvent used can include tetrahydrofuran, diethyl ether, 1,2-dimethoxyethane, 1,4-dioxane, mixed solvents of them, and the like. Examples of the lithium salt used can include lithium diisopropylamide, *n*-butyllithium, *sec*-butyllithium, and the like. The reaction temperature can usually be carried out at -78°C to 20°C, and it is preferable to perform the reaction at -78°C to -50°C. The reaction time differs depending on starting materials used, solvents, reaction temperature, and the like, but is usually 30 minutes to 3 days.

In this context, in the case where the trimethylsilyl group or the like introduced as a protective group in Step 1-6 is present in R⁵, it can be removed, for example, using tetrabutylammonium fluoride or the like in a solvent, after the completion of the above-described reaction. Examples of the solvent used can include tetrahydrofuran, diethyl ether, 1,2-dimethoxyethane, 1,4-dioxane, mixed solvents of them, and the like. The reaction temperature can usually be carried out at -78°C to a solvent reflux temperature, and it is preferable to perform the reaction at 0°C to 30°C. The reaction time differs depending on starting materials used, solvents, reaction temperature, and the like, but is usually 30 minutes to 3 days.

### Step 1-7b

The present step is a step of reacting the compound (9) with compound (10b) to produce compound (12). The compound (12) can be produced, for example, by lithiating the compound (9) using lithium salt or the like in a solvent, and then allowing zinc iodide to act thereon to prepare a zinc complex, which is then reacted with the compound (10b) in the presence or in the absence of a palladium catalyst. Examples of the solvent used can include tetrahydrofuran, diethyl ether, 1,2-dimethoxyethane, 1,4-dioxane, mixed solvents of them, and the like. Examples of the lithium salt used can include lithium diisopropylamide, *n*-butyllithium, *sec*-butyllithium, and the like. Examples of the palladium catalyst used can include tetrakistriphenylphosphinepalladium (0), and the like. The reaction temperature can usually be carried out at -78°C to 20°C, and it is preferable to perform the reaction at -78°C to -50°C. The reaction time differs depending on starting materials used, solvents, reaction temperature, and the like, but is usually 30 minutes to 3 days.

In this context, in the case where the trimethylsilyl group or the like introduced as a protective group in Step 1-6 is present in R⁵, it can be removed, for example, using tetrabutylammonium fluoride or the like in a solvent, after the completion of the above-described reaction. Examples of the solvent used can include tetrahydrofuran, diethyl ether, 1,2-dimethoxyethane, 1,4-dioxane, mixed solvents of them, and the like. The reaction temperature can usually be carried out at -78°C to a solvent reflux temperature, and it is preferable to perform the reaction at 0°C to 30°C. The reaction time differs depending on starting materials used, solvents, reaction temperature, and the like, but is usually 30 minutes to 3 days.

### Step 1-8

The present step is a step of replacing a hydroxy group in the compound (11) by a hydrogen atom to thereby produce compound (12). The compound (12) can be produced, for example, by allowing triethylsilane or the like to act thereon in the presence of a strong acid such as trifluoroacetic acid and the like in a solvent or without a solvent. Examples of the solvent used can include dichloromethane, chloroform, tetrahydrofuran, mixed solvents of them, and the like. The reaction temperature can usually be carried out at -78°C to a solvent reflux temperature, and it is preferable to perform the reaction at 0°C to 30°C. The reaction time differs depending on starting materials used, solvents, reaction temperature, and the like, but is usually 30 minutes to 3 days.

### Step 1-9

The present step is a step of hydrolyzing the ester moiety of the compound (12) to thereby produce compound (13). The compound (13) can be produced, for example, by reacting the compound (12) in the presence of a base in a solvent. Examples of the solvent can include tetrahydrofuran, 1,4-dioxane, diethyl ether, methanol, ethanol, propanol, 2-propanol, butanol, water, and the like. The reaction temperature can usually be carried out at 0°C to a solvent reflux temperature. Examples of the base used can include alkali metal salts such as potassium hydroxide ,sodium hydroxide, and the like. The reaction time differs depending on starting materials used, solvents, reaction temperature, and the like, but is usually 30 minutes to 3 days.

(Method B) 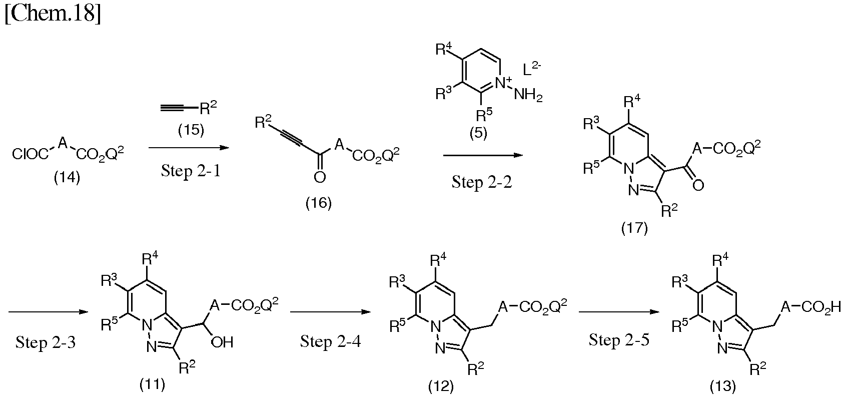 (wherein R², R³, R⁴, R⁵, A, L², and Q² have the same meanings as above).

### Step 2-1

The present step is a step of reacting compound (14) with compound (15) to produce compound (16). The compound (16) can be produced, for example, by reacting the compound (14) with the compound (15) in the presence of a base with a copper reagent and a palladium reagent as catalysts in a solvent. Examples of the solvent used can include tetrahydrofuran, 1,4-dioxane, dichloromethane, 1,2-dichloroethane, benzene, toluene, mixed solvents of them, and the like. Examples of the palladium catalyst used include dichlorobis(triphenylphosphine)palladium (II) and the like. Examples of the copper catalyst used include copper (I) iodide copper (I) bromide, and the like. Examples of the base used include triethylamine, diisopropylethylamine, pyridine, 2,6-dimethylpyridine, 2,4,6-trimethylpyridine, and the like. The reaction temperature can usually be carried out at -78°C to a solvent reflux temperature, and it is preferable to perform the reaction at 0 to 20°C. The reaction time differs depending on starting materials used, solvents, reaction temperature, and the like, but is usually 30 minutes to 3 days.

### Step 2-2

The present step is a step of reacting the compound (16) with compound (5) to produce compound (17). The compound (17) can be produced, for example, by reacting the compound (16) with the compound (5) in the presence of a base in a solvent. Examples of the solvent can include 1,4-dioxane, tetrahydrofuran, methanol, ethanol, 2-propanol, *N,N-*dimethylformamide, mixed solvents of them, and the like. Potassium carbonate, sodium carbonate, cesium carbonate, sodium acetate, or the like can be used as the base used. The reaction temperature can usually be carried out at -78°C to a solvent reflux temperature, and it is preferable to perform the reaction at 0 to 20°C. The reaction time differs depending on starting materials used, solvents, reaction temperature, and the like, but is usually 30 minutes to 3 days. Incidentally, the compound (5) can be produced in a manner similar to Step 1-2 of Method A described above.

### Step 2-3

The present step is a step of reducing the ketone moiety of the compound (17) to produce compound (11) having a hydroxy group. The compound (11) can be produced, for example, by allowing a reagent that becomes a hydride source, such as sodium borohydride, or the like, to act on the compound (17) in a solvent. An alcohol solvent such as methanol or ethanol is used alone as the solvent, and alternatively, mixed solvents of these solvents with dichloromethane, chloroform, tetrahydrofuran, 1,4-dioxane, or the like can be used. The reaction temperature can usually be carried out at -78°C to a solvent reflux temperature, and it is preferable to perform the reaction at -20 to 20°C. The reaction time differs depending on starting materials used, solvents, reaction temperature, and the like, but is usually 30 minutes to 3 days.

### Step 2-4

The present step is a step of replacing a hydroxy group in the compound (11) by a hydrogen atom to thereby produce compound (12). The present step can be performed according to Step 1-8 of Method A described above.

### Step 2-5

The present step is a step of hydrolyzing the ester moiety of the compound (12) to thereby produce compound (13). The present step can be performed according to Step 1-9 of Method A described above.

(Method C) 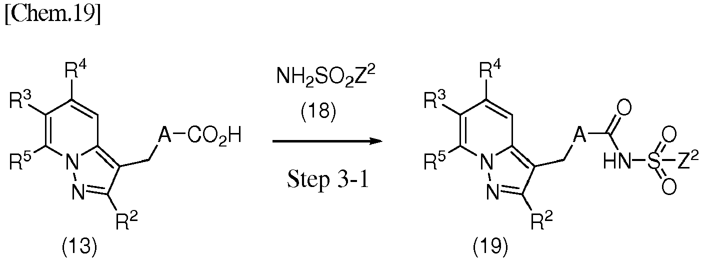 (wherein R², R³, R⁴, R⁵ A, and Z² have the same meanings as above).

### Step 3-1

The present step is a step of producing compound (19) by the condensation reaction between compound (13) and compound (18). The compound (19) can be produced, for example, by reacting the compound (13) with the compound (18) in the presence of a condensing agent and in the presence or in the absence of a base in a solvent. Examples of the solvent can include *N,N-*dimethylformamide, dichloromethane, 1,4-dioxane, tetrahydrofuran, mixed solvents of them, and the like. Examples of the condensing agent used include 1-ethyl-3-(3-dimethylaminopropyl)carbodiimide (EDCI), dicyclohexylcarbodiimide (DCC), 2-(1*H*-7-azabenzotriazol-1-yl)-1,1,3,3-tetramethyluronium hexafluorophosphate methanaminium (HATU), and the like. Moreover, as appropriate, *N,N*-dimethylaminopyridine, pyridine, 1-hydroxybenzotriazole (HOBT), or the like can also be used as a reaction accelerator. The reaction temperature can usually be carried out at -78°C to a solvent reflux temperature, and it is preferable to perform the reaction at 0 to 20°C. Potassium carbonate, sodium carbonate, triethylamine, diisopropylethylamine, or the like can be used as the base used. The reaction time differs depending on starting materials used, solvents, reaction temperature, and the like, but is usually 30 minutes to 3 days.

(Method D) 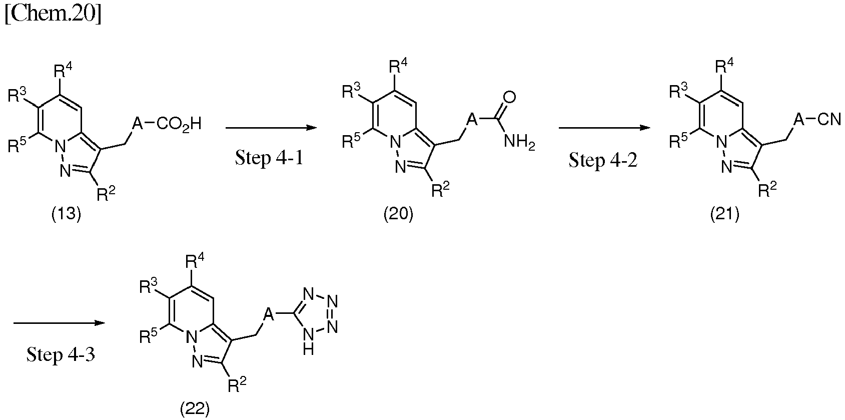 (wherein R², R³, R⁴, R⁵, and A have the same meanings as above).

### Step 4-1

The present step is a step of producing compound (20) from compound (13). The compound (20) can be produced, for example, by reacting the compound (13) with a compound that becomes an amine source, such as ammonium chloride, and the like, in the presence of a condensing agent and in the presence or in the absence of a base in a solvent. Examples of the solvent can include *N,N-*dimethylformamide, dichloromethane, 1,4-dioxane, tetrahydrofuran, mixed solvents of them, and the like. Examples of the condensing agent used include 1-ethyl-3-(3-dimethylaminopropyl)carbodiimide (EDCI), dicyclohexylcarbodiimide (DCC), 2-(1*H*-7-azabenzotriazol-1-yl)-1,1,3,3-tetramethyluronium hexafluorophosphate methanaminium (HATU), *O*-(benzotriazol-1-yl)-*N,N,N*'*N*'-tetramethyluronium hexafluorophosphate (HBTU), and the like. Moreover, as appropriate, *N,N*-dimethylaminopyridine, pyridine, 1-hydroxybenzotriazole (HOBT), or the like can also be used as a reaction accelerator. The reaction temperature can usually be carried out at -78°C to a solvent reflux temperature, and it is preferable to perform the reaction at 0 to 20°C. Potassium carbonate, sodium carbonate, triethylamine, diisopropylethylamine, or the like can be used as the base used. The reaction time differs depending on starting materials used, solvents, reaction temperature, and the like, but is usually 30 minutes to 3 days.

### Step 4-2

The present step is a step of producing compound (21) by the dehydration reaction of the compound (20). The compound (21) can be produced, for example, by allowing thionyl chloride, phosphorus oxychloride, or the like to act on the compound (20) in a solvent. Examples of the solvent can include *N,N-*dimethylformamide, dichloromethane, 1,4-dioxane, tetrahydrofuran, toluene, mixed solvents of them, and the like. The reaction temperature can usually be carried out at -78°C to a solvent reflux temperature, and it is preferable to perform the reaction at 0 to 50°C. The reaction time differs depending on starting materials used, solvents, reaction temperature, and the like, but is usually 30 minutes to 3 days.

### Step 4-3

The present step is a step of converting a nitrile group in the compound (21) to a tetrazolyl group to produce compound (22). The compound (22) can be produced, for example, by allowing sodium azide or the like to act on the compound (21) in a solvent. Examples of the solvent can include *N,N-*dimethylformamide, water, dichloromethane, 1,4-dioxane, tetrahydrofuran, mixed solvents of them, and the like. Moreover, as appropriate, zinc bromide, ammonium chloride, silver nitrate, acetic acid, or the like can be added. The reaction temperature can usually be carried out at -78°C to a solvent reflux temperature, and it is preferable to perform the reaction at 20°C to a solvent reflux temperature. The reaction time differs depending on starting materials used, solvents, reaction temperature, and the like, but is usually 30 minutes to 3 days.

(MethodE) 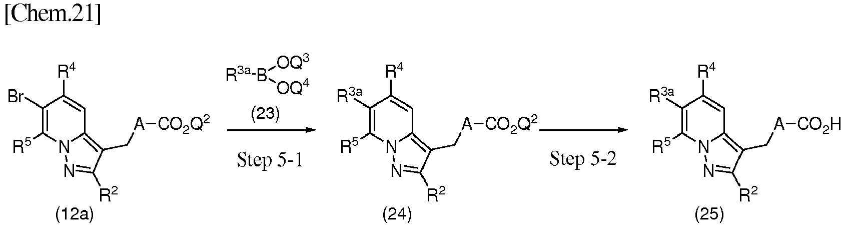 (wherein R², R⁴, R⁵, A, and Q² have the same meanings as above; Q³ and Q⁴ are each a hydrogen atom, a C₁₋₆ alkyl group (they may be bonded to each other to form a ring), or the like; and R^{3a} represents a C₁₋₆ alkyl group, a C₂₋₆ alkenyl group, a C₁₋₇ alkanoyl group, a cyano group, or the like).

### Step 5-1

The present step is a step of replacing a bromine atom in compound (12a) by R^{3a} to thereby produce compound (24). The compound (24) can be produced, for example, by reacting the compound (12a) with compound (23) in the presence of a base with a palladium reagent as a catalyst in a solvent. Examples of the solvent used can include tetrahydrofuran, 1,4-dioxane, dichloromethane, 1,2-dichloroethane, benzene, toluene, ethanol, propanol, *N,N-*dimethylformamide, dimethyl sulfoxide, water, mixed solvents of them, and the like. Examples of the palladium catalyst used include dichlorobis(triphenylphosphine)palladium (II), tetrakistriphenylphosphinepalladium (0), and the like. Examples of the base used include sodium carbonate, potassium carbonate, sodium bicarbonate, potassium bicarbonate, cesium carbonate, triethylamine, diisopropylethylamine, pyridine, 2,6-dimethylpyridine, 2,4,6-trimethylpyridine, and the like. The reaction temperature can usually be carried out at -78°C to a solvent reflux temperature, and it is preferable to perform the reaction at 0 to 20°C. The reaction time differs depending on starting materials used, solvents, reaction temperature, and the like, but is usually 30 minutes to 3 days. Incidentally, the compound (12a) can be produced according to Method A described above using compound (4) in which R³ is a bromine atom.

### Step 5-2

The present step is a step of hydrolyzing the ester moiety of the compound (24) to thereby produce compound (25). The present step can be performed according to Step 1-9 of Method A described above.

(Method F) 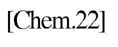 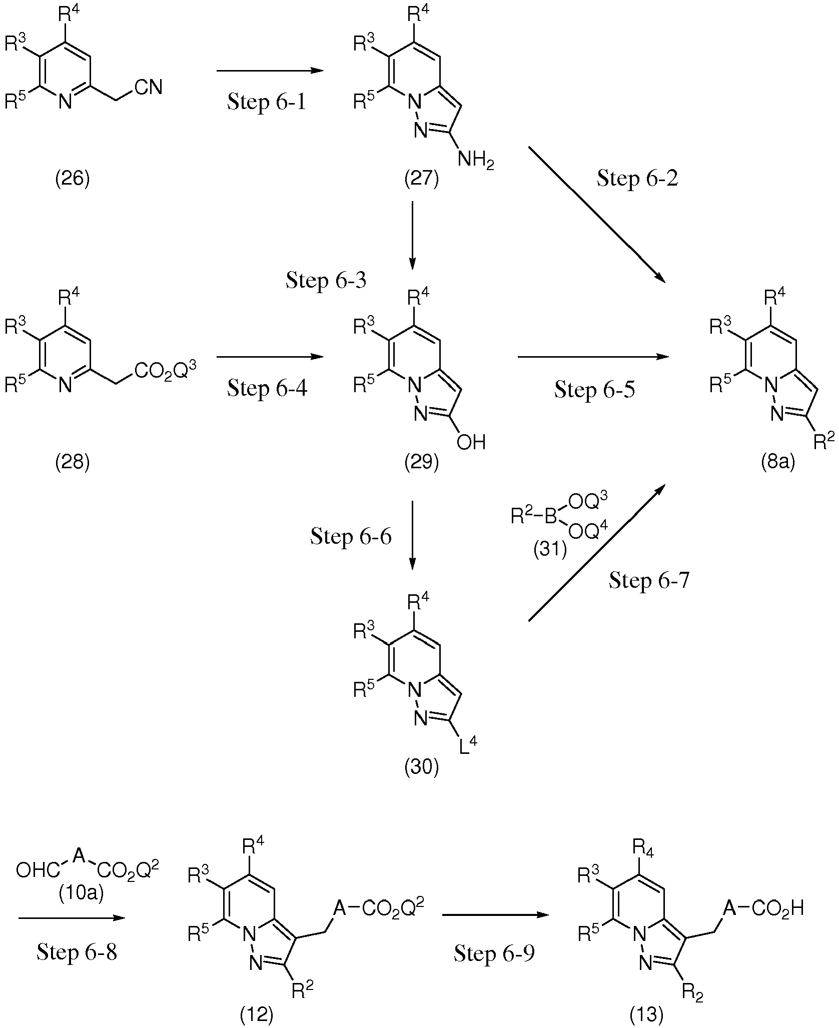 (wherein R², R³, R⁴, R⁵, Q², Q³, Q⁴, and A have the same meanings as above; and L⁴ represents a chlorine atom, a bromine atom, a trifluoromethanesulfonyloxy group, or the like).

### Step 6-1

The present step is a step of *N*-aminating compound (26) and then performing cyclization to thereby produce compound (27). The compound (27) can be produced, for example, by reacting the compound (26) with ethyl *O*-mesitylsulfonylacetohydroxamate or the like in the presence of an acid such as perchloric acid or the like in a solvent to perform N-amination and then treating it with a base in a solvent to close the ring. Examples of the solvent used in the *N*-amination reaction can include 1,4-dioxane, tetrahydrofuran, diethyl ether, 1,2-dimethoxyethane, mixed solvents of them, and the like. The reaction temperature can usually be carried out at -78°C to a solvent reflux temperature, and it is preferable to perform the reaction at -20 to 20°C. The reaction time differs depending on starting materials used, solvents, reaction temperature, and the like, but is usually 30 minutes to 3 days. Examples of the solvent used in the cyclization reaction can include methanol, ethanol, 2-propanol, *N,N-*dimethylformamide, 1,4-dioxane, tetrahydrofuran, mixed solvents of them, and the like. Examples of the base used include sodium carbonate, potassium carbonate, sodium bicarbonate, potassium bicarbonate, cesium carbonate, triethylamine, diisopropylethylamine, pyridine, 2,6-dimethylpyridine, 2,4,6-trimethylpyridine, and the like. The reaction temperature can usually be carried out at -78°C to a solvent reflux temperature, and it is preferable to perform the reaction at -20 to 20°C. The reaction time differs depending on starting materials used, solvents, reaction temperature, and the like, but is usually 30 minutes to 3 days.
Moreover, the compound (26) used in the present step can employ a commercially available product and alternatively, can also be produced according to methods described in literatures or methods equivalent thereto.

### Step 6-2

The present step is a step of alkylating a primary amino group in the compound (27) to thereby perform production in the case where the R² moiety of compound (8a) is a mono- or di-alkylamino group or a cyclic amino group. The compound (8a) can be produced, for example, by reacting the compound (27) with various alkyl halides in the presence or in the absence of a base in a solvent. Examples of the solvent can include tetrahydrofuran, 1,4-dioxane, *N*,*N*-dimethylformamide, dichloromethane, ethanol, diethyl ether, 1,2-dimethoxyethane, mixed solvents of them, and the like. Examples of the base used include sodium carbonate, potassium carbonate, sodium bicarbonate, potassium bicarbonate, cesium carbonate, triethylamine, diisopropylethylamine, pyridine, 2,6-dimethylpyridine, 2,4,6-trimethylpyridine, sodium hydride, and the like. The reaction temperature can usually be carried out at -20°C to a solvent reflux temperature, and it is preferable to perform the reaction at 0°C to a solvent reflux temperature. The reaction time differs depending on starting materials used, solvents, reaction temperature, and the like, but is usually 30 minutes to 3 days.

### Step 6-3

The present step is a step of producing compound (29) from the compound (27). The compound (29) can be produced, for example, by reacting the compound (27) with water in the presence of hydrochloric acid or sulfuric acid,and the like. The reaction temperature can usually be carried out at 20°C to a solvent reflux temperature, and it is preferable to perform the reaction at 50°C to 100°C. The reaction time differs depending on starting materials used, solvents, reaction temperature, and the like, but is usually 30 minutes to 3 days.

### Step 6-4

The present step is a step of producing compound (29) from the compound (28). The present step can be performed according to Step 6-1 of Method F described above.
Moreover, the compound (28) used in the present step can employ a commercially available product and alternatively, can also be produced according to methods described in literatures or methods equivalent thereto.

### Step 6-5

The present step is a step of alkylating a hydroxy group in the compound (29) to thereby perform production in the case where the R² moiety of compound (8a) is an alkoxy group. The compound (8a) can be produced, for example, by reacting the compound (29) with various alkyl halides in the presence or in the absence of a base in a solvent. Examples of the solvent can include tetrahydrofuran, 1,4-dioxane, *N,N-*dimethylformamide, dichloromethane, diethyl ether, 1,2-dimethoxyethane, mixed solvents of them, and the like. Examples of the base used include sodium carbonate, potassium carbonate, sodium bicarbonate, cesium carbonate, sodium hydride, and the like. The reaction temperature can usually be carried out at -20°C to a solvent reflux temperature, and it is preferable to perform the reaction at 0°C to a solvent reflux temperature. The reaction time differs depending on starting materials used, solvents, reaction temperature, and the like, but is usually 30 minutes to 3 days.

### Step 6-6

The present step is a step of producing compound (30) from the compound (29). In the case where L⁴ of the compound (30) is a trifluoromethanesulfonyloxy group, it can be produced, for example, by reacting the compound (29) with trifluoromethanesulfonic anhydride in the presence or in the absence of a base in a solvent. Examples of the solvent can include tetrahydrofuran, 1,4-dioxane, *N,N-*dimethylformamide, mixed solvents of them, and the like. Examples of the base used include sodium carbonate, potassium carbonate, cesium carbonate, triethylamine, diisopropylethylamine, pyridine, 2,6-dimethylpyridine, 2,4,6-trimethylpyridine, sodium hydride, and the like. The reaction temperature can usually be carried out at -20°C to a solvent reflux temperature, and it is preferable to perform the reaction at -20°C to 30°C. The reaction time differs depending on starting materials used, solvents, reaction temperature, and the like, but is usually 30 minutes to 3 days.

### Step 6-7

The present step is a step of performing production by the coupling reaction between the compound (30) and compound (31) in the case where the R² moiety of compound (8a) is an aryl group or a C₁₋₆ alkyl group. The present step can be performed according to Step 5-1 of Method E described above.

### Step 6-8

The present step is a step of producing compound (12) by the condensation reaction between the compound (8a) and compound (10a). The compound (12) can be produced, for example, by allowing trifluoroacetic acid and triethylsilane to act on a mixture of the compound (8a) and the compound (10a) in a solvent. Examples of the solvent used can include dichloromethane, chloroform, tetrahydrofuran, mixed solvents of them, and the like. The reaction temperature can usually be carried out at -78°C to a solvent reflux temperature, and it is preferable to perform the reaction at 0°C to 30°C. The reaction time differs depending on starting materials used, solvents, reaction temperature, and the like, but is usually 30 minutes to 3 days.

Moreover, the compound (8a) used in the present step can also be produced according to Method A described above or alternatively according to methods described in literatures or methods equivalent thereto.

### Step 6-9

The present step is a step of producing compound (13) from the compound (12). The present step can be performed according to Step 1-9 of Method A described above.

The pharmacologically acceptable salt of the compound (I) of the present invention can be produced according to a conventional method using the compound (I) of the present invention.

The schemes shown above are examples of methods for producing the compound (I) of the present invention or an intermediate for production thereof. These may be variously changed or modified to schemes readily understandable by those skilled in the art.

Moreover, in the case where a protective group is necessary depending on the type of a functional group, the operations of introduction and detachment can be appropriately carried out in combination according to a standard method. Examples as to the type, introduction, and detachment of the protective group can include methods described in Theodora W. Greene & Peter G M. Wuts ed., "Greene's Protective Groups in Organic Synthesis", fourth edition, Wiley-Interscience, 2006.

The intermediate used for producing the compound (I) of the present invention or the pharmacologically acceptable salt thereof can be isolated/purified, as appropriate, by solvent extraction, crystallization, recrystallization, chromatography, preparative high-performance liquid chromatography, and the like, which is isolation/purification means well known by those skilled in the art.

### Pharmaceutical composition containing compound (I) of the present invention or pharmacologically acceptable salt thereof

A pharmaceutical composition containing the compound (I) of the present invention or the pharmacologically acceptable salt thereof as an active ingredient is used in various dosage forms according to usage. Examples of such dosage forms can include powders, granules, fine granules, dry syrups, tablets, capsules, injections, liquids, ointments, suppositories, patches, sublinguals, and the like, which are orally or parenterally administered.

These pharmaceutical compositions can be formulated by a heretofore known approach according to the dosage forms thereof by appropriately performing mixing or dilution/dissolution with appropriate pharmaceutical additives such as excipients, disintegrants, binders, lubricants, diluents, buffers, tonicity agents, antiseptics, wetting agents, emulsifiers, dispersants, stabilizers, solubilizing agents, and the like. Moreover, in the case of being used in combination with an agent other than EP₁ receptor antagonists, the pharmaceutical compositions can be produced by simultaneously or separately formulating individual active ingredients in a manner similar to above.

### Pharmaceutical use of compound (I) of the present invention or pharmacologically acceptable salt thereof

The compound (I) of the present invention or the pharmacologically acceptable salt thereof exhibits a strong EP₁ receptor antagonistic effect in an EP₁ receptor antagonistic effect confirmation test, and the like. Hence, the compound (I) of the present invention or the pharmacologically acceptable salt thereof can suppress or reduce an intracellular calcium concentration. Thus, the pharmaceutical composition containing the compound (I) of the present invention or the pharmacologically acceptable salt thereof as an active ingredient can be used as a therapeutic agent or a prophylactic agent for diseases or symptoms attributed to the activation of the EP₁ receptor by the stimulating effect of PGE₂.

Moreover, examples of the diseases that activate the EP₁ receptor by the stimulating effect of PGE₂ include lower urinary tract symptoms (LUTS), inflammatory disease, pain disease, osteoporosis, cancer, and the like. It is preferable that the pharmaceutical composition containing the compound (I) of the present invention or the pharmacologically acceptable salt thereof as an active ingredient should be used as a therapeutic agent or a prophylactic agent for LUTS, inflammatory disease, or pain disease. One that is more preferred is LUTS.

Examples of causative diseases of the lower urinary tract symptoms include cystitis and prostatitis such as overactive bladder (OAB), benign prostatic hyperplasia (BPH), interstitial cystitis, and the like.

The "lower urinary tract symptoms" mean urinary storage symptoms, voiding symptoms, and post-voiding symptoms.

It is preferable that the compound (I) of the present invention or the pharmacologically acceptable salt thereof should be used for the treatment or prevention of urinary storage symptoms.

Urgency to urinate, daytime urinary frequency, nocturia, incontinence (stress urinary incontinence, urge incontinence, mixed incontinence, enuresis, enuresis nocturna, continuous incontinence, and the like), and bladder sensation (increase in bladder sensation, reduction in bladder sensation, lack of bladder sensation, nonspecific bladder sensation, and the like) are included as the "urinary storage symptoms". It is preferable that the compound (I) of the present invention or the pharmacologically acceptable salt thereof should be used for the treatment or prevention of urgency to urinate, daytime urinary frequency, nocturia, urge incontinence, mixed incontinence, enuresis, enuresis nocturna, increase in bladder sensation, or nonspecific bladder sensation. One that is more preferred is urgency to urinate, daytime urinary frequency, nocturia, urge incontinence or increase in bladder sensation. Moreover, the compound (I) of the present invention or the pharmacologically acceptable salt thereof is particularly preferable for the treatment or prevention of OABs.

### Combined use or combination agent of compound (I) of the present invention or pharmacologically acceptable salt thereof

The compound (I) of the present invention or the pharmacologically acceptable salt thereof can also be appropriately used in combination with at least one type of agent other than EP₁ receptor antagonists.

Examples of the agent that can be used in combination with the compound (I) of the present invention or the pharmacologically acceptable salt thereof include therapeutic agents for cystitis and prostatitis, and the like, such as overactive bladder (OAB), benign prostatic hyperplasia (BPH), and interstitial cystitis with a mechanism of action different from EP₁ receptor antagonists. Examples of such agents include anticholinergic agents, α₁ antagonists, β agonists, 5α-reductase inhibitors, PDE inhibitors, acetylcholinesterase inhibitors, antiandrogens, progesterone-based hormones, LH-RH analogs, neurokinin inhibitors, antidiuretics, calcium channel blockers, direct smooth muscle acting agents, tricyclic antidepressants, potassium channel modulators, sodium channel blockers, H₁ blockers, serotonin reuptake inhibitors, norepinephrine reuptake inhibitors, dopamine reuptake inhibitors, GABA agonists, TRPV1 modulators, endothelin antagonist, 5-HT_{1A} antagonists, α₁ agonists, opioid agonists, P₂X antagonists, COX inhibitors, σ agonists, muscarine agonists, and the like. One that is preferred is anticholinergic agents, α₁ antagonists, β agonists, 5α-reductase inhibitors, PDE inhibitors, progesterone-based hormones, antidiuretics, direct smooth muscle acting agents, or tricyclic antidepressants.

Moreover, although the agent used in combination will be described specifically as follows, the contents of the present invention are not limited to these. Moreover, specific compounds include free forms thereof and other pharmacologically acceptable salts.

Examples of the "anticholinergic agents" can include oxybutynin, propiverine, solifenacin, tolterodine, imidafenacin, temiverine, darifenacin, fesoterodine, trospium, propantheline, and the like.

Examples of the "α₁ antagonists" can include urapidil, naftopidil, tamsulosin, silodosin, prazosin, terazosin, alfuzosin, doxazosin, CR-2991, and fiduxosin.

Examples of the "β agonists" can include mirabegron, KUC-7483, KRP-204, SM-350300, TRK-380, amibegron, clenbuterol, SR-150640, solabegron, and the like.

Examples of the "5α-reductase inhibitors" can include dutasteride, TF-505, finasteride, izonsteride, and the like.

Examples of the "PDE inhibitors" mean phosphodiesterase inhibitors, and examples thereof can include tadalafil, vardenafil, sildenafil, avanafil, UK-369003, T-0156, AKP-002, etazolate, and the like.

Examples of the "acetylcholinesterase inhibitors" can include distigmine, donepezil, Z-338, rivastigmine, ganstigmine, BGC-20-1259, galanthamine, itopride, NP-61, SPH-1286, tolserine, ZT-1, and the like.

Examples of the "antiandrogens" can include gestonorone, oxendolone, bicalutamide, BMS-641988, CB-03-01, CH-4892789, flutamide, MDV-3100, nilutamide, TAK-700, YM-580, and the like.

Examples of the "progesterone-based hormones" can include chlormadinone allylestrenol, and the like.

The "LH-RH analogs" mean gonadotropic hormone-releasing hormone analogs. Moreover, the gonadotropic hormone-releasing hormones are also called luteinizing hormone-releasing hormones. Examples thereof can include AEZS-108, buserelin, deslorelin, goserelin, histrelin, leuprorelin, lutropin, nafarelin, triptorelin, AEZS-019, cetrorelix, degarelix, elagolix, ganirelix, ozarelix, PTD-634, TAK-385, teverelix, TAK-448, TAK-683, and the like.

Examples of the "neurokinin inhibitors" can include KRP-103, aprepitant, AV-608, casopitant, CP-122721, DNK-333, fosaprepitant, LY-686017, netupitant, orvepitant, rolapitant, TA-5538, T-2328, vestipitant, AZD-2624, Z-501, 1144814, MEN-15596, MEN-11420, SAR-102779, SAR-102279, saredutant, SSR-241586, and the like.

Examples of the "antidiuretics" can include desmopressin VA-106483, and the like.

Examples of the "calcium channel blockers" can include amlodipine, cilnidipine, propiverine, temiverine, PD-299685, aranidipine, azelnidipine, bamidipine, benidipine, bevantolol, clevidipine, CYC-381, diltiazem, efonidipine, fasudil, felodipine, gabapentin, gallopamil, isradipine, lacidipine, lercanidipine, lomerizine, manidipine, MEM-1003, nicardipine, nifedipine, nilvadipine, nimodipine, nisoldipine, SB-751689, verapamil, YM-58483, ziconotide, and the like.

Examples of the "direct smooth muscle acting agents" can include flavoxate and the like.

Examples of the "tricyclic antidepressants" can include imipramine, clomipramine, amitriptyline, and the like.

Examples of the "potassium channel modulators" can include nicorandil, NIP-141, NS-4591, NS-1643, andolast, diazoxide, ICA-105665, minoxidil, pinacidil, tilisolol, VRX-698, and the like.

Examples of the "sodium channel blockers" can include bepridil, dronedarone, propafenone, safinamide, SUN-N8075, SMP-986, 1014802, 552-02, A-803467, brivaracetam, cibenzoline, eslicarbazepine, F-15845, flecainide, fosphenytoin, lacosamide, lamotrigine, levobupivacaine, M-58373, mexiletine, moracizine, nerispirdine, NW-3509, oxcarbazepine, pilsicainide, pirmenol, propafenone, NW-1029, ropivacaine, vernakalant, and the like.

Examples of the "H₁ blockers" can include acrivastine, alcaftadine, bepotastine, bilastine, cetirizine, desloratadine, ebastine, efletirizine, epinastine, fexofenadine, GSK-835726, levocabastine, levocetirizine, loratadine, mequitazine, mizolastine, NBI-75043, ReN-1869, terfenadine, UCB-35440, vapitadine, YM-344484, diphenhydramine, chlorpheniramine, and the like.

Examples of the "serotonin reuptake inhibitors" can include UCB-46331, 424887, AD-337, BGC-20-1259, BMS-505130, citalopram, dapoxetine, desvenlafaxine, DOV-102677, DOV-216303, DOV-21947, duloxetine, escitalopram, F-2695, F-98214-TA, fluoxetine, fluvoxamine, IDN-5491, milnacipran, minaprine, NS-2359, NSD-644, paroxetine, PF-184298, SD-726, SEP-225289, SEP-227162, SEP-228425, SEP-228432, sertraline, sibutramine, tesofensine, tramadol, trazodone, UCB-46331, venlafaxine, vilazodone, WAY-426, WF-516, and the like.

Examples of the "norepinephrine reuptake inhibitors" can include AD-337, desvenlafaxine, DOV-102677, DOV-216303, DOV-21947, duloxetine, F-2695, F-98214-TA, milnacipran, NS-2359, NSD-644, PF-184298, SD-726, SEP-225289, SEP-227162, SEP-228425, SEP-228432, sibutramine, tesofensine, tramadol, venlafaxine, bupropion, radafaxine, atomoxetine, DDP-225, LY-2216684, neboglamine, NRI-193, reboxetine, tapentadol, WAY-256805, WAY-260022, and the like.

Examples of the "dopamine reuptake inhibitors" can include DOV-102677, DOV-216303, DOV-21947, IDN-5491, NS-2359, NSD-644, SEP-225289, SEP-228425, SEP-228432, sibutramine, tesofensine, tramadol, brasofensine, bupropion, NS-27100, radafaxine, safinamide, and the like.

Examples of the "GABA agonists" can include retigabine, eszopiclone, indiplon, pagoclone, SEP-225441, acamprosate, baclofen, AZD-7325, BL-1020, brotizolam, DP-VPA, progabide, propofol, topiramate, zopiclone, EVT-201, AZD-3043, ganaxolone, NS-11394, arbaclofen, AZD-3355, GS-39783, ADX-71441, ADX-71943, and the like.

Examples of the "TRPV1 modulators" can include capsaicin, resiniferatoxin, DE-096, GRC-6211, AMG-8562, JTS-653, SB-705498, A-425619, A-784168, ABT-102, AMG-628, AZD-1386, JNJ-17203212, NGD-8243, PF-3864086, SAR-115740, SB-782443, and the like.

Examples of the "endothelin antagonists" can include SB-234551, ACT-064992, ambrisentan, atrasentan, bosentan, clazosentan, darusentan, fandosentan, S-0139, TA-0201, TBC-3711, zibotentan, BMS-509701, PS-433540, and the like.

Examples of the "5-HT_{1A} antagonists" can include espindolol, lecozotan, lurasidone, E-2110, REC-0206, SB-649915, WAY-426, WF-516, and the like.

Examples of the "α₁ agonists" can include CM-2236, armodafinil, midodrine, modafinil, and the like.

Examples of the "opioid agonists" can include morphine, TRK-130, DPI-125, DPI-3290, fentanyl, LIF-301, loperamide, loperamide oxide, remifentanil, tapentadol, WY-16225, oxycodone, PTI-202, PTI-721, ADL-5747, ADL-5859, DPI-221, DPI-353, IPP-102199, SN-11, ADL-10-0101, ADL-10-0116, asimadoline, buprenorphine, CR-665, CR-845, eptazocine, nalbuphine, nalfurafine, pentazocine, XEN-0548, W-212393, ZP-120, nalmefene, and the like.

Examples of the "P₂X antagonists" can include A-740003, AZ-11657312, AZD-9056, GSK-1482160, GSK-31481A, and the like.

The "COX inhibitors" mean cyclooxygenase inhibitors, and examples thereof can include aceclofenac, ST-679, aspirin, bromfenac, dexketoprofen, flurbiprofen, FYO-750, ibuprofen, ketoprofen, ketorolac, licofelone, lornoxicam, loxoprofen, LT-NS001, diclofenac, mofezolac, nabumetone, naproxen, oxaprozin, piroxicam, pranoprofen, suprofen, tenoxicam, tiaprofenic acid, tolfenamic acid, zaltoprofen, 644784, ABT-963, ajulemic acid, apricoxib, celecoxib, cimicoxib, etoricoxib, iguratimod, lumiracoxib, meloxicam, nimesulide, parecoxib, RO-26-2198, valdecoxib, and the like.

Examples of the "σ agonists" can include ANAVEX-27-1041, PRS-013, SA-4503, ANAVEX-2-73, siramesine, ANAVEX-7-1037, and ANAVEX-1-41.

Examples of the "muscarine agonists" can include AC-260584, cevimeline, MCD-386, NGX-267, NGX-292, sabcomeline, pilocarpine, bethanechol, and the like.

In the case of using the compound (I) of the present invention or the pharmacologically acceptable salt thereof in combination with one type or more of the above-described agents, the present invention includes any one administration method selected from the following 1) to 5):
1) simultaneous administration with a combination agent,
2) simultaneous administration through the same administration route as separate formulations,
3) simultaneous administration through different administration routes as separate formulations,
4) administration at different times through the same administration route as separate formulations, and
5) administration at different times through different administration routes as separate formulations.
   Moreover, in the case of performing the administration at different times as separate formulations as in 4) or 5), the order of administration of the compound (I) of the present invention and the agent(s) is not particularly limited.

Moreover, the compound (I) of the present invention or the pharmacologically acceptable salt thereof can be appropriately used in combination with one type or more of the above-described agents to thereby obtain advantageous effects equal to or more than additive effects on the prevention or treatment of the above-described diseases. Alternatively, similarly, the amount of use thereof can be decreased compared with the case of being used alone, or the side effect of the agent(s) used in combination can be decreased, or the side effect of the agent(s) used in combination can be avoided or alleviated.

### Administration and dosage of compound (I) of the present invention

The pharmaceutical composition of the present invention can be administered systemically or locally and orally or parenterally (transnasally, transpulmonarily, intravenously, intrarectally, subcutaneously, intramuscularly, transdermally, and the like).

In the case of using the pharmaceutical composition of the present invention in actual treatment, the dose of the compound (I) of the present invention or the pharmacologically acceptable salt thereof which is an active ingredient thereof is appropriately determined depending on the age, sex, and body weight of a patient, a disease, and the degree of treatment, and the like. For example, in the case of oral administration, it can be appropriately administered in one portion or several divided portions within the range of generally 3 to 1000 mg/body per day in adult (body weight: 60 kg). The dose per day as an oral preparation is preferably 6 to 540 mg/body, more preferably 18 to 180 mg/body. In the case of parenteral administration, it can be appropriately administered in one portion or several divided portions within the range of generally 0.01 to 300 mg per day in adult. The dose per day as a parenteral preparation is preferably 1 to 100 mg/body, more preferably 6 to 60 mg/body. Moreover, the dose of the compound (I) of the present invention or the pharmacologically acceptable salt thereof can be decreased according to the dose of an agent other than EP₁ receptor antagonists.

### Examples

Hereinbelow, the present invention will be described in detail referring to Test Examples, Examples, and Reference Examples. Furthermore, because novel compounds are included in the starting material compounds used for the production of the compound according to the present invention (I), examples of producing starting material compounds will also be described as Reference Examples. The compound according to the present invention is not limited to compounds described in the following Examples and may be modified within the range not deviating from the scope of the present invention.

### <Reference Example 1>

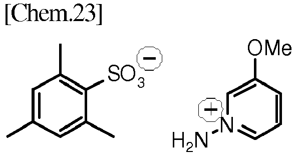

### 1-Amino-3-methoxypyridinium 2,4,6-trimethylbenzene sulfonate

A 70% perchloric acid aqueous solution (2.28 mL) was added to a 1,4-dioxane solution (5.5 mL) of ethyl *O*-mesitylsulfonylacetohydroxamate (6.28 g) under an argon atmosphere under ice-cooling, and then the mixture was stirred for 30 minutes under ice-cooling. Ice water (60 mL) was added to the reaction solution, the precipitated solid was filtered off, the obtained solid was dissolved in dichloromethane (18.5 mL), and the solution was divided into layers. The organic layer was dried over anhydrous magnesium sulfate and filtered off. A dichloromethane solution (18.5 mL) of 3-methoxypyridine (2.00 g) was added to the obtained filtrate under ice-cooling, the mixture was stirred at room temperature for 1 hour, and the reaction solution was evaporated to obtain a title compound as a colorless crystal (5.83 g).
¹H-NMR (400 MHz, DMSO-d₆) δ 2.17 (3H, s), 2.49 (6H, s), 3.96 (3H, s), 6.74 (2H, s), 7.90-7.93 (2H, m), 8.38-8.43 (1H, m), 8.43-8.50 (2H, brs), 8.57-8.60 (1H, m).

### <Reference Example 2-1>

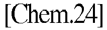

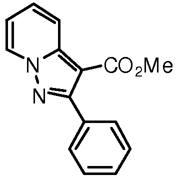

### Methyl 2-phenylpyrazolo[1,5-a]pyridine-3-carboxylate

Potassium carbonate (34.5 g) and methyl phenylpropiolate (10 g) were added to a methanol solution (312 mL) of 1-aminopyridinium iodide (27.8 g) under an argon atmosphere under ice-cooling, and then the mixture was stirred for 30 minutes under ice-cooling. Water was added to the reaction solution, and then the mixture was extracted with ethyl acetate. The organic layer was washed with water and saturated saline in this order, then dried over anhydrous magnesium sulfate, then the solvent was evaporated under vacuum, and the residue was then purified by silica gel column chromatography (ethyl acetate) to obtain a title compound as a yellow solid (12.1 g).
¹H-NMR (400 MHz, CDCl₃) δ 3.84 (3H, s), 6.98 (1H, td, J = 7.3, 1.2 Hz), 7.59-7.62 (1H, m), 7.71 (2H, t, J = 7.3 Hz), 7.78-7.80 (2H, m), 7.94-7.99 (1H, m), 8.21 (1H, d, J = 9.1 Hz), 8.54(1H,d,J=6.7Hz).

### <Reference Example 2-2>

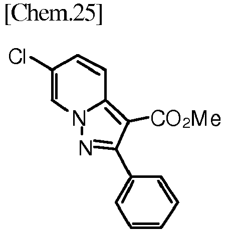

### Methyl 6-chloro-2-phenylpyrazolo[1,5-a]pyridine-3-carboxylate

Potassium carbonate (34.5 g) and 1-amino-3-chloropyridinium 2,4,6-trimethylbenzenesulfonate (41 g) were added to an *N*,*N*-dimethylformamide solution (312 mL) of methyl phenylpropiolate (10 g) at room temperature under an argon atmosphere, and then the mixture was stirred at room temperature for 20 hours. Water was added to the reaction solution, and then the mixture was extracted with ethyl acetate. The organic layer was washed with water and saturated saline in this order and then dried over anhydrous magnesium sulfate. The solvent was evaporated and then the residue was purified by silica gel column chromatography (*n*-hexane:ethyl acetate = 1:10) to obtain a title compound (3.6 g) as a colorless powder.
¹H-NMR (400 MHz, CDCl₃) δ 3.84 (3H, s), 7.40 (1H, dd, J = 9.7, 1.8 Hz), 7.45-7.48 (3H, m), 7.76-7.78 (2H, m), 8.17 (1H, d, J = 9.7 Hz), 8.57-8.58 (1H, m).

### <Reference Example 2-3>

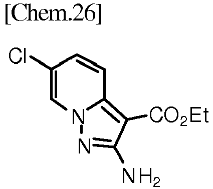

### Ethyl 2-amino-6-chloropyrazolo[1,5-a]pyridine-3-carboxylate

Potassium carbonate (70.5 g) was added to an ethanol (250 mL) solution of ethyl 3-ethoxy-3-iminopropionate hydrochloride (25.0 g) and 1-amino-3-chloropyridinium 2,4,6-trimethylbenzenesulfonate (75.8 g) at 0°C, and then the mixture was stirred at room temperature for 5 hours. The reaction solution was diluted with ethyl acetate, then washed with water, 5% hydrochloric acid, a saturated sodium bicarbonate aqueous solution, and saturated saline, and then dried over anhydrous sodium sulfate. The solvent was evaporated, then the residue thus obtained was purified by silica gel column chromatography (*n*-hexane:ethyl acetate = 2:1) to obtain a title compound as a yellow crystal (900 mg).
¹H-NMR (400 MHz, CDCl₃) δ 1.42 (3H, t, J = 7.3 Hz), 4.37 (2H, q, J = 7.3 Hz), 5.24 (2H, brs), 7.27 (1H, dd, J = 9.7 and 1.8 Hz), 7.74 (1H, d, J = 9.7 Hz), 8.26 (1H, d, J =1.8 Hz).

### <Reference Example 3-1>

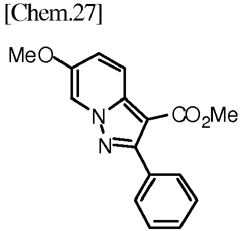

### Methyl 6-methoxy-2-phenylpyrazolo[1,5-a]pyridine-3-carboxylate

A 70% perchloric acid aqueous solution (28.5 mL) was added to a 1,4-dioxane solution (69 mL) of ethyl *O*-mesitylsulfonylacetohydroxamate (78.5 g) under an argon atmosphere under ice-cooling, and then the mixture was stirred for 30 minutes under ice-cooling. Ice water (750 mL) was added to the reaction mixture, then the precipitated solid was filtered off, and then the solid thus obtained was dissolved in dichloromethane (229 mL), and the solution was divided into layers. The organic layer was dried over anhydrous magnesium sulfate and filtered off. A dichloromethane solution (229 mL) of 3-methoxypyridine (25 g) was added to the obtained filtrate under ice-cooling, then the reaction mixture was stirred at room temperature for 1 hour, and then it was evaporated to obtain 1-amino-3-methoxypyridinium2,4,6-trimethylbenzenesulfonate.
Potassium carbonate (63 g) and methyl phenylpropiolate (18 mL) were added to 1-amino-3-methoxypyridinium 2,4,6-trimethylbenzenesulfonate in a mixed solution of tetrahydrofuran and *N*,*N*-dimethylformamide (253 mL, 10:1) at room temperature under an argon atmosphere. Then *N*,*N*-dimethylformamide (217 mL) was added until the reaction started to progress and then the mixture was stirred at room temperature for 20 hours. Water was added to the reaction solution, and then the mixture was extracted with ethyl acetate. The organic layer was washed with water and saturated saline in this order and then dried over anhydrous magnesium sulfate. The solvent was evaporated and then the residue was purified by silica gel column chromatography (*n*-hexane:ethyl acetate = 4:1) to obtain a title compound as a colorless powder (6.6 g).
¹H-NMR (400 MHz, CDCl₃) δ 3.83 (3H, s), 3.88 (3H, s), 7.21 (1H, dd, J = 9.7, 1.8 Hz), 7.42-7.48 (3H, m), 7.76-7.79 (2H, m), 8.09 (1H, d, J = 9.7 Hz), 8.12 (1H, d, J =1.8 Hz).

### <Reference Example 3-2>

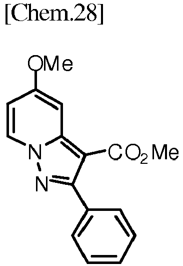

### Methyl 5-methoxy-2-phenylpyrazolo[1,5-a]pyridine-3-carboxylate

A 70% perchloric acid aqueous solution (21.7 mL) was added to a 1,4-dioxane solution (53 mL) of ethyl *O*-mesitylsulfonylacetohydroxamate (59.9 g) under an argon atmosphere under ice-cooling, and then the mixture was stirred for 30 minutes under ice-cooling. Ice water (600 mL) was added to the reaction mixture, then the precipitated solid was filtered off, and then the solid thus obtained was dissolved in dichloromethane (175 mL), and the solution was divided into layers. The organic layer was dried over anhydrous magnesium sulfate and filtered off. A dichloromethane solution (175 mL) of 4-methoxypyridine (19.1 g) was added to the obtained filtrate under ice-cooling, then the reaction mixture was stirred at room temperature for 2 hour, and then it was evaporated to obtain 1-amino-4-methoxypyridinium2,4,6-trimethylbenzenesulfonate.
1-Amino-4-methoxypyridinium2,4,6-trimethylbenzenesulfonate (39 g) was dissolved in a mixed solution of tetrahydrofuran and *N*,*N*-dimethylformamide (191 mL, 10:1) under an argon atmosphere, and potassium carbonate (48.4 g) and methyl phenylpropiolate (13.5 mL) were added at room temperature, and then the mixture was stirred for 19 hours. Water was added to the reaction solution, and then the mixture was extracted with ethyl acetate. The organic layer was washed with water and saturated saline in this order and then dried over anhydrous magnesium sulfate and then the solvent was evaporated under vacuum to obtain a title compound as brown liquid (23.6 g).
¹H-NMR (400 MHz, CDCl₃) δ 3.80 (3H, s), 3.96 (3H, s), 6.64 (1H, dd, J = 7.3, 2.4 Hz), 6.81 (1H, d, J = 6.1 Hz), 7.42-7.48 (2H, m), 7.50 (1H, d, J = 2.4 Hz), 7.71-7.77 (1H, m), 8.33 (1H, d, J = 7.3 Hz), 8.43 (1H, d, J = 6.1 Hz).

### <Reference Example 3-3>

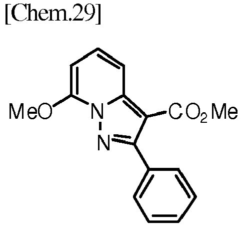

### Methyl 7-methoxy-2-phenylpyrazolo[1,5-a]pyridine-3-carboxylate

A 70% perchloric acid aqueous solution (28.5 mL) was added to a 1,4-dioxane solution (69 mL) of ethyl *O*-mesitylsulfonylacetohydroxamate (78.5 g) under an argon atmosphere under ice-cooling, and then the mixture was stirred for 30 minutes under ice-cooling. Ice water (750 mL) was added to the reaction solution, then the precipitated solid was filtered off, and then the obtained solid was dissolved in dichloromethane (229 mL), and the solution was divided into layers. The organic layer was dried over anhydrous magnesium sulfate and filtered off. A dichloromethane solution (229 mL) of 2-methoxypyridine (25 g) was added to the obtained filtrate under ice-cooling, then the mixture was stirred at room temperature for 1 hour, and then the reaction solution was evaporated to obtain 1-amino-2-methoxypyridinium 2,4,6-trimethylbenzenesulfonate.
Potassium carbonate (63 g) and methyl phenylpropiolate (18 mL) were added to an *N*,*N*-dimethylformamide solution (230 mL) of 1-amino-2-methoxypyridinium 2,4,6-trimethylbenzenesulfonate at room temperature under an argon atmosphere, and the mixture was stirred at room temperature for 17 hours. Water was added to the reaction solution, and then the mixture was extracted with ethyl acetate. The organic layer was washed with water and saturated saline in this order and then dried over anhydrous magnesium sulfate. The solvent was evaporated, then the residue was purified by silica gel column chromatography (*n*-hexane:ethyl acetate = 2:1 to 1:1) to obtain a title compound as a colorless powder (9.1 g).
¹H-NMR (400 MHz, CDCl₃) δ 3.82 (3H, s), 4.19 (3H, s), 6.32 (1H, dd, J = 7.9, 1.2 Hz), 7.41-7.46 (4H, m), 7.74-7.78 (2H, m), 7.86 (1H, dd, J = 9.1, 1.2 Hz).

### <Reference Example 4-1>

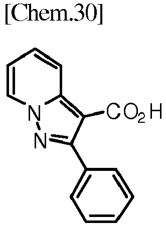

### 2-Phenylpyrazolo[1,5-a]pyridine-3-carboxylic acid

A 2 mol/L potassium hydroxide aqueous solution (119 mL) was added to a methanol solution (238 mL) of methyl 2-phenylpyrazolo[1,5-a]pyridine-3-carboxylate (12.0 g) under an argon atmosphere under ice-cooling, and then the mixture was stirred at 80°C for 5 hours. A1 mol/L hydrochloric acid aqueous solution (480 mL) was added to the reaction solution and the precipitated solid was filtered off. The obtained solid was dried under vacuum to obtain a title compound as an orange solid (10.2 g).
¹H-NMR (400 MHz, DMSO-d₆) δ 7.15 (1H, td, J = 6.7, 1.4 Hz), 7.42-7.46 (3H, m), 7.55-7.58 (1H, m), 7.74-7.77 (2H, m), 8.15 (1H, d, J = 4.5 Hz), 8.84 (1H, d, J = 6.8 Hz).

### <Reference Example 4-2>

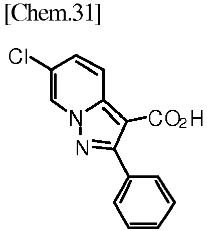

### 6-Chloro-2-phenylpyrazolo[1,5-a]pyridine-3-carboxylic acid

A 2 mol/L potassium hydroxide aqueous solution (16 mL) was added to a methanol solution (13 mL) of methyl 6-chloro-2-phenylpyrazolo[1,5-a]pyridine-3-carboxylate (1.8 g) under an argon atmosphere at room temperature, and then the mixture was stirred at 70°C for 5 hours. A 1 mol/L hydrochloric acid aqueous solution (40 mL) was added to the reaction mixture and the precipitated solid was filtered off. The obtained solid was dried under vacuum to obtain a title compound as a colorless solid (1.6 g).
¹H-NMR (400 MHz, DMSO-d₆) δ 7.43-7.47 (3H, m), 7.63 (1H, dd, J = 9.1, 1.8 Hz), 7.74 (2H, dd, J = 7.3, 1.8 Hz), 8.14 (1H, d, J = 9.1 Hz), 9.21 (1H, d, J = 1.8Hz).

### <Reference Example 4-3>

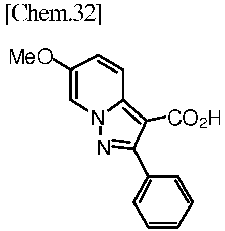

### 6-Methoxy-2-phenylpyrazolo[1,5-a]pyridine-3-carboxylic acid

A 2 mol/L potassium hydroxide aqueous solution (58 mL) was added to a methanol solution (117 mL) of methyl 6-methoxy-2-phenylpyrazolo[1,5-a]pyridine-3-carboxylate (6.6 g) at room temperature under an argon atmosphere, and the mixture was stirred at 80°C for 6 hours. A 1 mol/L hydrochloric acid was added to the reaction solution, and then the precipitated solid was filtered off to obtain a title compound as a colorless powder (6.0 g).
¹H-NMR (400 MHz, CDCl₃) δ 3.89 (3H, s), 7.20 (1H, dd, J = 9.1, 1.2 Hz), 7.40-7.43 (3H, m), 7.81-7.84 (2H, m), 8.14 (1H, s), 8.16 (1H, d, J = 9.1 Hz).

### <Reference Example 4-4>

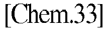

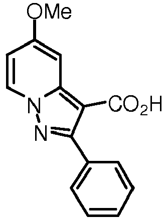

### 5-Methoxy-2-phenylpyrazolo[1,5-a]pyridine-3-carboxylic acid

A 2 mol/L potassium hydroxide aqueous solution (133 mL) was added to a methanol solution (266 mL) of methyl 5-methoxy-2-phenylpyrazolo[1,5-a]pyridine-3-carboxylate (15 g) under an argon atmosphere at room temperature, and then the mixture was stirred at 80°C for 16 hours. A 1 mol/L hydrochloric acid aqueous solution (250 mL) was added to the reaction mixture and the precipitated solid was filtered off. The obtained solid was dried under vacuum to obtain a title compound as a brown powder (14.2 g).
¹H-NMR (400 MHz, DMSO-d₆) δ 3.90 (3H, s), 6.81 (1H, dd, J = 7.3, 3.0 Hz), 7.39-7.44 (4H, m), 7.68-7.74 (2H, m), 8.69 (1H, d, J = 7.3 Hz).

### <Reference Example 4-5>

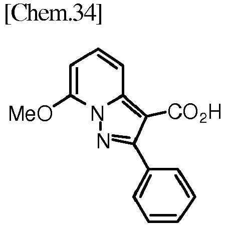

### 7-Methoxy-2-phenylpyrazolo[1,5-a]pyridine-3-carboxylic acid

A 2 mol/L potassium hydroxide aqueous solution (80 mL) was added to a methanol solution (160 mL) of methyl 7-methoxy-2-phenylpyrazolo[1,5-a]pyridine-3-carboxylate (9.0 g) at room temperature under an argon atmosphere, and the mixture was stirred at 80°C for 3 hours. A 1 mol/L hydrochloric acid was added to the reaction mixture, and then the precipitated solid was filtered off to obtain a title compound as a colorless powder (8.6 g).
¹H-NMR (400 MHz, CDCl₃) δ 4.19 (3H, s), 6.35 (1H, dd, J = 7.9, 1.2 Hz), 7.42-7.49 (4H, m), 7.77-7.83 (2H, m), 7.93 (1H, dd, J = 9.1,1.2 Hz).

### <Reference Example 4-6>

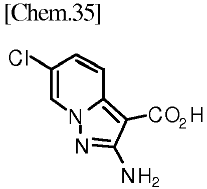

### 2-Amino-6-chloropyrazolo[1,5-a]pyridine-3-carboxylic acid

A 2 mol/L potassium hydroxide aqueous solution (9.0 mL) was added to a methanol solution (7.5 mL) of ethyl 2-amino-6-chloropyrazolo [1,5-a]pyridine-3-carboxylate (860 mg) under an argon atmosphere, and the mixture was heated and stirred at 80°C for 4 hours. After cooling down the reaction mixture to room temperature, a 1 mol/L hydrochloric acid (18.0 mL) was added to the reaction mixture, and then the precipitated solid was filtered off to obtain a title compound as a yellow crystal (616 mg).
¹H-NMR (400 MHz, DMSO-d₆) δ 6.15 (2H, brs), 7.43 (1H, dd, J = 9.7, 1.8 Hz), 7.66 (1H, d, J = 9.7 Hz), 8.78 (1H, d, J =1.8 Hz), 12.31 (1H, brs).

### <Reference Example 5-1>

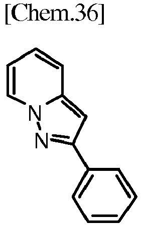

### 2-Phenylpyrazolo[1,5-a]pyridine

An *o*-dichlorobenzene solution (42 mL) of 2-phenylpyrazolo[1,5-a]pyridine-3-carboxylic acid (10.0 g) was stirred at 160°C for 2 hours under an argon atmosphere. The reaction solution was evaporated under vacuum and the obtained solid was washed with *n*-hexane to obtain a title compound as a brown solid (9.45 g).
¹H-NMR (400 MHz, CDCl₃) δ 6.72 (1H, td, J = 7.3,1.2 Hz), 6.79 (1H, s), 7.05-7.11 (1H, m), 7.18-7.21 (2H, m), 7.34-7.39 (1H, m), 7.44-7.49 (1H, m), 7.97 (2H, d, J = 7.3 Hz), 8.47 (1H, d, J = 7.9 Hz).

### <Reference Example 5-2>

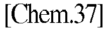

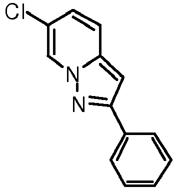

### 6-Chloro-2-phenylpyrazolo[1,5-a]pyridine

An *o*-dichlorobenzene solution (12 mL) of 6-chloro-2-phenylpyrazolo[1,5-a]pyridine-3-carboxylic acid (1.6 g) was stirred at 160°C for 2 hour under an argon atmosphere. The reaction mixture was purified by a silica gel column chromatography (*n*-hexane:ethyl acetate = 1:0 to 4:1) to obtain a title compound as a colorless powder (802 mg).
¹H-NMR (400 MHz, CDCl₃) δ 6.82 (1H, s), 7.08 (1H, dd, J = 9.1, 1.8 Hz), 7.38 (1H, t, J = 7.3 Hz), 7.46 (3H, t, J = 7.3 Hz), 7.94 (2H, d, J = 7.3 Hz), 8.52 (1H, s).

### <Reference Example 5-3>

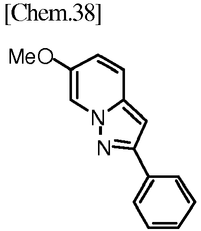

### 6-Methoxy-2-phenylpyrazolo[1,5-a]pyridine

An *o*-dichlorobenzene solution (22 mL) of 6-methoxy-2-phenylpyrazolo[1,5-a]pyridine-3-carboxylic acid (5.9 g) was stirred at 160°C for 1 hour under an argon atmosphere. The reaction mixture was purified by a silica gel chromatography (*n*-hexane:ethyl acetate = 1:0 to 2:1) to obtain a title compound as a colorless powder (3.2 g).
¹H-NMR (400 MHz, CDCl₃) δ 3.85 (3H, s), 6.74 (1H, s), 6.92 (1H, dd, J = 9.7, 1.8 Hz), 7.34 (1H, tt, J = 7.3, 1.8 Hz), 7.40 (1H, d, J = 9.7 Hz), 7.44 (2H, t, J = 7.3 Hz), 7.91-7.94 (2H, m), 8.09 (1H, d, J = 1. 8Hz).

### <Reference Example 5-4>

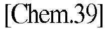

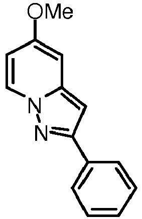

### 5-Methoxy-2-phenylpyrazolo[1,5-a]pyridine

A dimethylsulfoxide solution (37 mL) of 5-methoxy-2-phenylpyrazolo[1,5-a]pyridine-3-carboxylic acid (5.0 g) was stirred at 160°C for 2 hours under an argon atmosphere. The reaction solution was diluted with ethyl acetate, the organic layer was washed with water and saturated saline in this order and then dried over anhydrous magnesium sulfate, and then the solvent was evaporated under vacuum. The residue was purified by silica gel column chromatography (*n*-hexane:ethyl acetate = 1:10) to obtain a title compound as a colorless powder (3.61 g).
¹H-NMR (400 MHz, CDCl₃) δ 3.84 (3H, s), 6.43 (1H, dd, J = 7.3, 3.0 Hz), 6.61 (1H, s), 6.72 (1H, d, J = 3.0 Hz), 7.35 (1H, t, J = 7.3 Hz), 744 (2H, t, J = 7.3 Hz), 7.90-7.95 (2H, m), 8.28 (1H, d, J = 7.3 Hz).

### <Reference Example 5-5>

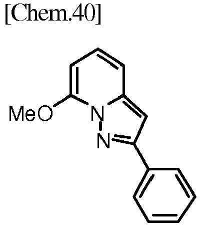

### 7-Methoxy-2-phenylpyrazolo[1,5-a]pyridine

An *o*-dichlorobenzene solution (32 mL) of 7-methoxy-2-phenylpyrazolo[1,5-a]pyridine-3-carboxylic acid (8.6 g) was stirred at 160°C for 1 hour under an argon atmosphere. The reaction solution was purified by silica gel chromatography (*n*-hexane:ethyl acetate = 1:0 to 2:1) to obtain a title compound as a colorless powder (5.8 g).
¹H-NMR (400 MHz, CDCl₃) δ 4.17 (3H, s), 6.08 (1H, dd, J = 7.3, 1.2 Hz), 6.81 (1H, s), 7.12 (1H, d, J = 7.3 Hz), 7.18 (1H, dd, J = 9.1, 1.2 Hz), 7.33-7.38 (1H, m), 7.41-7.46 (2H, m), 8.00-8.04 (2H, m).

### <Reference Example 5-6>

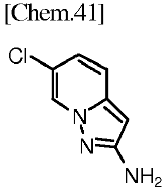

### 2-Amino-6-chloropyrazolo[1,5-a]pyridine

After concentrated sulfuric acid (0.1 mL) was added to an ethanol solution (28 mL) of 2-amino-6-chloropyrazolo[1,5-a]pyridine-3-carboxylic acid (605 mg) under an argon atmosphere, the mixture was heated and stirred at 100°C for 2 hours. A saturated sodium bicarbonate aqueous solution was added to the reaction solution, and then the mixture was extracted with dichloromethane and dried over anhydrous sodium sulfate. The solvent was evaporated and then the residue thus obtained was purified by silica gel chromatography (ethyl acetate:*n*-hexane = 2:1) to obtain a title compound as a colorless powder (289 mg).
¹H-NMR (400 MHz, DMSO-d6) δ 5.37 (2H, brs), 5.68 (1H, s), 7.02 (1H, dd, J = 9.7, 1.8 Hz), 7.27 (1H, d, J = 9.7 Hz), 8.51 (1H, d, J =1.8 Hz).

### <Reference Example 6-1>

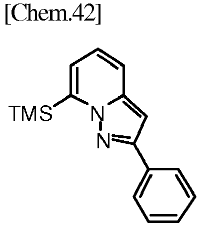

### 2-Phenyl-7-(trimethylsilyl)pyrazolo[1,5-a]pyridine

*n*-Butyllithium (3.8 mL, a 2.6 mol/L *n*-hexane solution) was added to a tetrahydrofuran solution (41 mL) of 2-phenylpyrazolo[1,5-a]pyridine (1.6 g) at -78°C under an argon atmosphere, and then the mixture was stirred at -78°C for 30 minutes. Trimethylsilyl chloride (5.3 mL) was added to the obtained mixture at -78°C and then the mixture was stirred at room temperature for 1 hour. A saturated ammonium chloride aqueous solution was added to the reaction solution and the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as colorless liquid (2.4 g).
¹H-NMR (400 MHz, CDCl₃) δ 0.61 (9H, s), 6.89 (1H, s), 6.92 (1H, dd, J = 6.7, 1.2 Hz), 7.13 (1H, dd, J = 8.5, 6.7 Hz), 7.45 (1H, tt, J = 7.3, 1.2 Hz), 7.52-7.57 (2H, m), 7.60 (1H, dd, J = 8.5, 1.2 Hz), 8.11 (2H, dd, J = 8.5, 1.2 Hz).

### <Reference Example 6-2>

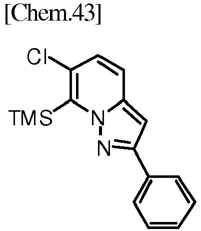

### 6-Chloro-2-phenyl-7-(trimethylsilyl)pyrazolo[1,5-a]pyridine

*n*-Butyllithium (1.4 mL, a 2.6 mol/L *n*-hexane solution) was added to a tetrahydrofuran solution (15 mL) of 6-chloro-2-phenylpyrazolo[1,5-a]pyridine (689 mg) at -78°C under an argon atmosphere, and then the mixture was stirred at -78°C for 30 minutes. Trimethylsilyl chloride (1.9 mL) was added to the obtained mixture at -78°C and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4:1) to obtain a title compound as yellow powder (830 mg).
¹H-NMR (400 MHz, CDCl₃) δ 0.63 (9H, s), 6.78 (1H, s), 7.00 (1H, d, J = 9.1 Hz), 7.36 (1H, tt, J = 7.3, 1.2 Hz), 7.40-7.47 (3H, m), 7.94-7.97 (2H, m).

### <Reference Example 6-3>

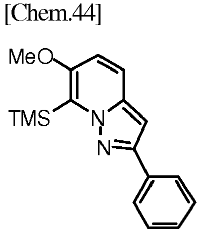

### 6-Methoxy-2-phenyl-7-(trimethylsilyl)pylazolo[1,5-a]pyridine

*n*-Butyllithium (2.1 mL, a 2.6 mol/L *n*-hexane solution) was added to a tetrahydrofuran solution (22 mL) of 6-methoxy-2-phenylpyrazolo[1,5-a]pyridine (1.0 g) at -78°C under an argon atmosphere, and then the mixture was stirred at -78°C for 30 minutes. Trimethylsilyl chloride (2.8 mL) was added to the obtained mixture at -78°C and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4:1) to obtain a title compound as yellow liquid (1.24 g).
¹H-NMR (400 MHz, CDCl₃) δ 0.54 (9H, s), 3.82 (3H, s), 6.74 (1H, s), 6.99 (1H, d, J = 9.7 Hz), 7.32 (1H, t, J = 7.9 Hz), 7.42 (2H, t, J = 7.9 Hz), 7.47 (1H, d, J = 9.7 Hz), 7.96 (2H, d, J =7.9Hz).

### <Reference Example 6-4>

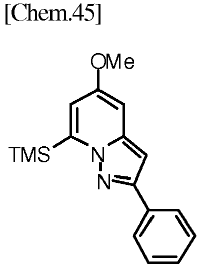

### 5-Methoxy-2-phenyl-7-(trimethylsilyl)pyrazolo[1,5-a]pyridine

*n*-Butyllithium (1.0 mL, a 2.6 mol/L *n*-hexane solution) was added to a tetrahydrofuran solution (11 mL) of 5-methoxy-2-phenylpyrazolo[1,5-a]pyridine (500 mg) at -78°C under an argon atmosphere, and then the mixture was stirred at -78°C for 30 minutes. Trimethylsilyl chloride (1.4 mL) was added to the obtained mixture at -78°C and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4:1) to obtain a title compound as pale yellow liquid (649 mg).
¹H-NMR (400 MHz, CDCl₃) δ 0.48 (9H, s), 3.84 (3H, s), 6.50 (1H, d, J = 3.0 Hz), 6.60 (1H, s), 6.72 (1H, d, J = 3.0 Hz), 7.34 (1H, dt, J = 7.3, 1.2 Hz), 7.40-7.45 (2H, m), 7.95-7.98 (2H, m).

### <Reference Example 6-5>

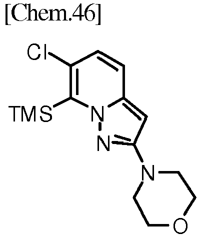

### 6-Chloro-2-(morpholin-4-yl)-7-(trimethylsilyl)pyrazolo[1,5-a]pyridine

*n*-Butyllithium (1.05 mL, a 1.6 mol/L *n*-hexane solution) was added to a tetrahydrofuran solution (7 mL) of 6-chloro-2-(morpholin-4-yl)pyrazolo[1,5-a]pyridine (330 mg) at -78°C under an argon atmosphere, and then the mixture was stirred at -78°C for 30 minutes. Trimethylsilyl chloride (0.9 mL) was added to the obtained mixture at -78°C and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 3:1) to obtain a title compound as a pale yellow crystal (422 mg).
¹H-NMR (400 MHz, CDCl₃) δ 0.54 (9H, s), 3.31 (4H, t, J = 4.9 Hz), 3.87 (4H, t, J = 4.9 Hz), 5.79(1H,s),6.94(1H,d,J=9.2Hz),7.18(1H,d,J=9.2Hz).

### <Reference Example 7-1>

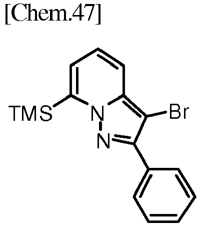

### 3-Bromo-2-phenyl-7-(trimethylsilyl)pyrazolo[1,5-a]pyridine

*N*-bromosuccinimide (1.8 g) was added to an acetonitrile solution (41 mL) of 2-phenyl-7-(trimethylsilyl)pyrazolo[1,5-a]pyridine (2.2 g) at room temperature under an argon atmosphere, and then the mixture was stirred at room temperature for 1 hour. Water was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4:1) to obtain a title compound as a pink powder (1.7 g).
¹H-NMR (400 MHz, CDCl₃) δ 0.61 (9H, s), 7.02 (1H, dd, J = 6.7, 1.2 Hz), 7.27 (1H, dd, J = 8.5, 6.7 Hz), 7.54 (1H, tt, J = 7.3, 1.2 Hz), 7.61 (2H, tt, J = 8.5, 1.2 Hz), 7.65 (1H, dd, J = 8.5, 1.2 Hz), 8.25-8.28 (2H, m).

### <Reference Example 7-2>

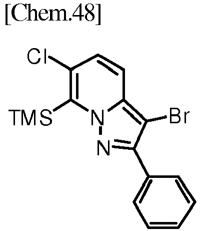

### 3-Bromo-6-chloro-2-phenyl-7-(trimethylsilyl)pyrazolo[1,5-a]pyridine

*N*-bromosuccinimide (589 mg) was added to an acetonitrile solution (14 mL) of 6-chloro-2-phenyl-7-(trimethylsilyl)pyrazolo[1,5-a]pyridine (830 mg) at room temperature under an argon atmosphere, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as a yellow powder (970 mg).
¹H-NMR (400 MHz, CDCl₃) δ 0.61 (9H, s), 7.11 (1H, d, J = 9.1 Hz), 7.42 (1H, d, J = 7.3 Hz), 7.44 (1H, d, J = 9.1 Hz), 7.49 (2H, t, J = 7.3 Hz), 8.11 (2H, d, J = 7.3 Hz).

### <Reference Example 7-3>

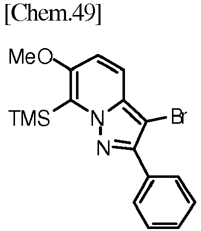

### 3-Bromo-6-methoxy-2-phenyl-7-(trimethylsilyl)pyrazolo[1,5-a]pyridine

*N*-bromosuccinimide (865 mg) was added to an acetonitrile solution (20 mL) of 6-methoxy-2-phenyl-7-(trimethylsilyl)pyrazolo[1,5-a]pyridine (1.2 g) at room temperature under an argon atmosphere, and then the mixture was stirred at room temperature for 1 hour. A saturated sodium bicarbonate aqueous solution was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 10:1) to obtain a title compound as a yellow powder (1.42 g).
¹H-NMR (400 MHz, CDCl₃) δ 0.52 (9H, s), 3.83 (3H, s), 7.09 (1H, d, J = 9.7 Hz), 7.39 (1H, tt, =7.3, 1.2 Hz), 7.44-7.49 (2H, m), 7.49 (1H, d, J = 9.7 Hz), 8.11-8.14 (2H, m).

### <Reference Example 7-4>

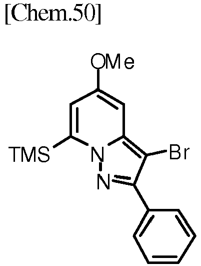

### 3-Bromo-5-methoxy-2-phenyl-7-(trimethylsilyl)pyrazolo[1,5-a]pyridine

*N*-bromosuccinimide (461 mg) was added to an acetonitrile solution (11 mL) of 5-methoxy-2-phenyl-7-(trimethylsilyl)pyrazolo[1,5-a]pyridine (640 mg) at room temperature under an argon atmosphere, and then the mixture was stirred at room temperature for 3 hour. A saturated sodium bicarbonate aqueous solution was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4:1) to obtain a title compound as a colorless powder (712 mg).
¹H-NMR (400 MHz, CDCl₃) δ 0.47 (9H, s), 3.90 (3H, s), 6.56 (1H, d, J = 3.0 Hz), 6.68 (1H, d, J = 3.0 Hz), 7.40 (1H, dt, J = 7.3, 1.2 Hz), 7.47 (2H, tt, J = 7.3,1.2 Hz), 8.09-8.12 (2H, m).

### <Reference Example 7-5>

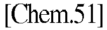

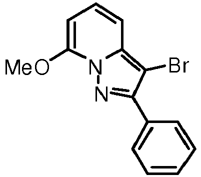

### 3-Bromo-7-methoxy-2-phenylpyrazolo[1,5-a]pyridine

*N*-bromosuccinimide (190 mg) was added to an acetonitrile solution (4.5 mL) of 7-methoxy-2-phenylpyrazolo[1,5-a]pyridine (200 mg) at room temperature under an argon atmosphere, and then the mixture was stirred at room temperature for 1 hour. A saturated sodium bicarbonate aqueous solution was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4: 1) to obtain a title compound as pale yellow liquid (270 mg).
¹H-NMR (400 MHz,, CDCl₃) δ 4.18 (3H, s), 6.16 (1H, dd, J = 7.3, 1.2 Hz), 7.18-7.24 (2H, m), 7.38-7.50 (3H, m), 8.03-8.07 (2H, m).

### <Reference Example 7-6>

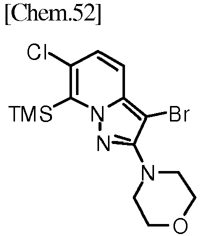

### 3-Bromo-6-chloro-2-(morpholin-4-yl)-7-(trimethylsilyl)pyrazolo[1,5-a]pyridine

[0214] *N*-bromosuccinimide (220 mg) was added to an acetonitrile solution (6 mL) of 6-chloro-2-(morpholin-4-yl)-7-(trimethylsilyl)pyrazolo[1,5-a]pyridine (376 mg) at 0°C under an argon atmosphere, and then the mixture was stirred at 0°C for 3 hour. Water was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 5:1) to obtain a title compound as a yellow oil (247 mg).
¹H-NMR (400 MHz, CDCl₃) δ 0.54 (9H, s), 3.45 (4H, t, J = 4.9 Hz), 3.88 (4H, t, J = 4.9 Hz), 7.03 (1H, d, J = 9.2 Hz), 7.23 (1H, d, J = 9.2 Hz).

### <Reference Example 8-1>

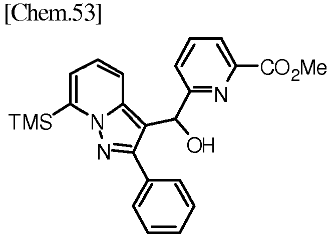

### Methyl 6-[hydroxy[2-phenyl-7-(trimethylsilyl)pyrazolo[1,5-a]pyridin-3-yl]methyl]pyridine-2-carbox ylate

*n*-Butyllithium (0.7 mL, a 2.6 mol/L *n*-hexane solution) was added to a tetrahydrofuran solution (4 mL) of 3-bromo-2-phenyl-7-(trimethylsilyl)pyrazolo[1,5-a]pyridine (550 mg) at -78°C under an argon atmosphere, and then the mixture was stirred at -78°C for 30 minutes. A tetrahydrofuran solution (4 mL) of methyl 6-formylpyridine-2-carboxylate (527 mg) was added to the obtained mixture at -78°C, and then the mixture was stirred at room temperature for 1 hour. A saturated ammonium chloride aqueous solution was added to the reaction solution and the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as yellow liquid (361 mg).
¹H-NMR (400 MHz, CDCl₃) δ 0.63 (9H, s), 4.21 (3H, s), 5.53 (1H, d, J = 2.4 Hz), 6.46 (1H, d, J = 2.4 Hz), 6.99 (1H, dd, J = 6.7, 1.8 Hz), 7.06 (1H, dd, J = 9.1, 6.7 Hz), 7.18 (1H, dd, J = 9.1, 1.8 Hz), 7.41-7.44 (2H, m), 7.62 (2H, tt, J = 7.3, 1.2 Hz), 7.86 (1H, t, J = 7.3 Hz), 8.03-8.07 (2H, m), 8.18 (1H, d, J = 7.3 Hz).

### <Reference Example 8-2>

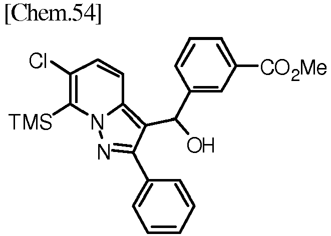

### Methyl 3-[[6-chloro-2-phenyl-7-(trimethylsilyl)pyrazolo[1,5-a]pyridin-3-yl](hydroxy)methyl] benzoate

*n*-Butyllithium (0.5 mL, a 2.6 mol/L *n*-hexane solution) was added to a tetrahydrofuran solution (2.5 mL) of 3-bromo-6-chloro-2-phenyl-7-(trimethylsilyl)pyrazolo[1,5-a]pyridine (400 mg) at -78°C under an argon atmosphere, and then the mixture was stirred at -78°C for 30 minutes. A tetrahydrofuran solution (2.5 mL) of methyl 3-formyl benzoate (346 mg) was added to the obtained mixture at -78°C, and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4:1) to obtain a title compound as yellow liquid (350 mg).
¹H-NMR (400 MHz, CDCl₃) δ 0.61 (9H, s), 3.91 (3H, s), 6.36 (1H, d, J = 3.6 Hz), 6.88 (1H, d, J = 9.7 Hz), 7.03 (1H, d, J = 9.7 Hz), 7.38-7.49 (4H, m), 7.59-7.62 (1H, m), 7.76-7.79 (2H, m), 7.96 (1H, d, J = 7.9 Hz), 8.16-8.18 (1H, m).

### <Reference Example 8-3>

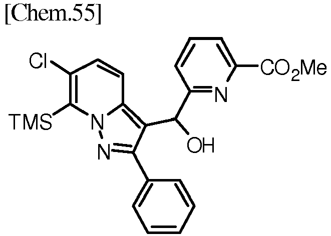

### Methyl 6-[[6-chloro-2-phenyl-7-(trimethylsilyl)pyrazolo[1,5-a]pyridin-3-yl] (hydroxy)methyl]pyridin e-2-carboxylate

*n*-Butyllithium (0.6 mL, a 2.6 mol/L *n*-hexane solution) was added to a tetrahydrofuran solution (3.5 mL) of 3-bromo-6-chloro-2-phenyl-7-(trimethylsilyl)pyrazolo[1,5-a]pyridine (530 mg) at -78°C under an argon atmosphere, and then the mixture was stirred at -78°C for 30 minutes. A tetrahydrofuran solution (3.5 mL) of methyl 6-formylpyridine-2-carboxylate (461 mg) was added to the obtained mixture at -78°C, and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 2:1) to obtain a title compound as yellow liquid (345 mg).
¹H-NMR (400 MHz, CDCl₃) δ 0.59 (9H, s), 4.05 (3H, s), 5.34 (1H, d, J = 3.0 Hz), 6.28 (1H, d, J = 3.0 Hz), 6.87 (1H, d, J = 9.7 Hz), 6.99 (1H, d, J = 9.7 Hz), 7.22 (1H, d, J = 7.9 Hz), 7.37-7.48 (3H, m), 7.71 (1H, t, J = 7.9 Hz), 7.83-7.87 (2H, m), 8.07 (1H, d, J = 7.9 Hz).

### <Reference Example 8-4>

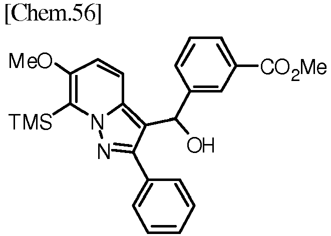

### Methyl 3-[hydroxy[6-methoxy-2-phenyl-7-(trimethylsilyl)pyrazolo[1,5-a]pyridin-3-yl]methyl] benzoate

*n*-Butyllithium (0.4 mL, a 2.6 mol/L *n*-hexane solution) was added to a tetrahydrofuran solution (2 mL) of 3-bromo-6-methoxy-2-phenyl-7-(trimethylsilyl)pyrazolo[1,5-a]pyridine (300 mg) at -78°C under an argon atmosphere, and then the mixture was stirred at -78°C for 30 minutes. A tetrahydrofuran solution (2 mL) of methyl 3-formyl benzoate (263 mg) was added to the obtained mixture at -78°C, and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4:1) to obtain a title compound as a yellow amorphous (185 mg).
¹H-NMR (400 MHz, CDCl₃) δ 0.54 (9H, s), 3.82 (3H, s), 3.97 (3H, s), 6.74 (1H, s), 6.99 (1H, d, J = 9.7 Hz), 7.33 (1H, tt, J = 7.9, 1.2 Hz), 7.40-7.45 (2H, m), 7.47 (1H, d, J = 9.7 Hz), 7.64 (1H, t, J = 7.9 Hz), 7.94-7.98 (2H, m), 8.09 (1H, dt, J = 7.9, 1.2 Hz), 8.31 (1H, dt, J = 7.9, 1.2 Hz), 8.54 (1H, t, J =1.2 Hz).

### <Reference Example 8-5>

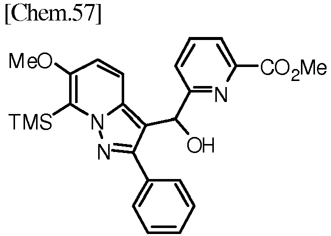

### Methyl 6-[hydroxy[6-methoxy-2-phenyl-7-(trimethylsilyl)pyrazolo[1,5-a]pyridin-3-yl]methyl]pyridi ne-2-carboxylate

*n*-Butyllithium (0.5 mL, a 2.6 mol/L *n*-hexane solution) was added to a tetrahydrofuran solution (2.8 mL) of 3-bromo-6-methoxy-2-phenyl-7-(trimethylsilyl)pyrazolo[1,5-a]pyridine (410 mg) at -78°C under an argon atmosphere, and then the mixture was stirred at -78°C for 30 minutes. A tetrahydrofuran solution (2.8 mL) of methyl 6-formylpyridine-2-carboxylate (360 mg) was added to the obtained mixture at -78°C, and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4:1) to obtain a title compound as yellow liquid (303 mg).
¹H-NMR (400 MHz, CDCl₃) δ 0.53 (9H, s), 3.77 (3H, s), 4.07 (3H, s), 5.35 (1H, d, J = 2.4 Hz), 6.31 (1H, d, J = 2.4 Hz), 6.87 (1H, d, J = 9.7 Hz), 7.04 (1H, d, J = 9.7 Hz), 7.28 (1H, d, J = 7.9 Hz), 7.40 (1H, tt, J = 7.9, 1.8 Hz), 7.47 (2H, t, J = 7.9 Hz), 7.72 (1H, t, J = 7.9 Hz), 7.87-7.91 (2H, m), 8.04 (1H, d, J = 7.9 Hz).

### <Reference Example 8-6>

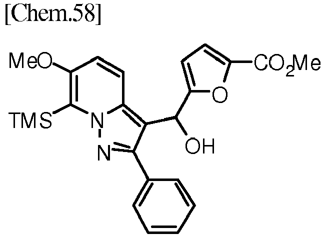

### Methyl 5-[hydroxy[6-methoxy-2-phenyl-7-(trimethylsilyl)pyrazolo[1,5-a]pyridin-3-yl]methyl]furan-carboxylate

*n*-Butyllithium (0.1 mL, a 2.6 mol/L *n*-hexane solution) was added to a tetrahydrofuran solution (0.7 mL) of 3-bromo-6-methoxy-2-phenyl-7-(trimethylsilyl)pyrazolo[1,5-a]pyridine (100 mg) at -78°C under an argon atmosphere, and then the mixture was stirred at -78°C for 30 minutes. A tetrahydrofuran solution (0.7 mL) of methyl 5-formyl-2-furan carboxylate (62 mg) was added to the obtained mixture at -78°C, and then the mixture was stirred at room temperature for 30 minutes. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 3:1) to obtain a title compound as yellow liquid (93 mg).
¹H-NMR (400 MHz, CDCl₃) δ 0.52 (9H, s), 3.81 (3H, s), 3.88 (3H, s), 6.28 (1H, d, J = 3.6 Hz),6.37(1H,d,J=3.6Hz),7.01(1H,d,J=9.7Hz),7.14(1H,d,J=3.6Hz),7.40(1H,d,J = 7.3 Hz), 7.42-7.47 (3H, m), 7.53 (1H, d, J = 9.7 Hz), 7.68-7.72 (2H, m).

### <Reference Example 8-7>

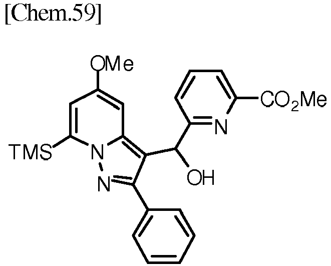

### Methyl 6-[hydroxy[5-methoxy-2-phenyl-7-(trimethylsilyl)pyrazolo[1,5-a]pyridin-3-yl]methyl]pyridi ne-2-carboxylate

*n*-Butyllithium (0.4 mL, a 2.6 mol/L *n*-hexane solution) was added to a tetrahydrofuran solution (2 mL) of 3-bromo-5-methoxy-2-phenyl-7-(trimethylsilyl)pyrazolo[1,5-a]pyridine (300 mg) at -78°C under an argon atmosphere, and then the mixture was stirred at -78°C for 30 minutes. A tetrahydrofuran solution (2 mL) of methyl 6-formylpyridine-2-carboxylate (198 mg) was added to the obtained mixture at -78°C, and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 2:1) to obtain a title compound as a colorless amorphous (330 mg). ¹H-NMR (400 MHz, CDCl₃) δ 0.45 (9H, s), 3.62 (3H, s), 4.04 (3H, s), 5.23 (1H, d, J = 3.0 Hz), 6.26 (1H, d, J = 3.0 Hz), 6.33 (1H, d, J = 3.0 Hz), 6.50 (1H, d, J = 3.0 Hz), 7.28 (1H, d, J = 7.9 Hz), 7.39 (1H, dt, J = 7.9, 1.8 Hz), 7.42-7.47 (2H, m), 7.71 (1H, t, J = 7.9 Hz), 7.83-7.87 (2H, m), 8.01 (1H, d, J = 7.9 Hz).

### <Reference Example 8-8>

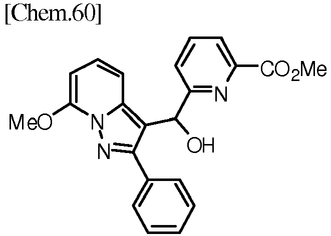

### Methyl 6-[hydroxy(7-methoxy-2-phenylpyrazolo[1,5-a]pyridin-3-yl)methyl]pyridine-2-carboxylate

*n*-Butyllithium (0.4 mL, a 2.6 mol/L *n*-hexane solution) was added to a tetrahydrofuran solution (2.2 mL) of 3-bromo-7-methoxy-2-phenylpyrazolo[1,5-a]pyridine (265 mg) at -78°C under an argon atmosphere, and then the mixture was stirred at -78°C for 30 minutes. A tetrahydrofuran solution (2.2 mL) of methyl 6-formylpyridine-2-carboxylate (216 mg) was added to the obtained mixture at -78°C, and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 1:1) to obtain a title compound as a pale yellow amorphous (248 mg).
¹H-NMR (400 MHz, CDCl₃) δ 4.04 (3H, s), 4.15 (3H, s), 5.38 (1H, d, J = 2.4 Hz), 6.09 (1H, dd, J = 7.3,1.2 Hz), 6.22 (1H, d, J = 2.4 Hz), 6.74 (1H, dd, J = 8.5, 1.2 Hz), 6.99 (1H, dd, J = 8.5, 7.3 Hz), 7.18 (1H, d, J = 7.9 Hz), 7.37-7.47 (3H, m), 7.68 (1H, t, J = 7.9 Hz), 7.83-7.87 (2H, m), 8.01 (1H, d, J = 7.9 Hz).

### <Reference Example 8-9>

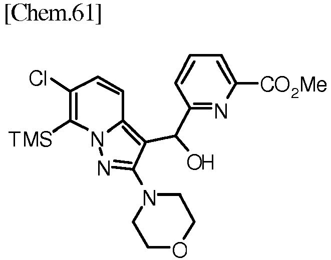

### Methyl 6-[[6-chloro-2-(morpholin-4-yl)-7-(trimethylsilyl)pyrazolo[1,5-a]pyridin-3-yl] (hydroxy)met hyl]pyridine-2-carboxylate

*n*-Butyllithium (0.25 mL, a 1.6 mol/L *n*-hexane solution) was added to a tetrahydrofuran solution (0.75 mL) of 3-bromo-6-chloro-2-(morpholin-4-yl)-7-(trimethylsilyl)pyrazolo[1,5-a]pyridine (117 mg) at -78°C under an argon atmosphere, and then the mixture was stirred at -78°C for 30 minutes. Methyl 6-formylpyridine-2-carboxylate (100 mg) was added to the obtained mixture at -78°C, and then the mixture was stirred at room temperature for 1 hour. A saturated ammonium chloride aqueous solution was added to the reaction mixture and the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 2:1) to obtain a title compound as a yellow foam (55.4 mg).
¹H-NMR (400 MHz, CDCl₃) δ 0.54 (9H, s), 3.20-3.33 (4H, m), 3.62-3.78 (4H, m), 4.03 (3H, s),5.44(1H,d,J=3.0Hz),6.12(1H,d,J=3.0Hz),6.87(1H,d,J=9.7Hz),6.95(1H,d,J= 9.7 Hz), 7.36 (1H, d, J = 7.9 Hz), 7.78 (1H, t, J = 7.9 Hz), 8.05 (1H, d, J = 7.9 Hz).

### <Reference Example 8-10>

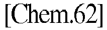

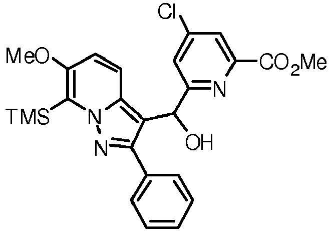

### Methyl 4-chloro-6-[hydroxy[6-methoxy-2-phenyl-7-(trimethylsilyl)pyrazolo[1,5-a]pyridin-3-yl]met hyl]pyridine-2-carboxylate

After a tetrahydrofuran solution (12.7 mL) of 3-bromo-6-methoxy-2-phenyl-7-(trimethylsilyl)pyrazolo[1,5-a]pyridine (952 mg) was cooled down to -78°C under an argon atmosphere, *n*-butyllithium (1.14 mL, a 2.66 mol/L *n*-hexane solution) was added, and the mixture was then stirred at -78°C for 30 minutes. 4-Chloro-2-formyl-6-methoxycarbonylpyridine (760 mg) was added to the obtained mixture at -78°C, and then the mixture was stirred at room temperature for 30 minutes. A saturated ammonium chloride aqueous solution was added to the reaction solution, and then the mixture was extracted with ethyl acetate. The organic layer was washed with saturated saline and then dried over anhydrous sodium sulfate. The solvent was evaporated under vacuum and then the residue was purified by silica gel column chromatography (*n*-hexane:ethyl acetate = 10:1 to 1:1) to obtain a title compound as a pale yellow solid (764 mg).
¹H-NMR (400 MHz, CDCl₃) δ 0.52 (9H, s), 3.26 (3H, s), 3.53 (3H, s), 4.42 (1H, d, J = 3.0 Hz), 5.74 (1H, d, J = 3.0 Hz), 6.38 (1H, d, J = 9.7 Hz), 6.59 (1H, d, J = 9.7 Hz), 6.76 (1H, d, J = 1.8 Hz), 6.83-6.90 (1H, m), 6.93 (2H, t, J = 7.9 Hz), 7.32 (2H, dd, J = 7.9, 1.8 Hz), 7.48 (1H, d,J=1.8Hz).

### <Reference Example 9-1>

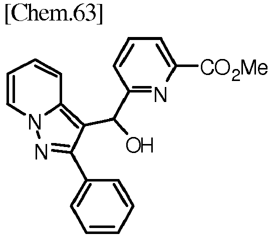

### Methyl 6-[hydroxy(2-phenylpyrazolo[1,5-a]pyridin-3-yl)methyl]pyridine-2-carboxylate

Tetrabutylammonium fluoride (1.6 mL, a 1 mol/L tetrahydrofuran solution) was added to a tetrahydrofuran solution (8.1 mL) of methyl 6-[hydroxy[2-phenyl-7-(trimethylsilyl)pyrazolo[1,5-a]pyridin-3-yl]methyl]pyridine-2-carbox ylate (350 mg) under an argon atmosphere under ice-cooling, and then the mixture was stirred for 1 hour under ice-cooling. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as a yellow amorphous (296 mg).
¹H-NMR (400 MHz, CDCl₃) δ 4.05 (3H, s), 5.37 (1H, d, J = 2.4 Hz), 6.25 (1H, d, J = 2.4 Hz), 6.75 (1H, td, J = 6.7, 1.2 Hz), 6.97 (1H, dd, J = 8.5, 6.7 Hz), 7.09 (1H, d, J = 8.5 Hz), 7.22(1H,d,J=7.9Hz),7.42(1H,d,J=7.3Hz),7.47(2H,t,J=7.3Hz),7.70(1H,t,J=7.9 Hz), 7.82-7.86 (2H, m), 8.02 (1H, d, J = 7.9 Hz), 8.48 (1H, d, J = 6.7 Hz).

### <Reference Example 9-2>

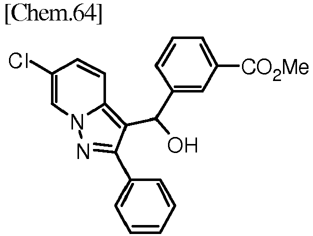

### Methyl 3-[(6-chloro-2-phenylpyrazolo[1,5-a]pyridin-3-yl)(hydroxy)methyl]benzoate

Tetrabutylammonium fluoride (1.5 mL, a 1 mol/L tetrahydrofuran solution) was added to a tetrahydrofuran solution (7.5 mL) of methyl 3-[[6-chloro-2-phenyl -7-(trimethylsilyl)pyrazolo[1,5-a]pyridin-3-yl](hydroxy)methylbenzoate (350 mg) under an argon atmosphere under ice-cooling, and then the mixture was stirred for 1 hour under ice-cooling. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 4:1) to obtain a title compound as a colorless powder (207 mg).
¹H-NMR (400 MHz, CDCl₃) δ 2.34 (1H, d, J = 3.6 Hz), 3.89 (3H, s), 6.30 (1H, d, J = 3.6 Hz), 6.97 (1H, dd, J = 9.7, 1.8 Hz), 7.14 (1H, dd, J = 9.7, 1.8 Hz), 7.38-7.50 (4H, m), 7.58 (1H, dt, J = 7.9, 1.2 Hz), 7.69-7.73 (2H, m), 7.96 (1H, dt, J = 7.9, 1.2 Hz), 8.12 (1H, s), 8.52 (1H,d,J=1.2Hz).
IR (ATR) νₘₐₓ 3182, 2952, 2865, 1723, 1589, 1514, 1469, 1436, 1327, 1285, 1271, 1181, 1077, 1026, 976, 884, 828, 769, 746, 696, 667, 584, 536, 478, 406 cm⁻¹.

### <Reference Example 9-3>

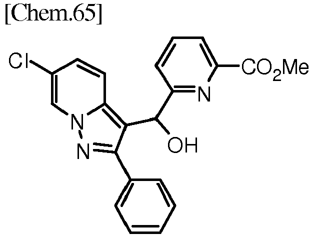

### Methyl 6-[(6-chloro-2-phenylpyrazolo[1,5-a]pyridin-3-yl)(hydroxy)methyl]pyridine-2-carboxylate

Tetrabutylammonium fluoride (1.1 mL, a 1 mol/L tetrahydrofuran solution) was added to a tetrahydrofuran solution (5.5 mL) of methyl 6-[[6-chloro-2-phenyl-7-(trimethylsilyl)pyrazolo[1,5-a]pyridin-3-yl](hydroxy)methyl]pyridine-2-carboxyl ate (216 mg) under an argon atmosphere under ice-cooling, and then the mixture was stirred for 1 hour under ice-cooling. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 2:1) to obtain a title compound as yellow liquid (162 mg).
¹H-NMR (400 MHz, CDCl₃) δ 4.03 (3H, s), 5.37 (1H, brs), 6.23 (1H, s), 6.95 (1H, dd, J = 9.7, 1.8 Hz), 7.12 (1H, d, J = 9.7 Hz), 7.20 (1H, d, J = 7.9 Hz), 7.39-7.49 (3H, m), 7.71 (1H, t, J = 7.9 Hz), 7.79-7.83 (2H, m), 8.02 (1H, d, J = 7.9 Hz), 8.50 (1H, d, J =1.8 Hz).

### <Reference Example 9-4>

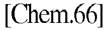

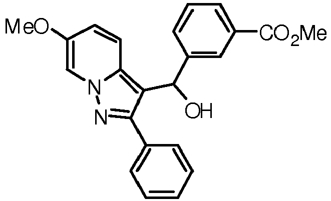

### Methyl 3-[hydroxy(6-methoxy-2-phenylpyrazolo[1,5-a]pyridin-3-yl)methyl]benzoate

Tetrabutylammonium fluoride (0.8 mL, a 1 mol/L tetrahydrofuran solution) was added to a tetrahydrofuran solution (4 mL) of methyl 3-[hydroxy[6-methoxy-2-phenyl-7-(trimethylsilyl)pyrazolo[1,5-a]pyridin-3-yl]methyl] benzoate (185 mg) under an argon atmosphere under ice-cooling, and then the mixture was stirred for 1 hour under ice-cooling. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 2:1) to obtain a title compound as a colorless amorphous (135 mg).
¹H-NMR (400 MHz, CDCl₃) δ 2.24 (1H, d, J = 3.6 Hz), 3.82 (3H, s), 3.89 (3H, s), 6.30 (1H, d, J = 3.6 Hz), 6.80 (1H, dd, J = 9.7, 2.4 Hz), 7.02 (1H, d, J = 9.7 Hz), 7.38-7.49 (4H, m), 7.59-7.63 (1H, m), 7.71-7.75 (2H, m), 7.95 (1H, d, J = 7.9 Hz), 8.09 (1H, d, J = 2.4 Hz), 8.15 (1H, s).

### <Reference Example 9-5>

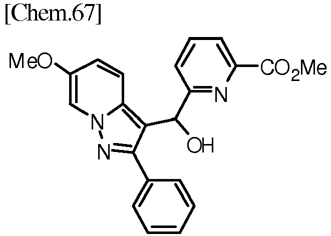

### Methyl 6-[hydroxy(6-methoxy-2-phenylpyrazolo[1,5-a]pyridin-3-yl)methyl]pyridine-2-carboxylate

Tetrabutylammonium fluoride (0.7 mL, a 1 mol/L tetrahydrofuran solution) was added to a tetrahydrofuran solution (3.2 mL) of methyl 6-[hydroxy[6-methoxy-2-phenyl-7-(trimethylsilyl)pyrazolo[1,5-a]pyridin-3-yl]methyl]pyridi ne-2-carboxylate (150 mg) under an argon atmosphere under ice-cooling, and then the mixture was stirred for 1 hour under ice-cooling. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 2:1) to obtain a title compound as yellow liquid (113 mg).
¹H-NMR (400 MHz, CDCl₃) δ 3.81 (3H, s), 4.04 (3H, s), 5.32 (1H, d, J = 2.4 Hz), 6.22 (1H, d, J = 2.4 Hz), 6.79 (1H, dd, J = 9.7, 2.4 Hz), 6.99 (1H, d, J = 9.7 Hz), 7.21 (1H, d, J = 7.9 Hz), 7.41 (1H, dt, J = 7.9, 1.2 Hz), 7.46 (2H, tt, J = 7.9, 1.2 Hz), 7.70 (1H, t, J = 7.9 Hz), 7.80-7.84 (2H, m), 8.02 (1H, d, J = 7.9 Hz), 8.08 (1H, d, J = 2.4 Hz).

### <Reference Example 9-6>

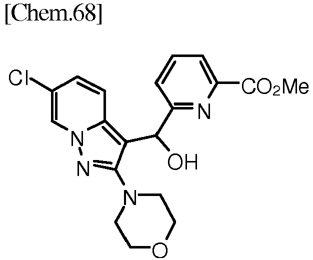

### Methyl 6-[[6-chloro-2-(morpholin-4-yl)pyrazolo[1,5-a]pyridin-3-yl)(hydroxy)methyl]pyridine-2-car boxylate

Tetrabutylammonium fluoride (0.22 mL, a 1 mol/L tetrahydrofuran solution) was added to a tetrahydrofuran solution (1mL) of methyl 6-[[6-chloro-2-(morpholin-4-yl)-7-(trimethylsilyl)pyrazolo[1,5-a]pyridin-3-yl] (hydroxy)met hyl]pyridine-2-carboxylate (52.0 mg) under an argon atmosphere under ice-cooling, and then the mixture was stirred for 1 hour at 0°C. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue was purified by silica gel column chromatography (ethyl acetate:*n*-hexane = 2: 1) to obtain a title compound as a yellow foam (31.2 mg).
¹H-NMR (400 MHz, CDCl₃) δ 3.23-3.35 (4H, m), 3.60-3.75 (4H, m), 4.33 (3H, s), 5.41 (1H, d, J = 3.0 Hz), 6.11 (1H, d, J = 3.0 Hz), 6.93 (1H, dd, J = 9.7, 1.8 Hz), 6.99 (1H, d, J = 9.7 Hz), 7.32 (1H, d, J = 7.9 Hz), 7.78 (1H, t, J = 7.9 Hz), 8.06 (1H, d, J = 7.9 Hz), 8.31 (1H, s).

### <Reference Example 10-1>

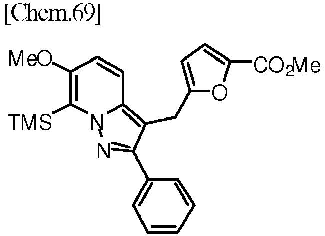

### Methyl 5-[[6-methoxy-2-phenyl-7-(trimethylsilyl)pyrazolo[1,5-a]pyridin-3-yl]methyl]furan-2-carbo xylate

Triethylsilane (0.1 mL) and trifluoroacetic acid (0.1 mL) were added to a dichloromethane solution (0.5 mL) of methyl 5-[hydroxy[6-methoxy-2-phenyl-7-(trimethylsilyl)pyrazolo[1,5-a]pyridin-3-yl]methyl]furan-2-carboxylate (48 mg) at room temperature under an argon atmosphere, and then the mixture was stirred at room temperature for 9 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 3:1) to obtain a title compound as yellow liquid (39 mg).
¹H-NMR (400 MHz, CDCl₃) δ 0.53 (9H, s), 3.82 (3H, s), 3.89 (3H, s), 4.28 (2H, s), 5.98 (1H, d, J = 3.0 Hz), 7.01 (1H, d, J = 9.7 Hz), 7.06 (1H, d, J = 3.0 Hz), 7.32-7.37 (2H, m), 7.42 (2H, t, J = 7.9 Hz), 7.72 (2H, d, J = 7.9 Hz).

### <Reference Example 10-2>

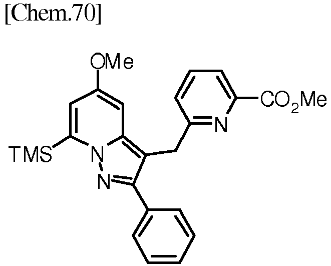

### Methyl 6-[[5-methoxy-2-phenyl-7-(trimethylsilyl)pyrazolo[1,5-a]pyridin-3-yl]methyl]pyridine-2-car boxylate

Triethylsilane (0.3 mL) and trifluoroacetic acid (0.3 mL) were added to a dichloromethane solution (1.7 mL) of methyl 6-[hydroxy[5-methoxy-2-phenyl-7-(trimethylsilyl)pyrazolo[1,5-a]pyridin-3-yl]methyl]pyridi ne-2-carboxylate (160 mg) at room temperature under an argon atmosphere, and then the mixture was stirred at room temperature for 1 hours. A saturated sodium bicarbonate aqueous solution was added to the reaction mixture, and then the mixture was extracted with ethyl acetate. The organic layer was washed with saturated saline and dried over anhydrous magnesium sulfate. The solvent was evaporated and then the residue was purified by silica gel column chromatography (*n*-hexane:ethyl acetate = 2:1) to obtain a title compound as yellow powder (130 mg).
¹H-NMR (400 MHz, CDCl₃) δ 0.48 (9H, s), 3.76 (3H, s), 4.04 (3H, s), 4.54 (2H, s), 6.53 (1H, d, J = 3.0 Hz), 6.58 (1H, d, J = 3.0 Hz), 7.18 (1H, d, J = 7.9 Hz), 7.31-7.36 (1H, m), 7.37-7.42 (2H, m), 7.65 (1H, t, J = 7.9 Hz), 7.73-7.76 (2H, m), 7.96 (1H, d, J = 7.9 Hz).

### <Reference Example 11-1>

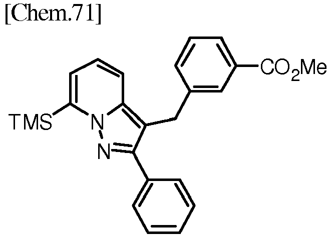

### Methyl 3-[[2-phenyl-7-(trimethylsilyl)pyrazolo[1,5-a]pyridin-3-yl]methyl]benzoate

*n*-Butyllithium (0.5 mL, a 2.6 mol/L *n*-hexane solution) was added to a tetrahydrofuran solution (3 mL) of 3-bromo-2-phenyl-7-(trimethylsilyl)pyrazolo[1,5-a]pyridine (400 mg) at -78°C under an argon atmosphere, and then the mixture was stirred at -78°C for 30 minutes. A tetrahydrofuran solution (3 mL) of zinc (II) iodide (389 mg) was added to the obtained mixture at -78°C, and then the mixture was stirred at room temperature for 30 minutes. Methyl 3-bromomethyl benzoate (291 mg) and tetrakistriphenylphosphinepalladium (0) (134 mg) were added to the obtained zinc compound, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as red liquid (197 mg).
¹H-NMR (400 MHz, CDCl₃) δ 0.63 (9H, s), 4.05 (3H, s), 4.61 (2H, s), 6.97 (1H, dd, J = 6.7, 1.2 Hz), 7.11 (1H, dd, J = 8.5, 6.7 Hz), 7.43-7.56 (4H, m), 7.85 (2H, d, J = 6.7 Hz), 7.92 (1H, t, J = 7.9 Hz), 7.99 (1H, dt, J = 6.7, 1.2 Hz), 8.03-8.08 (2H, m).

### <Reference Example 11-2>

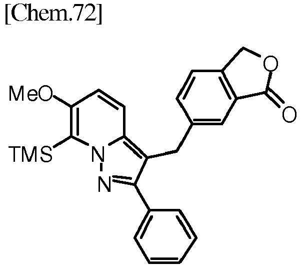

### 4-[[6-Methoxy-2-phenyl-7-(trimethylsilyl)pyrazolo[1,5-a]pyridin-3-yl]methyl]-1(3H)-isoben zofuranone

*n*-Butyllithium (0.3 mL, a 1.6 mol/L *n*-hexane solution) was added to a tetrahydrofuran solution (2.0 mL) of 3-bromo-6-methoxy-2-phenyl-7-(trimethylsilyl)pyrazolo[1,5-a]pyridine (200 mg) at -78°C under an argon atmosphere, and then the mixture was stirred at -78°C for 30 minutes. A tetrahydrofuran solution (0.7 mL) of zinc iodide (179 mg) was added to the obtained mixture at -78°C, and then the mixture was stirred at room temperature for 30 minutes. Then 6-(bromomethyl)-1(3*H*)-isobenzofuranone (134 mg) and tetrakistriphenylphosphinepalladium (0) (61 mg) were added, and then the mixture was stirred at room temperature for 6 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as a yellow amorphous (76 mg).
¹H-NMR (400 MHz, CDCl₃) δ 0.55 (9H, s), 3.81 (3H, s), 4.37 (2H, s), 5.28 (2H, s), 6.95 (1H, d, J = 9.7 Hz), 7.20 (1H, d, J = 9.7 Hz), 7.33 (1H, d, J = 7.3 Hz), 7.35-7.41 (3H, m), 7.52 (1H, dd, J = 7.9, 1.8 Hz), 7.66-7.69 (2H, m), 7.73 (1H, s).

### <Reference Example 11-3>

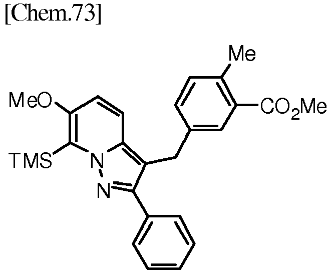

### Methyl 5-[[6-methoxy-2-phenyl-7-(trimethylsilyl)pyrazolo[1,5-a]pyridin-3-yl]methyl]-2-methylbenz oate

*n*-Butyllithium (0.2 mL, a 1.6 mol/L *n*-hexane solution) was added to a tetrahydrofuran solution (1.0 mL) of 3-bromo-6-methoxy-2-phenyl-7-(trimethylsilyl)pyrazolo[1,5-a]pyridine (100 mg) at -78°C under an argon atmosphere, and then the mixture was stirred at -78°C for 30 minutes. A tetrahydrofuran solution (0.4 mL) of zinc iodide (89 mg) was added to the obtained mixture at -78°C, and then the mixture was stirred at room temperature for 30 minutes. Then methyl 5-(bromomethyl)-2-methylbenzoate (71 mg) and tetrakistriphenylphosphinepalladium (0) (31 mg) were added, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 9:1) to obtain a title compound as yellow liquid (54 mg).
¹H-NMR (400 MHz, CDCl₃) δ 0.54 (9H, s), 2.54 (3H, s), 3.80 (3H, s), 3.86 (3H, s), 4.26 (2H,s),6.92(1H,d,J=9.7Hz),7.11(1H,d,J=7.9Hz),7.14(1H,d,J=1.8Hz),7.19(1H, d, J = 9.7 Hz), 7.33 (1H, d, J = 7.9 Hz), 7.37-7.42 (2H, m), 7.70-7.75 (2H, m), 7.78-7.81 (1H, m).

### <Reference Example 12-1>

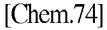

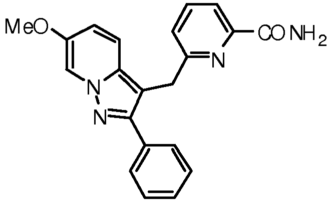

### 6-[(6-Methoxy-2-phenylpyrazolo[1,5-a]pyridin-3-yl)methyl]pyridine-2-carboxamide

Ammonium chloride (12 mg), o-(benzotriazol-1-yl)-*N,N,N',N'*-tetramethyl-uronium hexafluorophosphate (103 mg), and diisopropylethylamine (0.1 mL) were added to an *N,N-*dimethylformamide solution (1.1 mL) of 6-[(6-methoxy-2-phenylpyrazolo[1,5-a]pyridin-3-yl)methyl]pyridine-2-carboxylic acid (80 mg) at room temperature under an argon atmosphere, and then the mixture was stirred at room temperature for 6 hours. Water was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (ethyl acetate) to obtain a title compound as yellow liquid (75 mg).
¹H-NMR (400 MHz, CDCl₃) δ 3.85 (3H, s), 4.41 (2H, s), 6.91 (1H, dd, J = 9.7, 2.4 Hz), 7.21 (1H, d, J = 7.3 Hz), 7.27 (1H, d, J = 9.7 Hz), 7.35-7.40 (1H, m), 7.43 (2H, t, J = 7.3 Hz), 7.68-7.73 (4H, m), 8.10 (1H, d, J = 2.4 Hz).

### <Reference Example 13-1>

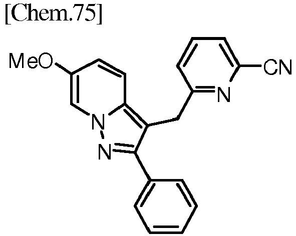

### 6-[(6-Methoxy-2-phenylpyrazolo[1,5-a]pyridin-3-yl)methyl]pyridine-2-carbonitrile

Thionyl chloride (0.03 mL) was added to an *N*,*N*-dimethylformamide solution (2.1 mL) of 6-[(6-methoxy-2-phenylpyrazolo[1,5-a]pyridin-3-yl)methyl]pyridine-2-carboxamide (75 mg) at room temperature under an argon atmosphere, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 1:1) to obtain a title compound as yellow liquid (70 mg).
¹H-NMR (400 MHz, CDCl₃) δ 3.85 (3H, s), 4.44 (2H, s), 6.93 (1H, dd, J = 9.7, 1.2 Hz), 7.16 (1H, d, J = 7.9 Hz), 7.31 (1H, d, J = 9.7 Hz), 7.39 (1H, d, J = 7.9 Hz), 7.44 (2H, t, J = 7.9 Hz), 7.51 (1H, d, J = 7.9 Hz), 7.61 (1H, d, J = 7.9 Hz), 7.63-7.68 (2H, m), 8.10 (1H, d, J =1.8 Hz).

### <Reference Example 14-1>

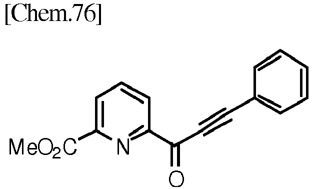

### Methyl 6-(1-oxo-3-phenyl-2-propyn-1-yl)pyridine-2-carboxylate

After thionyl chloride (20.0 mL) was added to a toluene (100 mL) solution of 2-carboxy-6-methoxycarbonylpyridine (5.00 g) at room temperature, the mixture was heated and stirred at 60°C for 3 hours. Dichlorobis(triphenylphosphine)palladium (II) (195 mg), copper (I) iodide (159 mg), phenyl acetylene (3.64 mL), and tetrahydrofuran (270 mL) were added to a colorless crystal obtained by evaporating the solvent, then triethylamine (4.80 mL) was added, and then the mixture was stirred at room temperature for 3 hours under an argon atmosphere. The solvent was evaporated and then the residue was purified by silica gel column chromatography (*n*-hexane:ethyl acetate = 3:1) to obtain a title compound as a green crystal (5.03 g).
¹H-NMR (400 MHz, CDCl₃) δ 4.08 (3H, s), 7.44 (2H, t, J = 7.9 Hz), 7.51 (1H, t, J = 7.9 Hz), 7.79(1H,d,J=7.9Hz),7.80(1H,d,J=7.9Hz),8.06(1H,t,J=7.9Hz),8.34(1H,d,J=7.9 Hz),8.35(1H,d,J=7.9Hz).

### <Reference Example 14-2>

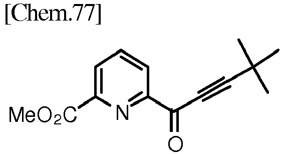

### Methyl 6-(4,4-dimethyl-1-oxo-2-pentyn-1-yl)pyridine-2-carboxylate

After thionyl chloride (7.50 mL) was added to a toluene (38 mL) solution of 2-carboxy-6-methoxycarbonylpyridine (1.82 g) at room temperature, the mixture was heated and stirred at 60°C for 3 hours. Dichlorobis(triphenylphosphine)palladium (II) (70.2 mg), copper (I) iodide (58.0 mg), 3,3-dimethyl-1-butyne (1.50 mL), and tetrahydrofuran (100 mL) were added to a colorless crystal obtained by evaporating the solvent, then triethylamine (1.70 mL) was added, and then the mixture was stirred at room temperature for 3 hours under an argon atmosphere. The solvent was evaporated and then the residue thus obtained was purified by silica gel column chromatography (*n*-hexane:ethyl acetate = 3:1) to obtain a title compound as a brown oil (1.51 g).
¹H-NMR (400 MHz, CDCl₃) δ 1.42 (9H, s), 4.03 (3H, s), 8.01 (1H, t, J = 7.9 Hz), 8.27 (1H, d, J = 7.9 Hz), 8.30 (1H, dd, J = 7.9 and 1.2 Hz).

### <Reference Example 14-3>

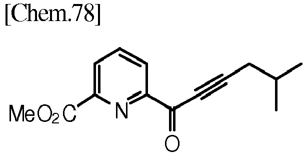

### Methyl 6-(5-methyl-1-oxo-2-hexyn-1-yl)pyridine-2-carboxylate

After thionyl chloride (7.50 mL) was added to a toluene (38 mL) solution of 2-carboxy-6-methoxycarbonylpyridine (1.82 g) at room temperature, the mixture was heated and stirred at 60°C for 3 hours. Dichlorobis(triphenylphosphine)palladium (II) (70.2 mg), copper (I) iodide (58.0 mg), 4,4-dimethyl-1-pentyne (1.45 mL), and tetrahydrofuran (100 mL) were added to a colorless crystal obtained by evaporating the solvent, then triethylamine (1.70 mL) was added, and then the mixture was stirred at room temperature for 3 hours under an argon atmosphere. The solvent was evaporated and then the residue was purified by silica gel column chromatography (*n*-hexane:ethyl acetate = 3:1) to obtain a title compound as a brown oil (1.53 g).
¹H-NMR (400 MHz, CDCl₃) δ 1.14 (6H, d, J = 6.6 Hz), 2.00-2.12 (1H, m), 2.47 (2H, d, J = 6.6 Hz), 4.02 (3H, s), 8.02 (1H, t, J = 7.9 Hz), 8.28 (1H, d, J = 7.9 Hz), 8.32 (1H, dd, J = 7.9 and 1.2 Hz).

### <Reference Example 14-4>

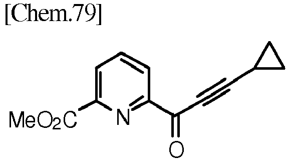

### Methyl 6-(3-cyclopropyl-1-oxo-2-propyn-1-yl)pyridine-2-carboxylate

After thionyl chloride (8.80 mL) was added to a toluene (45 mL) solution of 2-carboxy-6-methoxycarbonylpyridine (2.18 g) at room temperature, the mixture was heated and stirred at 60°C for 3 hours. Dichlorobis(triphenylphosphine)palladium (II) (85.0 mg), copper (I) iodide (69.0 mg), cyclopropyl acetylene (1.22 mL), and tetrahydrofuran (120 mL) were added to a colorless crystal obtained by evaporating the solvent, then triethylamine (2.00 mL) was added, and then the mixture was stirred at room temperature for 3 hours under an argon atmosphere. The solvent was evaporated and then the residue was purified by silica gel column chromatography (*n*-hexane:ethyl acetate = 3:1) to obtain a title compound as a dark green crystal (1.99 g).
¹H-NMR (400 MHz, CDCl₃) δ 1.05- 1.16 (4H, m),1.57-1.66 (1H, m), 4.04 (3H, s), 8.01 (1H, t, J = 7.9 Hz), 8.26 (1H, dd, J = 7.9 and 1.8Hz), 8.31 (1H, dd, J = 7.9 and 1.8 Hz).

### <Reference Example 14-5>

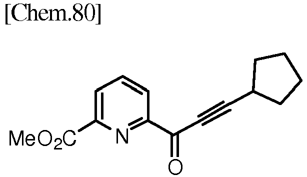

### Methyl 6-(3-cyclopentyl-1-oxo-2-propyn-1-yl)pyridine-2-carboxylate

After thionyl chloride (8.80 mL) was added to a toluene (45 mL) solution of 2-carboxy-6-methoxycarbonylpyridine (2.18 g) at room temperature, the mixture was heated and stirred at 60°C for 3 hours. Dichlorobis(triphenylphosphine)palladium (II) (85.0 mg), copper (I) iodide (69.0 mg), cyclopentyl acetylene (1.67 mL), and tetrahydrofuran (120 mL) were added to a colorless crystal obtained by evaporating the solvent, then triethylamine (2.00 mL) was added, and then the mixture was stirred at room temperature for 3 hours under an argon atmosphere. The solvent was evaporated and then the residue was purified by silica gel column chromatography (*n*-hexane:ethyl acetate = 3:1) to obtain a title compound as a dark brown crystal (2.17 g).
¹H-NMR (400 MHz, CDCl₃) δ 1.60-1.73 (2H, m), 1.79-1.95 (4H, m), 2.00-2.13 (2H, m), 2.94-3.03 (1H, m), 4.03 (3H, s), 8.01 (1H, t, J = 7.9 Hz), 8.27 (1H, dd, J = 7.9 and 1.2Hz), 8.31 (1H, dd, J = 7.9 and 1.2 Hz).

### <Reference Example 14-6>

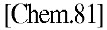

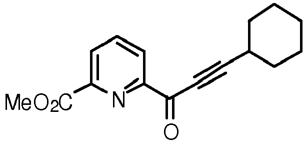

### Methyl 6-(3-cyclohexyl-1-oxo-2-propyn-1-yl)pyridine-2-carboxylate

After thionyl chloride (8.80 mL) was added to a toluene (45 mL) solution of 2-carboxy-6-methoxycarbonylpyridine (2.18 g) at room temperature, the mixture was heated and stirred at 60°C for 3 hours. Dichlorobis(triphenylphosphine)palladium (II) (85.0 mg), copper (I) iodide (69.0 mg), cyclohexylacetylene (1.89 mL), and tetrahydrofuran (120 mL) were added to a colorless crystal obtained by evaporating the solvent, then triethylamine (2.00 mL) was added, and then the mixture was stirred at room temperature for 3 hours under an argon atmosphere. The solvent was evaporated and then the residue was purified by silica gel column chromatography (*n*-hexane:ethyl acetate = 3:1) to obtain a title compound as a brown crystal (2.30 g).
¹H-NMR (400 MHz, CDCl₃) δ 1.35-1.60 (4H, m), 1.65-1.75 (2H, m), 1.78-1.88 (2H, m), 1.90-2.00 (2H, m), 2.73-2.83 (1H, m), 4.03 (3H, s), 8.01 (1H, t, J = 7.9 Hz), 8.28 (1H, dd, J = 7.9 and 1.2Hz), 8.31 (1H, dd, J = 7.9 and 1.2 Hz).

### <Reference Example 14-7>

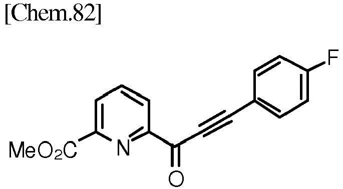

### Methyl 6-[3-(4-fluorophenyl)-1-oxo-2-propyn-1-yl]pyridine-2-carboxylate

After thionyl chloride (10.1 mL) was added to a toluene (47.0 mL) suspension of 2-carboxy-6-methoxycarbonylpyridine (2.54 g) under an argon atmosphere, the mixture was heated and stirred at 60°C for 3 hours. The solvent was evaporated under vacuum, then tetrahydrofuran (140 mL), 4-fluorophenylacetylene (2.02 g), copper (I) iodide (80.0 mg), dichlorobis(triphenylphosphine)palladium (II) (98.3 mg), and triethylamine (2.44 mL) were added, and then the mixture was stirred at room temperature for 2 hours. The reaction solution was concentrated and then purified by silica gel column chromatography (*n*-hexane:ethyl acetate = 4:1 to 1:1), and then the obtained crude product was triturated with diisopropyl ether to obtain a title compound as a white solid (960 mg).
¹H-NMR (400 MHz, CDCl₃) δ 4.07 (3H, s), 7.14 (2H, tt, J = 9.1,2.4 Hz), 7.77-7.83 (2H, m), 8.06 (1H, t, J = 7.9 Hz), 8.34 (2H, td, J = 7.9, 1.2 Hz).

### <Reference Example 14-8>

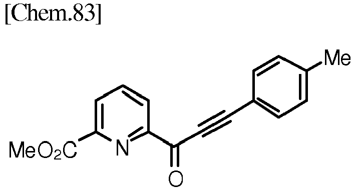

### Methyl 6-[3-(4-methylphenyl)-1-oxo-2-propyn-1-yl]pyridine-2-carboxylate

Thionyl chloride (10.1 mL) was added to a toluene (47.0 mL) suspension of 2-carboxy-6-methoxycarbonylpyridine (2.54 g) under an argon atmosphere, and then the mixture was heated and stirred at 60°C for 3 hours. The solvent was evaporated under vacuum, then tetrahydrofuran (140 mL), 4-methylphenylacetylene (1.95 g), copper (I) iodide (80.0 mg), dichlorobis(triphenylphosphine)palladium (II) (98.3 mg), and triethylamine (2.44 mL) were added, and then the mixture was stirred at room temperature for 2 hours. The reaction mixture was concentrated and purified by silica gel column chromatography (*n*-hexane:ethyl acetate = 4:1 to 1:1), and then the obtained crude product was triturated with diisopropyl ether to obtain a title compound as a gray solid (960 mg).
¹H-NMR (400 MHz, CDCl₃) δ 2.42 (3H, s), 4.08 (3H, s), 7.24 (1H, s), 7.69 (2H, d, J = 7.9 Hz), 8.05 (1H, t, J = 7.9 Hz), 8.34 (2H, d, J = 7.9 Hz).

### <Reference Example 14-9>

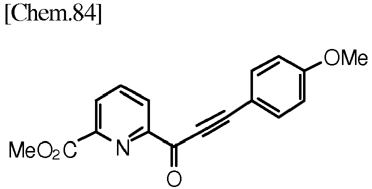

### Methyl 6-[3-(4-methoxyphenyl)-1-oxo-2-propyn-1-yl]pyridine-2-carboxylate

Thionyl chloride (10.1 mL) was added to a toluene (47.0 mL) suspension of 2-carboxy-6-methoxycarbonylpyridine (2.54 g) under an argon atmosphere, and then the mixture was heated and stirred at 60°C for 3 hours. The solvent was evaporated under vacuum, then tetrahydrofuran (140 mL), 4-methoxyphenylacetylene (1.95 g), copper (I) iodide (80.0 mg), dichlorobis(triphenylphosphine)palladium (II) (98.3 mg), and triethylamine (2.44 mL) were added, and then the mixture was stirred at room temperature for 2 hours. The reaction mixture was concentrated and purified by silica gel column chromatography (*n*-hexane:ethyl acetate = 4:1 to 1:1), and then the obtained crude product was triturated with diisopropyl ether to obtain a title compound as a gray solid (608 mg).
¹H-NMR (400 MHz, CDCl₃) δ 3.87 (3H, s), 4.08 (3H, s), 6.95 (2H, d, J = 9.1 Hz), 7.75 (2H, d, J = 9.1 Hz), 8.05 (1H, t, J = 7.9 Hz), 8.34 (2H, d, J = 7.9 Hz).

### <Reference Example 14-10>

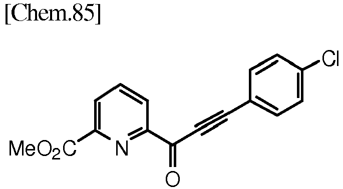

### Methyl 6-[3-(4-chlorophenyl)-1-oxo-2-propyn-1-yl]pyridine-2-carboxylate

Thionyl chloride (8.78 mL) was added to a toluene (40.9 mL) suspension of 2-carboxy-6-methoxycarbonylpyridine (2.21 g) under an argon atmosphere, and then the mixture was heated and stirred at 60°C for 3 hours. The solvent was evaporated under vacuum, then tetrahydrofuran (122 mL), 4-chlorophenylacetylene (2.00 g), copper (I) iodide (69.6 mg), dichlorobis(triphenylphosphine)palladium (II) (85.5 mg), and triethylamine (2.12 mL) were added, and then the mixture was stirred at room temperature for 2 hours. The reaction mixture was concentrated and purified by silica gel column chromatography (*n*-hexane:ethyl acetate = 4:1 to 1:1) to obtain a title compound as a gray solid (1.08 mg).
¹H-NMR (400 MHz, CDCl₃) δ 4.07 (3H, s), 7.42 (2H, dt, J = 8.7, 1.8 Hz), 7.72 (2H, dt, J = 8.7,1.8 Hz), 8.06 (1H, t, J = 7.9 Hz), 8.32-8.36 (2H, m).

### <Reference Example 14-11>

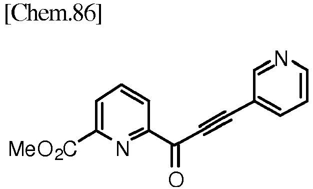

### Methyl 6-[3-(pyridin-3-yl)-1-oxo-2-propyn-1-yl]pyridine-2-carboxylate

After thionyl chloride (10.1 mL) was added to a toluene (47.0 mL) suspension of 2-carboxy-6-methoxycarbonylpyridine (2.54 g) under an argon atmosphere, the mixture was heated and stirred at 60°C for 3 hours. The solvent was evaporated under vacuum, then tetrahydrofuran (140 mL), 3-pyridylacetylene (1.73 g), copper (I) iodide (80.0 mg), dichlorobis(triphenylphosphine)palladium (II) (98.3 mg), and triethylamine (2.44 mL) were added, and then the mixture was stirred at room temperature for 2 hours. The reaction solution was concentrated and then purified by silica gel column chromatography (*n*-hexane:ethyl acetate = 4:1 to 1:1) to obtain a title compound as a reddish brown solid (207 mg).
¹H-NMR (400 MHz, DMSO-d₆) δ 3.97 (3H, s), 7.60 (1H, dd, J = 7.9,4.8 Hz), 8.20 (1H, dt, J = 7.9,1.8 Hz), 8.27 (1H, t, J = 7.9 Hz), 8.34-8.38 (2H, m), 8.77 (1H, dd, J = 4.8,1.8 Hz), 8.98 (1H,d,J=1.2Hz).

### <Reference Example 14-12>

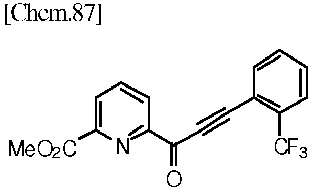

### Methyl 6-[1-oxo-3-[2-(trifluoromethyl)phenyl]-2-propyn-1-yl]pyridine-2-carboxylate

Thionyl chloride (8 mL) was added to a toluene (37 mL) solution of 2-carboxy-6-methoxycarbonylpyridine (2.0 g) at room temperature under an argon atmosphere, and then the mixture was stirred at 60°C for 3 hours. A colorless crystal obtained by evaporating the reaction solution was dissolved in tetrahydrofuran (37 mL), then 2-(trifluoromethyl)phenylacetylene (1.8 mL), copper (I) iodide (64 mg), dichlorobis(triphenylphosphine)palladium (II) (78 mg), and triethylamine (1.9 mL) were added at room temperature, and then the mixture was stirred at room temperature for 3 hours. The reaction solution was evaporated and then the residue was purified by silica gel column chromatography (*n*-hexane:ethyl acetate = 4:1) to obtain a title compound as brown liquid (1.29 g).
¹H-NMR (400 MHz, CDCl₃) δ 4.06 (3H, s), 7.58-7.66 (2H, m), 7.77 (1H, dd, J = 6.7, 1.8 Hz), 7.97 (1H, dd, J = 6.7, 1.8 Hz), 8.07 (1H, t, J = 7.9 Hz), 8.36 (2H, d, J = 7.9 Hz).

### <Reference Example 14-13>

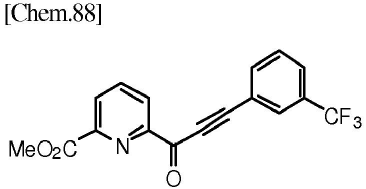

### Methyl 6-[1-oxo-3-[3-(trifluoromethyl)phenyl]-2-propyn-1-yl]pyridine-2-carboxylate

Thionyl chloride (8 mL) was added to a toluene (37 mL) solution of 2-carboxy-6-methoxycarbonylpyridine (2.0 g) at room temperature under an argon atmosphere, and then the mixture was stirred at 60°C for 3 hours. A colorless crystal obtained by evaporating the reaction mixture was dissolved in tetrahydrofuran (37 mL), then 3-(trifluoromethyl)phenylacetylene (1.9 mL), copper (I) iodide (64 mg), dichlorobis(triphenylphosphine)palladium (II) (78 mg), and triethylamine (1.9 mL) were added at room temperature, and then the mixture was stirred at room temperature for 2 hours. The reaction mixture was evaporated and then the residue was purified by silica gel column chromatography (*n*-hexane:ethyl acetate = 4:1) to obtain a title compound as brown liquid (302 mg).
¹H-NMR (400 MHz, CDCl₃) δ4.08 (3H, s), 7.59 (1H, t, J = 7.9 Hz), 7.76 (1H, d, J = 7.9 Hz), 7.95 (1H, d, J = 7.9 Hz), 8.05-8.10 (2H, m), 8.33 (1H, dd, J = 7.9,1.2 Hz), 8.38 (1H, dd, J = 7.9, 1.2 Hz).

### <Reference Example 14-14>

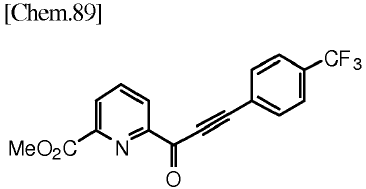

### Methyl 6-[1-oxo-3-[4-(trifluoromethyl)phenyl]-2-propyn-1-yl]pyridine-2-carboxylate

Thionyl chloride (4 mL) was added to a toluene (18 mL) solution of 2-carboxy-6-methoxycarbonylpyridine (1.0 g) at room temperature under an argon atmosphere, and then the mixture was stirred at 60°C for 3 hours. A colorless crystal obtained by evaporating the reaction mixture was dissolved in tetrahydrofuran (55 mL), then 4-(trifluoromethyl)phenylacetylene (1.0 mL), copper (I) iodide (32 mg), dichlorobis(triphenylphosphine)palladium (II) (39 mg), and triethylamine (1.0 mL) were added at room temperature, and then the mixture was stirred at room temperature for 3 hours. The reaction mixture was evaporated and then the residue was purified by silica gel column chromatography (*n*-hexane:ethyl acetate = 4:1) to obtain a title compound as brown liquid (314 mg).
¹H-NMR (400 MHz, CDCl₃) δ 4.07 (3H, s), 7.71 (2H, d, J = 7.9 Hz), 7.89 (2H, d, J = 7.9 Hz), 8.07 (1H, t, J = 7.9 Hz), 8.33 (1H, d, J = 7.9 Hz), 8.37 (1H, dd, J = 7.9, 1.2 Hz).

### <Reference Example 14-15>

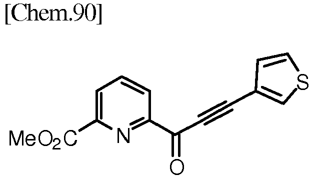

### Methyl 6-[1-oxo-3-(thiophen-3-yl)-2-propyn-1-yl]pyridine-2-carboxylate

Thionyl chloride (8 mL) was added to a toluene (37 mL) solution of 2-carboxy-6-methoxycarbonylpyridine (2.0 g) at room temperature under an argon atmosphere, and then the mixture was stirred at 60°C for 3 hours. A colorless crystal obtained by evaporating the reaction mixture was dissolved in tetrahydrofuran (37 mL), then (thiophen-3-yl)acetylene (1.3 mL), copper (I) iodide (64 mg), dichlorobis(triphenylphosphine)palladium (II) (78 mg), and triethylamine (1.9 mL) were added at room temperature, and then the mixture was stirred at room temperature for 4 hours. The reaction mixture was evaporated and then the residue was purified by silica gel column chromatography (*n*-hexane:ethyl acetate = 4:1) to obtain a title compound as brown powder (1.55 g).
¹H-NMR (400 MHz, CDCl₃) δ 4.07 (3H, s), 7.36-7.42 (2H, m), 7.97 (1H, dd, J = 3.0, 1.2 Hz), 8.05 (1H, t, J = 7.9 Hz), 8.32-8.36 (2H, m).

### <Reference Example 14-16>

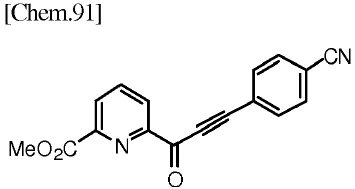

### Methyl 6-[3-(4-cyanophenyl)-1-oxo-2-propyn-1-yl]pyridine-2-carboxylate

Thionyl chloride (8.8 mL) was added to a toluene (45 mL) solution of 2-carboxy-6-methoxycarbonylpyridine (2.18 g) at room temperature, and then the mixture was heated and stirred at 60°C for 3 hours. Dichlorobis(triphenylphosphine)palladium (II) (85.0 mg), copper (I) iodide (69.0 mg), 4-cyanophenylacetylene (1.83 g), and tetrahydrofuran (120 mL) were added to the colorless crystal obtained by evaporating the solvent, then triethylamine (2.00 mL) was added, and then the mixture was stirred at room temperature for 3 hours under an argon atmosphere. The solvent was evaporated and then the residue was purified by silica gel column chromatography (*n*-hexane:ethyl acetate = 3:1) to obtain a title compound as a pale yellow crystal (273 mg).
¹H-NMR (400 MHz, CDCl₃) δ 4.07 (3H, s), 7.73 (2H, d, J = 8.5 Hz), 7.87 (1H, d, J = 8.5 Hz), 8.07 (1H, t, J = 7.9 Hz), 8.32 (1H, dd, J = 7.9 and 1.2 Hz), 8.36 (1H, dd, J = 7.9 and 1.2 Hz).

### <Reference Example 14-17>

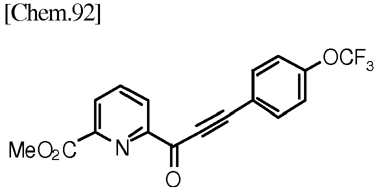

### Methyl 6-[1-oxo-3-[4-(trifluoromethoxy)phenyl]-2-propyn-1-yl]pyridine-2-carboxylate

After thionyl chloride (8.8 mL) was added to a toluene (45 mL) solution of 2-carboxy-6-methoxycarbonylpyridine (2.18 g) at room temperature, the mixture was heated and stirred at 60°C for 3 hours. Dichlorobis(triphenylphosphine)palladium (II) (85.0 mg), copper (I) iodide (69.0 mg), 4-(triffuoromethoxy)phenylacetylene (2.68 g), and tetrahydrofuran (120 mL) were added to the colorless crystal obtained by evaporating the solvent, then triethylamine (2.00 mL) was added, and then the mixture was stirred at room temperature for 3 hours under an argon atmosphere. The solvent was evaporated and then the residue thus obtained was purified by silica gel column chromatography (*n*-hexane:ethyl acetate = 3:1) to obtain a title compound as a violet crystal (1.92 g).
¹H-NMR (400 MHz, CDCl₃) δ 4.07 (3H, s), 7.28 (2H, d, J = 8.4 Hz), 7.83 (1H, d, J = 8.4 Hz), 8.06 (1H, t, J = 7.9 Hz), 8.33 (1H, d, J = 7.91.2 Hz), 8.36 (1H, d, J = 7.9 Hz).

### <Reference Example 15-1>

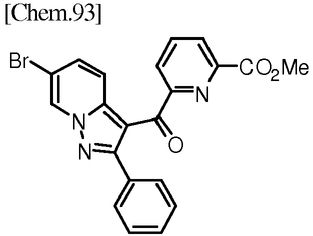

### Methyl 6-[(6-bromo-2-phenylpyrazolo[1,5-a]pyridin-3-yl)carbonyl]pyridine-2-carboxylate

After potassium carbonate (6.25 g) was added to an *N*,*N-*dimethylformamide (22.6 mL) solution of methyl 6-(1-oxo-3-phenyl-2-propyn-1-yl)pyridine-2-carboxylate (3.00 g) and 1-amino-3-bromopyridinium 2,4,6-trimethylbenzenesulfonate (7.71 g) at room temperature, the mixture was stirred at room temperature for 2 hours. The reaction solution was diluted with ethyl acetate, washed with water and saturated saline, and then dried over anhydrous sodium sulfate. The solvent was evaporated and then the residue thus obtained was purified by silica gel column chromatography (*n*-hexane:ethyl acetate = 3:1) to obtain a title compound as a yellow crystal (1.35 g).
¹H-NMR (400 MHz, CDCl₃) δ 3.85 (3H, s), 7.05 (2H, t, J = 7.9 Hz), 7.13 (1H, t, J = 7.9 Hz), 7.23 (2H, d, J = 7.9 Hz), 7.59 (1H, dd, J = 9.1 and 1.8 Hz), 7.86 (1H, t, J = 7.9 Hz), 7.94 (1H, d, J = 7.9 Hz), 8.00 (1H, dd, J = 7.9 and 1.2 Hz), 8.28 (1H, d, J = 9.1 Hz), 8.73 (1H, d, J =1.8 Hz).

### <Reference Example 15-2>

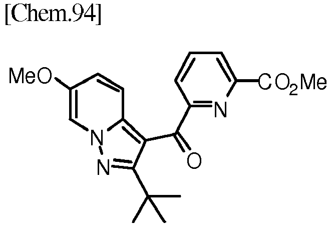

### Methyl 6-[(2-tert-butyl-6-methoxypyrazolo[1,5-a]pyridin-3-yl)carbonyl]pyridine-2-carboxylate

Potassium carbonate (4.06 g) was added to a 1,4-dioxane (15 mL) solution of methyl 6-(4,4-dimethyl-1-oxo-2-pentyn-1-yl)pyridine-2-carboxylate (1.79 g) and 1-amino-3-methoxypyridinium 2,4,6-trimethylbenzenesulfonate (4.75 g) at room temperature, and then the mixture was stirred at room temperature for 2 hours. The reaction mixture was diluted with ethyl acetate, washed with water and saturated saline, and dried over anhydrous sodium sulfate. The solvent was evaporated and then the residue was purified by silica gel column chromatography (*n*-hexane:ethyl acetate = 1:1) to obtain a title compound as a yellow crystal (295 mg).
¹H-NMR (400 MHz, CDCl₃) δ 1.55 (9H, s), 3.83 (3H, s), 3.93 (3H, s), 6.58 (1H, d, J = 9.7 Hz), 6.86 (1H, dd, J = 9.7 and 1.9 Hz), 8.00-8.07 (3H, m), 8.25-8.32 (1H, m).

### <Reference Example 15-3>

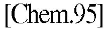

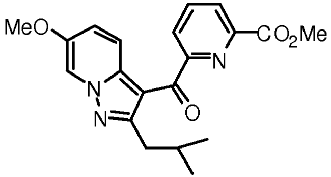

### Methyl 6-[(2-isobutyl-6-methoxypyrazolo[1,5-a]pyridin-3-yl)carbonyl]pyridine-2-carboxylate

Potassium carbonate (3.43 g) was added to a 1,4-dioxane (13 mL) solution of methyl 6-(5-methyl-1-oxo-2-hexyn-1-yl)pyridine-2-carboxylate (1.52 g) and 1-amino-3-methoxypyridinium 2,4,6-trimethylbenzenesulfonate (4.02 g) at room temperature, and then the mixture was stirred at room temperature for 2 hours. The reaction mixture was diluted with ethyl acetate, washed with water and saturated saline, and dried over anhydrous sodium sulfate. The solvent was evaporated and then the residue was purified by silica gel column chromatography (n-hexane:ethyl acetate = 1:1) to obtain a title compound as a yellow crystal (302 mg).
¹H-NMR (400 MHz, CDCl₃) δ 0.81 (6H, d, J = 6.7 Hz), 1.80-1.93 (1H, m), 2.75 (2H, d, J = 7.3 Hz), 3.87 (3H, s), 3.98 (3H, s), 7.14 (1H, dd, J = 9.7 and 2.4 Hz), 7.84 (1H, d, J = 9.7 Hz), 8.03-8.13 (3H, m), 8.30 (1H, dd, J = 7.3 and 2.4 Hz),.

### <Reference Example 15-4>

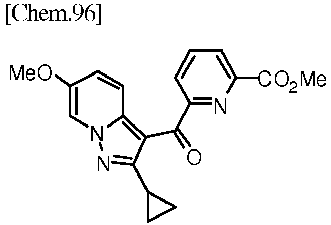

### Methyl 6-[(2-cyclopropyl-6-methoxypyrazolo[1,5-a]pyridin-3-yl)carbonyl]pyridine-2-carboxylate

Potassium carbonate (3.67 g) was added to a 1,4-dioxane (13 mL) solution of methyl 6-(3-cyclopropyl-1-oxo-2-propyn-1-yl)pyridine-2-carboxylate (1.52 g) and 1-amino-3-methoxypyridinium 2,4,6-trimethylbenzenesulfonate (4.30 g) at room temperature, and then the mixture was stirred at room temperature for 2 hours. The reaction mixture was diluted with ethyl acetate, washed with water and saturated saline, and dried over anhydrous sodium sulfate. The solvent was evaporated and then the residue was purified by silica gel column chromatography (*n*-hexane:ethyl acetate = 1:1) to obtain a title compound as a yellow crystal (370 mg).
¹H-NMR (400 MHz, CDCl₃) δ 0.75-0.82 (2H, m), 0.98-1.03 (2H, m), 1.98-2.07 (1H, m), 3.85 (3H, s), 3.98 (3H, s), 7.14 (1H, dd, J = 9.7 and 2.4 Hz), 7.82 (1H, d, J = 9.7 Hz), 8.01 (1H, d, J = 1.8 Hz), 8.03-8.10 (2H, m), 8.29 (1H, dd, J = 7.3 and 1.8 Hz),.

### <Reference Example 15-5>

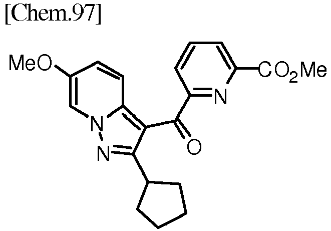

### Methyl 6-[(2-cyclopentyl-6-methoxypyrazolo[1,5-a]pyridin-3-yl)carbonyl]pyridine-2-carboxylate

Potassium carbonate (3.59 g) was added to a 1,4-dioxane (13 mL) solution of methyl 6-(3-cyclopentyl-1-oxo-2-propyn-1-yl)pyridine-2-carboxylate (1.67 g, 6.49 mmol) and 1-amino-3-methoxypyridinium 2,4,6-trimethylbenzenesulfonate (4.21 g) at room temperature, and then the mixture was stirred at room temperature for 2 hours. The reaction mixture was diluted with ethyl acetate, washed with water and saturated saline, and dried over anhydrous sodium sulfate. The solvent was evaporated and then the residue was purified by silica gel column chromatography (n-hexane:ethyl acetate = 1:1) to obtain a title compound as a yellow crystal (295 mg).
¹H-NMR (400 MHz, CDCl₃) δ 1.50-1.60 (2H, m), 1.70-1.92 (4H, m), 1.92-2.03 (2H, m), 3.40-3.50 (1H, m), 3.85 (3H, s), 3.97 (3H, s), 7.10 (1H, dd, J = 9.7 and 2.4 Hz), 7.67 (1H, d, J = 9.7 Hz), 8.04-8.13 (3H, m), 8.30 (1H, dd, J = 7.9 and 1.8 Hz),.

### <Reference Example 15-6>

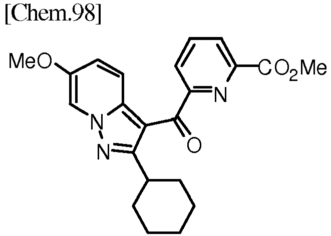

### Methyl 6-[(2-cyclohexyl-6-methoxypyrazolo[1,5-a]pyridin-3-yl)carbonyl]pyridine-2-carboxylate

Potassium carbonate (3.67 g) was added to a 1,4-dioxane (13 mL) solution of methyl 6-(3-cyclohexyl-1-oxo-2-propyn-1-yl)pyridine-2-carboxylate (1.80 g, 6.63 mmol) and 1-amino-3-methoxypyridinium 2,4,6-trimethylbenzenesulfonate (4.30 g) at room temperature, and then the mixture was stirred at room temperature for 2 hours. The reaction mixture was diluted with ethyl acetate, washed with water and saturated saline, and dried over anhydrous sodium sulfate. The solvent was evaporated and then the residue was purified by silica gel column chromatography (*n*-hexane:ethyl acetate = 1:1) to obtain a title compound as a yellow crystal (319 mg).
¹H-NMR (400 MHz, CDCl₃) δ 1.01-1.15 (2H, m), 1.18-1.34 (2H, m), 1.53-1.69 (2H, m), 1.72-1.80 (2H, m), 1.92-2.05 (2H, m), 2.75-2.85 (1H, m), 3.86 (3H, s), 3.97 (3H, s), 7.13 (1H, dd, J = 9.7 and 2.4 Hz), 7.77 (1H, d, J = 9.7 Hz), 8.07 (1H, d, J = 1.8 Hz), 8.08 (1H, s), 8.11 (1H, d, J = 2.4 Hz), 8.28-8.35 (1H, m).

### <Reference Example 15-7>

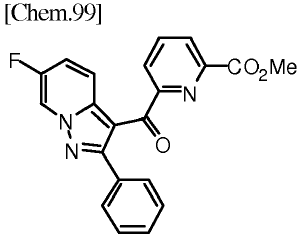

### Methyl 6-[(6-fluoro-2-phenylpyrazolo[1,5-a]pyridin-3-yl)carbonyl]pyridine-2-carboxylate

A 70% perchloric acid aqueous solution (1.2 mL) was added to a 1,4-dioxane solution (3 mL) of ethyl *O*-mesitylsulfonylacetohydroxamate (3.4 g) under an argon atmosphere under ice-cooling, and then the mixture was stirred for 30 minutes under ice-cooling. Ice water (35 mL) was added to the reaction mixture, the precipitated solid was filtered off, the solid thus obtained was dissolved in dichloromethane (10 mL), and the solution was divided into layers. The organic layer was dried over anhydrous magnesium sulfate and filtered off. A dichloromethane solution (10 mL) of 3-fluoropyridine (0.9 mL) was added to the obtained filtrate under ice-cooling, and the mixture was stirred at normal temperature for 1 hour. The residue obtained by evaporating the reaction mixture was dissolved in an *N*,*N-*dimethylformamide solution (10 mL), then potassium carbonate (2.8 g) and methyl 6-(1-oxo-3-phenyl-2-propyn-1-yl)pyridine-2-carboxylate (1.3 g) were added at normal temperature, and then the mixture was stirred at normal temperature for 1 hour. Water was added to the reaction mixture, and then the reaction mixture was extracted with ethyl acetate. The organic layer was washed with water and saturated saline in this order and then dried over anhydrous magnesium sulfate. The solvent was evaporated and then the residue was purified by silica gel column chromatography (*n*-hexane:ethyl acetate = 2:1) to obtain a title compound as yellow amorphous (329 mg).
¹H-NMR (400 MHz, CDCl₃) δ 3.85 (3H, s), 7.04 (2H, tt, J = 7.3, 1.2 Hz), 7.12 (1H, tt, J = 7.3, 1.2 Hz), 7.21-7.24 (2H, m), 7.46 (1H, ddd, J = 9.7, 7.9, 2.4 Hz), 7.86 (1H, t, J = 7.9 Hz), 7.94 (1H, dd, J = 7.9, 1.2 Hz), 8.00 (1H, dd, J = 7.9, 1.2 Hz), 8.40 (1H, dd, J = 9.7, 5.4 Hz), 8.53-8.55 (1H, m).

### <Reference Example 15-8>

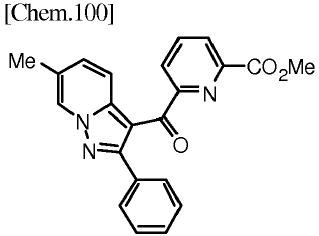

### Methyl 6-[(6-methyl-2-phenylpyrazolo[1,5-a]pyridin-3-yl)carbonyl]pyridine-2-carboxylate

A 70% perchloric acid aqueous solution (0.6 mL) was added to a 1,4-dioxane solution (1.5 mL) of ethyl *O*-mesitylsulfonylacetohydroxamate (1.7 g) under an argon atmosphere under ice-cooling, and then the mixture was stirred for 30 minutes under ice-cooling. Ice water (17.5 mL) was added to the reaction mixture, the precipitated solid was filtered off, the solid thus obtained was dissolved in dichloromethane (5 mL), and the solution was divided into layers. The organic layer was dried over anhydrous magnesium sulfate and filtered off. A dichloromethane solution (5 mL) of 3-methylpyridine (0.5 mL) was added to the obtained filtrate under ice-cooling, and the mixture was stirred at normal temperature for 1 hour. The residue obtained by evaporating the reaction mixture was dissolved in an *N*,*N-*dimethylformamide-tetrahydrofuran mixed solution (5 mL, 1:1), then potassium carbonate (1.4 g) and methyl 6-(1-oxo-3-phenyl-2-propyn-1-yl)pyridine-2-carboxylate (663 mg) were added at normal temperature, and then the mixture was stirred at normal temperature for 1 hour. Water was added to the reaction mixture, and then the reaction mixture was extracted with ethyl acetate. The organic layer was washed with water and saturated saline in this order and then dried over anhydrous magnesium sulfate. The solvent was evaporated and then the residue was purified by silica gel column chromatography (*n*-hexane:ethyl acetate = 2:1) to obtain a title compound as a yellow amorphous (299 mg).
¹H-NMR (400 MHz, CDCl₃) δ 2.45 (3H, s), 3.86 (3H, s), 7.03 (2H, t, J = 7.3 Hz), 7.07-7.13 (1H, m), 7.20-7.24 (2H, m), 7.40 (1H, dd, J = 9.1, 1.2 Hz), 7.83 (1H, t, J = 7.9 Hz), 7.92 (1H, dd, J = 7.9, 1.2 Hz), 7.97 (1H, dd, J = 7.9, 1.2 Hz), 8.31 (1H, d, J = 9.1 Hz), 8.39 (1H, s).

### <Reference Example 15-9>

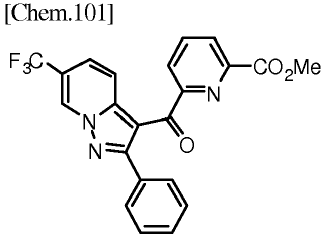

### Methyl 6-[[2-phenyl-6-(trifluoromethyl)pyrazolo[1,5-a]pyridin-3-yl)carbonyl]pyridine-2-carboxylate

A 70% perchloric acid aqueous solution (0.6 mL) was added to a 1,4-dioxane solution (1.5 mL) of ethyl *O*-mesitylsulfonylacetohydroxamate (1.7 g) under an argon atmosphere under ice-cooling, and then the mixture was stirred for 30 minutes under ice-cooling. Ice water (17.5 mL) was added to the reaction mixture, the precipitated solid was filtered off, the solid thus obtained was dissolved in dichloromethane (5 mL), and the solution was divided into layers. The organic layer was dried over anhydrous magnesium sulfate and filtered off. A dichloromethane solution (5 mL) of 3-trifluoromethylpyridine (0.6 mL) was added to the obtained filtrate under ice-cooling, and the mixture was stirred at normal temperature for 1 hour. The residue obtained by evaporating the reaction mixture was dissolved in an *N*,*N-*dimethylformamide-tetrahydrofuran mixed solution (5 mL, 1:1), then potassium carbonate (1.4 g) and methyl 6-(1-oxo-3-phenyl-2-propyn-1-yl)pyridine-2-carboxylate (663 mg) were added at normal temperature, and then the mixture was stirred at normal temperature for 1 hour. Water was added to the reaction mixture, and then the reaction mixture was extracted with ethyl acetate. The organic layer was washed with water and saturated saline in this order and then dried over anhydrous magnesium sulfate. The solvent was evaporated and then the residue was purified by silica gel column chromatography (n-hexane:ethyl acetate = 4:1) to obtain a title compound as a colorless powder (378 mg).
¹H-NMR (400 MHz, CDCl₃) δ 3.85 (3H, s), 7.08 (2H, t, J = 7.3 Hz), 7.16 (1H, tt, J = 7.3, 1.8 Hz), 7.24-7.28 (2H, m), 7.65 (1H, dd, J = 9.1,1.8 Hz), 7.89 (1H, t, J = 7.9 Hz), 7.97 (1H, dd, J = 7.9, 1.2 Hz), 8.05 (1H, dd, J = 7.9,1.2 Hz), 8.45 (1H, d, J = 9.1 Hz), 8.93 (1H, s).

### <Reference Example 15-10>

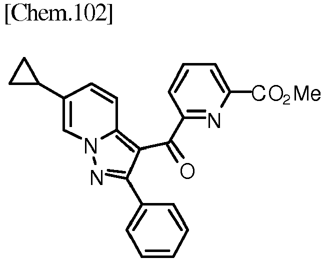

### Methyl 6-[(6-cyclopropyl-2-phenylpyrazolo[1,5-a]pyridin-3-yl)carbonyl]pyridine-2-carboxylate

A 70% perchloric acid aqueous solution (0.6 mL) was added to a 1,4-dioxane solution (1.5 mL) of ethyl O-mesitylsulfonylacetohydroxamate (1.7 g) under an argon atmosphere under ice-cooling, and then the mixture was stirred for 30 minutes under ice-cooling. Ice water (17.5 mL) was added to the reaction solution, the precipitated solid was filtered off, the obtained solid was dissolved in dichloromethane (5 mL), and the solution was divided into layers. The organic layer was dried over anhydrous magnesium sulfate and filtered off. A dichloromethane solution (5 mL) of 3-cyclopropylpyridine (596 mg) was added to the obtained filtrate under ice-cooling, and the mixture was stirred at normal temperature for 1 hour. The residue obtained by evaporating the reaction solution was dissolved in an *N,N*-dimethylformamide-tetrahydrofuran mixed solution (5 mL, 1:1), then potassium carbonate (1.4 g) and methyl 6-(1-oxo-3-phenyl-2-propyn-1-yl)pyridine-2-carboxylate (663 mg) were added at normal temperature, and then the mixture was stirred at normal temperature for 1 hour. Water was added to the reaction solution, and then the mixture was extracted with ethyl acetate. The organic layer was washed with water and saturated saline in this order and then dried over anhydrous magnesium sulfate. The solvent was evaporated and then the residue was purified by silica gel column chromatography (*n*-hexane:ethyl acetate = 4:1) to obtain a title compound as yellow liquid (112 mg).
¹H-NMR (400 MHz, CDCl₃) δ 1.04-1.11 (2H, m), 1.26 (2H, t, J = 7.3 Hz), 1.96-2.03 (1H, m), 3.85 (3H, s), 6.91 (2H, t, J = 7.3 Hz), 7.22 (2H, dd, J = 7.9, 1.8 Hz), 7.31 (1H, dd, J = 9.1, 1.8 Hz), 7.83 (1H, d, J = 7.9 Hz), 7.92 (1H, dd, J = 7.9, 1.2 Hz), 8.12 (2H, dd, J = 7.9, 1.2 Hz), 8.29 (1H, d, J = 9.1 Hz), 8.37 (1H, s).

### <Reference Example 15-11>

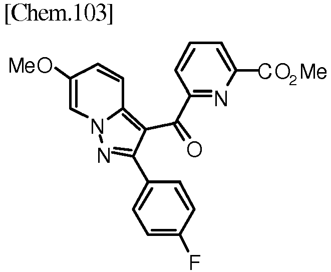

### Methyl 6-[[2-(4-fluorophenyl)-6-methoxypyrazolo[1,5-a]lpyridin-3-yl]carbonyl]pyridine-2-carboxyla te

After methyl 6-[3-(4-fluorophenyl)-1-oxo-2-propyn-1-yl]pyridine-2-carboxylate (950 mg) was added to a 1,4-dioxane (6.7 mL) suspension of 1-amino-3-methoxypyridinium 2,4,6-trimethylbenzenesulfonate (2.18 g), potassium carbonate (1.84 g) was added, and then the mixture was stirred for 1 hour. Subsequently, water and ethyl acetate were added to the reaction solution to divide it into layers, and then the organic layer was washed with saturated saline and dried over anhydrous sodium sulfate. The solvent was evaporated under vacuum, and then the residue was purified by silica gel column chromatography (*n*-hexane:ethyl acetate = 2:1 to 1:1), and the crude product thus obtained was triturated with diisopropyl ether to obtain a title compound as a colorless solid (132 mg).
¹H-NMR (400 MHz, DMSO-d₆) δ 3.76 (3H, s), 3.91 (3H, s), 6.89 (2H, t, J = 9.1 Hz), 7.17-7.23 (2H, m), 7.51 (1H, dd, J = 9.1, 1.8 Hz), 7.91-7.97 (2H, m), 8.03 (1H, t, J = 7.6 Hz), 8.08 (1H, d, J = 9.7 Hz), 8.73 (1H, d, J =1.8 Hz).

### <Reference Example 15-12>

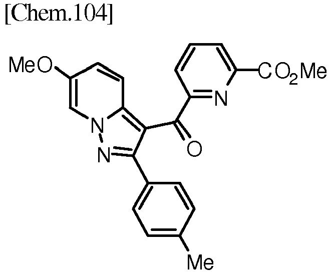

### Methyl 6-[[6-methoxy-2-(4-methylphenyl)pyrazolo[1,5-a]pyridin-3-yl]carbonyl]pyridine-2-carboxyl ate

Methyl 6-[3-(4-methylphenyl)-1-oxo-2-propyn-1-yl]pyridine-2-carboxylate (1.00 g) was added to a 1,4-dioxane (7.2 mL) suspension of 1-amino-3-methoxypyridinium 2,4,6-trimethylbenzenesulfonate (2.32 g), then potassium carbonate (1.97 g) was added to the mixture, and then the mixture was stirred for 1 hour. Subsequently, water and ethyl acetate were added to the reaction mixture to divide it into layers, and then the organic layer was washed with saturated saline and dried over anhydrous sodium sulfate. The solvent was evaporated under vacuum, and then the residue was purified by silica gel column chromatography (*n*-hexane:ethyl acetate = 2:1 to 1:1), and the crude product thus obtained was triturated with diisopropyl ether to obtain a title compound as a gray solid (212 mg).
¹H-NMR (400 MHz, DMSO-d₆) δ 2.17 (3H, s), 3.75 (3H, s), 3.90 (3H, s), 6.84 (2H, d, J = 7.9 Hz), 7.03 (2H, d, J = 7.9 Hz), 7.49 (1H, dd, J = 9.7, 2.4 Hz), 7.87-7.93 (2H, m), 8.00 (1H, t, J = 7.9 Hz), 8.05 (1H, d, J = 9.7 Hz), 8.72 (1H, d, J = 1.8 Hz).

### <Reference Example 15-13>

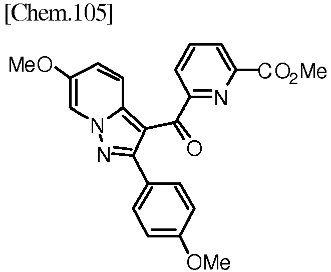

### Methyl 6-[[6-methoxy-2-(4-methoxyphenyl)pyrazolo[1,5-a]pyridin-3-yl]carbonyl]pyridine-2-carbox ylate

Methyl 6-[3-(4-methoxyphenyl)-1-oxo-2-propyn-1-yl]pyridine-2-carboxylate (600 mg) was added to a 1,4-dioxane (4.0 mL) suspension of 1-amino-3-methoxypyridinium 2,4,6-trimethylbenzenesulfonate (1.32 g), then potassium carbonate (1.12g) was added to the mixture, and then the mixture was stirred for 1 hour. Subsequently, water and ethyl acetate were added to the reaction mixture to divide it into layers, and then the organic layer was washed with saturated saline and dried over anhydrous sodium sulfate. The solvent was evaporated under vacuum, and then the residue was purified by silica gel column chromatography (*n*-hexane:ethyl acetate = 2:1 to 1:1), and the crude product thus obtained was triturated with diisopropyl ether to obtain a title compound as a pale yellow solid (115 mg).
¹H-NMR (400 MHz, DMSO-d₆) δ 3.66 (3H, s), 3.76 (3H, s), 3.90 (3H, s), 6.61 (2H, td, J = 9.7, 1.8 Hz), 7.09 (2H, td, J = 9.7, 1.8 Hz), 7.48 (1H, dd, J = 9.7, 2.4 Hz), 7.89-7.93 (2H, m), 8.01 (2H, q, J = 9.7 Hz), 8.71 (1H, d, J =1.8 Hz).

### <Reference Example 15-14>

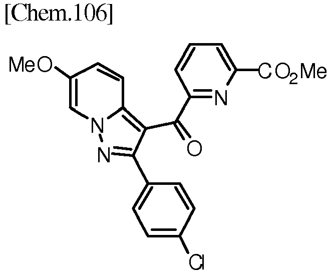

### Methyl 6-[[2-(4-chlorophenyl)-6-methoxypyrazolo[1,5-a]pyridin-3-yl]carbonyl]pyridine-2-carboxyl ate

Methyl 6-[3-(4-chlorophenyl)-1-oxo-2-propyn-1-yl]pyridine-2-carboxylate (1.00 g) was added to a 1,4-dioxane (6.7 mL) suspension of 1-amino-3-methoxypyridinium 2,4,6-trimethylbenzenesulfonate (2.16 g), then potassium carbonate (1.85 g) was added to the mixture, and then the mixture was stirred for 1 hour. Subsequently, water and ethyl acetate were added to the reaction mixture to divide it into layers, and then the organic layer was washed with saturated saline and dried over anhydrous sodium sulfate. The solvent was evaporated under vacuum, and then the residue was purified by silica gel column chromatography (n-hexane:ethyl acetate = 2:1 to 1:1), and the crude product thus obtained was triturated with diisopropyl ether to obtain a title compound as a white solid (151 mg).
¹H-NMR (400 MHz, DMSO-d₆) δ 3.76 (3H, s), 3.91 (3H, s), 7.12 (2H, dt, J = 9.1, 2.1 Hz), 7.18 (2H, dt, J = 9.1, 2.1 Hz), 7.51 (1H, dd, J = 9.7, 2.4 Hz), 7.93-7.99 (2H, m), 8.06 (2H, q, J = 9.7 Hz), 8.74 (1H, d, J = 2.4 Hz).

### <Reference Example 15-15>

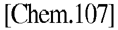

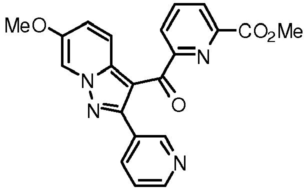

### Methyl 6-[[6-methoxy-2-(pyridin-3-yl)pyrazolo[1,5-a]pyridin-3-yl]carbonyl]pyridine-2-carboxylate

Methyl 6-[3-pyridin-3-yl)-1-oxo-2-propyn-1-yl]pyridine-2-carboxylate (200 mg) was added to a 1,4-dioxane (1.5 mL) suspension of 1-amino-3-methoxypyridinium 2,4,6-trimethylbenzenesulfonate (487 mg), then potassium carbonate (414 mg) was added to the mixture, and then the mixture was stirred for 1 hour. Subsequently, water and ethyl acetate were added to the reaction mixture to divide it into layers, and then the organic layer was washed with saturated saline and dried over anhydrous sodium sulfate. The solvent was evaporated under vacuum, and then the residue was purified by silica gel column chromatography (*n*-hexane:ethyl acetate = 2:1 to 1:1), and the crude product thus obtained was triturated with diisopropyl ether to obtain a title compound as a yellow solid (24.8 mg).
¹H-NMR (400 MHz, CDCl₃) δ 3.87 (3H, s), 3.92 (3H, s), 7.02 (1H, ddd, J = 7.9, 4.8, 1.2 Hz), 7.34 (1H, dd, J = 9.7, 2.4 Hz), 7.60 (1H, dt, J = 7.9, 1.8 Hz), 7.93 (1H, t, J = 7.9 Hz), 8.00 (1H, dd, J = 7.9, 1.2 Hz), 8.09 (1H, dd, J = 7.9, 1.2 Hz), 8.19 (1H, d, J =1.8 Hz), 8.28 (1H, d, J = 9.7 Hz), 8.35 (1H, dd, J = 4.8, 1.8 Hz), 8.46 (1H, d, J =1.2 Hz).

### <Reference Example 15-16>

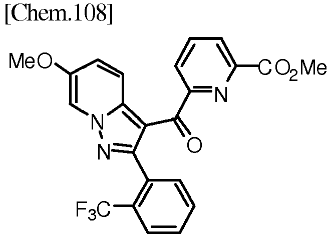

### Methyl 6-[[6-methoxy-2-[2-(wifluoromethyl)phenyl]pyrazolo[1,5-a]pyridin-3-yl]carbonyl]pyridine-2 -carboxylate

1-Amino-3-methoxypyridinium 2,4,6-trimethylbenzenesulfonate (2.3 g) and potassium carbonate (2.0 g) were added to a 1,4-dioxane solution (7.2 mL) of methyl 6-[1-oxo-3-[2-(trifluoromethyl)phenyl]-2-propyn-1-yl]pyridine-2-carboxylate (1.2 g) at room temperature under an argon atmosphere, and then the mixture was stirred at room temperature for 3 hours. Water was added to the reaction solution and the mixture was extracted with ethyl acetate. The organic layer was washed with water and saturated saline in this order and then dried over anhydrous magnesium sulfate. The solvent was evaporated and then the residue was purified by silica gel column chromatography (*n*-hexane:ethyl acetate = 2:1) to obtain a title compound as orange liquid (257 mg).
¹H-NMR (400 MHz, CDCl₃) δ 3.90 (3H, s), 3.94 (3H, s), 7.21-7.27 (2H, m), 7.32-7.37 (2H, m), 7.45 (1H, dd, J = 7.9, 1.2 Hz), 7.77 (1H, t, J = 7.9 Hz), 7.84 (1H, dd, J = 7.9, 1.2 Hz), 7.88 (1H, dd, J = 7.9, 1.2 Hz), 8.17 (1H, d, J =1.2 Hz), 8.35 (1H, d, J = 9.7 Hz).

### <Reference Example 15-17>

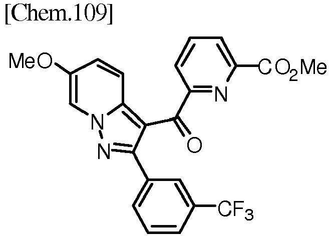

### Methyl 6-[[6-methoxy-2-[3-(trifluoromethyl)phenyl]pyrazolo[1,5-a]pyridin-3-yl]carbonyl]pyridine-2 -carboxylate

1-Amino-3-methoxypyridinium 2,4,6-trimethylbenzenesulfonate (587 mg) and potassium carbonate (500 mg) were added to a 1,4-dioxane solution (1.8 mL) of methyl 6-[1-oxo-3-[3-(trifluoromethyl)phenyl]-2-propyn-1-yl]pyridine-2-carboxylate (302 mg) at room temperature under an argon atmosphere, and then the mixture was stirred at room temperature for 16 hours. Water was added to the reaction mixture and the reaction mixture was extracted with ethyl acetate. The organic layer was washed with water and saturated saline in this order and then dried over anhydrous magnesium sulfate. The solvent was evaporated and then the residue thus obtained was purified by silica gel column chromatography (*n*-hexane:ethyl acetate = 2:1) to obtain a title compound as a colorless amorphous (52 mg).
¹H-NMR (400 MHz, CDCl₃) δ 3.82 (3H, s), 3.92 (3H, s), 7.18 (1H, t, J = 7.9 Hz), 7.34 (1H, dd, J = 9.7, 1.8 Hz), 7.38 (1H, d, J = 7.9 Hz), 7.44 (1H, d, J = 7.9 Hz), 7.49 (1H, s), 7.89 (1H, t, J = 7.9 Hz), 7.95 (1H, dd, J = 7.9, 1.8 Hz), 8.04 (1H, dd, J = 7.9, 1.2 Hz), 8.19 (1H, d, J = 1.2 Hz), 8.30 (1H, d, J = 9.7 Hz).

### <Reference Example 15-18>

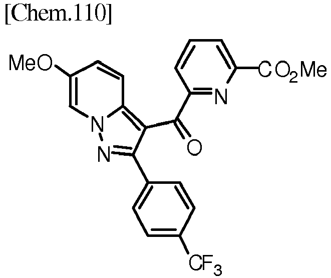

### Methyl 6-[[6-methoxy-2-[4-(trifluoromethyl)phenyl]pyrazolo[1,5-a]pyridin-3-yl]carbonyl]pyridine-2 -carboxylate

1-Amino-3-methoxypyridinium 2,4,6-trimethylbenzenesulfonate (2.9 g) and potassium carbonate (2.5 g) were added to a 1,4-dioxane solution (9.0 mL) of methyl 6-[1-oxo-3-[4-(trifluoromethyl)phenyl]-2-propyn-1-yl]pyridine-2-carboxylate (1.5 g) at room temperature under an argon atmosphere, and then the mixture was stirred at room temperature for 1 hour. Water was added to the reaction mixture and the reaction mixture was extracted with ethyl acetate. The organic layer was washed with water and saturated saline in this order and then dried over anhydrous magnesium sulfate. The solvent was evaporated and then the residue thus obtained was purified by silica gel column chromatography (*n*-hexane:ethyl acetate = 2:1) to obtain a title compound as brown liquid (408 mg).
¹H-NMR (400 MHz, CDCl₃) δ 3.84 (3H, s), 3.92 (3H, s), 7.31 (2H, d, J = 8.5 Hz), 7.37 (2H, d, J = 8.5 Hz), 7.55-7.57 (1H, m), 7.91 (1H, d, J = 7.9 Hz), 7.96 (1H, dd, J = 7.9, 1.2 Hz), 8.04 (1H, dd, J = 7.9,1.2 Hz), 8.18 (1H, d, J =1.8 Hz), 8.28 (1H, d, J = 9.7 Hz).

### <Reference Example 15-19>

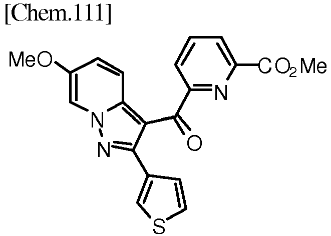

### Methyl 6-[[6-methoxy-2-(thiophen-3-yl)pyrazolo[1,5-a]pyridin-3-yl]carbonyl]pyridine-2-carboxylat e

1-Amino-3-methoxypyridinium 2,4,6-trimethylbenzenesulfonate (3.6 g) and potassium carbonate (3.1 g) were added to a 1,4-dioxane solution (11.1 mL) of methyl 6-[1-oxo-3-(thiophen-3-yl)-2-propyn-1-yl]pyridine-2-carboxylate (1.5 g) at room temperature under an argon atmosphere, and then the mixture was stirred at room temperature for 4 hours. Water was added to the reaction mixture and the reaction mixture was extracted with ethyl acetate. The organic layer was washed with water and saturated saline in this order and then dried over anhydrous magnesium sulfate. The solvent was evaporated and then the residue thus obtained was purified by silica gel column chromatography (*n*-hexane:ethyl acetate = 2:1) to obtain a title compound as a yellow amorphous (183 mg).
¹H-NMR (400 MHz, CDCl₃) δ 3.89 (3H, s), 3.90 (3H, s), 6.87 (1H, dd, J = 4.8,1.2 Hz), 6.99 (1H, dd, J = 4.8, 3.0 Hz), 7.26-7.30 (2H, m), 7.90 (1H, t, J = 7.9 Hz), 7.99 (1H, dd, J = 7.9, 1.2 Hz), 8.06 (1H, dd, J = 7.9, 1.2 Hz), 8.15 (1H, s), 8.16 (1H, d, J = 7.9 Hz).

### <Reference Example 15-20>

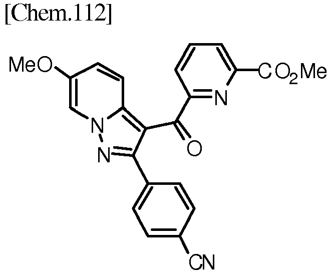

### Methyl 6-[[2-(4-cyanophenyl)-6-methoxypyrazolo[1,5-a]pyridin-3-yl]carbonyl]pyridine-2-carboxyla te

Potassium carbonate (516 mg) was added to a 1,4-dioxane (2.0 mL) solution of methyl 6-[3-(4-cyanophenyl)-1-oxo-2-propyn-1-yl]pyridine-2-carboxylate (270 mg) and 1-amino-3-methoxypyridinium 2,4,6-trimethylbenzenesulfonate (605 mg) at room temperature, and then the mixture was stirred at room temperature for 2 hours. The reaction mixture was diluted with ethyl acetate, then washed with water and saturated saline, and then dried over anhydrous sodium sulfate. The solvent was evaporated and the residue thus obtained was purified by silica gel column chromatography (n-hexane:ethyl acetate = 3:1) to obtain a title compound as a pale yellow crystal (83.0 mg).
¹H-NMR (400 MHz, CDCl₃) δ 3.87 (3H, s), 3.92 (3H, s), 7.33 (2H, dd, J = 9.7, 2.4 Hz), 7.35-7.43 (4H, m), 7.93 (1H, t, J = 7.9 Hz), 8.00 (1H, dd, J = 7.9, 1.2 Hz), 8.17 (1H, dd, J = 7.9, 1.2 Hz), 8.17 (1H, d, J = 2.4 Hz), 8.23 (1H, d, J = 9.7 Hz).

### <Reference Example 15-21>

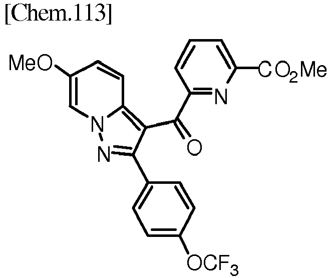

### Methyl 6-[[6-methoxy-2-[4-(trifluoromethoxy)phenyl]pyrazolo[1,5-a]pyridin-3-yl]carbonyl]pyridine -2-carboxylate

After potassium carbonate (2.90 g) was added to a 1,4-dioxane (10.5 mL) solution of methyl 6-[1-oxo-3-[4-(trifluoromethoxy)phenyl]-2-propyn-1-yl]pyridine-2-carboxylate (1.83 g, 5.24 mmol) and 1-amino-3-methoxypyridinium 2,4,6-trimethylbenzenesulfonate (3.40 g) at room temperature, the mixture was stirred at room temperature for 2 hours. The reaction solution was diluted with ethyl acetate, then washed with water and saturated saline, and then dried over anhydrous sodium sulfate. The solvent was evaporated and the residue thus obtained was purified by silica gel column chromatography (*n*-hexane:ethyl acetate = 3:1) to obtain a title compound as a yellow crystal (533 mg).
¹H-NMR (400 MHz, CDCk₃) δ 3.85 (3H, s), 3.92 (3H, s), 6.90 (2H, d, J = 7.9 Hz), 7.28 (2H, d, J = 7.9 Hz), 7.33 (1H, dd, J = 9.7, 2.4 Hz), 7.88 (1H, t, J = 7.9 Hz), 7.97 (1H, dd, J = 7.9, 1.2 Hz), 8.00 (1H, dd, J = 7.9, 1.2 Hz), 8.17 (1H, d, J = 2.4 Hz), 8.27 (1H, d, J = 9.7 Hz).

### <Reference Example 16-1>

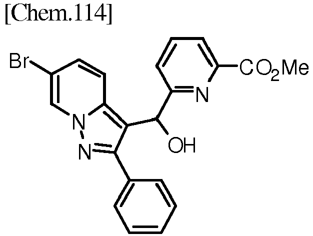

### Methyl 6-[(6-bromo-2-phenylpyrazolo[1,5-a]pyridin-3-yl)(hydroxy)methyl]pyridine-2-carboxylate

After a methanol (3.3 mL) solution of sodium borohydride (150 mg) was added to a dichloromethane (33 mL) solution of methyl 6-[(6-bromo-2-phenylpyrazolo[1,5-a]pyridin-3-yl)carbonyl]pyridine-2-carboxylate (1.44 g) at 0°C, the mixture was stirred at 0°C for 1 hour. Water was added to the reaction solution and then the mixture was extracted with ethyl acetate. The extract was then washed with saturated saline and then dried over anhydrous sodium sulfate. The solvent was evaporated and the residue thus obtained was purified by silica gel column chromatography (*n*-hexane:ethyl acetate = 2:1) to obtain a title compound as a colorless crystal (1.40 g).
¹H-NMR (400 MHz, CDCl₃) δ 4.05 (3H, s), 5.31 (1H, d, J = 3.0 Hz), 6.23 (1H, d, J = 3.0 Hz), 7.05 (2H, d, J =1.2 Hz), 7.18 (1H, d, J = 7.9 Hz), 7.40-7.50 (3H, m), 7.71 (1H, t, J = 7.9 Hz), 7.81 (1H, d, J = 7.9 Hz), 7.82 (1H, d, J = 7.9 Hz), 8.03 (1H, d, J = 7.9 Hz), 8.62 (1H, s).

### <Reference Example 16-2>

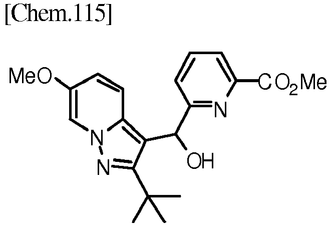

### Methyl 6-[(2-tert-butyl-6-methoxypyrazolo[1,5-a]pyridin-3-yl)(hydroxy)methyl]pyridine-2-carboxyl ate

A methanol (0.7 mL) solution of sodium borohydride (30.0 mg) was added to a dichloromethane (6.5 mL) solution of methyl 6-[(2-*tert*-butyl-6-methoxypyrazolo[1,5-a]pyridin-3-yl)carbonyl]pyridine-2-carboxylate (240 mg) at 0°C, and then the mixture was stirred at 0°C for 1 hour. Water was added to the reaction mixture and the reaction mixture was extracted with ethyl acetate. The extract thus obtained was then washed with saturated saline and then dried over anhydrous sodium sulfate. The solvent was evaporated and the residue thus obtained was purified by silica gel column chromatography (*n*-hexane:ethyl acetate = 1:1) to obtain a title compound as a colorless crystal (172 mg).
¹H-NMR (400 MHz, CDCl₃) δ 1.56 (9H, s), 3.76 (3H, s), 4.05 (3H, s), 5.27 (1H, d, J =1.8 Hz), 6.43 (1H, d, J = 7.9 Hz), 6.66 (1H, dd, J = 9.7, 1.8 Hz), 6.78 (1H, d, J = 9.7 Hz), 7.22 (1H, d, J = 7.9 Hz), 7.72 (1H, t, J = 7.9 Hz), 7.99 (1H, d, J = 1.8 Hz), 8.34 (1H, d, J = 7.9 Hz).

### <Reference Example 16-3>

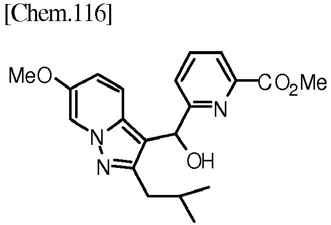

### Methyl 6-[(2-isobutyl-6-methoxypyrazolo[1,5-a]pyridin-3-yl)(hydroxy)methyl]pyridine-2-carboxyla te

A methanol (0.7 mL) solution of sodium borohydride (31.0 mg) was added to a dichloromethane (7.0 mL) solution of methyl 6-[(2-isobutyl-6-methoxypyrazolo[1,5-a]pyridin-3-yl)carbonyl]pyridine-2-carboxylate (253 mg) at 0°C, and then the mixture was stirred at 0°C for 1 hour. Water was added to the reaction mixture and the reaction mixture was extracted with ethyl acetate. The extract thus obtained was then washed with saturated saline and then dried over anhydrous sodium sulfate. The solvent was evaporated and the residue thus obtained was purified by silica gel column chromatography (*n*-hexane:ethyl acetate = 1:1) to obtain a title compound as a colorless crystal (213 mg).
¹H-NMR (400 MHz, CDCl₃) δ 0.98 (6H, t, J = 6.1 Hz), 2.03-2.18 (1H, m), 2.69 (2H, d, J = 7.3 Hz), 3.78 (3H, s), 4.04 (3H, s), 5.17 (1H, brs), 6.07 (1H, brs), 6.76 (1H, dd, J = 9.7, 1.8 Hz), 7.02 (1H, d, J = 9.7 Hz), 7.23 (1H, d, J = 7.9 Hz), 7.74 (1H, t, J = 7.9 Hz), 7.99 (1H, d, J = 1.8 Hz), 8.04 (1H, d, J = 7.9 Hz).

### <Reference Example 16-4>

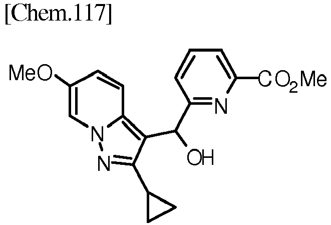

### Methyl 6-[(2-cyclopropyl-6-methoxypyrazolo[1,5-a]pyridin-3-yl)(hydroxy)methyl]pyridine-2-carbo xylate

A methanol (1.0 mL) solution of sodium borohydride (43.5 mg) was added to a dichloromethane (9.5 mL) solution of methyl 6-[(2-cyclopropyl-6-methoxypyrazolo[1,5-a]pyridin-3-yl)carbonyl]pyridine-2-carboxylate (336 mg) at 0°C, and then the mixture was stirred at 0°C for 1 hour. Water was added to the reaction mixture and the reaction mixture was extracted with ethyl acetate. The extract thus obtained was then washed with saturated saline and then dried over anhydrous sodium sulfate. The solvent was evaporated and the residue thus obtained was purified by silica gel column chromatography (n-hexane:ethyl acetate = 1:1) to obtain a title compound as a pale yellow amorphous (257 mg).
¹H-NMR (400 MHz, CDCl₃) δ 0.84-1.07 (4H, m),1.95-2.04 (1H, m), 3.76 (3H, s), 4.04 (3H, s), 5.24 (1H, d, J = 2.4 Hz), 6.24 (1H, d, J = 2.4 Hz), 6.77 (1H, dd, J = 9.7, 1.8 Hz), 7.09 (1H, d, J = 9.7Hz), 7.31 (1H, d, J = 7.9 Hz), 7.76 (1H, t, J = 7.9 Hz), 7.91 (1H, d, J = 1.8 Hz), 8.04 (1H, d, J = 7.9 Hz).

### <Reference Example 16-5>

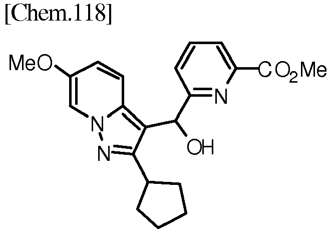

### Methyl 6-[(2-cyclopentyl-6-methoxypyrazolo[1,5-a]pyridin-3-yl)(hydroxy)methyl]pyridine-2-carbo xylate

A methanol (0.7 mL) solution of sodium borohydride (31.0 mg) was added to a dichloromethane (7.0 mL) solution of methyl 6-[(2-cyclopentyl-6-methoxypyrazolo[1,5-a]pyridin-3-yl)carbonyl]pyridine-2-carboxylate (257 mg) at 0°C, and then the mixture was stirred at 0°C for 1 hour. Water was added to the reaction mixture and the reaction mixture was extracted with ethyl acetate. The extract thus obtained was then washed with saturated saline and then dried over anhydrous sodium sulfate. The solvent was evaporated and the residue thus obtained was purified by silica gel column chromatography (*n*-hexane:ethyl acetate = 1:1) to obtain a title compound as a pale yellow amorphous (221 mg).
¹H-NMR (400 MHz, CDCl₃) δ 1.60-1.75 (2H, m), 1.80-2.16 (6H, m), 3.27-3.38 (1H, m), 3.77 (3H, s), 4.04 (3H, s), 5.17 (1H, d, J = 2.4 Hz), 6.13 (1H, d, J = 2.4 Hz), 6.74 (1H, dd, J = 9.7,2.4 Hz), 7.02 (1H, d, J = 9.7 Hz), 7.24 (1H, d, J = 7.9 Hz), 7.74 (1H, t, J = 7.9 Hz), 8.00 (1H, d, J = 1.8 Hz), 8.03 (1H, d, J = 7.9 Hz).

### <Reference Example 16-6>

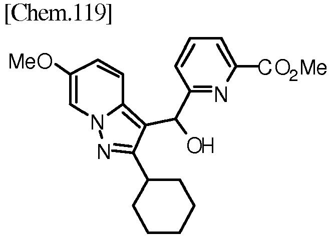

### Methyl 6-[(2-cyclohexyl-6-methoxypyrazolo[1,5-a]pyridin-3-yl)(hydroxy)methyl]pyridine-2-carbox ylate

A methanol (0.75 mL) solution of sodium borohydride (34.1 mg) was added to a dichloromethane (7.5 mL) solution of methyl 6-[(2-cyclohexyl-6-methoxypyrazolo[1,5-a]pyridin-3-yl)carbonyl]pyridine-2-carboxylate (295 mg) at 0°C, and then the mixture was stirred at 0°C for 1 hour. Water was added to the reaction mixture and the reaction mixture was extracted with ethyl acetate. The extract thus obtained was then washed with saturated saline and then dried over anhydrous sodium sulfate. The solvent was evaporated and the residue thus obtained was purified by silica gel column chromatography (*n*-hexane:ethyl acetate = 1:1) to obtain a title compound as a colorless crystal (248 mg).
¹H-NMR (400 MHz, CDCl₃) δ 1.25-1.45 (3H, m), 1.65-2.03 (7H, m), 2.84-2.94 (1H, m), 3.77 (3H, s), 4.04 (3H, s), 5.16 (1H, d, J = 2.4 Hz), 6.13 (1H, d, J = 2.4 Hz), 6.74 (1H, dd, J = 9.7, 2.4 Hz), 7.00 (1H, d, J = 9.7 Hz), 7.24 (1H, d, J = 7.9 Hz), 7.75 (1H, t, J = 7.9 Hz), 8.01 1 (1H, d, J = 1.8 Hz), 8.04 (1H, d, J = 7.9 Hz).

### <Reference Example 16-7>

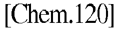

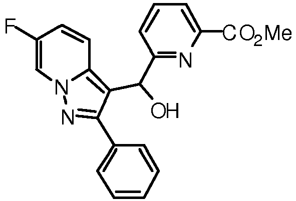

### Methyl 6-[(6-fluoro-2-phenylpyrazolo[1,5-a]pyridin-3-yl)(hydroxy)methyl]pyridine-2-carboxylate

A methanol solution (0.5 mL) of sodium borohydride (27 mg) was added to a dichloromethane solution (5.5 mL) of methyl 6-[(6-fluoro-2-phenylpyrazolo[1,5-a]pyridin-3-yl)carbonyl]pyridine-2-carboxylate (225 mg) at normal temperature under an argon atmosphere, and then the mixture was stirred for 30 minutes under ice-cooling. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as a colorless amorphous (190 mg).
¹H-NMR (400 MHz, CDCl₃) δ 4.05 (3H, s), 5.32 (1H, d, J = 2.4 Hz), 6.24 (1H, d, J = 2.4 Hz), 6.94 (1H, ddd, J = 10.3, 7.9, 2.4 Hz), 7.14 (1H, dd, J = 9.7,5.4 Hz), 7.20 (1H, d, J = 7.9 Hz), 7.42-7.50 (3H, m), 7.72 (1H, t, J = 7.9 Hz), 7.79-7.83 (2H, m), 8.03 (1H, d, J = 7.3 Hz), 8.40-8.43 (1H, m).

### <Reference Example 16-8>

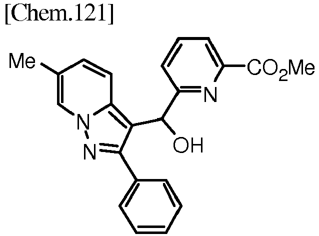

### Methyl 6-[(6-methyl-2-phenylpyrazolo[1,5-a]pyridin-3-yl)(hydroxy)methyl]pyridine-2-carboxylate

A methanol solution (0.7 mL) of sodium borohydride (35 mg) was added to a dichloromethane solution (7.1 mL) of methyl 6-[(6-methyl-2-phenylpyrazolo[1,5-a]pyridin-3-yl)carbonyl]pyridine-2-carboxylate (290 mg) at normal temperature under an argon atmosphere, and then the mixture was stirred under ice-cooling for 1 hour. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue thus obtained was purified by silica gel column chromatography (n-hexane:ethyl acetate = 2:1) to obtain a title compound as a colorless amorphous (283 mg).
¹H-NMR (400 MHz, CDCl₃) δ 2.29 (3H, s), 4.04 (3H, s), 5.33 (1H, d, J = 2.4 Hz), 6.22 (1H, d, J = 2.4 Hz), 6.83 (1H, d, J = 9.1 Hz), 6.98 (1H, d, J = 9.1 Hz), 7.20 (1H, d, J = 7.9 Hz), 7.41 (1H, d, J = 7.3 Hz), 7.46 (2H, t, J = 7.3 Hz), 7.69 (1H, t, J = 7.9 Hz), 7.80-7.85 (2H, m), 8.01 (1H, d, J = 7.3 Hz), 8.27 (1H, s).

### <Reference Example 16-9>

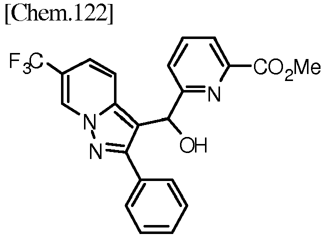

### Methyl 6-[[2-phenyl-6-(trifluoromethyl)pyrazolo[1,5-a]pyridin-3-yl](hydroxy)methyl]pyridine-2-car boxylate

A methanol solution (0.8 mL) of sodium borohydride (39 mg) was added to a dichloromethane solution (7.7 mL) of methyl 6-[[2-phenyl-6-(trifluoromethyl)pyrazolo[1,5-a]pyridin-3-yl]carbonyl]pyridine-2-carboxylate (360 mg) at normal temperature under an argon atmosphere, and then the mixture was stirred under ice-cooling for 1 hour. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue thus obtained was purified by silica gel column chromatography (*n*-hexane:ethyl acetate = 2:1) to obtain a title compound as a colorless amorphous (322 mg).
¹H-NMR (400 MHz, CDCl₃) δ 4.05 (3H, s), 5.31 (1H, d, J = 3.0 Hz), 6.26 (1H, d, J = 3.0 Hz), 7.11 (1H, dd, J = 9.1, 1.2 Hz), 7.19 (1H, d, J = 7.9 Hz), 7.28 (1H, d, J = 7.3 Hz), 7.43-7.52 (3H, m), 7.73 (1H, t, J = 7.9 Hz), 7.82-7.86 (2H, m), 8.04 (1H, d, J = 7.9 Hz), 8.81 (1H, s).

### <Reference Example 16-10>

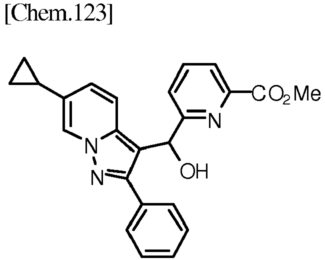

### Methyl 6-[(6-cyclopropyl-2-phenylpyrazolo[1,5-a]pyridin-3-yl)(hydroxy)methyl]pyridine-2-carboxy late

A methanol solution (0.7 mL) of sodium borohydride (37 mg) was added to a dichloromethane solution (7.4 mL) of methyl 6-[(6-cyclopropyl-2-phenylpyrazolo[1,5-a]pyridin-3-yl)carbonyl]pyridine-2-carboxylate (320 mg) at normal temperature under an argon atmosphere, and then the mixture was stirred under ice-cooling for 1 hour. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue thus obtained was purified by silica gel column chromatography (*n*-hexane:ethyl acetate = 2:1) to obtain a title compound as a colorless amorphous (282 mg).
¹H-NMR (400 MHz, CDCl₃) δ 0.61-0.66 (2H, m), 0.90-0.96 (2H, m), 1.81-1.88 (1H, m), 4.02 (3H, s), 5.40 (1H, d, J = 2.4 Hz), 6.22 (1H, d, J = 2.4 Hz), 6.73 (1H, dd, J = 9.1, 1.2 Hz), 6.98 (1H, d, J = 9.1 Hz), 7.23 (1H, d, J = 7.9 Hz), 7.38 (1H, tt, J = 7.3, 1.2 Hz), 7.42-7.47 (2H, m), 7.68 (1H, t, J = 7.9 Hz), 7.81-7.85 (2H, m), 7.99 (1H, d, J = 7.9 Hz), 8.24 (1H, s).

### <Reference Example 16-11>

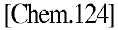

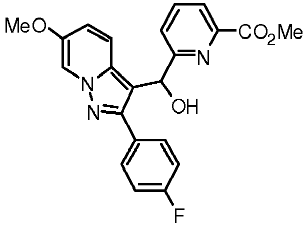

### Methyl 6-[[2-(4-fluorophenyl)-6-methoxypyrazolo[1,5-a]pyridin-3-yl] (hydroxy)methyl]pyridine-2-c arboxylate

A dichloromethane (2.5 mL) solution of methyl 6-[[2-(4-fluorophenyl)-6-methoxypyrazolo[1,5-a]pyridin-3-yl]carbonyl]pyridine-2-carboxyla te (110 mg) was ice-cooled under an argon atmosphere and a methanol (0.25 mL) suspension of sodium borohydride (20.0 mg) was added. The mixture was then stirred for 1 hour under ice-cooling, and then a saturated ammonium chloride aqueous solution was added. The mixture was extracted with ethyl acetate, washed with saturated saline, and then dried over anhydrous sodium sulfate. The solvent was evaporated under vacuum and then the residue was purified by silica gel column chromatography (n-hexane:ethyl acetate = 5:1 to 1:1) to obtain a title compound as a white solid (111 mg).
¹H-NMR (400 MHz, CDCl₃) δ 3.81 (3H, s), 4.04 (3H, s), 5.29 (1H, d, J = 3.0 Hz), 6.17 (1H, d, J = 2.4 Hz), 6.80 (1H, dd, J = 9.7, 2.4 Hz), 6.98 (1H, d, J = 9.7 Hz), 7.14 (2H, tt, J = 8.8, 2.4 Hz), 7.21 (1H, d, J = 7.9 Hz), 7.71 (1H, t, J = 7.9 Hz), 7.78-7.83 (2H, m), 8.03 (1H, d, J = 7.9 Hz), 8.06 (1H, d, J = 2.4 Hz).

### <Reference Example 16-12>

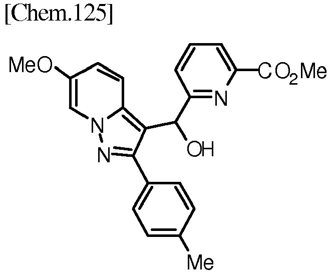

### Methyl 6-[hydroxy[6-methoxy-2-(4-methylphenyl)pyrazolo[1,5-a]pyridin-3-yl]methyl]pyridine-2-ca rboxylate

A methanol (0.41 mL) suspension of sodium borohydride (20.0 mg) was added to a dichloromethane (4.1 mL) solution of methyl 6-[[6-methoxy-2-(4-methylphenyl)pyrazolo[1,5-a]pyridin-3-yl]carbonyl]pyridine-2-carboxyl ate (180 mg) under an argon atmosphere under ice-cooling. The mixture was then stirred for 1 hour under ice-cooling, and then a saturated ammonium chloride aqueous solution was added. The mixture was extracted with ethyl acetate, washed with saturated saline, and dried over anhydrous sodium sulfate. The solvent was evaporated under vacuum and then the residue was purified by silica gel column chromatography (n-hexane:ethyl acetate = 5:1 to 1:1) to obtain a title compound as a colorless solid (158 mg).
¹H-NMR (400 MHz, CDCl₃) δ 2.41 (3H, s), 3.80 (3H, s), 4.05 (3H, s), 5.32 (1H, d, J = 2.4 Hz), 6.22 (1H, d, J = 2.4 Hz), 6.77 (1H, dd, J = 9.7, 2.4 Hz), 6.96 (1H, d, J = 9.7 Hz), 7.20 (1H, d, J = 7.9 Hz), 7.29 (1H, s), 7.68-7.73 (3H, m), 8.02 (1H, d, J = 7.9 Hz), 8.08 (1H, d, J = 2.4 Hz).

### <Reference Example 16-13>

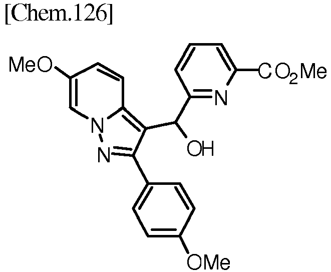

### Methyl 6-[hydroxy[6-methoxy-2-(4-methoxyphenyl)pyrazolo[1,5-a]pyridin-3-yl]methyl]pyridine-2-carboxylate

A methanol (0.24 mL) suspension of sodium borohydride (10.9 mg) was added to a dichloromethane (2.4 mL) solution of methyl 6-[[6-methoxy-2-(4-methoxyphenyl)pyrazolo[1,5-a]pyridin-3-yl]carbonyl]pyridine-2-carbox ylate (110 mg) under an argon atmosphere under ice-cooling. The mixture was then stirred for 1 hour under ice-cooling, and then a saturated ammonium chloride aqueous solution was added. The mixture was extracted with ethyl acetate, washed with saturated saline, and dried over anhydrous sodium sulfate. The solvent was evaporated under vacuum and then the residue was purified by silica gel column chromatography (n-hexane:ethyl acetate = 5:1 to 1:1) to obtain a title compound as a pale yellow solid (103 mg).
¹H-NMR (400 MHz, CDCl₃) δ 3.80 (3H, s), 3.86 (3H, s), 4.04 (3H, s), 5.30 (1H, d, J = 2.4 Hz), 6.20 (1H, d, J = 1.8 Hz), 6.77 (1H, dd, J = 9.7, 2.4 Hz), 6.95 (1H, d, J = 9.7 Hz), 7.00 (2H, td, J = 5.8, 3.6 Hz), 7.21 (1H, d, J = 7.3 Hz), 7.70 (1H, t, J = 7.9 Hz), 7.76 (2H, td, J = 5.8,3.6 Hz), 8.02 (1H, d, J = 7.9 Hz), 8.07 (1H, d, J =1.8 Hz).

### <Reference Example 16-14>

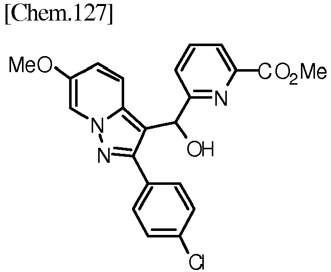

### Methyl 6-[[2-(4-chlorophenyl)-6-methoxypyrazolo[1,5-a]pyridin-3-yl](hydroxy)methyl]pyridine-2-c arboxylate

A methanol (0.26 mL) suspension of sodium borohydride (12.9 mg) was added to a dichloromethane (2.6 mL) solution of methyl 6-[[2-(4-chlorophenyl)-6-methoxypyrazolo[1,5-a]pyridin-3-yl]carbonyl]pyridine-2-carboxyl ate (120 mg) under an argon atmosphere under ice-cooling. The mixture was then stirred for 1 hour under ice-cooling, and then a saturated ammonium chloride aqueous solution was added. The mixture was extracted with ethyl acetate, washed with saturated saline, and dried over anhydrous sodium sulfate. The solvent was evaporated under vacuum and then the residue was purified by silica gel column chromatography (n-hexane:ethyl acetate = 5:1 to 1:1) to obtain a title compound as a white solid (119 mg).
¹H-NMR (400 MHz, CDCl₃) δ 3.81 (3H, s), 4.05 (3H, s), 5.29 (1H, d, J = 2.4 Hz), 6.18 (1H, d, J = 2.4 Hz), 6.80 (1H, dd, J = 9.7, 1.8 Hz), 6.98 (1H, d, J = 9.7 Hz), 7.21 (1H, d, J = 7.9 Hz), 7.43 (2H, dt, J = 8.9, 1.8 Hz), 7.71 (1H, t, J = 7.9 Hz), 7.78 (2H, dt, J = 8.9, 1.8 Hz), 8.03 (1H, d, J = 7.9 Hz), 8.06 (1H, d, J = 1.8 Hz).

### <Reference Example 16-15>

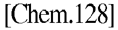

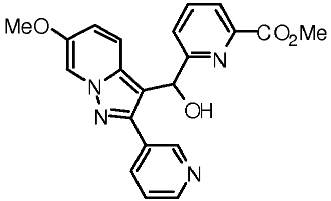

### Methyl 6-[hydroxy[6-methoxy-2-(pyridin-3-yl)pyrazolo[1,5-a]pyridin-3-yl]methyl]pyridine-2-carbo xylate

A methanol (0.056 mL) suspension of sodium borohydride (2.8 mg) was added to a dichloromethane (1.5 mL) solution of methyl 6-[[6-methoxy-2-(pyridin-3-yl)pyrazolo[1,5-a]pyridin-3-yl]carbonyl]pyridine-2-carboxylate (24.0 mg) under an argon atmosphere under ice-cooling. The mixture was then stirred for 1 hour under ice-cooling, and then a saturated ammonium chloride aqueous solution was added. The mixture was extracted with ethyl acetate, washed with saturated saline, and dried over anhydrous sodium sulfate. The solvent was evaporated under vacuum and then the residue was purified by silica gel column chromatography (n-hexane:ethyl acetate = 5:1 to 1:1) to obtain a title compound as a colorless oil (18.6 mg).
¹H-NMR (400 MHz, CDCl₃) δ 3.83 (3H, s), 4.04 (3H, s), 5.31 (1H, d, J = 2.4 Hz), 6.17 (1H, d, J = 2.4 Hz), 6.84 (1H, dd, J = 9.7, 2.1 Hz), 7.05 (1H, d, J = 9.7 Hz), 7.25 (1H, d, J = 7.9 Hz), 7.37 (1H, dd, J = 7.9, 4.8 Hz), 7.72 (1H, t, J = 7.9 Hz), 8.03 (1H, d, J = 7.9 Hz), 8.08 (1H, d, J = 2.4 Hz), 8.18 (1H, td, J = 7.9, 1.8 Hz), 8.63 (1H, dd, J = 4.8, 1.8 Hz), 9.04 (1H, d, J = 2.4 Hz).

### <Reference Example 16-16>

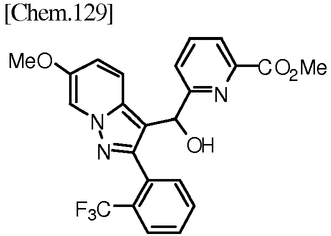

### Methyl 6-[hydroxy[6-methoxy-2-[2-(trifluoromethyl)phenyl]pyrazolo[1,5-a]pyridin-3-yl]methyl]pyr idine-2-carboxylate

A methanol solution (0.5 mL) of sodium borohydride (25 mg) was added to a dichloromethane solution (5.0 mL) of methyl 6-[[6-methoxy-2-[2-(trifluoromethyl)phenyl]pyrazolo[1,5-a]pyridin-3-yl]carbonyl]pyridine-2 -carboxylate (250 mg) at room temperature under an argon atmosphere, and then the mixture was stirred for 1 hour under ice-cooling. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 2:1) to obtain a title compound as a colorless amorphous (152 mg).
¹H-NMR (400 MHz, CDCl₃) δ 3.81 (3H, s), 4.02 (3H, s), 5.13 (1H, d, J = 2.4 Hz), 5.80 (1H, d, J = 2.4 Hz), 6.84 (1H, dd, J = 9.7, 1.8 Hz), 7.07 (1H, d, J = 9.7 Hz), 7.19 (1H, d, J = 7.3 Hz), 7.54 (1H, t, J = 7.3 Hz), 7.61 (1H, t, J = 7.3 Hz), 7.66 (1H, d, J = 7.9 Hz), 7.70 (1H, t, J = 7.9 Hz), 7.79 (1H, d, J = 7.9 Hz), 7.98 (1H, d, J = 7.9 Hz), 8.05 (1H, d, J = 1.8 Hz).

### <Reference Example 16-17>

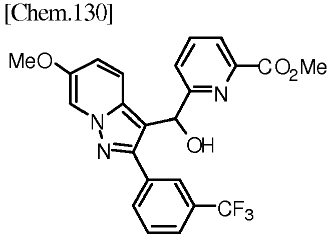

### Methyl 6-[hydroxy[6-methoxy-2-[3-(trifluoromethyl)phenyl]pyrazolo[1,5-a]pyridin-3-yl]methyl]pyr idine-2-carboxylate

A methanol solution (0.1 mL) of sodium borohydride (5 mg) was added to a dichloromethane solution (1.0 mL) of methyl 6-[[6-methoxy-2-[3-(trifluoromethyl)phenyl]pyrazolo[1,5-a]pyridin-3-yl]carbonyl]pyridine-2 -carboxylate (52 mg) at room temperature under an argon atmosphere, and then the mixture was stirred for 1 hour under ice-cooling. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue thus obtained was purified by silica gel column chromatography (n-hexane:ethyl acetate = 3:2) to obtain a title compound as yellow liquid (39 mg).
¹H-NMR (400 MHz, CDCl₃) δ 3.83 (3H, s), 4.04 (3H, s), 5.30 (1H, d, J = 3.0 Hz), 6.19 (1H, d, J = 3.0 Hz), 6.84 (1H, dd, J = 9.7, 1.8 Hz), 7.04 (1H, d, J = 9.7 Hz), 7.23 (1H, d, J = 7.9 Hz), 7.55 (1H, t, J = 7.9 Hz), 7.63 (1H, d, J = 7.9 Hz), 7.71 (1H, t, J = 7.9 Hz), 8.02 (1H, d, J = 7.9 Hz), 8.03 (1H, d, J = 7.9 Hz), 8.08-8.10 (2H, m).

### <Reference Example 16-18>

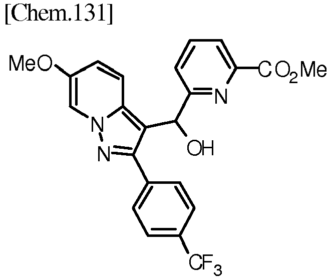

### Methyl 6-[hydroxy[6-methoxy-2-[4-(trifluoromethyl)phenyl]pyrazolo[1,5-a]pyridin-3-yl]methyl]pyr idine-2-carboxylate

A methanol solution (0.8 mL) of sodium borohydride (40 mg) was added to a dichloromethane solution (8.0 mL) of methyl 6-[[6-methoxy-2-[4-(trifluoromethyl)phenyl]pyrazolo[1,5-a]pyridin-3-yl]carbonyl]pyridine-2 -carboxylate (400 mg) at room temperature under an argon atmosphere, and then the mixture was stirred for 1 hour under ice-cooling. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue thus obtained was purified by silica gel column chromatography (n-hexane:ethyl acetate = 2:1) to obtain a title compound as a yellow amorphous (365 mg).
¹H-NMR (400 MHz, CDCl₃) δ 3.82 (3H, s), 4.04 (3H, s), 5.30 (1H, brs), 6.20 (1H, s), 6.83 (1H, dd, J = 9.7, 1.2 Hz), 7.01 (1H, d, J = 9.7 Hz), 7.22 (1H, d, J = 7.9 Hz), 7.71 (3H, t, J = 7.9 Hz), 7.97 (2H, d, J = 7.9 Hz), 8.03 (1H, d, J = 7.9 Hz), 8.08 (1H, d, J =1.2 Hz).

### <Reference Example 16-19>

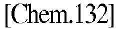

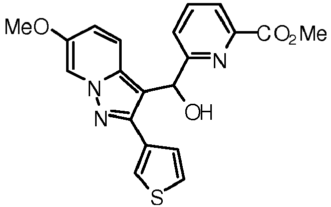

### Methyl 6-[hydroxy[6-methoxy-2-(thiophen-3-yl)pyrazolo[1,5-a]pyridin-3-yl]methyl]pyridine-2-carb oxylate

A methanol solution (0.4 mL) of sodium borohydride (21 mg) was added to a dichloromethane solution (4.2 mL) of methyl 6-[[6-methoxy-2-(thiophen-3-yl)pyrazolo[1,5-a]pyridin-3-yl]carbonyl]pyridine-2-carboxylat e (182 mg) at room temperature under an argon atmosphere, and then the mixture was stirred for 1 hour under ice-cooling. A saturated ammonium chloride aqueous solution was added to the reaction mixture, and then the reaction mixture was extracted with ethyl acetate. The organic layer was washed with saturated saline and dried over anhydrous magnesium sulfate. The solvent was evaporated and then the residue thus obtained was purified by silica gel column chromatography (*n*-hexane:ethyl acetate = 3:2) to obtain a title compound as yellow liquid (131 mg).
¹H-NMR (400 MHz, CDCl₃) δ 3.81 (3H, s), 4.04 (3H, s), 5.15 (1H, d, J = 2.4 Hz), 6.30 (1H, d, J = 2.4 Hz), 6.79 (1H, dd, J = 9.7, 1.8 Hz), 6.99 (1H, d, J = 9.7 Hz), 7.28 (1H, d, J = 7.9 Hz), 7.41 (1H, dd, J = 4.8, 3.0 Hz), 7.58 (1H, dd, J = 4.8, 1.2 Hz), 7.73 (1H, t, J = 7.9 Hz), 7.81 (1H, dd, J = 3.0,1.2 Hz), 8.04 (1H, d, J = 9.7 Hz), 8.05 (1H, s).

### <Reference Example 16-20>

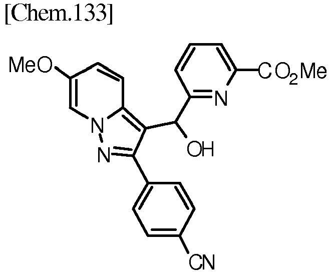

### Methyl 6-[[2-(4-cyanophenyl)-6-methoxypyrazolo[1,5-a]pyridin-3-yl](hydroxy)methyl]pyridine-2-c arboxylate

A methanol (0.5 mL) solution of sodium borohydride (11.0 mg) was added to a dichloromethane (2.5 mL) solution of methyl 6-[[2-(4-cyanophenyl)-6-methoxypyrazolo[1,5-a]pyridin-3-yl]carbonyl]pyridine-2-carboxyla te (100 mg) at 0°C, and then the mixture was stirred at 0°C for 1 hour. Water was added to the reaction mixture and the reaction mixture was extracted with ethyl acetate. The extract thus obtained was then washed with saturated saline and then dried over anhydrous sodium sulfate. The solvent was evaporated and the residue thus obtained was purified by silica gel column chromatography (*n*-hexane:ethyl acetate = 2:1) to obtain a title compound as a colorless crystal (45.8 mg).
¹H-NMR (400 MHz, CDCl₃) δ 3.83 (3H, s), 4.05 (3H, s), 5.26 (1H, d, J = 2.4 Hz), 6.19 (1H, d, J = 2.4 Hz), 6.84 (1H, dd, J = 9.7, 1.8 Hz), 7.01 (1H, d, J = 9.7 Hz), 7.22 (1H, d, J = 7.9 Hz), 7.70-7.75 (3H, m), 7.99 (2H, d, J = 7.9 Hz), 8.03 (1H, d, J = 7.9 Hz), 8.06 (1H, d, J =1.8 Hz).

### <Reference Example 16-21>

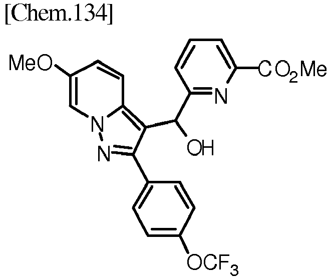

### Methyl 6-[hydroxy[6-methoxy-2-[4-(trifluoromethoxy)phenyl]pyrazolo[1,5-a]pyridin-3-yl]methyl]p yridine-2-carboxylate

After a methanol (1 mL) solution of sodium borohydride (39.0 mg) was added to a dichloromethane (8.5 mL) solution of methyl 6-[[6-methoxy-2-[4-(trifluoromethoxy)phenyl]pyrazolo[1,5-a]pyridin-3-yl]carbonyl]pyridine -2-carboxylate (400 mg) at 0°C, the mixture was stirred at 0°C for 1 hour. Water was added to the reaction solution and then the mixture was extracted with ethyl acetate. The extract was then washed with saturated saline and then dried over anhydrous sodium sulfate. The solvent was evaporated and the residue thus obtained was purified by silica gel column chromatography (n-hexane:ethyl acetate = 2:1) to obtain a title compound as a yellow foam (346 mg).
¹H-NMR (400 MHz, CDCl₃) δ 3.82 (3H, s), 4.04 (3H, s), 5.29 (1H, brs), 6.19 (1H, brs), 6.82 (1H, dd, J = 9.7, 1.8 Hz), 6.99 (1H, d, J = 9.7 Hz), 7.29 (2H, d, J = 7.9 Hz), 7.71 (1H, t, J = 7.9 Hz), 7.83-7.88 (2H, m), 8.02 (1H, d, J = 7.9 Hz), 8.06 (1H, d, J = 1.8 Hz).

### <Reference Example 16-22>

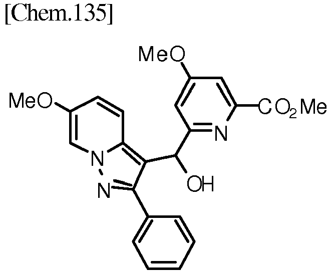

### Methyl 6-[hydroxy(6-methoxy-2-phenylpyrazolo[1,5-a]pyridin-3-yl)methyl]-4-methoxypyridine-2-c arboxylate

A methanol (0.72 mL) suspension of sodium borohydride (35.9 mg) was added to a dichloromethane (7.2 mL) solution of methyl 4-methoxy-6-[(6-methoxy-2-phenylpyrazolo[1,5-a]pyridin-3-yl)carbonyl]pyridine-2-carbox ylate (330 mg) at 0°C, and the mixture was stirred for 1 hour. A saturated ammonium chloride aqueous solution was slowly added to the reaction solution to achieve pH = 2 and then the mixture was purified by silica gel column chromatography (*n*-hexane:ethyl acetate = 5:1 to 1:1) to obtain a title compound as a colorless solid (299 mg).
¹H-NMR (400 MHz, CDCl₃) δ 3.74 (3H, s), 3.82 (3H, s), 4.03 (3H, s), 5.33 (1H, d, J = 3.0 Hz), 6.15 (1H, d, J = 3.0 Hz), 6.65 (1H, d, J = 2.4 Hz), 6.81 (1H, dd, J = 9.7, 1.8 Hz), 7.07 (1H, d, J = 9.7 Hz), 7.38-7.49 (3H, m), 7.56 (1H, d, J = 2.4 Hz), 7.80-7.83 (2H, m), 8.08 (1H, d, J = 1.8 Hz).

### <Reference Example 16-23>

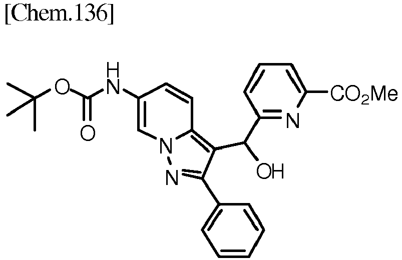

### Methyl 6-[[6-[(tert-butoxycarbonyl)amino]-2-phenylpyrazolo[1,5-a]pyridin-3-yl](hydroxy)methyl]p yridine-2-carboxylate

Sodium borohydride (20 mg) was added to a methanol solution (21 mL) of methyl 6-[[6-[(*tert*-butoxycarbonyl)amino]-2-phenylpyrazolo[1,5-a]pyridin-3-yl]carbonyl]pyridine-2-carboxylate (1.0 g) at 0°C under an argon atmosphere, and then the mixture was stirred at 0°C for 2 hours. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 2:1) to obtain a title compound as a colorless amorphous (229 mg).
¹H-NMR (400 MHz, CDCl₃) δ 1.30 (9H, s), 4.01 (3H, s), 5.57 (1H, s), 6.25 (1H, s), 6.82 (1H, t, J = 7.3 Hz), 7.07 (1H, d, J = 7.9 Hz), 7.41-7.47 (3H, m), 7.61-7.66 (3H, m), 7.73 (1H, t, J = 7.9 Hz), 8.03 (1H, d, J = 7.9 Hz), 8.27 (1H, d, J = 7.9 Hz), 8.95 (1H, s).

### <Reference Example 16-24>

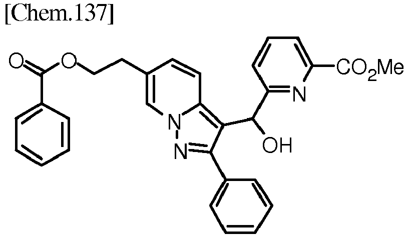

### Methyl 6-[[6-[2-(benzoyloxy)ethyl]-2-phenylpyrazolo[1,5-a]pyridin-3-yl](hydroxy)methyl]pyridine-2-carboxylate

A methanol solution (3.8 mL) of sodium borohydride (189 mg) was added to a dichloromethane solution (37.7 mL) of methyl 6-[[6-[2-(benzoyloxy)ethyl]-2-phenylpyrazolo[1,5-a]pyridin-3-yl]carbonyl]pyridine-2-carbo xylate (2.1 g) at room temperature under an argon atmosphere, and then the mixture was stirred at 0°C for 1 hour. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as a colorless amorphous (1.44 g).
¹H-NMR (400 MHz, CDCl₃) δ 3.05 (2H, t, J = 6.7 Hz), 4.04 (3H, s), 4.53 (2H, t, J = 6.7 Hz), 5.33 (1H, s), 6.23 (1H, s), 6.94 (1H, dd, J = 9.1, 1.2 Hz), 7.06 (1H, d, J = 9.1 Hz), 7.22 (1H, d, J = 7.9 Hz), 7.40-7.49 (5H, m), 7.56 (1H, tt, J = 7.3, 1.2 Hz), 7.69 (1H, t, J = 7.3 Hz), 7.80-7.84 (2H, m), 7.99 (2H, dd, J = 9.1, 1.2 Hz), 8.03 (1H, d, J = 7.9 Hz), 8.41 (1H, s).

### <Reference Example 17-1>

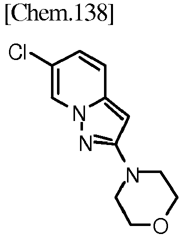

### 6-Chloro-2-(morpholin-4-yl)pyrazolo[1,5-a]pyridine

After 2-bromoethylether (0.35 mL) and diisopropylethylamine (0.6 mL) were added to an N,N-dimethylacetamide solution (1.7 mL) of 2-amino-6-chloropyrazolo[1,5-a]pyridine (278 mg) under an argon atmosphere, the mixture was heated and stirred at 110°C for 2 hours. The reaction solution was diluted with ethyl acetate, then washed with water and saturated saline, and then dried over anhydrous sodium sulfate. The solvent was evaporated and the residue thus obtained was purified by silica gel chromatography (ethyl acetate:n-hexane = 1:1) to obtain a title compound as a colorless crystal (340 mg).
¹H-NMR (400 MHz, CDCl₃) δ 3.33 (4H, t, J = 4.9 Hz), 3.86 (4H, t, J = 4.9 Hz), 5.80 (1H, s), 7.00 (1H, dd, J = 9.2,1.8 Hz), 7.20 (1H, d, J = 9.2 Hz), 8.28 (1H, s).

### <Reference Example 18-1>

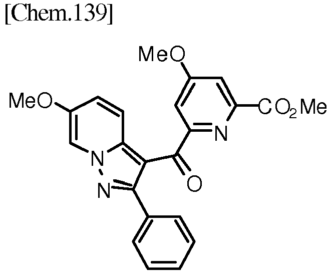

### Methyl 4-methoxy-6-[(6-methoxy-2-phenylpyrazolo[1,5-a]pyridin-3-yl)carbonyl]pyridine-2-carbox ylate

Thionyl chloride (1.08 mL) was added to a toluene (5.0 mL) suspension of 2-carboxy-4-methoxy-6-methoxycarbonylpyridine (336 mg), and then the mixture was heated under reflux for 4 hours. The reaction solution was evaporated under vacuum, then 1,4-dioxane (1.3 mL) and 6-methoxy-2-phenylpyrazolo[1,5-a]pyridine (336 mg) were added, and then the mixture was heated under reflux for 3 hours. The mixture was then cooled down to room temperature, then a saturated sodium bicarbonate aqueous solution was slowly added to the reaction solution to achieve pH = 9, and then the mixture was extracted with ethyl acetate. The organic layer was washed with saturated saline and then dried over anhydrous sodium sulfate. The solvent was evaporated under vacuum, then the residue was purified by silica gel column chromatography (*n*-hexane:ethyl acetate = 10:1 to 1:1), and then the crude product thus obtained was triturated with diisopropyl ether to obtain a title compound as a colorless solid (373 mg).
¹H-NMR (400 MHz, DMSO-d₆) δ 3.74 (3H, s), 3.87 (3H, s), 3.90 (3H, s), 7.06-7.16 (3H, m), 7.19 (2H, d, J = 7.3 Hz), 7.35 (1H, d, J = 2.4 Hz), 7.43 (1H, d, J = 2.4 Hz), 7.49 (1H, dd, J = 9.7, 2.4 Hz), 8.05 (1H, d, J = 9.7 Hz), 8.72 (1H, d, J = 2.4 Hz).

### <Reference Example 19-1>

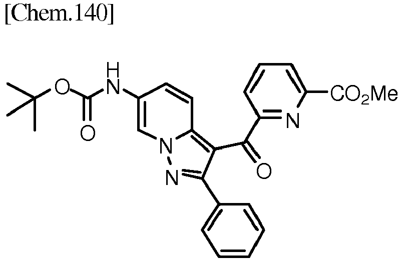

### Methyl 6-[[6-[(tert-butoxycarbonyl)amino]-2-phenylpyrazolo[1,5-a]pyridin-3-yl]carbonyl]pyridine-2-carboxylate

A 70% perchloric acid aqueous solution (6.4 mL) was added to a 1,4-dioxane solution (15 mL) of ethyl *O*-mesitylsulfonylacetohydroxamate (17.6 g) at 0°C under an argon atmosphere, and then the mixture was stirred at 0°C for 30 minutes. Ice water (170 mL) was added to the reaction mixture, the precipitated solid was filtered off, the solid thus obtained was dissolved in dichloromethane (52 mL), and the mixture was divided into layers. The organic layer was dried over anhydrous magnesium sulfate and filtered off. A dichloromethane solution (52 mL) of 3-[(*tert*-butoxycarbonyl)amino]pyridine (10 g) was added to the filtrate thus obtained at 0°C, the mixture was stirred at room temperature for 1 hour, and then the reaction mixture was evaporated to obtain 1-amino-3-[(*tert*-butoxycarbonyl)amino]pyridinium 2,4,6-trimethylbenzenesulfonate.
Potassium carbonate (14.2 g) and methyl 6-(1-oxo-3-phenyl-2-propyn-1-yl)pyridine-2-carboxylate (6.8 g) were added to a 1,4-dioxane solution (52 mL) of 1-amino-3-[(*tert*-butoxycarbonyl)amino]pyridinium 2,4,6-trimethylbenzenesulfonate at room temperature under an argon atmosphere, and then the mixture was stirred at room temperature for 1 hours. Water was added to the reaction mixture and the reaction mixture was extracted with ethyl acetate. The organic layer was washed with water and saturated saline in this order and then dried over anhydrous magnesium sulfate. The solvent was evaporated and then the residue thus obtained was purified by silica gel column chromatography (*n*-hexane:ethyl acetate = 2:1) to obtain a title compound as yellow liquid (2.59 g).
¹H-NMR (400 MHz, CDCl₃) δ 1.52 (9H, s), 4.01 (3H, s), 5.84 (1H, s), 7.23 (2H, dd, J = 8.5, 4.8 Hz), 7.44 (2H, dd, J = 7.9, 2.4 Hz), 7.86 (1H, dd, J = 7.9, 1.2 Hz), 7.93 (1H, t, J = 7.9 Hz), 8.11 (1H, dd, J = 7.3,1.2 Hz), 8.27 (2H, dd, J = 4.8,1.2 Hz), 8.46 (2H, d, J = 2.4 Hz).

### <Reference Example 20-1>

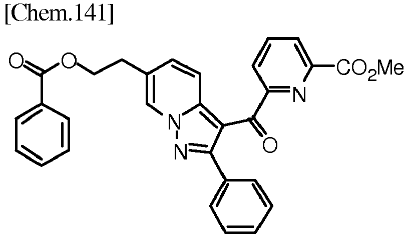

### Methyl 6-[[6-[2-(benzoyloxy)ethyl]-2-phenylpyrazolo[1,5-a]pyridin-3-yl]carbonyl]pyridine-2-carbo xylate

A 70% perchloric acid aqueous solution (3.4 mL) was added to a 1,4-dioxane solution (8.3 mL) of ethyl *O*-mesitylsulfonylacetohydroxamate (9.4 g) at 0°C under an argon atmosphere, and then the mixture was stirred at 0°C for 30 minutes. Ice water (90 mL) was added to the reaction solution, the precipitated solid was filtered off, the obtained solid was dissolved in dichloromethane (28 mL), and the solution was divided into layers. The organic layer was dried over anhydrous magnesium sulfate and filtered off. A dichloromethane solution (28 mL) of 2-(pyridin-3-yl)ethanol (3.4 g) was added to the obtained filtrate at 0°C, the mixture was stirred at room temperature for 1 hour, and then the reaction solution was evaporated to obtain 1-amino-3-[1-(2-hydroxyethyl)]pyridinium 2,4,6-trimethylbenzenesulfonate.
Potassium carbonate (7.6 g) and methyl 6-(1-oxo-3-phenyl-2-propyn-1-yl)pyridine-2-carboxylate (3.7 g) were added to a 1,4-dioxane solution (55.2 mL) of 1-amino-3-[1-(2-hydroxyethyl)]pyridinium 2,4,6-trimethylbenzenesulfonate at room temperature under an argon atmosphere, and then the mixture was stirred at room temperature for 16 hours. Water was added to the reaction solution and the mixture was extracted with ethyl acetate. The organic layer was washed with water and saturated saline in this order and then dried over anhydrous magnesium sulfate. The solvent was evaporated and then the residue was purified by silica gel column chromatography (*n*-hexane:ethyl acetate = 2:1 1 to 0:1) to obtain a yellow powder (3.52 g).
Benzoyl chloride (1.2 mL) and pyridine (2.1 mL) were added to a dichloromethane solution (44 mL) of the above-described yellow powder (3.5 g) at 0°C under an argon atmosphere, and then the mixture was stirred at 0°C for 1 hour. Water was added to the reaction solution and the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as a yellow powder (2.18 g).
¹H-NMR (400 MHz, CDCl₃) δ 3.20 (2H, t, J = 6.4 Hz), 3.85 (3H, s), 4.64 (2H, t, J = 6.4 Hz), 7.03 (2H, tt, J = 7.3, 1.2 Hz), 7.11 (1H, tt, J = 7.3, 1.2 Hz), 7.21-7.24 (2H, m), 7.44 (2H, tt, J = 7.9, 1.8 Hz), 7.51 (1H, dd, J = 9.1, 1.8 Hz), 7.56 (1H, tt, J = 7.9, 1.8 Hz), 7.84 (1H, t, J = 7.9 Hz), 7.93 (1H, dd, J = 7.9, 1.2 Hz), 7.98 (1H, dd, J = 7.9, 1.2 Hz), 7.99-8.03 (2H, m), 8.37 (1H, d, J = 9.1 Hz), 8.53 (1H, s).

### <Reference Example 21-1>

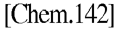

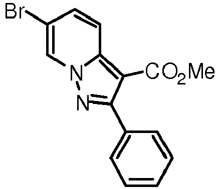

### Methyl 6-bromo-2-phenylpyrazolo[1,5-a]pyridine-3-carboxylate

A 70% perchloric acid aqueous solution (12.9 mL) was added to a 1,4-dioxane solution (31 mL) of ethyl *O*-mesitylsulfonylacetohydroxamate (35.7 g) under an argon atmosphere under ice-cooling, and then the mixture was stirred for 30 minutes under ice-cooling. Ice water (360 mL) was added to the reaction solution, the precipitated solid was filtered off, the obtained solid was dissolved in dichloromethane (104 mL), and the solution was divided into layers. The organic layer was dried over anhydrous magnesium sulfate and filtered off. A dichloromethane solution (104 mL) of 3-bromopyridine (10 mL) was added to the obtained filtrate under ice-cooling, the mixture was stirred at room temperature for 1 hour, and the reaction solution was evaporated to obtain a crude product *N*-amino-3-bromopyridinium 2,4,6-trimethylbenzenesulfonate.
Methyl phenylpropiolate (7.7 mL) and potassium carbonate (28.7 g) were added to an *N,N*-dimethylformamide solution (104 mL) of the crude product *N*-amino-3-bromopyridinium 2,4,6-trimethylbenzenesulfonate at room temperature under an argon atmosphere, and then the mixture was stirred at room temperature for 16 hours. Water was added to the reaction solution and the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as a yellow powder (8.0 g).
¹H-NMR (400 MHz, CDCl₃) δ 3.84 (3H, s), 7.43-7.51 (4H, m), 7.74-7.79 (2H, m), 8.12 (1H, d, J = 9.1 Hz), 8.68 (1H, s).

The following Reference Examples 22-1 to 22-8 were obtained by using corresponding pyridine derivatives in the similar manner as Reference Example 15-7.

### <Reference Example 22-1>

### Methyl 6-[(6-chloro2-cyclopropylpyrazolo[1,5-a]pyridin-3-yl)carbonyl]pyridine-2-carboxylate

¹H NMR (400 MHz, CDCl₃) δ 0.81-0.85 (2H, m), 1.02-1.06 (2H, m), 2.04-2.11 (1H, m), 3.98 (3H, s), 7.31(1H, dd, J = 9.2, 1.8 Hz), 7.87 (1H, d, J = 9.2 Hz), 8.08 (1H, dd, J = 7.6, 7.3 Hz), 8.13 (1H, dd, J = 7.6, 1.2 Hz), 8.31 (1H, dd, J = 7.3, 1.2 Hz), 8.44 (1H, d, J = 1.8 Hz).

### <Reference Example 22-2>

### Methyl 6-[(6-chloro-2-cyclopentylpyrazolo[1,5-a]pyridin-3-yl)carbonyl]pyridine-2-carboxylate

¹H NMR (400 MHz, CDCl₃) δ 1.55-1.59 (2H, m), 1.79-1.88 (4H, m), 1.93-2.01 (2H, m), 3.45-3.53 (1H, m), 3.97 (3H, s), 7.28 (1H, dd, J = 9.8,1.8 Hz), 7.76 (1H, d, J = 9.8 Hz), 8.08 (1H, dd, J = 7.9, 7.3 Hz), 8.15 (1H, dd, J = 7.9, 1.2 Hz), 8.32 (1H, dd, J = 7.3, 1.2 Hz), 8.53 (1H,d,J=1.8Hz).

### <Reference Example 22-3>

### Methyl 6-[(6-chloro-2-cyclohexylpyrazolo[1,5-a]pyridin-3-yl)carbonyl]pyridine-2-carboxylate

¹H NMR (400 MHz, CDCl₃) δ 1.08-1.12 (2H, m), 1.23-1.27 (1H, m), 1.75-1.77 (2H, m), 1.97-2.01 (2H, m), 2.83-2.89 (1H, m), 3.97 (3H, s), 7.31 (1H, dd, J = 9.8, 1.8 Hz), 7.85 (1H, d, J = 9.8 Hz), 8.09 (1H, dd, J = 7.9, 7.3 Hz), 8.13 (1H, dd, J = 7.9,1.8 Hz), 8.33 (1H, dd, J = 7.3, 1.8 Hz), 8.54 (1H, d, J = 1.8 Hz).

### <Reference Example 22-4>

### Methyl 6-[(2-tert-butyl-6-chloropyrazolo[ 1,5-a]pyridin-3-yl)carbonyl]pyridine-2-carboxylate

¹H NMR (400 MHz, CDCl₃) δ 1.53 (9H, s), 3.93 (3H, s), 6.79 (1H, d, J = 9.7 Hz) 7.05 (dd, J = 9.7, 1.8 Hz), 8.06 (1H, dd, J = 7.9, 7.3 Hz), 8.13 (1H, dd, J = 7.9, 1.2 Hz), 8.30 (1H, dd, J = 7.3, 1.2 Hz), 8.51 (1H,d,J=1.8Hz).

### <Reference Example 22-5>

### Methyl 6-[[6-chloro-2-(2-methyl-propyl)pyrazolo[1,5-a]pyridin-3-yl]carbonyl]pyridine-2-carboxylat e

¹H NMR (400 MHz, CDCl₃) δ 0.82 (6H, d, J = 6.7 Hz), 1.83-1.94 (1H, m), 2.80 (2H, d, J = 6.7 Hz), 3.98 (3H, s), 7.32 (1H, dd, J = 9.2, 1.8 Hz), 7.92 (1H, d, J = 9.2 Hz), 8.08 (1H, dd, J = 7.9, 7.3 Hz), 8.14 (1H, dd, J = 7.3, 1.2 Hz), 8.32 (1H, dd, J = 7.9, 1.2 Hz), 8.53 (1H, d, J = 1.8 Hz).

### < Reference Example 22-6>

### Methyl 6-[[2-(2-fluorophenyl)-6-methoxypyrazolo[1,5-a]pyridin-3-yl]carbonyl]pyridine-2-carboxyla te

¹H NMR (400 MHz, DMSO-d₆) δ 3.75 (3H, s), 3.91 (3H, s), 6.80 (1H, t, J = 9.7 Hz), 7.02 (1H, td, J = 7.3, 1.2 Hz), 7.15-7.23 (1H, m), 7.35 (1H, td, J = 7.3, 1.6 Hz), 7.52 (1H, dd, J = 9.7,2.4 Hz), 7.90 (1H, dd, J = 7.9, 1.2 Hz), 7.98 (1H, dd, J = 7.9, 1.2 Hz), 8.04 (1H, t, J = 7.3 Hz), 8.14 (1H, d, J = 9.7 Hz), 8.75 (1H, d, J = 1.8 Hz).

### <Reference Example 22-7>

### Methyl 6-[[2-(3-fluorophenyl)-6-methoxypyrazolo[1,5-a]pyridin-3-yl]carbonyl]pyridine-2-carboxyla te

¹H NMR (400 MHz, DMSO-d₆) δ 3.75 (3H, s), 3.91 (3H, s), 6.93-7.10 (4H, m), 7.53 (1H, dd, J = 9.7, 2.4 Hz), 7.92 (1H, dd, J = 7.9, 1.2 Hz), 7.98 (1H, dd, J = 7.9, 1.2 Hz), 8.04 (1H, t, J = 7.9 Hz), 8.12 (1H, d, J = 9.1 Hz), 8.74 (1H, d, J =1.8 Hz).

### <Reference Example 22-8>

### Methyl 6-[(6-chloro-5-methyl-2-phenylpyrazolo[1,5-a]pyridin-3-yl)carbonyl]pyridine-2-carboxylate

¹H NMR (400 MHz, CDCl₃) δ 2.56 (3H, d, J = 1.2 Hz), 3.86 (3H, s), 7.01-7.06 (2H, m), 7.09-7.14 (1H, m), 7.19-7.22 (2H, m), 7.84 (1H, t, J = 7.9 Hz), 7.93 (1H, dd, J = 7.9, 1.2 Hz), 7.97 (1H, dd, J = 7.9, 1.2 Hz), 8.30 (1H, d, J = 1.2 Hz), 8.62 (1H, s).

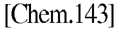

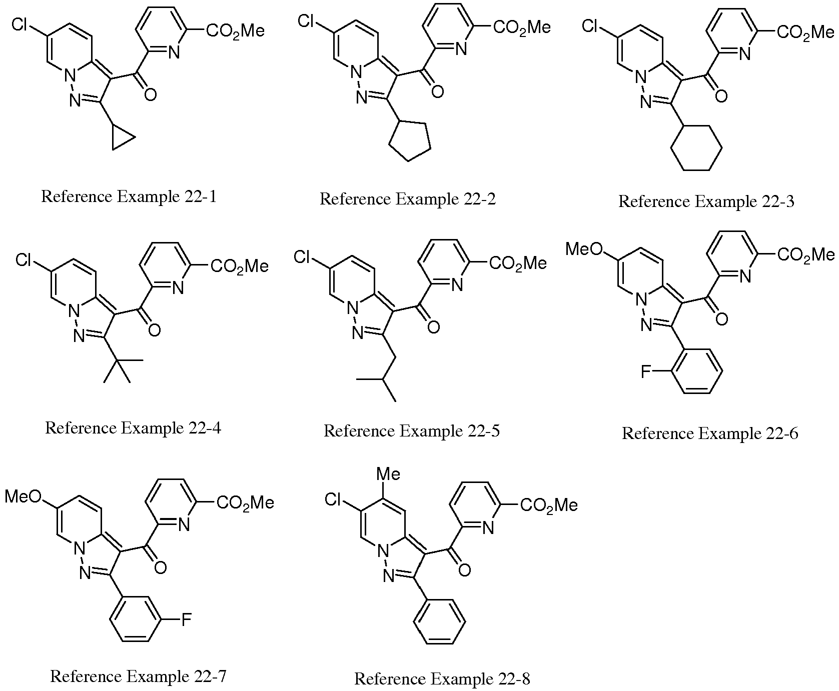

### <Reference Example 23-1>

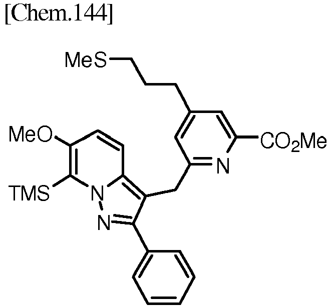

### Methyl 6-[[6-methoxy-2-phenyl-7-(trimethylsilyl)pyrazolo[1,5-a]pyridin-3-yl]methyl]-4-[3-(methylt hio)propyl]pyridine-2-carboxylate

After tetrahydrofuran (2.7 mL) solution of 3-bromo-6-methoxy-2-phenyl-7-(trimethylsilyl)pyrazolo[1,5-a]pyridine (200 mg) was cooled down to -78°C under an argon atmosphere, *n*-butyllithium (0.39 mL, a 1.65 mol/L n-hexane solution) was added, and then the mixture was stirred at -78°C for 30 minutes. Next, methyl 6-formyl-4-[3-(methylthio)propyl]pyridine-2-carboxylate (202 mg) was added at -78°C and then the mixture was stirred at room temperature for 30 minutes. A saturated ammonium chloride aqueous solution was added to the reaction solution, and then the mixture was extracted with ethyl acetate. The organic layer was washed with saturated saline and then dried over anhydrous sodium sulfate. The solvent was evaporated under vacuum, then a dichloromethane (1.4 mL) solution of the residue was ice-cooled, and then triethylsilane (0.217 mL) and trifluoroacetic acid (0.202 mL) were added. The solution was heated up to room temperature, stirred for 45 minutes, and then ice-cooled again. Next, a saturated sodium bicarbonate aqueous solution was slowly added to achieve pH = 9. The organic solvent was evaporated under vacuum and the residue was purified by silica gel column chromatography (*n*-hexane:ethyl acetate = 5:1 to 2:1) to obtain a title compound as a colorless oil (30.8 mg).
¹H NMR (400 MHz, CDCl₃) δ 0.55 (9H, s), 1.71-1.79 (2H, m), 1.97 (3H, s), 2.34 (2H, t, J = 7.3 Hz), 2.60 (2H, t, J = 7.9 Hz), 3.81 (3H, s), 4.04 (3H, s), 4.56 (2H, s), 6.95 (1H, s), 6.96 (1H, d, J = 9.7 Hz), 7.29-7.34 (2H, m), 7.38 (2H, t, J = 7.6 Hz), 7.74-7.82 (3H, m).

### <Reference Example 24-1>

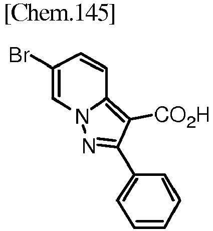

### 6-Bromo-2-phenylpyrazolo[1,5-a]pyridine-3-carboxylic acid

A 2 mol/L potassium hydroxide aqueous solution (60 mL) was added to a methanol solution (121 mL) of methyl 6-bromo-2-phenylpyrazolo[1,5-a]pyridine-3-carboxylate (8.0 g) at room temperature under an argon atmosphere, and then the mixture was stirred at 60°C for 1 hour. A 1 mol/L hydrochloric acid (120 mL) was added to the reaction solution and the precipitated solid was filtered off to obtain a title compound as a colorless powder (6.4 g).
¹H-NMR (400 MHz, DMSO-d₆) δ 7.42-7.47 (3H, m), 7.70 (1H, dd, J = 9.1, 1.8 Hz), 7.73-7.76 (2H, m), 8.09 (1H, d, J = 9.1 Hz), 9.27 (1H, s), 12.57 (1H, s).

### <Reference Example 25-1>

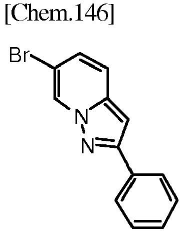

### 6-Bromo-2-phenylpyrazolo[1,5-a]pyridine

An *o*-dichlorobenzene solution (40 mL) of 6-bromo-2-phenylpyrazolo[1,5-a]pyridine-3-carboxylic acid (6.3 g) was stirred at 160°C for 7 hours under an argon atmosphere. The reaction solution was purified by silica gel chromatography (n-hexane:ethyl acetate = 1:0 to 2:1) to obtain a title compound as a pale yellow powder (4.6 g).
¹H-NMR (400 MHz, CDCl₃) δ 6.82 (1H, s), 7.17 (1H, dd, J = 9.7, 1.8 Hz), 7.39 (1H, d, J = 7.3 Hz), 7.42 (1H, d, J = 9.7 Hz), 7.45 (2H, t, J = 7.3 Hz), 7.94 (2H, d, J = 7.3 Hz), 8.63 (1H, s).

### <Reference Example 26-1>

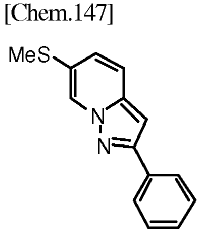

### 6-(Methylthio)-2-phenylpyrazolo[1,5-a]pyridine

Sodium thiomethoxide (462 mg) and copper (I) iodide (209 mg) were added to an *N,N*-dimethylformamide solution (11 mL) of 6-bromo-2-phenylpyrazolo[1,5-a]pyiidine (1.5 g) at room temperature under an argon atmosphere, and then the mixture was stirred at 150°C for 6 hours. The reaction solution was filtered by Celite, then water was added, and the solution was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as a pale yellow powder (812 mg).
¹H-NMR (400 MHz, CDCl₃) δ 2.50 (3H, s), 6.78 (1H, s), 7.09 (1H, dd, J = 9.1, 1.8 Hz), 7.38 (1H, d, J = 7.3 Hz), 7.42-7.47 (3H, m), 7.94 (2H, d, J = 7.3 Hz), 8.41 (1H, s).

### <Reference Example 27-1>

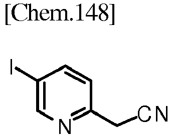

### 5-Iodo-2-pyridineacetonitrile

*n*-Butyllithium (21.0 mL, a 1.6 mol/L *n*-hexane solution) was diluted with tetrahydrofuran (75 mL) under an argon atmosphere, then acetonitrile (1.75 mL) was added at -78°C over 10 minutes, and then the mixture was stirred at -78°C for 45 minutes. A tetrahydrofuran (25 mL) solution of 2-fluoro-5-iodopyridine (3.35 g) was added to the reaction solution, then the mixture was stirred at -78°C for 2 hours, and then the mixture was gradually heated up to room temperature. A saturated ammonium chloride aqueous solution was added to the reaction solution and then the mixture was extracted with ethyl acetate, washed with saturated saline, and then dried over anhydrous sodium sulfate. The solvent was evaporated and the residue thus obtained was purified by silica gel chromatography (ethyl acetate:n-hexane = 1:1) to obtain a title compound as a brown crystal (3.35 g).
¹H-NMR (400 MHz, CDCl₃) δ 3.88 (2H, s), 7.25 (1H, d, J = 7.9 Hz), 8.05 (1H, dd, J = 7.9, 1.8Hz), 8.79(1H,d,J=1.8Hz).

### <Reference Example 27-2>

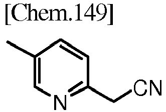

### 5-Methyl-2-pyridineacetonitrile

*n*-Butyllithium (21.0 mL, a 1.6 mol/L *n*-hexane solution) was diluted with tetrahydrofuran (75 mL) under an argon atmosphere, then acetonitrile (1.75 mL) was added at -78°C over 10 minutes, and then the mixture was stirred at -78°C for 45 minutes. A tetrahydrofuran (25 mL) solution of 2-fluoro-5-methylpyridine (1.67 g) was added to the reaction mixture, then the reaction mixture was stirred at -78°C for 2 hours, and then it was gradually heated up to room temperature. A saturated ammonium chloride aqueous solution was added to the reaction mixture and then the mixture was extracted with ethyl acetate, washed with saturated saline, and dried over anhydrous sodium sulfate. The solvent was evaporated and the residue thus obtained was purified by silica gel chromatography (ethyl acetate:n-hexane = 1:1) to obtain a title compound as a colorless crystal (1.05 g).
¹H-NMR (400 MHz, CDCl₃) δ 2.05 (3H, s), 3.90 (2H, s), 7.32 (1H, d, J = 7.9 Hz), 7.54 (1H, dd, J = 7.9,1.8 Hz), 8.40 (1H, d, J =1.8 Hz).

### <Reference Example 28-1>

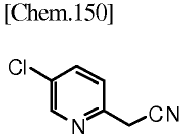

### 5-Chloro-2-pyridineacetonitrile

*tert*-Butyl cyanoacetate (25.7 mL) was added to a dimethylsulfoxide (160 mL) suspension of sodium hydride (7.20g, a 60%-oil suspension) at room temperature for 1 hour under an argon atmosphere, and then the mixture was stirred at room temperature for 0.5 hours. A dimethylsulfoxide (7 mL) solution of 2,5-dichloropyridine (10.0 g) was added to the reaction mixture, and then the reaction mixture was stirred at 120°C for 3 hours. The reaction mixture was cooled and then poured into a saturated ammonium chloride aqueous solution, the precipitated solid was filtered off, and the product was washed with water and ethanol to obtain *tert*-butyl 2-(5-chloropyridin-2-yl)-2-cyanoacetate.
The *tert*-butyl 2-(5-chloropyridin-2-yl)-2-cyanoacetate thus obtained was dissolved in acetonitrile (160 mL), *p*-toluenesulfonic acid (6.43 g) was added, and then the mixture was heated under reflux for 1 hour. The reaction mixture was diluted with ethyl acetate, washed with water and saturated saline, and then dried over anhydrous sodium sulfate. The solvent was evaporated and the residue thus obtained was purified by silica gel chromatography (*n*-hexane:ethyl acetate = 3:1) to obtain a title compound as a yellow oil (6.35 g).
¹H-NMR (400 MHz, CDCl₃) δ 3.93 (2H, s), 7.40 (1H, d, J = 7.9 Hz), 7.72 (1H, dd, J = 7.9, 2.4 Hz), 8.55 (1H, d, J = 2.4 Hz).

### <Reference Example 28-2>

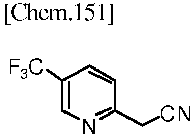

### 5-(Trifluoromethyl)-2-pyridineacetonitrile

*tert*-Butyl cyanoacetate (21.3 mL) was added to a dimethylsulfoxide (130 mL) suspension of sodium hydride (5.80 g, a 60%-oil suspension) at room temperature over 1 hour under an argon atmosphere, and then the mixture was stirred at room temperature for 0.5 hours. A dimethylsulfoxide (5 mL) solution of 5-(trifluoromethyl)-2-chloropyridine (10.0 g) was added to the reaction solution, and then the mixture was stirred at 120°C for 3 hours. The reaction solution was cooled and then poured into a saturated ammonium chloride aqueous solution, the precipitated solid was filtered off, and the product was washed with water and ethanol to obtain *tert*-butyl 2-cyano-2-[5-(trifluoromethyl)pyridin-2-yl] acetate.
The *tert*-butyl 2-cyano-2-[5-(trifluoromethyl)pyridin-2-yl]acetate thus obtained was dissolved in acetonitrile (130 mL), *p*-toluenesulfonic acid (5.24 g) was added, and then the mixture was heated under reflux for 1 hour. The reaction solution was diluted with ethyl acetate, washed with water and saturated saline, and then dried over anhydrous sodium sulfate. The solvent was evaporated and the residue thus obtained was purified by silica gel chromatography (*n*-hexane:ethyl acetate = 3:1) to obtain a title compound as a pale yellow crystal (8.30 g).
¹H-NMR (400 MHz, CDCl₃) δ 4.04 (2H, s), 7.60 (1H, d, J = 8.5 Hz), 8.00 (1H, d, J = 8.5 Hz), 8.87 (1H, s).

### <Reference Example 29-1>

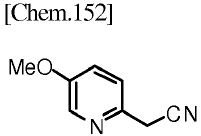

### 5-Methoxy-2-pyridineacetonitrile

After sodium cyanide (4.85 g) was added to a dimethylsulfoxide (125 mL) solution of 2-(chloromethyl)-5-methoxypyridine (7.80 g) at room temperature under an argon atmosphere, and then the mixture was stirred at room temperature for 2 hours. Ice water was added to the reaction solution and then the mixture was extracted with ethyl acetate, washed with saturated saline, and dried over anhydrous sodium sulfate. The solvent was evaporated and the residue thus obtained was purified by silica gel chromatography (*n*-hexane:ethyl acetate = 3:2) to obtain a title compound as a yellow oil (6.77 g).
¹H-NMR (400 MHz, CDCl₃) δ 3.87 (3H, s), 3.88 (2H, s), 7.22 (1H, dd, J = 8.6, 3.1 Hz), 7.34 (1H, d, J = 8.6 Hz), 8.26 (1H, d, J = 3.1 Hz)

### <Reference Example 30-1>

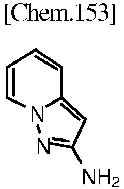

### 2-Aminopyrazolo[1,5-a]pyridine

A 70% perchloric acid aqueous solution (0.27 mL) was added to a 1,4-dioxane solution (1.3 mL) of ethyl *O*-mesitylsulfonylacetohydroxamate (736 mg) under an argon atmosphere under ice-cooling, and then the mixture was stirred for 30 minutes under ice-cooling. Ice water (3 mL) was added to the reaction solution, the precipitated solid was filtered off, the obtained solid was dissolved in dichloromethane (12 mL), and the solution was divided into layers. The organic layer was dried over anhydrous magnesium sulfate and filtered off. A dichloromethane solution (12 mL) of pyridine-2-acetonitrile (200 mg) was added to the obtained filtrate under ice-cooling, the mixture was stirred at room temperature for 1 hour, and the reaction solution was evaporated to obtain 1-amino-(2-cyanomethyl)-1-pyridinium 2,4,6-trimethylbenzenesulfonate.
Potassium carbonate (720 mg) was added to a methanol solution (8.5 mL) of 1-amino-(2-cyanomethyl)-1-pyridinium 2,4,6-trimethylbenzenesulfonate at 0°C under an argon atmosphere, and then the mixture was stirred at room temperature for 2 hours. Water was added to the reaction solution and the mixture was extracted with ethyl acetate. The organic layer was washed with water and saturated saline in this order, and then dried over anhydrous magnesium sulfate. The solvent was evaporated and then the residue was purified by silica gel column chromatography (*n*-hexane:ethyl acetate = 2:1) to obtain a title compound as a yellow solid (139 mg).
¹H-NMR (400 MHz, DMSO-d₆) δ 5.22 (2H, s), 5.60 (1H, s), 6.47 (1H, td, J = 6.7, 1.2 Hz), 6.96 (1H, td, J = 6.7, 1.2 Hz), 7.22 (1H, dd, J = 6.7,1.2 Hz), 8.22 (1H, dd, J = 6.7, 1.2 Hz).

The following Reference Examples 30-2 to 30-7 were obtained by using corresponding pyridine-2-acetonitrile derivatives in the similar manner as Reference Example 30-1.

### <Reference Example 30-2>

### 2-Amino-6-methylpyrazolo[1,5-a]pyridine

¹H-NMR (400 MHz, DMSO-d₆) δ 2.17 (3H, d, J = 1.2 Hz), 5.11 (2H, s), 5.53 (1H, s), 6.84 (1H, dd, J = 8.7, 1.2 Hz), 7.15 (1H, d, J = 8.7 Hz), 8.06 (1H, s)

### <Reference Example 30-3>

### 2-Amino-6-bromopyrazolo[ 1,5-a]pyridine

¹H-NMR (400 MHz, DMSO-d₆) δ 5.38 (2H, s), 5.67 (1H, s), 7.08 (1H, dd, J = 9.1, 1.8 Hz), 7.22 (1H, d, J = 9.1 Hz), 8.56 (1H, d, J = 1.8 Hz)

### <Reference Example 30-4>

### 2-Amino-6-iodopyrazolo[ 1,5-a]pyridine

¹H-NMR (400 MHz, DMSO-d₆) δ 5.36 (2H, s), 5.64 (1H, s), 7.10 (1H, d, J = 9.1 Hz), 7.15 (1H, d, J = 9.1 Hz), 8.55 (1H, s)

### <Reference Example 30-5>

### 2-Amino-6-(trifluoromethyl)pyrazolo[1,5-a]pyridine

¹H-NMR (400 MHz, DMSO-d₆) δ 5.66 (2H, s), 5.80 (1H, s), 7.17 (1H, dd, J = 9.2, 1.2 Hz), 7.39 (1H, d, J = 9.2 Hz), 8.78 (1H, d, J =1.2 Hz).

### <Reference Example 30-6>

### 2-Amino-6-chloropyrazolo[1,5-a]pyridine

¹H-NMR (400 MHz, DMSO-d₆) δ 5.37 (2H, brs), 5.68 (1H, s), 7.02 (1H, dd, J = 9.7, 1.8 Hz), 7.27 (1H, d, J = 9.7 Hz), 8.51 (1H, d, J = 1.8 Hz).

### <Reference Example 30-7>

### 2-Amino-6-methoxypyrazolo[1,5-a]pyridine

¹H-NMR (400 MHz, DMSO-d₆) δ 3.72 (3H, s), 5.03 (2H, s), 5.54 (1H, s), 6.81 (1H, dd, J = 9.2, 1.8 Hz), 7.17 (1H, d, J = 9.2 Hz), 7.97 (1H, s)

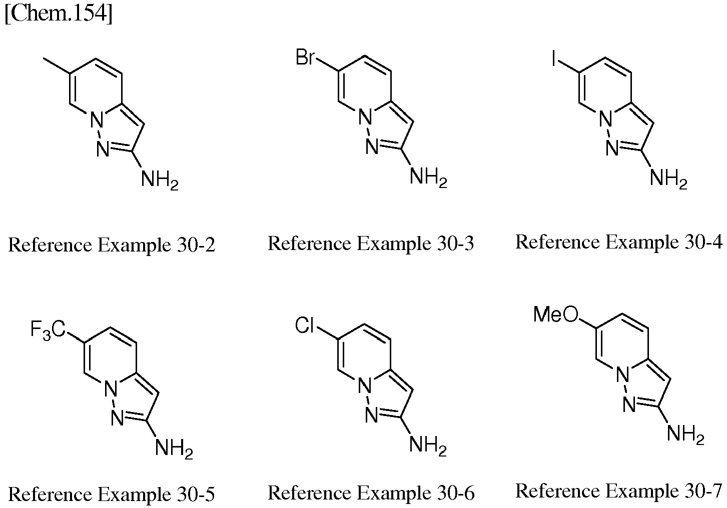

### <Reference Example 31-1>

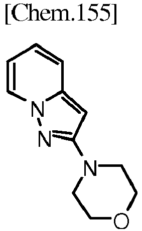

### 2-(Morpholin-4-yl)pyrazolo[1,5-a]pyridine

After 2-bromoethylether (0.21 mL) and diisopropylethylamine (0.36 mL) were added to an *N,N*-dimethylacetamide solution (1.0 mL) of 2-aminopyrazolo[1,5-a]pyiidine (134 mg) under an argon atmosphere, the mixture was stirred at 110°C for 2 hours. The reaction solution was diluted with ethyl acetate, then washed with water and saturated saline, and then dried over anhydrous sodium sulfate. The solvent was evaporated and the residue thus obtained was purified by silica gel chromatography (ethyl acetate:n-hexane = 1:1) to obtain a title compound as a colorless crystal (135 mg).
¹H-NMR (400 MHz, CDCl₃) δ 3.34 (4H, t, J = 4.8 Hz), 3.86 (4H, t, J = 4.8 Hz), 5.78 (1H, s), 6.54 (1H, t, J = 6.7 Hz), 7.01 (1H, t, J = 6.7 Hz), 7.25 (1H, d, J = 6.7 Hz), 8.21 (1H, d, J = 6.7 Hz).

The following Reference Examples 31-2 to 31-12 were obtained by using corresponding 2-aminopyrazolo[1,5-a]pyridine derivatives in the similar manner as Reference Example 31-1.

### <Reference Example 31-2>

### 6-Methyl-2-(morpholin-4-yl)pyrazolo[1,5-a]pyridine

¹H-NMR (400 MHz, CDCl₃) δ 2.26 (3H, s), 3.32 (4H, t, J = 4.8 Hz), 3.86 (4H, t, J = 4.8 Hz), 5.72 (1H, s), 6.87 (1H, dd, J = 9.1, 1.2 Hz), 7.17 (1H, d, J = 9.1 Hz), 8.03 (1H, s).

### <Reference Example 31-3>

### 6-Bromo-2-(morpholin-4-yl)pyrazolo[1,5-a]pyridine

¹H-NMR (400 MHz, CDCl₃) δ 3.33 (4H, t, J = 4.8 Hz), 3.85 (4H, t, J = 4.8 Hz), 5.79 (1H, s), 7.07 (1H, dd, J = 9.1,1.8 Hz), 7.15 (1H, d, J = 9.1 Hz), 8.36 (1H, s).

### <Reference Example 31-4>

### 6-Iodo-2-(morpholin-4-yl)pyrazolo[1,5-a]pyridine

¹H-NMR (400 MHz, CDCl₃) δ 3.32 (4H, t, J = 4.8 Hz), 3.85 (4H, t, J = 4.8 Hz), 5.78 (1H, s), 7.05 (1H, d, J = 9.1 Hz), 7.17 (1H, dd, J = 9.1, 1.2 Hz), 8.47 (1H, s).

### <Reference Example 31-5>

### 2-(Morpholin-4-yl)-6-(trifluoromethyl)pyrazolo[1,5-a]pyridine

¹H-NMR (400 MHz, CDCl₃) δ 3.36 (4H, t, J = 4.8 Hz), 3.86 (4H, t, J = 4.8 Hz), 5.88 (1H, s), 7.14 (1H, dd, J = 9.1,1.2 Hz), 7.32 (1H, d, J = 9.1 Hz), 8.55 (1H, s).

### <Reference Example 31-6>

### 6-Methoxy-2-(morpholin-4-yl)pyrazolo[1,5-a]pyridine

¹H-NMR (400 MHz, CDCl₃) δ 3.31 (4H, t, J = 4.9 Hz), 3.79 (3H, s), 3.87 (4H, t, J = 4.9 Hz), 5.73 (1H, s), 6.87 (1H, dd, J = 9.8, 1.8 Hz), 7.18 (1H, d, J = 9.8 Hz), 7.94 (1H, s).

### <Reference Example 31-7>

### 6-Chloro-2-(pyrrolidin-1-yl)pyrazolo[1,5-a]pyridine

¹H NMR (400 MHz, CDCl₃) δ 1.97-2.04 (4H, m), 3.37-3.40 (4H, m), 5.62 (1H, s), 6.94 (1H, dd, J = 9.1, 1.8 Hz), 7.13 (1H, d, J = 9.1 Hz), 8.27 (1H, d, J =1.8 Hz).

### <Reference Example 31-8>

### 6-Chloro-2-(piperidin-1-yl)pyrazolo[1,5-a]pyridine

¹H NMR (400 MHz, CDCl₃) δ 1.60-1.62 (2H, m), 1.67-1.71 (4H, m), 3.31-3.32 (4H, m), 5.78 (1H,s), 6.94 (1H, dd, J = 9.2, 1.8 Hz), 7.15 (1H, d, J = 9.2 Hz), 8.24 (1H, d, J =1.8 Hz).

### <Reference Example 31-9>

### 6-Chloro-2-(hexahydro-1H-azepin-1-yl)pyrazolo[1,5-a]pyridine

¹H NMR (400 MHz, CDCl₃) δ 1.62-1.66 (4H, m), 1.84-1.86 (4H, m), 3.55-3.56 (4H, m), 5.68 (1H, s), 6.97 (1H, dd, J = 9.1, 1.8 Hz), 7.15 (1H, d, J = 9.1 Hz), 8.30 (1H, d, J =1.8 Hz).

### <Reference Example 31-10>

### 6-Chloro-2-(dimethylamino)pyrazolo[1,5-a]pyridine

¹H NMR (400 MHz, CDCl₃) δ 2.98 (6H, s), 5.72 (1H, s), 6.95 (1H, dd, J = 9.1, 1.8 Hz), 7.15 (1H, d, J = 9.1 Hz), 8.26 (1H, d, J =1.8 Hz).

### <Reference Example 31-11>

### 6-Chloro-2-[bis(2-hydroxyethyl)amino]pyrazolo[1,5-a]pyridine

¹H NMR (400 MHz, CDCl₃) δ 3.16 (2H, s), 3.59-3.60 (4H, m), 3.90-3.91 (4H, m), 5.70 (1H, s), 6.97 (1H, dd, J = 9.7, 1.8 Hz), 7.14 (1H, d, J = 9.7 Hz), 8.21 (1H, d, J =1.8 Hz).

### <Reference Example 31-12>

### 6-Chloro-2-(thiomorpholin-4-yl)pyrazolo[1,5-a]pyridine

¹H NMR (400 MHz, CDCl₃) δ 2.67-2.69 (4H, m), 3.67-3.69 (4H, m), 5.71 (1H, s), 6.93 (1H, dd, J = 9.1, 1.8 Hz), 7.13 (1H, d, J = 9.1 Hz), 8.20 (1H, d, J =1.8 Hz).

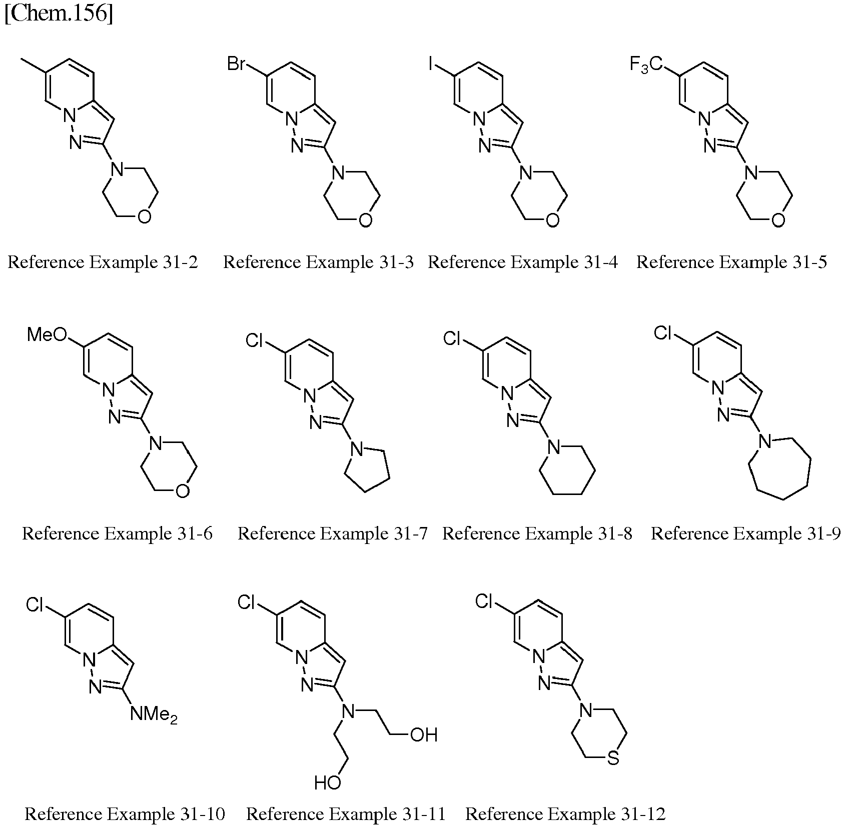

### <Reference Example 32-1>

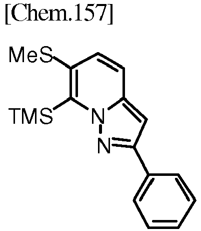

### 6-(Methylthio)-2-phenyl-7-(trimethylsilyl)pyrazolo[1,5-a]pyridine

*n*-Butyllithium (2.5 mL, a 1.6 mol/L *n*-hexane solution) was added to a tetrahydrofuran solution (17 mL) of 6-(methylthio)-2-phenylpylazolo[1,5-a]pyridine (812 mg) at -78°C under an argon atmosphere, and then the mixture was stirred at -78°C for 30 minutes. Trimethylsilyl chloride (2.2 mL) was added to the mixture thus obtained at -78°C, and then the mixture was stirred at room temperature for 1 hour. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 9:1) to obtain a title compound as yellow liquid (1.05 g).
¹H-NMR (400 MHz, CDCl₃) δ 0.66 (9H, s), 2.43 (3H, s), 6.75 (1H, s), 7.16 (1H, d, J = 9.1 Hz), 7.34 (1H, t, J = 7.3 Hz), 7.40-7.46 (3H, m), 7.97 (2H, d, J = 7.3 Hz).

### <Reference Example 32-2>

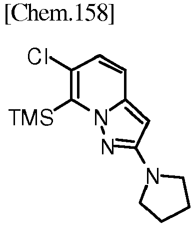

### 6-Chloro-2-(pyrrolidin-1-yl)-7-(trimethylsilyl)pyrazolo[1,5-a]pyridine

After 6-chloro-2-(pyrrolidine-1-yl)pyrazolo[1,5-a]pyridine (700 mg) was dissolved in tetrahydrofuran (20.0 mL), and *n*-butyllithium (2.46 mL, a 1.6 mol/L *n*-hexane solution) was added at -78°C, the mixture was stirred at the same temperature for 1 hour. Trimethylsilyl chloride (0.80 mL) was added thereto at -78°C and then the mixture was stirred at room temperature for 1 hour. A saturated ammonium chloride aqueous solution was added to the reaction solution, and then the mixture was extracted with ethyl acetate. The organic layer was washed with water and saturated saline in this order and then dried over anhydrous sodium sulfate. The desiccant was filtered off, the filtrate was evaporated under vacuum, and the residue thus obtained was purified by silica gel column chromatography (*n*-hexane/ethyl acetate = 4:1) to obtain a title compound as a yellow amorphous (762 mg).
¹H-NMR (400 MHz, CDCl₃) δ 0.56 (9H, s),1.96-2.02 (4H, m), 3.35-3.38 (4H, m), 5.59 (1H, s), 6.88 (1H,d,J=9.1Hz),7.12(1H,d,J=9.1Hz).

### <Reference Example 32-3>

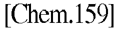

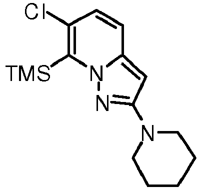

### 6-Chloro-2-(piperidin-1-yl)-7-(trimethylsilyl)pyrazolo[1,5-a]pyridine

After 6-chloro-2-(piperidin-1-yl)pyrazolo[1,5-a]pyridine (700 mg) was dissolved in tetrahydrofuran (20.0 mL), and *n*-butyllithium (2.46 mL, a 1.6 mol/L *n*-hexane solution) was added at -78°C, the mixture was stirred at the same temperature for 1 hour. Trimethylsilyl chloride (0.80 mL) was added thereto at -78°C and then the mixture was stirred at room temperature for 1 hour. A saturated ammonium chloride aqueous solution was added to the reaction solution, and then the mixture was extracted with ethyl acetate. The organic layer was washed with water and saturated saline in this order and then dried over anhydrous sodium sulfate. The desiccant was filtered off, the filtrate was evaporated under vacuum, and the residue thus obtained was purified by silica gel column chromatography (n-hexane/ethyl acetate = 4:1) to obtain a title compound as a yellow solid (881 mg).
¹H-NMR (400 MHz, CDCl₃) δ 0.54 (9H, s),1.59-1.62 (2H, m),1.67-1.71 (4H, m), 3.30-3.31 (4H, m), 5.75 (1H, s), 6.89 (1H, d, J = 9.2 Hz), 7.14 (1H, d, J = 9.2 Hz).

### <Reference Example 32-4>

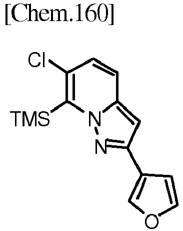

### 6-Chloro-2-(furan-3-yl)-7-(trimethylsilyl)pyrazolo[1,5-a]pyridine

*n*-Butyllithium (564 µL, a 1.63 mol/L *n*-hexane solution) was added to a tetrahydrofuran (4.6 mL) solution of 6-chloro-2-(furan-3-yl)pyrazolo[1,5-a]pyridine (200 mg) at -78°C, and then the mixture was stirred for 30 minutes. Subsequently, trimethylsilyl chloride (151 µL) was added at -78°C and then the mixture was stirred at room temperature for 1.5 hours. After the reaction was completed, a saturated ammonium chloride aqueous solution (10 mL) was added, then the mixture was extracted with ethyl acetate (30 mL) and washed with a saturated ammonium chloride aqueous solution (10 mL) and saturated saline (10 mL). The organic layer was dried over anhydrous sodium sulfate, the solvent was evaporated under vacuum, and then the residue thus obtained was purified by silica gel column chromatography (*n*-hexane:ethyl acetate = 10:0 to 2:8) to obtain a title compound as a brown oil (200 mg).
¹H-NMR (400 MHz, CDCl₃) δ 0.60 (9H, s), 6.55 (1H, s), 6.83 (1H, d, J =1.2 Hz), 6.99 (1H, dd, J = 9.7 Hz), 7.37 (1H, d, J = 9.7 Hz), 7.48 (1H, t, J =1.8 Hz), 7.89 (1H, s).

### <Reference Example 33-1>

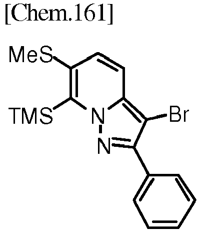

### 3-Bromo-6-(methylthio)-2-phenyl-7-(trimethylsilyl)pyrazolo[1,5-a]pyridine

*N*-bromosuccinimide (752 mg) was added to an acetonitrile solution (18 mL) of 6-(methylthio)-2-phenyl-7-(trimethylsilyl)pyrazolo[1,5-a]pyridine (1.1 g) at room temperature under an argon atmosphere, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 19:1) to obtain a title compound as yellow liquid (961 mg).
¹H-NMR (400 MHz, CDCl₃) δ 0.63 (9H, s), 2.45 (3H, s), 7.27 (1H, d, J = 7.3 Hz), 7.45-7.54 (3H, m), 8.08 (1H, d, J = 9.1 Hz), 8.12 (2H, d, J = 7.3 Hz).

### <Reference Example 33-2>

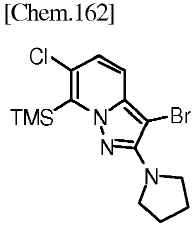

### 3-Bromo-6-chloro-2-(pylrolidin-1-yl)-7-(trimethylsilyl)pylazolo[1,5-a]pyridine

6-Chloro-2-(pyrrolidin-1-yl)-7-(trimethylsilyl)pyrazolo[1,5-a]pyridine (700 mg) was dissolved in acetonitrile (14.0 mL), *N*-bromosuccinimide (509 mg) was added under ice-cooling, and then the mixture was stirred at room temperature for 2 hours. A saturated sodium bicarbonate aqueous solution was added to the reaction solution, and then the mixture was extracted with ethyl acetate. The organic layer was washed with water and saturated saline in this order and then dried over anhydrous sodium sulfate. The desiccant was filtered off, the filtrate was evaporated under vacuum, and the residue thus obtained was purified by silica gel column chromatography (*n*-hexane:ethyl acetate = 5:1) to obtain a title compound as a pale yellow solid (608 mg).
¹H-NMR (400 MHz, CDCl₃) δ 0.54 (9H, s),1.94-1.96 (4H, m), 3.60-3.64 (4H, m), 6.98 (1H, d,J=9.1Hz),7.13(1H,d,J=9.1Hz).

### <Reference Example 33-3>

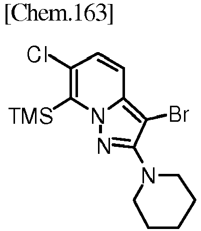

### 3-Bromo-6-chloro-2-(piperidin-1-yl)-7-(trimethylsilyl)pyrazolo[1,5-a]pyridine

6-Chloro-2-(piperidin-1-yl)-7-(trimethylsilyl)pyrazolo[1,5-a]pyridine (860 mg) was dissolved in acetonitrile (16.0 mL), *N*-bromosuccinimide (611 mg) was added under ice-cooling, and then the mixture was stirred at room temperature for 2 hours. A saturated sodium bicarbonate aqueous solution was added to the reaction solution, and then the mixture was extracted with ethyl acetate. The organic layer was washed with water and saturated saline in this order and then dried over anhydrous sodium sulfate. The desiccant was filtered off, the filtrate was evaporated under vacuum, and the residue thus obtained was purified by silica gel column chromatography (*n*-hexane:ethyl acetate = 5:1) to obtain a title compound as an orange oil (835 mg).
¹H-NMR (400 MHz, CDCl₃) δ 0.53 (9H, s),1.59-1.64 (2H, m),1.69-1.74 (4H, m), 3.38-3.41 (4H, m), 6.99 (1H, d, J = 9.7 Hz), 7.20 (1H, d, J = 9.7 Hz).

### <Reference Example 33-4>

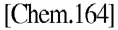

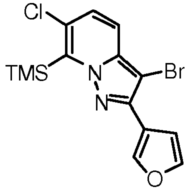

### 3-Bromo-6-chloro-2-(furan-3-yl)-7-(trimethylsilyl)pyrazolo[ 1,5-a]pyridine

*N*-bromosuccinimide was added to an acetonitrile (3.3 mL) solution of 6-chloro-2-(furan-3-yl)-7-(trimethylsilyl)pyrazolo[1,5-a]pyridine (190 mg) at 0°C, and the mixture was stirred at 0°C for 1 hour. After the reaction was completed, a saturated sodium bicarbonate aqueous solution (5 mL) was added, and then the mixture was extracted with ethyl acetate (20 mL) and washed with a saturated sodium bicarbonate aqueous solution (5 mL) and water (10 mL). The organic layer was dried over anhydrous sodium sulfate, the solvent was evaporated under vacuum, and then the residue thus obtained was purified by silica gel column chromatography (*n*-hexane:ethyl acetate = 10:0 to 9:1) to obtain a title compound as a colorless oil (220 mg).
¹H-NMR (400 MHz, CDCl₃) δ 0.60 (9H, s), 7.03 (1H, d, J = 1.2 Hz), 7.09 (1H, d, J = 9.8 Hz), 7.38 (1H, d, J = 9.8 Hz), 7.51 (1H, t, J =1.8 Hz), 8.26 (1H, s).

### <Reference Example 34-1>

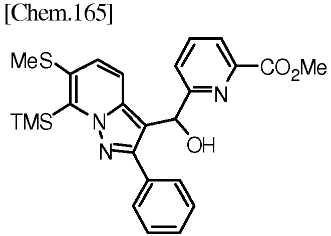

### Methyl 6-[hydroxy[6-(methylthio)-2-phenyl-7-(trimethylsilyl)pyrazolo[1,5-a]pyridin-3-yl]methyl]py ridine-2-carboxylate

*n*-Butyllithium (1.8 mL, a 1.6 mol/L n-hexane solution) was added to a tetrahydrofuran solution (6.0 mL) of 3-bromo-6-(methylthio)-2-phenyl-7-(trimethylsilyl)pyrazolo[1,5-a]pyridine (960 mg) at -78°C under an argon atmosphere, and then the mixture was stirred at -78°C for 30 minutes. A tetrahydrofuran solution (6.0 mL) of methyl 6-formylpyridine-2-carboxylate (608 mg) was added to the obtained mixture at -78°C, and then the mixture was stirred at room temperature for 1 hour. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as yellow liquid (383 mg).
¹H-NMR (400 MHz, CDCl₃) δ 0.62 (9H, s), 2.37 (3H, s), 4.05 (3H, s), 5.32 (1H, d, J = 2.4 Hz), 6.28 (1H, d, J = 2.4 Hz), 7.24 (1H, d, J = 7.9 Hz), 7.40 (1H, d, J = 7.3 Hz), 7.45 (2H, t, J = 7.3 Hz), 7.70 (1H, t, J = 7.9 Hz), 7.87 (2H, d, J = 7.3 Hz), 8.03 (1H, d, J = 7.9 Hz), 8.16 (1H, dd, J = 7.9, 1.2 Hz), 8.36 (1H, dd, J = 7.9, 1.2 Hz).

### <Reference Example 34-2>

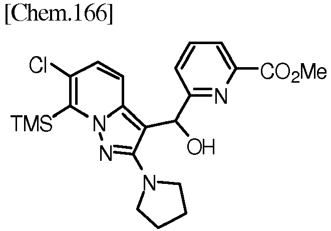

### Methyl 6-[[6-chloro-2-(pyrrolidin-1-yl)-7-(trimethylsilyl)pyrazolo[1,5-a]pyridin-3-yl](hydroxy)meth yl]pyridine-2-carboxylate

3-Bromo-6-chloro-2-(pyrrolidin-1-yl)-7-(trimethylsilyl)pyrazolo[1,5-a]pyridine (550 mg) was dissolved in tetrahydrofuran (7.38 mL), n-butyllithium (1.15 mL, a 1.54 mol/L *n*-hexane solution) was added at -78°C, and then the mixture was stirred at -78°C for 1 hour. A tetrahydrofuran solution (13.0 mL) of methyl 6-formyl-2-pyridinecarboxylate (489 mg) was added at the same temperature and then the mixture was stirred at room temperature for 1 hour. A saturated ammonium chloride aqueous solution was added to the reaction solution, and then the mixture was extracted with ethyl acetate. The organic layer was washed with saturated saline and then dried over anhydrous sodium sulfate. The desiccant was filtered off, the filtrate was evaporated under vacuum, and the residue thus obtained was purified by silica gel column chromatography (*n*-hexane:ethyl acetate = 2:1) to obtain a title compound as a yellow amorphous (441 mg).
¹H-NMR (400 MHz, CDCl₃) δ 0.55 (9H, s),1.91-1.99 (4H, m), 3.42-3.48 (2H, m), 3.53-3.59 (2H, m), 4.04 (3H, s), 5.30 (1H, s), 6.25 (1H, s), 6.61 (1H, d, J = 9.1 Hz), 6.74 (1H, d, J = 9.1 Hz), 7.38 (1H, d, J = 7.9 Hz), 7.76 (1H, dd, J = 7.9,7.3 Hz), 8.05 (1H, d, J = 7.3 Hz).

### <Reference Example 34-3>

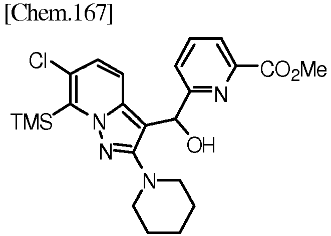

### Methyl 6-[[6-chloro-2-(piperidin-1-yl)-7-(trimethylsilyl)pyrazolo[1,5-a]pyridin-3-yl] (hydroxy)methy 1]pyridine-2-carboxylate

3-Bromo-6-chloro-2-(piperidin-1-yl)-7-(trimethylsilyl)pyrazolo[1,5-a]pyridine (1.00 g) was dissolved in tetrahydrofuran (7.5 mL), n-butyllithium (1.38 mL, a 1.54 mol/L *n*-hexane solution) was added at -78°C, and then the mixture was stirred at -78°C for 1 hour. A tetrahydrofuran solution (15.0 mL) of methyl 6-formyl-2-pyridinecarboxylate (855 mg) was added at the same temperature and then the mixture was stirred at room temperature for 1 hour. A saturated ammonium chloride aqueous solution was added to the reaction solution, and then the mixture was extracted with ethyl acetate. The organic layer was washed with saturated saline and then dried over anhydrous sodium sulfate. The desiccant was filtered off, the filtrate was evaporated under vacuum, and the residue thus obtained was purified by silica gel column chromatography (*n*-hexane:ethyl acetate = 2:1) to obtain a title compound as yellow amorphous (678 mg).
¹H-NMR (400 MHz, CDCl₃) δ 0.56 (9H, s),1.57-1.66 (6H, m), 3.18-3.23 (4H, m), 4.05 (3H, s), 5.89 (1H, s), 6.16 (1H, s), 6.86 (1H, d, J = 9.2 Hz), 7.04 (1H, d, J = 9.2 Hz), 7.46 (1H, d, J = 7.9 Hz), 7.80 (1H, dd, J = 7.9,7.3 Hz), 8.06 (1H, d, J = 7.3 Hz).

### <Reference Example 34-4>

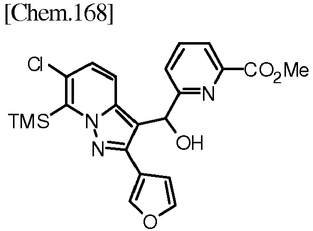

### Methyl 6-[[6-chloro-2-(furan-3-yl)-7-(trimethylsilyl)pyrazolo[1,5-a]pyridin-3-yl] (hydroxy)methyl]p yridine-2-carboxylate

*n*-Butyllithium (440 µL, a 1.63 mol/L *n*-hexane solution) was added to a tetrahydrofuran (3 mL) solution of 3-bromo-6-chloro-2-(furan-3-yl)-7-(trimethylsilyl)pyrazolo[1,5-a]pyridine (220 mg) at -78°C, then the mixture was stirred at -78°C for 10 minutes, then a tetrahydrofuran (1 mL) solution of methyl 6-formyl-2-pyridinecarboxylate (147 mg) was added, and then the mixture was stirred at -78°C for 30 minutes. A saturated ammonium chloride aqueous solution (10 mL) was added and the mixture was extracted with ethyl acetate (30 mL) and washed with a saturated ammonium chloride aqueous solution (10 mL) and saturated saline (10 mL). The organic layer was dried over anhydrous sodium sulfate, the solvent was evaporated under vacuum, and then the residue thus obtained was purified by silica gel column chromatography (*n*-hexane:ethyl acetate = 10:0 to 1:1) to obtain a title compound as a colorless oil (100 mg).
¹H-NMR (400 MHz, CDCl₃) δ 0.60 (9H, s), 4.07 (1H, s), 6.26 (1H, s), 6.88- 6.90 (2H, m), 7.07(1H,d,J=9.1Hz), 7.30(1H,d,J=7.9Hz), 7.48(1H,d,J=1.8Hz), 7.74(1H,t,J=7.9 Hz), 7.95 (1H, s), 8.04 (1H, d, J = 7.9 Hz).

### <Reference Example 34-5>

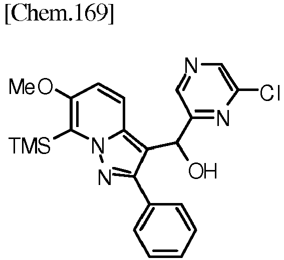

### (6-Chloropyrazin-2-yl)[6-methoxy-2-phenyl-7-(trimethylsilyl)pyrazolo[1,5-a]pyridin-3-yl]m ethanol

After a tetrahydrofuran (2.0 mL) solution of 3-bromo-6-methoxy-2-phenyl-7-(trimethylsilyl)pyrazolo[1,5-a]pyridine (150 mg) was cooled down to -78°C under an argon atmosphere, *n*-butyllithium (0.290 mL, a 1.65 mol/L *n*-hexane solution) was added, and then the mixture was stirred at -78°C for 30 minutes. The obtained mixture was slowly added to a tetrahydrofuran (2.0 mL) solution of 6-chloropyrazine-2-carbaldehyde at -78°C and the mixture was stirred for 30 minutes. A saturated ammonium chloride aqueous solution was added to the reaction solution, and then the mixture was extracted with ethyl acetate. The organic layer was washed with saturated saline and then dried over anhydrous sodium sulfate. The solvent was evaporated under vacuum and then the residue was purified by silica gel column chromatography (n-hexane:ethyl acetate =1:0 to 1:1) to obtain a title compound as a pale yellow oil (121 mg).
¹H NMR (400 MHz, CDCl₃) δ 0.51 (9H, s), 3.73 (1H, d, J = 3.0 Hz), 3.79 (3H, s), 6.32 (1H, d, J = 3.0 Hz), 6.96 (1H, d, J = 9.7 Hz), 7.23 (1H, d, J = 9.7 Hz), 7.40 (1H, dt, J = 7.3, 1.8 Hz), 7.43-7.47 (2H, m), 7.79 (2H, dd, J = 8.2, 1.8 Hz), 8.44 (1H, s), 8.47 (1H, s).

### <Reference Example 35-1>

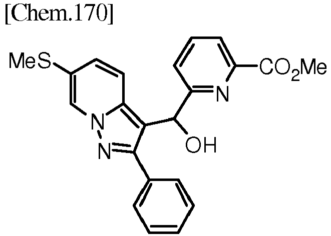

### Methyl 6-[hydroxy[6-(methylthio)-2-phenylpyrazolo[1,5-a]pyridin-3-yl]methyl]pyridine-2-carboxyl ate

Tetrabutylammonium fluoride (1.6 mL, a 1 mol/L tetrahydrofuran solution) was added to a tetrahydrofuran solution (4.0 mL) of methyl 6-[hydroxy[6-(methylthio)-2-phenyl-7-(trimethylsilyl)pyrazolo[1,5-a]pyridin-3-yl]methyl]py ridine-2-carboxylate (383 mg) under an argon atmosphere under ice-cooling, and then the mixture was stirred for 1 hour under ice-cooling. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 1:1) to obtain a title compound as a pale yellow amorphous (290 mg).
¹H-NMR (400 MHz, CDCl₃) δ 2.46 (3H, s), 4.05 (3H, s), 5.32 (1H, d, J = 2.4 Hz), 6.23 (1H, d, J = 2.4 Hz), 6.96 (1H, dd, J = 9.7, 1.8 Hz), 7.04 (1H, d, J = 9.7 Hz), 7.21 (1H, d, J = 7.9 Hz), 7.42 (1H, t, J = 7.3 Hz), 7.47 (2H, t, J = 7.3 Hz), 7.71 (1H, t, J = 7.9 Hz), 7.83 (2H, d, J = 7.3 Hz), 8.03 (1H, d, J = 7.9 Hz), 8.39 (1H, d, J =1.8 Hz).

### <Reference Example 36-1>

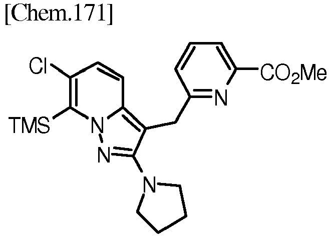

### Methyl 6-[[6-chloro-2-(pyrrolidin-1-yl)-7-(trimethylsilyl)pyrazolo[1,5-a]pyridin-3-yl]methyl]pyridin e-2-carboxylate

Methyl 6-[[6-chloro-2-(pyrrolidin-1-yl)-7-(trimethylsilyl)pyrazolo[1,5-a]pyridin-3-yl](hydroxy)meth yl]pyridine-2-carboxylate (430 mg) was dissolved in dichloromethane (1.90 mL), then triethylsilane (0.90 mL) and trifluoroacetic acid (0.84 mL) were added at room temperature, and then the mixture was stirred at the same temperature for 3 hours. A saturated sodium bicarbonate aqueous solution was added to the reaction solution, and then the mixture was extracted with ethyl acetate. The organic layer was washed with water and saturated saline in this order and then dried over anhydrous sodium sulfate. The desiccant was filtered off, the filtrate was evaporated under vacuum, and the residue thus obtained was purified by silica gel column chromatography (n-hexane:ethyl acetate = 2:1) to obtain a title compound as a yellow solid (246 mg).
¹H NMR (400 MHz, CDCl₃) δ 0.57 (9H, s), 1.86-1.90 (4H, m), 3.40-3.44 (4H, m), 4.03 (3H, s), 4.41 (2H, s), 6.87 (1H, d, J = 9.1 Hz), 6.99 (1H, d, J = 9.1 Hz), 7.18 (1H, d, J = 7.9 Hz), 7.67 (1H, dd, J = 7.9, 7.3 Hz), 7.95 (1H, d, J = 7.3 Hz).

### <Reference Example 36-2>

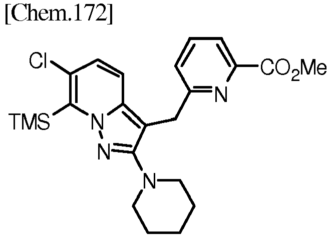

### Methyl 6-[[6-chloro-2-(piperidin-1-yl)-7-(trimethylsilyl)pyrazolo[1,5-a]pyridin-3-yl]methyl]pyridine -2-carboxylate

Methyl 6-[[6-chloro-2-(piperidin-1-yl)-7-(trimethylsilyl)pyrazolo[1,5-a]pyridin-3-yl] (hydroxy)methy 1]pyridine-2-carboxylate (600 mg) was dissolved in dichloromethane (2.50 mL), then triethylsilane (1.2 mL) and trifluoroacetic acid (1.13 mL) were added at room temperature, and then the mixture was stirred at the same temperature for 3 hours. A saturated sodium bicarbonate aqueous solution was added to the reaction solution, and then the mixture was extracted with ethyl acetate. The organic layer was washed with water and saturated saline in this order and then dried over anhydrous sodium sulfate. The desiccant was filtered off, the filtrate was evaporated under vacuum, and the residue thus obtained was purified by silica gel column chromatography (n-hexane:ethyl acetate = 2:1) to obtain a title compound as a pale brown solid (479 mg).
¹H-NMR (400 MHz, CDCl₃) δ 0.59 (9H, s), 1.55-1.62 (6H, m), 3.19-3.20 (4H, m), 4.06 (3H, s), 4.34 (2H, s), 6.89 (1H, d, J = 9.1 Hz), 7.05 (1H, d, J = 9.1 Hz), 7.24 (1H, d, J = 7.3 Hz), 7.70 (1H, t, J = 7.3 Hz), 7.99 (1H, d, J = 7.3 Hz).

### <Reference Example 36-3>

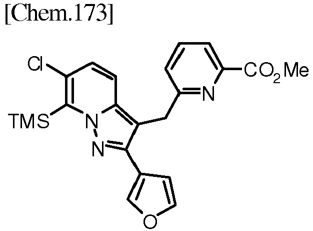

### Methyl 6-[[6-chloro-2-(furan-3-yl)-7-(trimethylsilyl)pyrazolo[1,5-a]pyridin-3-yl]methyl]pyridine-2-c arboxylate

Trifluoroacetic acid (201 µL) and triethylsilane (210 µL) were added to a dichloromethane (1.1 mL) solution of methyl 6-[[6-chloro-2-(furan-3-yl)-7-(trimethylsilyl)pyrazolo[1,5-a]pyridin-3-yl] (hydroxy)methyl]p yridine-2-carboxylate (100 m), and then the mixture was stirred at room temperature for 1 hour. After the reaction was completed, a saturated sodium bicarbonate aqueous solution (10 mL) was added, then the mixture was extracted with ethyl acetate (30 mL) and washed with a saturated sodium bicarbonate aqueous solution (10 mL) and saturated saline (10 mL). The organic layer was dried over anhydrous sodium sulfate, the solvent was evaporated under vacuum, and then the residue thus obtained was purified by silica gel column chromatography (n-hexane:ethyl acetate = 10:0 to 7:3) to obtain a title compound as a colorless oil (30 mg).
¹H-NMR (400 MHz, CDCl₃) δ 0.61 (9H, s), 4.05 (3H, s), 4.48 (2H, s), 6.87 (1H, d, J =1.2 Hz), 6.99 (1H, d, J = 9.1 Hz), 7.10 (1H, d, J = 7.9 Hz), 7.32 (1H, d, J = 9.1 Hz), 7.46 (1H, t, J =1.2 Hz), 7.64 (1H, t, J = 7.9 Hz), 7.83 (1H, s), 7.96 (1H, d, J = 7.9 Hz).

### <Reference Example 36-4>

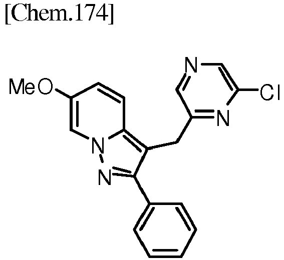

### 3-[(6-Chloropyrazin-2-yl)methyl]-6-methoxy-2-phenylpyrazolo[1,5-a]pyridine

Triethylsilane (0.310 mL) and trifluoroacetic acid (0.290 mL) were added to a dichloromethane (1.63 mL) solution of (6-chloropyrazin-2-yl)[6-methoxy-2-phenyl-7-(trimethylsilyl)pyrazolo[1,5-a]pyridin-3-yl]me thanol (143 mg) under an argon atmosphere, and then the mixture was stirred at room temperature for 15 hours. A saturated sodium bicarbonate aqueous solution was added to the reaction solution, and then the mixture was extracted with ethyl acetate. The organic layer was washed with saturated saline and then dried over anhydrous sodium sulfate. The solvent was evaporated under vacuum and then the residue was purified by silica gel column chromatography (n-hexane:ethyl acetate = 1:0 to 10:1) to obtain a title compound as a colorless oil (57.8 mg).
¹H NMR (400 MHz, CDCl₃) δ 3.85 (3H, s), 4.39 (2H, s), 6.95 (1H, dd, J = 9.7, 1.8 Hz), 7.33-7.41 (2H, m), 7.45 (2H, tt, J = 7.3, 1.8 Hz), 7.66 (2H, dd, J = 7.3, 1.2 Hz), 8.10 (1H, d, J =1.8 Hz), 8.16 (1H, s), 8.40 (1H, s).

### <Reference Example 37-1>

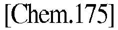

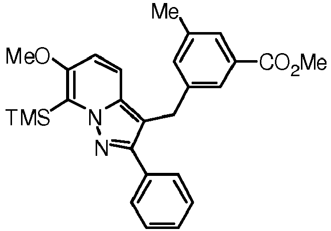

### Methyl 3-[[6-methoxy-2-phenyl-7-(trimethylsilyl)pyrazolo[1,5-a]pyridin-3-yl]methyl]-5-methylbenz oate

n-Butyllithium (0.3 mL, a 1.6 mol/L n-hexane solution) was added to a tetrahydrofuran solution (1.1 mL) of 3-bromo-6-methoxy-2-phenyl-7-(trimethylsilyl)pyrazolo[1,5-a]pyridine (158 mg) at -78°C under an argon atmosphere, and then the mixture was stirred at -78°C for 30 minutes. A tetrahydrofuran solution (1.0 mL) of zinc iodide (140 mg) was added to the obtained mixture at -78°C, and then the mixture was stirred at room temperature for 30 minutes. Then methyl 3-(bromomethyl)-5-methylbenzoate (112 mg) and tetrakistriphenylphosphinepalladium (0) (49 mg) were added, and then the mixture was stirred at room temperature for 4 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 9:1) to obtain a title compound as yellow liquid (92 mg).
¹H-NMR (400 MHz, CDCl₃) δ 0.55 (9H, s), 2.30 (3H, s), 3.81 (3H, s), 3.88 (3H, s), 4.27 (2H, s), 6.93 (1H, d, J = 9.7 Hz), 7.14 (1H, s), 7.19 (1H, d, J = 9.7 Hz), 7.33 (1H, d, J = 7.3 Hz), 7.39 (2H, d, J = 7.3 Hz), 7.67-7.71 (2H, m), 7.73 (2H, s).

### <Reference Example 37-2>

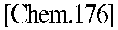

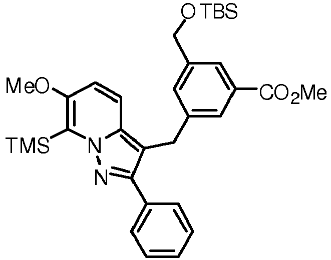

### Methyl 3-[[(tert-butyldimethylsilyl)oxy]methyl]-5-[[6-methoxy-2-phenyl-7-(trimethylsilyl)pyrazolo[ 1,5-a]pyridin-3-yl]methyl]benzoate

n-Butyllithium (0.5 mL, a 1.6 mol/L n-hexane solution) was added to a tetrahydrofuran solution (2.1 mL) of 3-bromo-6-methoxy-2-phenyl-7-(trimethylsilyl)pyrazolo[1,5-a]pyridine (304 mg) at -78°C under an argon atmosphere, and then the mixture was stirred at -78°C for 30 minutes. A tetrahydrofuran solution (2.0 mL) of zinc iodide (271 mg) was added to the obtained mixture at -78°C, and then the mixture was stirred at room temperature for 30 minutes. Then methyl 3-(bromomethyl)-5-[[(tert-butyldimethylsilyl)oxy]methyl]benzoate (334 mg) and tetrakistriphenylphosphinepalladium (0) (94 mg) were added, and then the mixture was stirred at room temperature for 2 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 9:1) to obtain a title compound as yellow liquid (309 mg).
¹H-NMR (400 MHz, CDCl₃) δ -0.03 (6H, s), 0.55 (9H, s), 0.82 (9H, s), 3.80 (3H, s), 3.89 (3H, s), 4.31 (2H, s), 4.68 (2H, s), 6.92 (1H, d, J = 9.7 Hz), 7.19 (1H, d, J = 9.7 Hz), 7.30 (1H, s), 7.32 (1H, d, J = 7.3 Hz), 7.38 (2H, t, J = 7.3 Hz), 7.70 (2H, d, J = 7.3 Hz), 7.79 (1H, s), 7.83 (1H, s).

### <Reference Example 38-1>

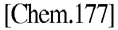

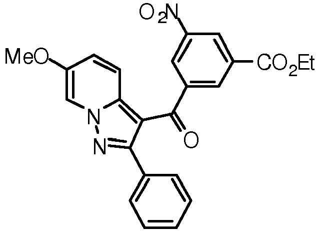

### Ethyl 3-[(6-methoxy-2-phenylpyrazolo[1,5-a]pyridin-3-yl)carbonyl]-5-nitrobenzoate

Thionyl chloride (0.5 mL) was added to a toluene (1.5 mL) solution of monoethyl 5-nitro-1,3-benzenedicarboxylate (160 mg) at room temperature under an argon atmosphere, and then the mixture was stirred at 60°C for 3 hours. The reaction solution thus obtained was concentrated under vacuum, a 1,4-dioxane solution (0.9 mL) of 6-methoxy-2-phenylpyrazolo[1,5-a]pyridine (100 mg) was added, and then the mixture was stirred at 150°C for 3 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 4:1) to obtain a title compound as pale yellow liquid (92 mg).
¹H-NMR (400 MHz, CDCl₃) δ 1.42 (3H, t, J = 7.3 Hz), 3.94 (3H, s), 4.39 (2H, q, J = 7.3 Hz), 7.09 (2H, d, J = 7.3 Hz), 7.13 (1H, d, J = 7.3 Hz), 7.23-7.26 (2H, m), 7.38 (1H, dd, J = 9.7, 1.8 Hz), 8.23 (1H, d, J =1.8 Hz), 8.29 (1H, d, J = 9.7 Hz), 8.39-8.43 (2H, m), 8.68 (1H, t, J = 1.8 Hz).

### <Reference Example 39-1>

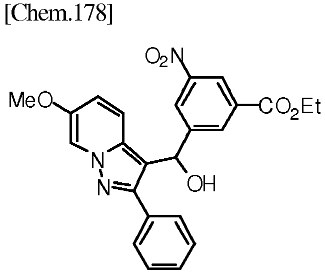

### Ethyl 3-[hydroxy(6-methoxy-2-phenylpyrazolo[1,5-a]pyridin-3-yl)methyl]-5-nitrobenzoate

A methanol solution (0.2 mL) of sodium borohydride (9 mg) was added to a dichloromethane solution (1.9 mL) of ethyl 3-[(6-methoxy-2-phenylpyrazolo[1,5-a]pyridin-3-yl)carbonyl]-5-nitrobenzoate (92 mg) under an argon atmosphere under ice-cooling, and then the mixture was stirred for 1 hour under ice-cooling. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 2:1) to obtain a title compound as a yellow amorphous (74 mg).
¹H-NMR (400 MHz, CDCl₃) δ 1.40 (3H, t, J = 7.3 Hz), 3.84 (3H, s), 4.40 (2H, q, J = 7.3 Hz), 6.35 (1H, d, J = 3.6 Hz), 6.87 (1H, dd, J = 9.7, 2.4 Hz), 7.04 (1H, d, J = 9.7 Hz), 7.42-7.50 (3H, m), 7.67-7.71 (2H, m), 8.11 (1H, d, J = 1.8 Hz), 8.37 (1H, d, J = 1.2 Hz), 8.49 (1H, s), 8.73 (1H, s).

The following Reference Examples 39-2 to 39-9 were obtained by using corresponding pyrazolopyridine derivatives in the similar manner as Reference Example 39-1.

### <Reference Example 39-2>

### Methyl 6-[(6-chloro-2-cyclopropylpyrazolo[1,5-a]pyridin-3-yl)(hydroxy)methyl]pyridine-2-carboxyl ate

¹H-NMR (400 MHz, CDCl₃) δ 0.88-1.06 (4H, m),1.92-2.04 (1H, m), 4.04 (3H, s), 5.24 (1H, d, J = 2.4 Hz), 6.25 (1H, d, J = 2.4 Hz), 6.93 (1H, dd, J = 9.2, 1.8 Hz), 7.20 (1H, d, J = 9.2 Hz), 7.30 (1H, d, J = 7.9 Hz), 7.78 (1H, dd, J = 7.9, 7.3 Hz), 8.05 (1H, d, J = 7.3 Hz), 8.33 (1H,d,J=1.8Hz).

### <Reference Example 39-3>

### Methyl 6-[(6-chloro-2-cyclopentylpyrazolo[1,5-a]pyridin-3-yl)(hydroxy)methyl]pyridine-2-carboxyl ate

¹H-NMR (400 MHz, CDCl₃) δ 1.65-1.72 (2H, m), 1.88-1.94 (5H, m), 2.09-2.11 (1H, m), 3.30-3.38 (1H, m), 4.04 (3H, s), 5.16 (1H, d, J = 3.1 Hz), 6.14 (1H, d, J = 3.1 Hz), 6.91 (1H, dd, J = 9.2, 1.8 Hz), 7.12 (1H, d, J = 9.2 Hz), 7.23 (1H, d, J = 7.9 Hz), 7.76 (1H, dd, J = 7.9, 7.3 Hz), 8.05 (1H, d, J = 7.3 Hz), 8.41 (1H, d, J =1.8 Hz).

### <Reference Example 39-4>

### Methyl 6-[(6-chloro-2-cyclohexylpyrazolo[1,5-a]pyridin-3-yl)(hydroxy)methyl]pyridine-2-carboxyl ate

¹H-NMR (400 MHz, CDCl₃) δ 1.30-1.38 (3H, m), 1.67-1.74 (3H, m), 1.84-1.98 (4H, m), 2.90 (1H, tt, J =11.5, 3.0 Hz), 4.04 (3H, s), 5.17 (1H, d, J = 3.0 Hz), 6.14 (1H, d, J = 3.0 Hz), 6.90 (1H, dd, J = 9.1, 1.8 Hz), 7.10 (1H, d, J = 9.1 Hz), 7.22 (1H, d, J = 7.9 Hz), 7.76 (1H, dd, J = 7.9,7.3 Hz), 8.05 (1H, d, J = 7.3 Hz), 8.42 (1H, d, J =1.8 Hz).

### <Reference Example 39-5>

### Methyl 6-[(2-tert-butyl-6-chloropyrazolo[1,5-a]pyridin-3-yl)(hydroxy)methyl]pyridine-2-carboxylat e

¹H-NMR (400 MHz, CDCl₃) δ 1.58 (9H, s), 4.08 (3H, s), 5.30 (1H, s), 6.46 (1H, s), 6.84 (1H, dd, J = 9.7,1.8 Hz), 6.91 (1H, d, J = 9.7 Hz), 7.22 (1H, d, J = 7.9 Hz), 7.76 (1H, dd, J = 7.9,7.3 Hz), 8.08 (1H, d, J = 7.3 Hz), 8.44 (1H, d, J = 1.8 Hz).

### <Reference Example 39-6>

### Methyl 6-[[6-chloro-2-(2-methyl-propyl)pyrazolo[1,5-a]pyridin-3-yl] (hydroxy)methyl]pyridine-2-ca rboxylate

¹H-NMR (400 MHz, CDCl₃) δ 0.97 (6H, dd, J = 7.3, 6.7 Hz), 2.06-2.13 (1H, m), 2.71 (2H, d, J = 7.3 Hz), 4.04 (3H, s), 5.16 (1H, s), 6.08 (1H, s), 6.93 (1H, dd, J = 9.1, 1.8 Hz), 7.13 (1H, d, J = 9.1 Hz), 7.21 (1H, d, J = 7.9 Hz), 7.76 (1H, dd, J = 7.9, 7.3 Hz), 8.05 (1H, d, J = 7.3 Hz), 8.41 (1H, d, J = 1.8 Hz).

### <Reference Example 39-7>

### Methyl 6-[[2-(2-fluorophenyl)-6-methoxypyrazolo[1,5-a]pyridin-3-yl] (hydroxy)methyl]pyridine-2-c arboxylate

¹H NMR (400 MHz, CDCl₃) δ 3.81 (3H, s), 4.03 (3H, s), 5.30 (1H, d, J =1.8 Hz), 6.01 (1H, s), 6.82 (1H, dd, J = 9.7, 2.4 Hz), 7.10 (1H, d, J = 9.7 Hz), 7.17 (1H, t, J = 9.7 Hz), 7.24-7.28 (1H, m), 7.34 (1H, d, J = 7.9 Hz), 7.37-7.44 (1H, m), 7.68 (1H, td, J = 7.3, 1.8 Hz), 7.73 (1H, t, J = 7.3 Hz), 8.02 (1H, d, J = 7.3 Hz), 8.08 (1H, d, J =1.8 Hz).

### <Reference Example 39-8>

### Methyl 6-[[2-(3-fluorophenyl)-6-methoxypyrazolo[1,5-a]pyridin-3-yl] (hydroxy)methyl]pyridine-2-c arboxylate

¹H NMR (400 MHz, CDCl₃) δ 3.78 (3H, s), 4.00 (3H, s), 5.41 (1H, d, J = 2.4 Hz), 6.22 (1H, d, J = 2.4 Hz), 6.77 (1H, dd, J = 9.7, 2.4 Hz), 7.03 (1H, d, J = 9.7 Hz), 7.08 (1H, td, J =7.9, 2.4 Hz), 7.29 (1H, d, J = 7.3 Hz), 7.35-7.42 (1H, m), 7.60-7.66 (2H, m), 7.70 (1H, t, J =7.3 Hz), 7.99 (1H, d, J = 7.9 Hz), 8.02 (1H, d, J = 1.8 Hz).

### <Reference Example 39-9>

### Methyl 6-[(6-chloro-5-methyl-2-phenylpyrazolo[1,5-a]pyridin-3-yl)(hydroxy)methyl]pyridine-2-car boxylate

¹H NMR (400 MHz, CDCl₃) δ 2.28 (3H, s), 4.05 (3H, s), 5.24 (1H, s), 6.20 (1H, s), 7.06 (1H, s), 7.19 (1H, d, J = 7.9 Hz), 7.41 (1H, tt, J = 7.3, 1.2 Hz), 7.43-7.49 (2H, m), 7.71 (1H, t, J = 7.9 Hz), 7.76-7.80 (2H, m), 8.03 (1H, d, J = 7.9,1.2 Hz), 8.51 (1H, s).

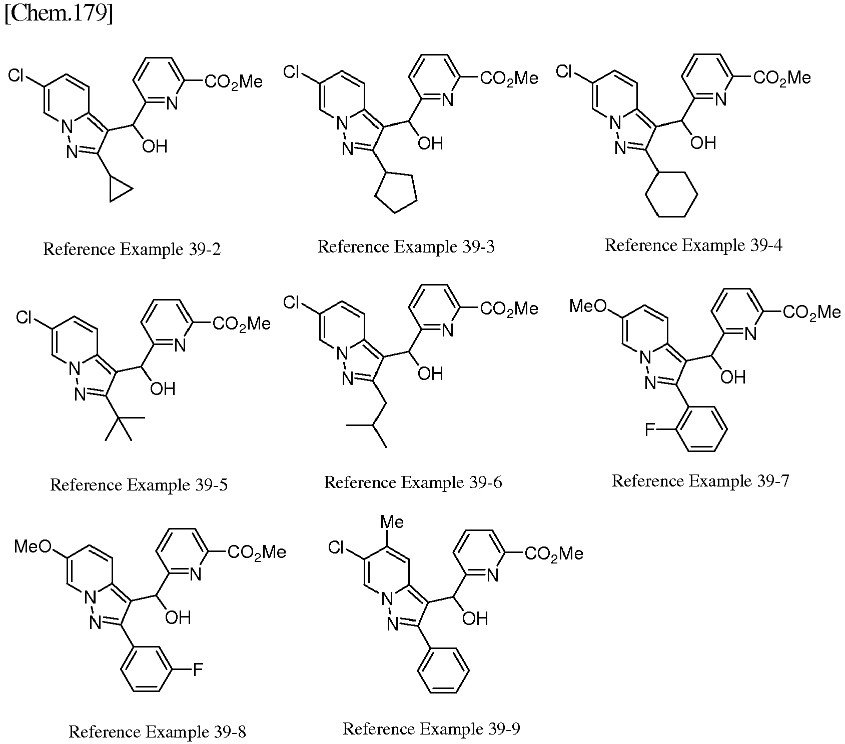

### <Reference Example 40-1>

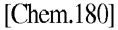

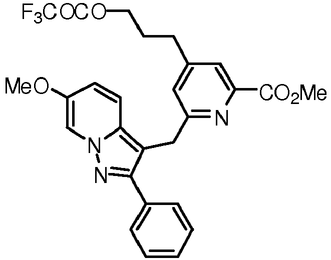

### Methyl 6-[(6-methoxy-2-phenylpyrazolo[1,5-a]pyridin-3-yl)methyl]-4-[3-[(2,2,2-triffuoroacetoxy)pr opyl]pyridine-2-carboxylate

After a tetrahydrofuran (1.3 mL) solution of 3-bromo-6-methoxy-2-phenyl-7-(trimethylsilyl)pyrazolo[1,5-a]pyridine (100 mg) was cooled down to -78°C under an argon atmosphere, n-butyllithium (0.19 mL, a 1.65 mol/L n-hexane solution) was added, and then the mixture was stirred at -78°C for 30 minutes. Next, methyl 4-[3-[(tert-butyldimethylsilyl)oxy]propyl]-6-formylpyridine-2-carboxylate (135 mg) was added at -78°C, and then the mixture was stirred at room temperature for 30 minutes. A saturated ammonium chloride aqueous solution was added to the reaction solution, and then the mixture was extracted with ethyl acetate. The organic layer was washed with saturated saline and then dried over anhydrous sodium sulfate. The solvent was evaporated under vacuum, a dichloromethane (1.0 mL) solution of the residue was ice-cooled, and triethylsilane (0.160 mL) and trifluoroacetic acid (0.150 mL) were added. The mixture was heated up to room temperature, stirred for 17 hours, and then ice-cooled again. Next, a saturated sodium bicarbonate aqueous solution was slowly added to achieve pH = 9. The organic solvent was evaporated under vacuum and then the residue was purified by silica gel column chromatography (n-hexane:ethyl acetate = 5:1 to 1:1) to obtain a title compound as a pale yellow oil (27.3 mg).
¹H NMR (400 MHz, CDCl₃) δ 1.87-1.95 (2H, m), 2.60 (2H, t, J = 7.9 Hz), 3.85 (3H, s), 4.03 (3H, s), 4.21 (2H, t, J = 6.4 Hz), 4.54 (2H, s), 6.88-6.91 (2H, m), 7.27 (1H, d, J = 9.7 Hz), 7.32-7.42 (3H, m), 7.67-7.72 (2H, m), 7.79 (1H, d, J =1.2 Hz), 8.11 (1H, d, J =1.8 Hz).

### <Reference Example 41-1>

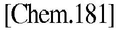

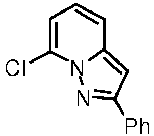

### 7-Chloro-2-phenylpyrazolo[1,5-a]pyridine

A 1.3 M iPrMgCl/LiCl tetrahydrofuran solution (0.59 mL) was added dropwise to a tetrahydrofuran solution (2.5 mL) of 2-phenylpyrazolo[1,5-a]pyridine (100 mg) under ice-cooling, then the mixture was stirred at room temperature for 2 hours, then hexachloroethane (183 mg) was added, and then the mixture was stirred at room temperature for 2 hours. A saturated ammonium chloride aqueous solution was added to the reaction solution and the mixture was extracted with ethyl acetate. The organic layer was dried over anhydrous sodium sulfate and then filtered off, and the solvent was evaporated under vacuum. The residue thus obtained was purified by silica gel chromatography (n-hexane:ethyl acetate =10:1) to obtain a title compound as a pale yellow crystal (82.2 mg).
¹H-NMR (400 MHz, CDCl₃) δ 6.89 (1H, d, J=7.3 Hz), 6.93 (1H, s), 7.07 (1H, dd, J= 9.1, 7.3 Hz), 7.38 (1H, t, J=7.6 Hz), 7.44 - 7.51 (3 H, m), 8.02 (2H, d, J = 7.3 Hz).

### <Reference Example 42-1>

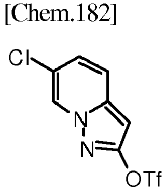

### 6-Chloro-2-(trifluoromethanesulfonyloxy)pyrazolo[1,5-a]pyridine

N-phenylbis(trifluoromethanesulfonimide) (3.89 g) was added to a mixed solution of tetrahydrofuran (10 mL) and NN-dimethylformamide (10 mL) of 6-chloro-2-hydroxypyrazolo[1,5-a]pyridine (1.59 g), sodium hydride (414 mg) was added under ice-cooling, and then the mixture was stirred at room temperature for 1 hour. After the reaction was completed, ice water (20 mL) was added, the mixture was extracted with ethyl acetate (50 mL) and washed with water (20 mL × 2) and saturated saline (20 mL) in this order, and then the organic layer was dried over anhydrous sodium sulfate. The solvent was evaporated under vacuum and then the residue was purified by silica gel column chromatography (n-hexane:ethyl acetate = 10:0 to 9:1) to obtain a title compound as a brown oil (2.68 g).
¹H-NMR (400 MHz, CDCl₃) δ 6.42 (1H, s), 7.24 (1H, dd, J = 9.7, 1.8 Hz), 7.47 (1H, d, J = 9.7 Hz), 8.43 (1H, s).

### <Reference Example 43-1>

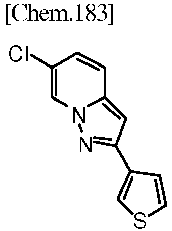

### 6-Chloro-2-(thiophen-3-yl)pyrazolo[1,5-a]pyridine

3-Thiopheneboronic acid (375 mg) and a 2 mol/L sodium carbonate aqueous solution (4 mL) were added to an *N,N*-dimethylformamide (4 mL) solution of 6-chloro-2-(trifluoromethanesulfonyloxy)pyrazolo[1,5-a]pyridine (800 mg), the reaction vessel was purged with argon, dichlorobis(triphenylphosphine)palladium (II) (373 mg) was added, and then the mixture was heated and stirred at 90°C for 1 hour. After the reaction was completed, the reaction mixture was filtered by Celite and washed with ethyl acetate, then water (10 mL) was added, the mixture was extracted with ethyl acetate (50 mL) and washed with water (10 mL× 3) and saturated saline (10 mL× 2) in this order, and the organic layer was dried over anhydrous sodium sulfate. The solvent was evaporated under vacuum and then the residue was purified by silica gel column chromatography (n-hexane:ethyl acetate = 10:0 to 9:1) and triturated with n-hexane (2 mL) to obtain a title compound as a colorless solid (312 mg).
¹H-NMR (400 MHz, CDCl₃) δ 6.70 (1H, s), 7.07 (1H, dd, J = 9.7, 1.5 Hz), 7.24 (1H, dd, J = 4.8, 3.0 Hz), 7.44 (1H, d, J = 9.7 Hz), 7.57 (1H, dd, J = 4.8, 1.2 Hz), 7.76 (1H, dd, J = 3.0, 1.2 Hz), 8.49 (1H, d, J = 1.5Hz).

The following Reference Examples 43-2 to 43-19 were obtained by using corresponding boronic acid derivatives in the same manner as Reference Example 43-1.

### <Reference Example 43-2>

### 6-Chloro-2-(furan-3-yl)pyrazolo[1,5-a]pyridine

¹H-NMR (400 MHz, CDCl₃) δ 6.60 (1H, s), 6.84 (1H, s), 7.07 (1H, dd, J = 9.1, 1.8 Hz), 7.42 (1H, d, J = 9.1 Hz), 7.50 (1H, t, J =1.8 Hz), 7.93 (1H, s), 8.48 (1H, s).

### <Reference Example 43-3>

### 6-Chloro-2-(thiophen-2-yl)pyrazolo[1,5-a]pyridine

¹H-NMR (400 MHz, CDCl₃) δ 6.71 (1H, s), 7.07 (1H, dd, J = 9.7, 1.8 Hz), 7.11 (1H, d, J = 4.8, 3.6 Hz), 7.34 (1H, dd, J = 4.8, 1.2 Hz), 7.41 (1H, d, J = 9.7 Hz), 7.50 (1H, d, J = 3.6, 1.2 Hz), 8.48 (1H, s).

### <Reference Example 43-4>

### 6-Chloro-2-(furan-2-yl)pyrazolo[1,5-a]pyridine

¹H-NMR (400 MHz, CDCl₃) δ 6.51 (1H, dd, J = 3.6, 1.8 Hz), 6.72 (1H, s), 6.84 (1H, d, J = 3.6 Hz), 7.06 (1H, dd,J = 9.7,1.8 Hz), 7.42 (1H, d,J = 9.7 Hz), 7.52 (1H, d,J = 1.8 Hz), 8.47 (1H, s).

### <Reference Example 43-5>

### 6-Chloro-2-(4-fluorophenyl)pyrazolo[1,5-a]pyridine

¹H-NMR (400 MHz, CDCl₃) δ 6.76 (1H, s), 7.08 (1H, dd, J = 9.4, 1.5 Hz), 7.14 (2H, t, J = 8.5 Hz), 7.46 (1H, d, J = 9.1 Hz), 7.91 (1H, dd, J = 8.5, 5.4 Hz), 8.50 (1H, s).

### <Reference Example 43-6>

### 6-Chloro-2-(3-fluorophenyl)pyrazolo[1,5-a]pyridine

¹H-NMR (400 MHz, CDCl₃) δ 6.81 (1H, s), 7.06 (1H, dd, J = 8.0, 1.8 Hz), 7.10 (2H, dd, J = 9.4, 1.8 Hz), 7.41 (1H, td, J = 8.0, 6.1 Hz), 7.48 (1H, d, J = 9.4 Hz), 7.65 (1H, dt, J = 10.3, 1.8 Hz), 7.71 (1H, d, J = 8.0, Hz), 8.52 (1H, s).

### <Reference Example 43-7>

### 6-Chloro-2-(2-fluorophenyl)pyrazolo[1,5-a]pyridine

¹H-NMR (400 MHz, CDCl₃) δ 7.01 (1H, d, J = 3.6 Hz), 7.09 (1H, dd, J = 9.4, 1.5 Hz), 7.18 (2H, dd, J = 11.2, 8.2 Hz), 7.24 (2H, t, J = 7.7 Hz), 7.32-7.38 (1H, m), 7.50 (1H, d, J = 9.4 Hz), 8.14 (1H, td, J = 7.7, 1.5 Hz), 8.54 (1H, s).

### <Reference Example 43-8>

### 6-Chloro-2-(4-methoxyphenyl)pyrazolo[1,5-a]pyridine

¹H-NMR (400 MHz, CDCl₃) δ 3.85 (3H, s), 6.72 (1H, s), 6.98 (2H, d, J = 9.1 Hz), 7.04 (1H, dd, J = 9.7, 1.8 Hz), 7.41 (1H, d, J = 9.7 Hz), 7.86 (2H, d, J = 9.1 Hz), 8.49 (1H, s).

### <Reference Example 43-9>

### 6-Chloro-2-(3-methoxyphenyl)pyrazolo[1,5-a]pyridine

¹H-NMR (400 MHz, CDCl₃) δ 3.90 (3H, s), 6.81 (1H, s), 6.93 (1H, dd, J = 8.2,1.8 Hz), 7.08 (1H, dd, J = 9.7, 1.8 Hz), 7.36 (1H, t, J = 8.2 Hz), 7.46 (1H, d, J = 9.7 Hz), 7.50-7.52 (2H, m), 8.52 (1H, s).

### <Reference Example 43-10>

### 6-Chloro-2-(2-methoxyphenyl)pyrazolo[1,5-a]pyridine

¹H-NMR (400 MHz, CDCl₃) δ 3.95 (3H, s), 7.04-7.08 (4H, m), 7.34-7.38 (1H, m), 7.46 (1H, d, J = 9.1 Hz), 8.07 (1H, dd, J = 7.9, 1.8 Hz), 8.53 (1H, s).

### <Reference Example 43-11>

### 6-Chloro-2-(4-methylphenyl)pyrazolo[1,5-a]pyridine

¹H-NMR (400 MHz, CDCl₃) δ 2.40 (3H, s), 6.78 (1H, s), 7.06 (1H, dd, J = 9.1, 1.8 Hz), 7.26 (2H, d, J =7.9 Hz), 7.45 (1H, d, J = 9.1 Hz), 7.83 (2H, d, J = 7.9 Hz), 8.51 (1H, s).

### <Reference Example 43-12>

### 6-Chloro-2-(3-methylphenyl)pyrazolo[1,5-a]pyridine

¹H-NMR (400 MHz, CDCl₃) δ 2.43 (3H, s), 6.81 (1H, s), 7.07 (1H, d, J = 9.7 Hz), 7.20 (1H, d, J = 7.9 Hz), 7.34 (1H, t, J = 7.9 Hz), 7.46 (1H, d, J = 9.7 Hz), 7.72 (1H, d, J = 9.7 Hz), 7.78 (1H, s), 8.51 (1H, s).

### <Reference Example 43-13>

### 6-Chloro-2-(2-methylphenyl)pyrazolo[1,5-a]pyridine

¹H-NMR (400 MHz, CDCl₃) δ 2.51 (3H, s), 6.66 (1H, s), 7.09 (1H, dd, J = 9.7, 1.8 Hz), 7.26-7.30 (3H, m), 7.48 (1H, d, J = 9.7 Hz), 7.62-7.64 (1H, m), 8.53 (1H, s).

### <Reference Example 43-14>

### 6-Chloro-2-[4-(trifluoromethyl)phenyl]pyrazolo[1,5-a]pyridine

¹H-NMR (400 MHz, CDCl₃) δ 6.87 (1H, s), 7.12 (1H, dd, J = 9.1, 1.8 Hz), 7.50 (1H, d, J = 9.1 Hz), 7.71 (2H, d, J = 7.9 Hz), 8.05 (2H, d, J = 7.9 Hz), 8.53 (1H, s).

### <Reference Example 43-15>

### 6-Chloro-2-[3-(trifluoromethyl)phenyl]pyrazolo[1,5-a]pyridine

¹H-NMR (400 MHz, CDCl₃) δ 6.87 (1H, s), 7.12 (1H, dd, J = 9.1, 1.8 Hz), 7.50 (1H, d, J = 9.1 Hz), 7.57 (1H, t, J = 7.6 Hz), 7.63 (1H, d, J = 7.6 Hz), 8.12 (1H, d, J = 7.6 Hz), 8.20 (1H, s), 8.53 (1H, s).

### <Reference Example 43-16>

### 6-Chloro-2-[2-(trifluoromethyl)phenyl]pyrazolo[1,5-a]pyridine

¹H-NMR (400 MHz, CDCl₃) δ 6.75 (1H, s), 7.13 (1H, dd, J = 9.7, 1.8 Hz), 7.49-7.55 (2H, m), 7.62 (1H, t, J = 7.3 Hz), 7.72(1H, d, J = 7.3 Hz), 7.80 (1H, d, J = 7.3 Hz), 8.53 (1H, s).

### <Reference Example 43-17>

### 6-Chloro-2-(4-chlorophenyl)pyrazolo[1,5-a]pyridine

¹H-NMR (400 MHz, CDCl₃) δ 6.79 (1H, s), 7.09 (1H, dd, J = 9.1, 1.8 Hz), 7.42 (2H, d, J = 8.5 Hz), 7.47 (1H, d, J = 8.5 Hz), 7.87 (2H, d, J = 8.5 Hz), 8.50 (1H, d, J =1.8 Hz).

### <Reference Example 43-18>

### 6-Chloro-2-(3-chlorophenyl)pyrazolo[1,5-a]pyridine

¹H-NMR (400 MHz, CDCl3) δ 6.81 (1H, s), 7.10 (1H, dd, J = 9.2, 1.8 Hz), 7.30-7.40 (2H, m), 7.48 (1H, d, J = 9.2 Hz), 7.82 (1H, d, J = 7.3 Hz), 7.94 (1H, s), 8.52 (1H, s).

### <Reference Example 43-19>

### 6-Chloro-2-(2-chlorophenyl)pyrazolo[1,5-a]pyridine

¹H-NMR (400 MHz, CDCl₃) δ 7.06 (1H, s), 7.11 (1H, dd, J = 9.7, 1.8 Hz), 7.30-7.39 (2H, m), 7.49-7.53 (2H, m), 7.89 (1H, dd, J = 7.3,1.8 Hz), 8.54 (1H, d, J = 1.8 Hz).

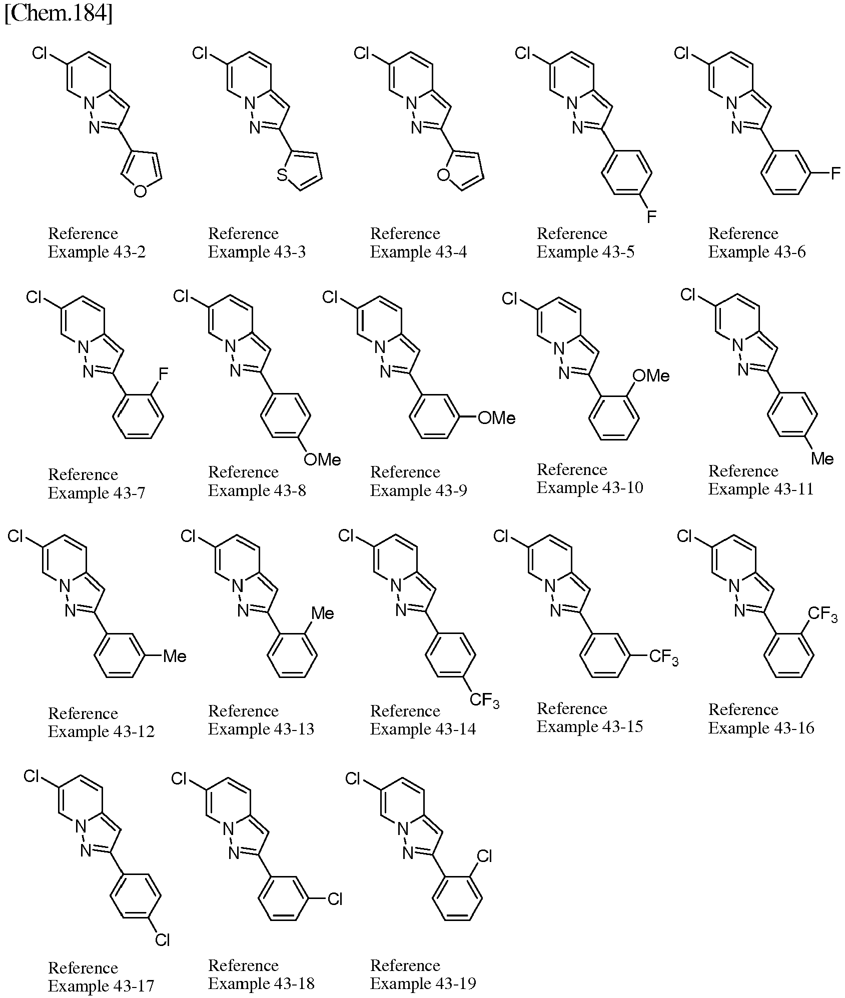

### <Reference Example 44-1>

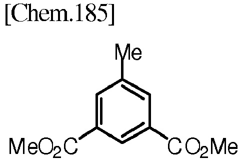

### 5-Methyl-1,3-benzenedicarboxylate 1,3-dimethyl ester

Thionyl chloride (4.0 mL) was added to a methanol solution (28 mL) of 5-methyl-1,3-benzenedicarboxylic acid (1.0 g) at room temperature under an argon atmosphere, and then the mixture was stirred at 60°C for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was washed with n-hexane to obtain a title compound as a pale yellow powder (1.08 g).
¹H-NMR (400 MHz, CDCl₃) δ 2.46 (3H, s), 3.94 (6H, s), 8.05 (2H, s), 8.49 (1H, s).

### <Reference Example 45-1>

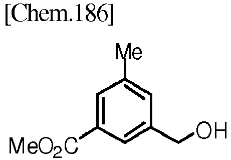

### Methyl 3-(hydroxymethyl)-5-methylbenzoate

Diisobutylaluminum hydride (9.2 mL, a 0.04 mol/L *n*-hexane solution) was added to a tetrahydrofuran solution (9.6 mL) of 5-methyl-1,3-benzenedicarboxylate 1,3-dimethyl ester (1.0 g) at -78°C under an argon atmosphere, and then the mixture was stirred at room temperature for 30 minutes. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as colorless liquid (692 mg).
¹H-NMR (400 MHz, CDCl₃) δ 1.69 (1H, t, J = 6.1 Hz), 2.41 (3H, s), 3.91 (3H, s), 4.72 (2H, d, J = 6.1 Hz), 7.40 (1H, s), 7.79 (1H, s), 7.83 (1H, s).

### <Reference Example 46-1>

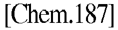

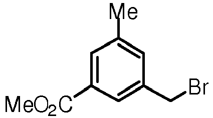

### Methyl 3-(bromomethyl)-5-methylbenzoate

Phosphorus tribromide (0.1 mL) was added to a chloroform solution (5.0 mL) of methyl 3-(hydroxymethyl)-5-methylbenzoate (180 mg) under an argon atmosphere under ice-cooling, and then the mixture was stirred at room temperature for 1 hour. A sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 4:1) to obtain a title compound as colorless liquid (112 mg).
¹H-NMR (400 MHz, CDCl₃) δ 2.40 (3H, s), 3.92 (3H, s), 4.48 (2H, s), 7.40 (1H, s), 7.79 (1H, s), 7.86 (1H, s).

### <Reference Example 47-1>

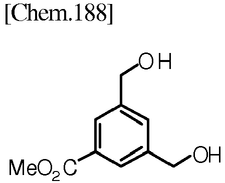

### Methyl 3,5-bis(hydroxymethyl)benzoate

A tetrahydrofuran solution (49 mL) of trimethyl 1,3,5-benzenetricarboxylate (5.0 g) was added to a tetrahydrofuran solution (50 mL) of lithium aluminium hydride (751 mg) under an argon atmosphere under ice-cooling, and then the mixture was stirred at room temperature for 1 hour. Water (0.8 mL), a 15% sodium hydroxide aqueous solution (0.8 mL), and water (2.3 mL) were added to the reaction solution and the suspension thus obtained was filtered off by Celite. The solvent was evaporated to obtain a title compound as a colorless powder (2.1 g).
¹H-NMR (400 MHz, CDCl₃) δ 1.78 (2H, t, J = 6.1 Hz), 3.93 (3H, s), 4.77 (4H, d, J = 6.1 Hz), 7.61 (1H, s), 7.96 (2H, s).

### <Reference Example 48-1>

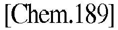

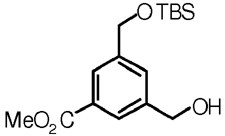

### Methyl 3-[[(tert-butyldimethylsilyl)oxy]methyl]-5-(hydroxymethyl)benzoate

*tert*-Butyldimethylchlorosilane (323 mg) and imidazole (175 mg) were added to an *N,N*-dimethylformamide solution (4.3 mL) of methyl 3,5-bis(hydroxymethyl)benzoate (420 mg) under an argon atmosphere under ice-cooling, and then the mixture was stirred at room temperature for 2 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as a yellow oil (282 mg).
¹H-NMR (400 MHz, CDCl₃) δ 0.11 (6H, s), 0.95 (9H, s), 1.77 (1H, t, J = 6.1 Hz), 3.92 (3H, s), 4.75 (2H, d, J = 6.1 Hz), 4.78 (2H, s), 7.56 (1H, s), 7.92 (2H, s).

### <Reference Example 49-1>

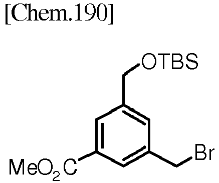

### Methyl 3-(bromomethyl)-5-[[(tert-butyldimethylsilyl)oxy]methyl]benzoate

Triphenylphosphine (286 mg) and carbon tetrabromide (421 mg) were added to a dichloromethane solution (4.5 mL) of methyl 3-[[(*tert*-butyldimethylsilyl)oxy]methyl]-5-(hydroxymethyl)benzoate (281 mg) at room temperature under an argon atmosphere, and then the mixture was stirred at room temperature for 30 minutes. The reaction solution was evaporated and then the residue was purified by silica gel column chromatography (*n*-hexane:ethyl acetate = 9:1) to obtain a title compound as a colorless oil (334 mg).
¹H-NMR (400 MHz, CDCl₃) δ 0.11 (6H, s), 0.95 (9H, s), 3.92 (3H, s), 4.52 (2H, s), 4.77 (2H, s), 7.58 (1H, s), 7.91 (1H, s), 7.94 (1H, s).

### <Reference Example 50-1>

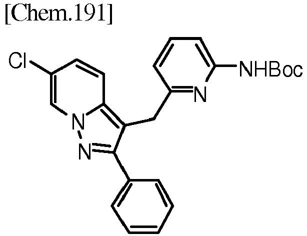

### tert-Butyl 6- [(6-chloro-2-phenylpyrazolo[1,5-a]pyridin-3-yl)methyl]pyridylcarbamate

Triethylamine (0.192 mL) and diphenylphosphoryl azide (0.296 mL) were added to a t-butanol solution (5.50 mL) of 6-[(6-chloro-2-phenylpyrazolo[1,5-a]pyridin-3-yl)methyl]pyridine-2-carboxylic acid (400 mg), and then the mixture was heated under reflux for 2 hours. Water was added to the reaction solution and the mixture was extracted twice with ethyl acetate. The combined ethyl acetate layer was washed with saturated saline and dried over anhydrous sodium sulfate, and then the solvent was evaporated under vacuum. The residue thus obtained was suspended in diisopropyl ether-ethyl acetate (1:1), the insolubles were filtered off, and then the filtrate was concentrated under vacuum. The residue thus obtained was purified by column chromatography (*n*-hexane:ethyl acetate = 80:20 to 0:100) to obtain a title compound as a yellow oil (543 mg).
¹H-NMR (400 MHz, CDCl₃) δ 1.52 (9H, s), 4.25 (2H, s), 6.66 (1H, d, J = 7.3 Hz), 7.04 (1H, dd, J = 9.6, 1.5 Hz), 7.26-7.27 (1H, m), 7.32 (1H, d, J = 9.6 Hz), 7.40-7.46 (5H, m), 7.53 (1H, t, J = 7.8 Hz), 7.70 (2H, d, J = 7.3 Hz), 8.51-8.52 (1H, m).

### <Reference Example 51-1>

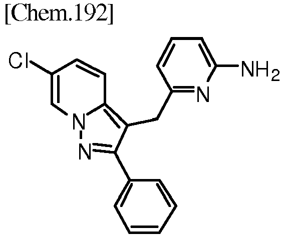

### 3-(6-Aminopyridin-2-yl)methyl-6-chloro-2-phenylpyrazolo[1,5-a]pyridine

4N ethyl acetate solution of hydrogen chloride (4.25 mL) was added to a methanol solution (2.00 mL) of *tert*-butyl 6-[(6-chloro-2-phenylpyrazolo[1,5-a]pyridin-3-yl)methyl]pyridylcarbamate (543 mg), and then the mixture was stirred at room temperature for 96 hours. A 1 mol/L sodium hydroxide aqueous solution was added to achieve pH 9 to 10 and the mixture was extracted twice with ethyl acetate. The mixture was washed with saturated saline together with the ethyl acetate layer and dried over anhydrous sodium sulfate, and then the solvent was evaporated under vacuum. The residue thus obtained was purified with triturate (diisopropyl ether:ethyl acetate = 3:1) to obtain a title compound as a colorless solid (320 mg).
¹H-NMR (400 MHz, CDCl₃) δ 4.20 (2H, s), 4.40 (2H, s), 6.31 (1H, d, J = 6.9 Hz), 6.34 (1H, d, J = 7.6 Hz), 7.02 (1H, dd, J = 9.4, 1.7 Hz), 7.27-7.30 (1H, m), 7.35-7.40 (2H, m), 7.42-7.46 (2H, m), 7.72-7.74 (2H, m), 8.51-8.52 (1H, m).

### <Reference Example 52-1>

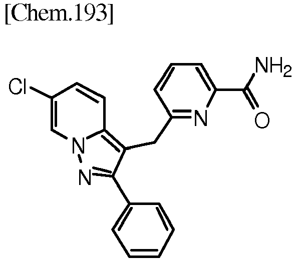

### 6-[(6-Chloro-2-phenylpyrazolo[1,5-a]pyridin-3-yl)methyl]pyridine-2-carboxamide

Ammonium chloride (220 mg), diisopropylethylamine (2.15 mL), and 2-(1*H*-7-azabenzotriazol-1-yl)-1,1,3,3-tetramethyluronium hexafluorophosphate methanaminium (HATU) (1.88 g) were added to an *N,N*-dimethylformamide solution (20.6 mL) of 6-[(6-chloro-2-phenylpyrazolo[1,5-a]pyridin-3-yl)methyl]pyridine-2-carboxylic acid (1.50 g) under ice-cooling, and then the mixture was stirred at room temperature for 2 hours. Water was added to the reaction solution, the insolubles were filtered off, and the filtrate was extracted twice with ethyl acetate. The combined ethyl aceteate layer was washed with water and saturated saline and dried over anhydrous sodium sulfate and then the solvent was evaporated under vacuum. The residue thus obtained was purified with triturate (ethyl acetate) and then mixed with the above-described product obtained by filtration to obtain a title compound as a yellow solid (1.31 g).
¹H-NMR (400 MHz, CDCl₃) δ 4.47 (2H, s), 7.26-7.30 (2H, m), 7.38-7.42 (1H, m), 7.43-7.48 (2H, m), 7.61 (1H, s), 7.65 (1H, s), 7.75-7.78 (2H, m), 7.79-7.83 (3H, m), 9.02 (1H, d, J =1.8 Hz).

### <Reference Example 53-1>

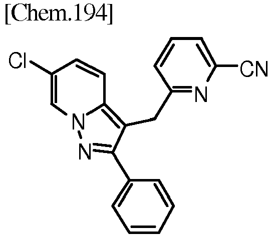

### 6-[(6-Chloro-2-phenylpyrazolo[1,5-a]pyridin-3-yl)methyl]pyridine-2-carbonitrile

Thionyl chloride (0.527 mL) was added to an *N,N*-dimethylformamide solution (18.1 mL) of 6-[(6-chloro-2-phenylpyrazolo[1,5-a]pyridin-3-yl)methyl]pyridine-2-carboxamide (1.31 g), and then the mixture was stirred at room temperature for 2 hours. Water and a saturated sodium bicarbonate aqueous solution were added to the reaction solution to achieve pH 7 and then the mixture was extracted twice with ethyl acetate. The combined ethyl acetate layer was washed with saturated saline and dried over anhydrous sodium sulfate, and then the solvent was evaporated under vacuum. The residue thus obtained was purified with triturate (ethyl acetate) and by column chromatography (n-hexane:ethyl acetate = 2:1) to obtain a title compound as a yellow solid (1.09 g).
¹H-NMR (400 MHz, CDCl₃) δ 4.44 (2H, s), 7.10 (1H, dd, J = 9.4, 1.7 Hz), 7.15 (1H, d, J = 8.0 Hz), 7.40-7.48 (4H, m), 7.52 (1H, d, J = 7.6 Hz), 7.62-7.67 (3H, m), 8.52-8.53 (1H, m).

### <Reference Example 54-1>

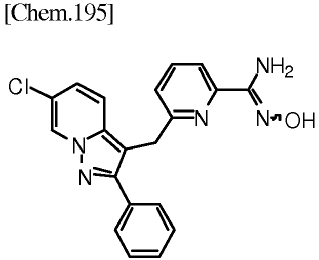

### 6-[(6-Chloro-2-phenylpyrazolo[1,5-a]pyridin-3-yl)methyl]-N-hydroxypyridine-2-carboxami dine

Hydroxylamine hydrochloride (12 mg) and triethylamine (0.024 mL) were added to an ethanol solution (0.435 mL) of 6-[(6-chloro-2-phenylpyrazolo[1,5-a]pyridin-3-yl)methyl]pyridine-2-carbonitrile (30 mg), and then the mixture was stirred at room temperature for 2 hours. The reaction solution was filtered off and then the obtained solid was washed with water to obtain a title compound as a pale yellow solid (33 mg).
¹H-NMR (400 MHz, CDCl₃) δ 4.40 (2H, s), 5.53 (2H, s), 6.50 (1H, s), 7.04-7.07 (2H, m), 7.36-7.47 (4H, m), 7.57 (1H, t, J = 7.8 Hz), 7.71-7.74 (3H, m), 8.53-8.54 (1H, m).

### <Reference Example 55-1>

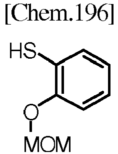

### 2-(Methoxymethoxy)benzenethiol

A methanol (35 mL) solution of iodine (10.0 g) was added to a water (50 mL) suspension of 2-mercaptophenol (10.0 g), then the mixture was stirred at room temperature for 10 minutes. Water and ethyl acetate were added to the reaction solution to separate it. The organic layer was washed with a saturated sodium thiosulfate aqueous solution, water, and saturated saline in this order, and dried over anhydrous sodium sulfate, and then the solvent was evaporated under vacuum to obtain bis(2-hydroxyphenyl)disulfide as a crude purified product. Diisopropylethylamine (30.7 g) and methoxymethyl chloride (15.9 g) were serially added to a tetrahydrofuran (10 mL) solution of the bis(2-hydroxyphenyl)disulfide (about 39.7 mmol) thus obtained at room temperature, and then the mixture was stirred at the same temperature for 3 hours. Water and ethyl acetate were added to the reaction solution to separate it. The organic layer was washed with a 10% citric acid aqueous solution and saturated saline in this order, and dried over anhydrous sodium sulfate, and then the solvent was evaporated under vacuum to obtain a methoxymethoxy (MOM) derivative as a crude purified product. Sodium borohydride (7.50 g) was added to an ethanol (50 mL) solution of the MOM derivative (14.0 g) thus obtained, and then the mixture was stirred at room temperature for 3 hours. Ethyl acetate and a 10% citric acid aqueous solution were added to the reaction solution to separate it. The organic layer was extracted with a 2 mol/L sodium hydroxide aqueous solution and the aqueous layer was washed with diethyl ether to achieve pH = 6 with concentrated hydrochloric acid. The aqueous layer was extracted with diethyl ether, the organic layer was washed with saturated saline and then dried over anhydrous sodium sulfate. The solvent was evaporated under vacuum to obtain a title compound as a colorless oil (10.0 g).
¹H-NMR (400 MHz, CDCl₃) δ 3.51 (3H, s), 3.78 (1H, s), 5.24 (2H, s), 6.88-7.12 (4H, m),

### <Reference Example 56-1>

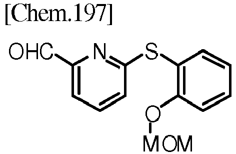

### 6-[2-(Methoxymethoxy)phenylthio]-2-pyridinecarboxaldehyde

2-(Methoxymethoxy)benzenethiol (1.10 g) and potassium carbonate (1.12 g) were added to an *N,N*-dimethylformamide (5 mL) solution of 6-bromo-2-pyridinecarboxaldehyde (1.00 g), and then the mixture was stirred at 100°C for 1 hour. The reaction solution was cooled down to room temperature, water and ethyl acetate were added, and the mixture was separated. The organic layer was washed with water and saturated saline and dried over anhydrous sodium sulfate. The solvent was concentrated under vacuum, the residue thus obtained was purified by silica gel chromatography (n-hexane:ethyl acetate = 7:1), and the fraction-concentrated residue thus obtained was washed with diisopropyl ether to obtain a title compound as a colorless crystal (1.10 g).
¹H-NMR (400 MHz, CDCl₃ δ 3.34 (3H, s), 5.17 (2H, s), 7.04 (1H, dd, J = 8.6, 1.2 Hz), 7.07-7.12 (1H, m), 7.25-7.29 (1H, m), 7.40-7.50 (1H, m), 7.56-7.68 (3H, m), 9.67 (1H, s).

### <Reference Example 57-1>

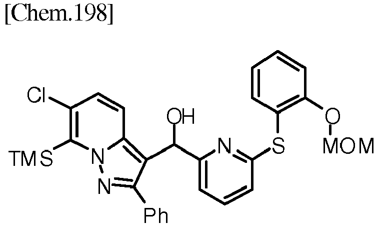

### 6-Chloro-3-[hydroxy[6-[2-(methoxymethoxy)phenylthio]pyridin-2-yl]methyl]-2-phenyl-7-(t rimethylsilyl)pyrazolo[1,5-a]pyridine

*n*-Butyllithium (0.77 mL, a 1.63 mol/L *n*-hexane solution) was added to a tetrahydrofuran (3 mL) solution of 3-bromo-6-chloro-2-phenyl-7-(trimethylsilyl)pyrazolo[1,5-a]pyridine (0.400 g) at -78°C, and then the mixture was stirred at the same temperature for 15 minutes. Then a tetrahydrofuran (3 mL) solution of 6-[2-(methoxymethoxy)phenylthio]-2-pyridinecarboxaldehyde (0.433 g) was added at the same temperature, and then the mixture was stirred for 15 minutes. Subsequently, the mixture was heated up to room temperature, water and ethyl acetate were added, and the mixture was separated. The organic layer was washed with water and saturated saline and dried over anhydrous sodium sulfate. The solvent was concentrated under vacuum and the residue thus obtained was purified by silica gel chromatography (*n*-hexane:ethyl acetate = 4:1), and then the fraction-concentrated residue thus obtained was washed with acetonitrile to obtain a title compound as a colorless crystal (0.353 g).
¹H-NMR (400 MHz, CDCl₃) δ 0.53 (9H, s), 3.38 (3H, s), 5.10 (1H, d, J =1.8 Hz), 5.19 (2H, s), 6.08 (1H, br), 6.68 (1H, d, J = 8.6 Hz), 6.83-6.90 (3H, m), 7.05-7.15 (1H, m), 7.23-7.28 (1H, m), 7.33 (1H, t, J = 8.6 Hz), 7.38-7.48 (4H, m), 7.64 (1H, dd, J = 8.6, 1.8 Hz), 7.79-7.84 (2H, m).

### <Reference Example 58-1>

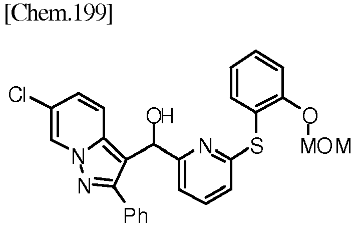

### 6-Chloro-3-[hydroxy[6-[2-(methoxymethoxy)phenylthio]pyridin-2-yl]methyl]-2-phenylpyra zolo[1,5-a]pyridine

A 1 mol/L tetrahydrofuran solution of tetrabutylammonium fluoride (1.12 mL) was added to a tetrahydrofuran solution (5 mL) of 6-chloro-3-[hydroxy[6-[2-(methoxymethoxy)phenylthio]pyridin-2-yl]methyl]-2-phenyl-7-(tr imethylsilyl)pyrazolo[1,5-a]pyridine (0.323 g), and then the mixture was stirred at room temperature for 30 minutes. A saturated ammonium chloride aqueous solution and ethyl acetate were added to the reaction solution to separate it. The organic layer was washed with saturated saline and dried over anhydrous sodium sulfate and then the solvent was evaporated under vacuum to obtain a title compound as a colorless amorphous (0.272 g).
¹H-NMR (400 MHz, CDCl₃) δ 3.38 (3H, s), 5.10 (1H, d, J = 3.1 Hz), 5.19 (2H, s), 6.02 (1H, d, J = 3.1 Hz), 6.63 (1H, d, J = 8.6 Hz), 6.85-6.99 (3H, m), 7.08-7.13 (1H, m), 7.23-7.27 (1H, m), 7.33 (1H, t, J = 8.6 Hz), 7.40-7.50 (4H, m), 7.64 (1H, dd, J = 8.6, 1.8 Hz), 7.75-7.79 (2H, m), 8.48 (1H, d,J =1.8 Hz).

### <Reference Example 59-1>

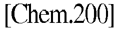

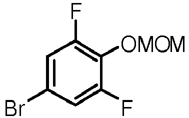

### 5-Bromo-1,3-difluoro-2-(methoxymethoxy)benzene

Diisopropylethylamine (3.10 g) and methoxymethyl chloride (1.45 g) were serially added to a tetrahydrofuran (1 mL) solution of 4-bromo-2,6-difluorophenol (2.50 g) at room temperature, and then the mixture was stirred at the same temperature for 1 hour. Water and ethyl acetate were added to the reaction solution to separate it. The organic layer was washed with a 10% citric acid aqueous solution and saturated saline in this order, and dried over anhydrous sodium sulfate, and then the solvent was evaporated under vacuum to obtain a title compound as a colorless oil (3.05 g).
¹H-NMR (400 MHz, CDCl₃) δ 3.58 (3H, s), 5.13 (2H, s), 7.05-7.14 (2H, m).

### <Reference Example 60-1>

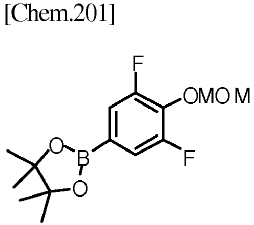

### 2-[3,5-Difluoro-4-(methoxymethoxy)phenyl]-4,4,5,5-tetramethyl-1,3,2-dioxaborolane

Bispinacoldiboron (2.21 g), potassium 2-ethylhexanoate (1.73 g), and Pd(dppf)Cl₂·CH₂Cl₂ (0.322 g) were serially added to a 1,4-dioxane (30 mL) solution of 5-bromo-1,3-difluoro-2-(methoxymethoxy)benzene (2.00 g), and then the mixture was stirred at 80°C for 4 hours. The reaction solution was cooled down to room temperature, the solvent was evaporated under vacuum, and then the residue thus obtained was purified by silica gel chromatography (n-hexane:ethyl acetate = 10:1) to obtain a title compound as a colorless oil (2.50 g). The compound thus obtained was not particularly purified and used in the next process.
¹H-NMR (400 MHz, CDCl₃) δ 1.32 (12H, s), 3.60 (3H, s), 5.19 (2H, s), 7.29-7.32 (2H, m).

### <Reference Example 61-1>

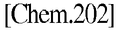

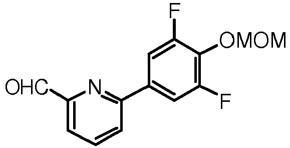

### 6-[3,5-difluoro-4-(methoxymethoxy)phenyl]pyridine-2-carboxaldehyde

A water (3 mL) solution of potassium carbonate (3.63 g) and tetrakistriphenylphosphinepalladium (0) (0.608 g) were serially added to a 1,4-dioxane (10 mL) solution of 2-[3,5-difluoro-4-(methoxymethoxy)phenyl]-4,4,5,5-tetramethyl-1,3,2-dioxaborolane (2.50 g) and 6-bromo-2-pyridinealdehyde (0.979 g), and then the mixture was heated and stirred under reflux for 3 hours. The reaction solution was cooled down to room temperature and water and ethyl acetate were added and the mixture was separated. The organic layer was washed with water and saturated saline and dried over anhydrous sodium sulfate. The solvent was concentrated under vacuum, the residue thus obtained was purified by silica gel chromatography (n-hexane:ethyl acetate = 8:1), and then the fraction-concentrated residue thus obtained was recrystallized from *n*-hexane:ethyl acetate = 4:1 to obtain a title compound as a colorless crystal (1.00 g).
¹H-NMR (400 MHz, CDCl₃) δ 3.63 (3H, s), 5.24 (2H, s), 7.66-7.75 (2H, m), 7.85-7.98 (3H, m), 10.14 (1H, s).

### <Reference Example 62-1>

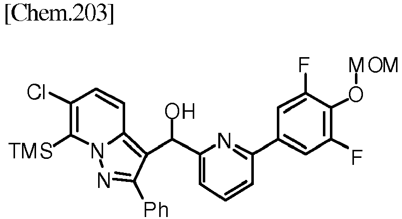

### 6-Chloro-3-[hydroxy[6-[3,5-difluoro-4-(methoxymethoxy)phenyl]pyridin-2-yl]methyl]-2-ph enyl-7-(trimethylsilyl)pyrazolo[1,5-a]pyridine

*n*-Butyllithium (0.89 mL, a 1.63 mol/L *n*-hexane solution) was added to a tetrahydrofuran (3 mL) solution of 3-bromo-6-chloro-2-phenyl-7-(trimethylsilyl)pyrazolo[1,5-a]pyridine (0.500 g) at -78°C, and then the mixture was stirred at the same temperature for 15 minutes. Subsequently, a tetrahydrofuran (3 mL) solution of 6-[3,5-difluoro-4-(methoxymethoxy)phenyl]pyridine-6-carboxaldehyde (0.478 g) was added at the same temperature, and then the mixture was stirred for 15 minutes. Then the mixture was heated up to room temperature and water and ethyl acetate were added and the mixture was separated. The organic layer was washed with water and saturated saline and dried over anhydrous sodium sulfate. The solvent was concentrated under vacuum and the residue thus obtained was purified by silica gel chromatography (*n*-hexane:ethyl acetate = 10:1 1 to 6:1) to obtain a title compound as a colorless amorphous (0.589 g).
¹H-NMR (400 MHz, CDCl₃) δ 0.60 (9H, s), 3.64 (3H, s), 5.25 (2H, s), 5.44 (1H, br), 6.27 (1H, br), 6.85 (1H, d, J = 8.6 Hz), 6.94 (1H, d, J = 8.6 Hz), 7.01 (1H, d, J = 8.6 Hz), 7.35-7.45 (1H, m), 7.46-7.50 (2H, m), 7.57 (1H, d, J = 8.6 Hz), 7.63-7.69 (3H, m), 7.84-7.89 (2H, m).

### <Reference Example 63-1>

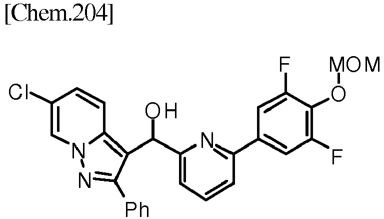

### 6-Chloro-3-[hydroxy[6-[3,5-difluoro-4-(methoxymethoxy)phenyl]pyridin-2-yl]methyl]-2-ph enylpyrazolo[1,5-a]pyridine

A 1 mol/L tetrahydrofuran solution of tetrabutylammonium fluoride (1.97 mL) was added to a tetrahydrofuran solution (3 mL) of 6-chloro-3-[hydroxy[6-[3,5-difluoro-4-(methoxymethoxy)phenyl]pyridin-2-yl]methyl]-2-ph enyl-7-(trimethylsilyl)pyrazolo[1,5-a]pyridine (0.570 g), and then the mixture was stirred at room temperature for 30 minutes. A saturated ammonium chloride aqueous solution and ethyl acetate were added to the reaction solution to separate it. The organic layer was washed with saturated saline and dried over anhydrous sodium sulfate, and then the solvent was evaporated under vacuum. The residue thus obtained was purified by silica gel chromatography (*n*-hexane:ethyl acetate = 8:1 to 1:1) to obtain a title compound as a colorless amorphous (0.500 g).
¹H-NMR (400 MHz, CDCl₃) δ 3.64 (3H, s), 5.26 (2H, s), 5.41 (1H, d, J = 3.1 Hz), 6.21 (1H, d, J = 3.1 Hz), 6.92-6.99 (2H, m), 7.02-7.06 (1H, m), 7.40-7.60 (3H, m), 7.57 (1H, d, J = 8.6 Hz), 7.64-7.69 (3H, m), 7.81-7.85 (2H, m), 8.51 (1H, d, J =1.8 Hz).

### <Reference Example 64-1>

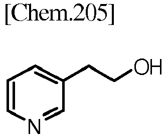

### 2-(Pyridin-3-yl)ethanol

Ethyl 3-pyridylacetate (5 g) was added to a tetrahydrofuran solution (101 mL) of lithium aluminium hydride (3.4 g) under an argon atmosphere under ice-cooling, and then the mixture was stirred for 1 hour under ice-cooling. Water (3.4 mL), a 15% sodium hydroxide aqueous solution (3.4 mL), and water (10.2 mL) were added to the reaction solution and then the suspension thus obtained was filtered off on Celite. The solvent was evaporated to obtain a title compound as yellow liquid (4.44 g).
¹H-NMR (400 MHz, CDCl₃) δ 2.83 (2H, t, J = 6.7 Hz), 3.84 (2H, t, J = 6.7 Hz), 7.20 (1H, ddd, J = 7.9, 4.8, 1.8 Hz), 7.57 (1H, dt, J = 7.9, 1.8 Hz), 8.33 (1H, dd, J = 4.8, 1.8 Hz), 8.36 (1H, d, J = 1.8 Hz).

### <Reference Example 65-1>

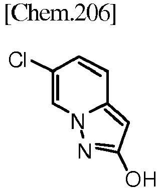

### 6-Chloro-2-hydroxypyrazolo[1,5-a]pyridine

50% Sulfuric acid (7.5 mL) was added to 2-amino-6-chloropyrazolo[1,5-a]pyridine (250 mg), and then the mixture was heated and stirred at 100°C for 2 hours. The reaction solution was cooled down and then poured into a saturated sodium bicarbonate aqueous solution to achieve pH = 11, then extracted with ethyl acetate, and dried over anhydrous sodium sulfate. The solvent was evaporated to obtain a title compound as a yellow solid (196 mg).
¹H-NMR (400 MHz, DMSO-d₆) δ 7.13 (1H, dd, J = 9.1, 1.8 Hz), 7.40 (1H, d, J = 9.1 Hz), 8.65 (1H, d, J = 1.8 Hz), 10.62 (1H, brs).

### <Reference Example 66-1>

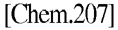

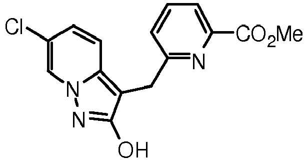

### Methyl 6-[(6-chloro-2-hydroxypyrazolo[1,5-a]pyridin-3-yl)methyl]pyridine-2-carboxylate

Trifluoroacetic acid (0.15 mL) and triethylsilane (0.43 mL) were serially added to a dichloromethane (9.5 mL) solution of 6-chloro-2-hydroxypyrazolo[1,5-a]pyridine (160 mg) and methyl 6-formylpyridine-2-carboxylate (235 mg) at room temperature, and then the mixture was stirred at room temperature for 5 hours. A saturated sodium bicarbonate aqueous solution was added to the reaction solution (pH = 7 to 8) under ice-cooling and then the mixture was extracted with ethyl acetate and dried over anhydrous sodium sulfate. The solvent was evaporated and the residue thus obtained was purified by silica gel chromatography (*n*-hexane:ethyl acetate = 1:1) to obtain a title compound as a colorless crystal (243 mg).
¹H-NMR (400 MHz, DMSO-d₆) δ 3.88 (3H, s), 4.11 (2H, s), 7.11 (1H, dd, J = 9.9, 1.8 Hz), 7.35-7.43(1H, m), 7.42 (1H, d, J = 9.8 Hz), 7.83-7.93 (2H, m), 8.65 (1H, s), 10.94 (1H, brs).

### <Reference Example 67-1>

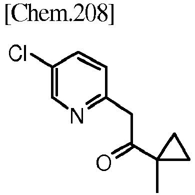

### 2-(5-Chloropyridin-2-yl)-1-(1-methylcyclopropyl)ethanone

Methyl 1-methyl cyclopropanecarboxylate (3.70 mL) and lithium bistrimethylsilylamide (50.0 mL, a 1.00 mol/L tetrahydrofuran solution) were added to a tetrahydrofuran solution (30 mL) of 5-chloro-2-methylpyridine (2.00 g) under an argon atmosphere under ice-cooling, and then the mixture was stirred for 2 hours under ice-cooling. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 3:1) to obtain a title compound as a colorless oil (2.70 g).
¹H-NMR (400 MHz, CDCl₃) δ 0.80 (2H, q, J = 3.6 Hz), 1.37 (2H, q, J = 3.6 Hz),1.40 (3H, s), 3.89 (2H, s), 7.19 (1H, d, J = 8.5 Hz), 7.61 (1H, dd, J = 8.5, 2.4 Hz), 8.48 (1H, d, J = 2.4 Hz).

### <Reference Example 67-2>

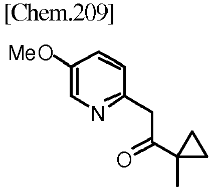

### 2-(5-Methoxypyridin-2-yl)-1-(1-methylcyclopropyl)ethanone

Methyl 1-methylcyclopropanecarboxylate (3.70 mL) and lithium bistrimethylsilylamide (50.0 mL, a 1.00 mol/L tetrahydrofuran solution) were added to a tetrahydrofuran solution (30 mL) of 5-methoxy-2-methylpyridine (2.00 g) under an argon atmosphere under ice-cooling, and then the mixture was stirred under ice-cooling for 2 hours, then at room temperature for 12 hours, and then at 70°C for 12 hours. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 1:1) to obtain a title compound as a yellow oil (2.11 g).
¹H-NMR (400 MHz, CDCl₃) δ 0.77 (2H, dd, J = 6.7, 3.6 Hz), 1.37 (2H, dd, J = 6.7, 3.6 Hz), 1.39 (3H, s), 3.84 (3H, s), 3.85 (2H, s), 7.16 (2H, s), 8.22 (1H, s).

### <Reference Example 68-1>

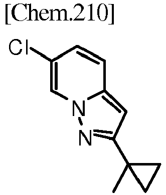

### 6-Chloro-2-(1-methylcyclopropyl)pyrazolo[1,5-a]pyridine

A 70% perchloric acid aqueous solution (1.80 mL) was added to a 1,4-dioxane solution (4.2 mL) of ethyl O-mesitylsulfonylacetohydroxamate (4.80 g) under an argon atmosphere under ice-cooling, and then the mixture was stirred for 30 minutes under ice-cooling. Ice water was added to the reaction solution, the precipitated solid was filtered off, the obtained solid was dissolved in dichloromethane (7.0 mL), and the solution was divided into layers. The organic layer was dried over anhydrous magnesium sulfate and filtered off. A dichloromethane solution (7.0 mL) of 2-(5-chloropyridin-2-yl)-1-(1-methylcyclopropyl)ethanone (2.00 g) was added to the obtained filtrate under ice-cooling and then the mixture was stirred at room temperature for 1 hour and then the reaction solution was concentrated under vacuum.
An *N,N*-dimethylformamide solution (14.0 mL) of the crude product was stirred at room temperature for 24 hours under an argon atmosphere. Water was added to the reaction solution and the mixture was extracted with ethyl acetate. The organic layer was washed with saturated saline and then dried over anhydrous magnesium sulfate. The solvent was evaporated and then the residue was purified by silica gel column chromatography *(n-hexane:ethyl* acetate = 10:1) to obtain a title compound as a yellow solid (919 mg).
¹H-NMR (400 MHz, CDCl₃) δ 0.84 (2H, q, J = 3.6 Hz), 1.10 (2H, q, J = 3.6 Hz), 1.53 (3H, s), 6.27 (1H, s), 7.00 (1H, dd, J = 9.1,1.8 Hz), 7.32 (1H, d, J = 9.1 Hz), 8.39 (1H, s).

### <Reference Example 68-2>

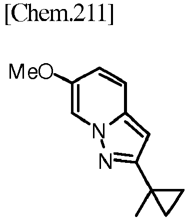

### 6-Methoxy-2-(1-methylcyclopropyl)pyrazolo[1,5-a]pyridine

A 70% perchloric acid aqueous solution (1.40 mL) was added to a 1,4-dioxane solution (3.5 mL) of ethyl *O*-mesitylsulfonylacetohydroxamate (3.70 g) under an argon atmosphere under ice-cooling, and then the mixture was *stirred* for 30 minutes under ice-cooling. Ice water was added to the reaction solution, the precipitated solid was filtered off, the obtained solid was dissolved in dichloromethane (6.0 mL), and the solution was divided into layers. The organic layer was dried over anhydrous magnesium sulfate and filtered off. A dichloromethane solution (6.0 mL) of 2-(5-methoxypyridin-2-yl)-1-(1-methylcyclopropyl)ethanone (2.00 g) was added to the obtained filtrate under ice-cooling and then the mixture was stirred at room temperature for 1 hour and then the reaction solution was concentrated under vacuum.
An *N,N*-dimethylformamide solution (11.0 mL) of the crude product was stirred at room temperature for 24 hours under an argon atmosphere. Water was added to the reaction solution and the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 5:1) to obtain a title compound as a colorless solid (1.07 g).
¹H-NMR (400 MHz, CDCl₃) δ 0.81 (2H, dd, J = 6.1, 4.2 Hz), 1.08 (2H, dd, J = 6.1, 4.2 Hz), 1.53 (3H, s), 6.19 (1H, s), 6.84 (1H, dd, J = 9.7, 2.4 Hz), 7.26 (1H, d, J = 9.7 Hz), 7.98 (1H, d, J = 2.4 Hz).

### <Reference Example 69-1>

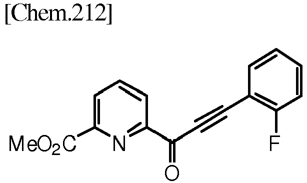

### Methyl 6-[3-(2-fluorophenyl)-1-oxo-2-propyn-1-yl]pyridine-2-carboxylate

After thionyl chloride (10.1 mL) was added to a toluene (47.0 mL) suspension of 2-carboxy-6-(methoxycarbonyl)pyridine (2.54 g) under an argon atmosphere, the mixture was heated and stirred at 60°C for 3 hours. The solvent was evaporated under vacuum, then tetrahydrofuran (140 mL), 1-ethynyl-2-fluorobenzene (2.02 g), copper (I) iodide (80.0 mg), dichlorobis(triphenylphosphine)palladium (II) (98.3 mg), and triethylamine (2.44 mL) were added, and then the mixture was stirred at room temperature for 2 hours. The reaction solution was concentrated and then purified by silica gel column chromatography (*n*-hexane:ethyl acetate = 4:1 to 1:1) to obtain a title compound as a dark green solid (1.45 g). ¹H NMR (400 MHz, CDCl₃) δ 4.08 (3H, s), 7.16-7.25 (2H, m), 7.48-7.54 (1H, m), 7.76 (1H, td, J = 7.9, 1.2 Hz), 8.07 (1H, t, J = 7.9 Hz), 8.36 (2H, dd, J = 7.9, 1.2 Hz).

### <Reference Example 69-2>

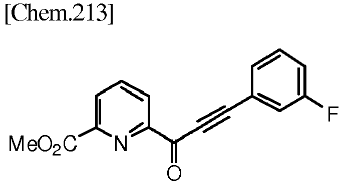

### Methyl 6-[3-(3-fluorophenyl)-1-oxo-2-propyn-1-yl]pyridine-2-carboxylate

After thionyl chloride (10.1 mL) was added to a toluene (47.0 mL) suspension of 2-carboxy-6-(methoxycarbonyl)pyridine (2.54 g) under an argon atmosphere, the mixture was heated and stirred at 60°C for 3 hours. The solvent was evaporated under vacuum, then tetrahydrofuran (140 mL), 1-ethynyl-3-fluorobenzene (2.02 g), copper (I) iodide (80.0 mg), dichlorobis(triphenylphosphine)palladium (II) (98.3 mg), and triethylamine (2.44 mL) were added, and then the mixture was stirred at room temperature for 2 hours. The reaction solution was concentrated and then purified by silica gel column chromatography (*n*-hexane:ethyl acetate = 4:1 to 1:1), then the crude product thus obtained was triturated with diisopropyl ether to obtain a title compound as a gray solid (993 mg).
¹H NMR (400 MHz, CDCl₃) δ 4.08 (3H, s), 7.22 (1H, tt, J = 8.5, 1.8 Hz), 7.42 (1H, td, J = 7.9, 6.1 Hz), 7.50 (1H, dq, J = 9.1, 1.2 Hz), 7.57 (1H, dt, J = 7.9, 1.2 Hz), 8.06 (1H, t, J = 7.9 Hz), 8.33 (1H, d, J = 7.9 Hz), 8.36 (1H, dd, J = 7.9, 1.2 Hz).

### <Reference Example 70-1>

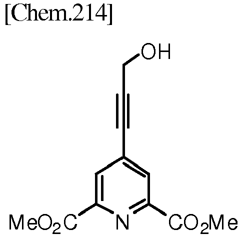

### Dimethyl 4-(3-hydroxy-1-propyn-1-yl)pyridine-2,6-dicarboxylate

Copper (I) iodide (180 mg), propargylalcohol (1.33 g), dichlorobis(triphenylphosphine)palladium (II) (332 mg), and diisopropylamine (95 mL) were added to a tetrahydrofuran (95 mL) solution of dimethyl 4-bromopyridine-2,6-dicarboxylate (7.31 g) under an argon atmosphere, and then the mixture was stirred at room temperature for 6 hours. The insolubles were filtered off, then the filtrate was evaporated under vacuum, and ethyl acetate and water were added to the residue thus obtained to extract it. The organic layer was washed with saturated saline and then dried over anhydrous sodium sulfate. The solvent was evaporated under vacuum and then the residue was purified by silica gel column chromatography (*n*-hexane:ethyl acetate = 1:1 to 1:2) to obtain a title compound as a colorless solid (3.08 g).
¹H NMR (400 MHz, CDCl₃) δ 1.75 (1H, t, J = 6.1 Hz), 4.03 (6H, s), 4.56 (2H, d, J = 6.1 Hz), 8.28 (2H, s).

### <Reference Example 71-1>

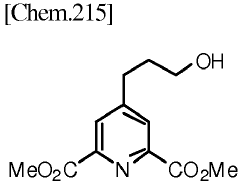

### Dimethyl 4-(3-hydroxypropyl)pyridine-2,6-dicarboxylate

10% Pd/C (300 mg) was added to an ethyl acetate (120 mL) solution under an argon atmosphere and the reaction vessel was purged with hydrogen and vigorously stirred. The reaction vessel was then purged with argon again, then dimethyl 4-(3-hydroxy-1-propyn-1-yl)pyridine-2,6-dicarboxylate (3.00 g) was added, then the mixture was replaced with hydrogen and vigorously stirred at room temperature for 6 hours. The insolubles were filtered off on Celite, then the filtrate was evaporated under vacuum, and then the residue was triturated with diisopropyl ether to obtain a title compound as a colorless solid (2.63 g).
¹H NMR (400 MHz, CDCl₃) δ 1.35 (1H, t, J = 4.8 Hz), 1.92-2.02 (2H, m), 2.91 (2H, t, J = 7.9 Hz), 3.71 (2H, q, J = 5.7 Hz), 4.02 (6H, s), 8.18 (2H, s).

### <Reference Example 72-1>

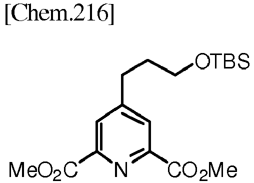

### Dimethyl 4-[3-[(tert-butyldimethylsilyl)oxy]propyl]pyridine-2,6-dicarboxylate

Imidazole (645 mg) and *tert*-butyldimethylchlorosilane (714 mg) were added to an *N,N*-dimethylformamide (11.8 mL) solution of dimethyl 4-(3-hydroxypropyl)pyridine-2,6-dicarboxylate (600 mg) under an argon atmosphere, and then the mixture was stirred at room temperature for 3 hours. Water was added to the reaction solution and the mixture was extracted with ethyl acetate. The organic layer was washed with saturated saline and then dried over anhydrous sodium sulfate. The solvent was evaporated under vacuum and then the residue was purified by silica gel column chromatography (*n*-hexane:ethyl acetate = 1:0 to 1:1) to obtain a title compound as a colorless oil (820 mg).
¹H NMR (400 MHz, CDCl₃) δ 0.06 (6H, s), 0.91 (9H, s), 1.87-1.96 (2H, m), 2.88 (2H, t, J = 7.6 Hz), 3.64 (2H, t, J = 5.8 Hz), 4.03 (6H, s), 8.17 (2H, s).

### <Reference Example 73-1>

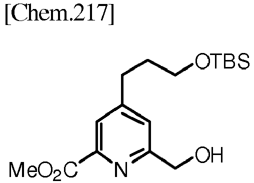

### Methyl 4-[3-[(tert-butyldimethylsilyl)oxy]propyl]-6-(hydroxymethyl)pyridine-2-carboxylate

Sodium borohydride (123 mg) was added to a methanol (48 mL) solution of dimethyl 4-[3-[(*tert*-butyldimethylsilyl)oxy]propyl]pyridine-2,6-dicarboxylate (800 mg) under ice-cooling, and then the mixture was stirred for 3 hours. A hydrogen chloride aqueous solution (1 mol/L) was slowly added to the reaction solution to achieve pH = 7 and the solvent was evaporated under vacuum. The residue thus obtained was purified by silica gel column chromatography (*n*-hexane:ethyl acetate = 2:3 to 1:4) to obtain a title compound as a colorless oil (508 mg).
¹H NMR (400 MHz, CDCl₃) δ 0.05 (6H, s), 0.91 (9H, s), 1.82-1.91 (2H, m), 2.79 (2H, t, J = 7.6 Hz), 3.31 (1H, t, J = 5.4 Hz), 3.63 (2H, t, J = 6.1 Hz), 3.99 (3H, s), 4.82 (2H, d, J = 5.4 Hz), 7.35 (1H, s), 7.89 (1H, s).

### <Reference Example 73-2>

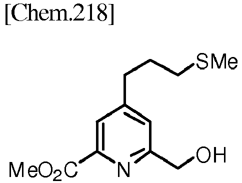

### Methyl 6-(hydroxymethyl)-4-[3-(methylthio)-propyl]pyridine-2-carboxylate

Sodium borohydride (340 mg) was added to a methanol (133 mL) solution of dimethyl 4-[3-(methylthio)propyl]pyridine-2,6-dicarboxylate (1.70 g) under ice-cooling, and then the mixture was stirred for 3 hours. A hydrogen chloride aqueous solution (1 mol/L) was slowly added to the reaction solution to achieve pH = 7 and then the solvent was evaporated under vacuum. The residue thus obtained was purified by silica gel column chromatography (*n*-hexane:ethyl acetate = 3:1 to 1:4) to obtain a title compound as a colorless oil (1.17 g).
¹H NMR (400 MHz, CDCl₃) δ 1.92-2.02 (2H, m), 2.11 (3H, d, J = 2.4 Hz), 2.52 (2H, td, J = 7.0,2.4 Hz), 2.81-2.85 (2H, m), 3.52-3.70 (1H, m), 3.99 (3H, d, J = 3.0 Hz), 4.84 (2H, d, J = 5.4 Hz), 7.40 (1H, s), 7.89 (1H, s).

### <Reference Example 74-1>

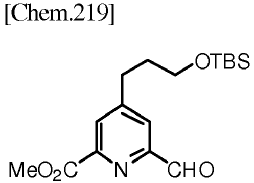

### Methyl 4-[3-[(tert-butyldimethylsilyl)oxy]propyl]-6-formylpyridine-2-carboxylate

Manganese dioxide (1.28 g) was added to a dichloromethane (18 mL) solution of methyl 4-[3-[(*tert*-butyldimethylsilyl)oxy]propyl]-6-(hydroxymethyl)pyridine-2-carboxylate (500 mg) under an argon atmosphere, and then the mixture was stirred at room temperature for 20 hours. The insolubles were filtered off on Celite, then the filtrate was evaporated under vacuum, and then the residue was purified by silica gel column chromatography (*n*-hexane:ethyl acetate = 1:0 to 2:1) to obtain a title compound as a colorless solid (320 mg).
¹H NMR (400 MHz, CDCl₃) δ 0.05 (6H, s), 0.90 (9H, s), 1.85-1.95 (2H, m), 2.88 (2H, t, J = 7.9 Hz), 3.64 (2H, t, J = 6.1 Hz), 4.06 (3H, s), 7.99 (1H, d, J = 1.2 Hz), 8.20 (1H, d, J = 1.2 Hz),10.18(1H,s).

### <Reference Example 74-2>

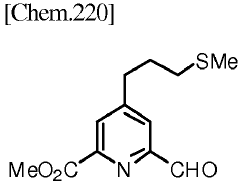

### Methyl 6-formyl-4-[3-(methylthio)propyl]pyridine-2-carboxylate

Manganese dioxide (3.98 g) was added to a dichloromethane (57 mL) solution of methyl 6-(hydroxymethyl)-4-[3-(methylthio)propyl]pyridine-2-carboxylate (1.17 g) under an argon atmosphere, and then the mixture was stirred at room temperature for 3 hours. The insolubles were filtered off on Celite, then the filtrate was evaporated under vacuum, and then the residue was purified by silica gel column chromatography (*n*-hexane:ethyl acetate = 1:0 to 2:1) to obtain a title compound as a pale yellow solid (786 mg).
¹H NMR (400 MHz, CDCl₃) δ 1.96-2.04 (2H, m), 2.11 (3H, s), 2.54 (2H, t, J = 7.0 Hz), 2.92 (2H, t, J = 7.6 Hz), 4.07 (3H, s), 7.99 (1H, d, J = 1.2 Hz), 8.21 (1H, d, J = 1.2 Hz), 10.18 (1H, s).

### <Reference Example 75-1>

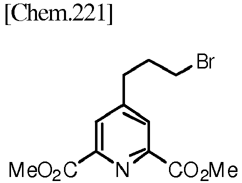

### Dimethyl 4-(3-bromopropyl)pyridine-2,6-dicarboxylate

Imidazole (755 mg), carbon tetrabromide (3.68 g), and triphenylphosphine (2.49 g) were added to a dichloromethane (78.9 mL) solution of dimethyl 4-(3-hydroxypropyl)pyridine-2,6-dicarboxylate (2.00 g) under an argon atmosphere, and then the mixture was stirred at room temperature for 10 minutes. The reaction solution was purified by silica gel column chromatography *(n-hexane:ethyl* acetate = 1:0 to 1:1) to obtain a title compound as a colorless solid (2.23 g).
¹H NMR (400 MHz, DMSO-d₆) δ 2.13-2.20 (2H, m), 2.91 (2H, t, J = 7.6 Hz), 3.51 (2H, t, J = 6.7 Hz), 3.90 (6H, s), 8.14 (2H, s).

### <Reference Example 76-1>

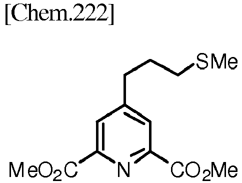

### Dimethyl 4-[3-(methylthio)propyl]pyridine-2,6-dicarboxylate

Sodium thiomethoxide (585 mg) was added to a methanol (34.8 mL) suspension of dimethyl 4-(3-bromopropyl)pyridine-2,6-dicarboxylate (2.20 g) under an argon atmosphere, and then the mixture was stirred at room temperature for 2 hours. A saturated ammonium chloride aqueous solution was added to the reaction solution, and then the mixture was extracted with ethyl acetate. The organic layer was washed with saturated saline and then dried over anhydrous sodium sulfate. The solvent was evaporated under vacuum and then the residue was purified by silica gel column chromatography (*n*-hexane:ethyl acetate = 1:0 to 1:1) to obtain a title compound as a colorless solid (1.75 g).
¹H NMR (400 MHz, CDCl₃) δ 1.97-2.04 (2H, m), 2.11 (3H, s), 2.54 (2H, t, J = 7.0 Hz), 2.91 (2H, t, J = 7.9 Hz), 4.02 (6H, s), 8.17 (2H, s).

### <Example 1-1>

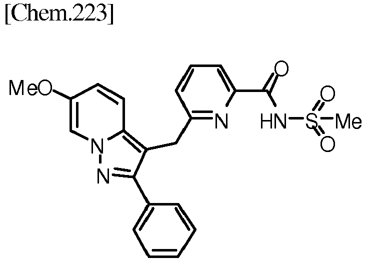

### N-Methanesulfonyl-6-[(6-methoxy-2-phenylpyrazolo[1,5-a]pyridin-3-yl)methyl]pyridine-2-carboxamide

Methanesulfonamide (67 mg), dimethylaminopyridine (21 mg), and 1-(3-dimethylaminopropyl)-3-ethylcarbodiimide hydrochloride (33 mg) were added to an *N,N*-dimethylformamide solution (0.7 mL) of 6-[(6-methoxy-2-phenylpyrazolo[1,5-a]pyridin-3-yl)methyl]pyridine-2-carboxylic acid (50 mg) at room temperature under an argon atmosphere, and then the mixture was stirred at room temperature for 1 hour. A 1 mol/L hydrochloric acid was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (ethyl acetate) to obtain a title compound as colorless liquid (45 mg).
¹H-NMR (400 MHz, CDCl₃) δ 3.40 (3H, s), 3.86 (3H, s), 4.43 (2H, s), 6.98 (1H, dd, J = 9.7, 1.8 Hz), 7.30 (1H, d, J = 9.7 Hz), 7.39 (1H, dt, J = 7.3, 1.8 Hz), 7.44 (2H, t, J = 7.3 Hz), 7.62-7.66 (2H, m), 7.76 (1H, t, J = 7.9 Hz), 8.00-8.05 (3H, m), 8.13 (1H, d, J = 1.8 Hz).

### <Example 2-1>

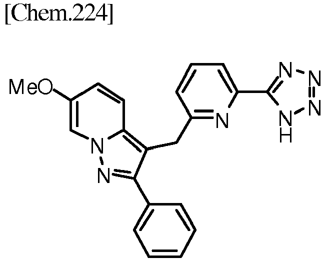

### 6-Methoxy-2-phenyl-3-[[6-(1H-tetrazol-5-yl)pyridin-2-yl]methyl]pyrazolo[1,5-a]pyridine

Sodium azide (23 mg) and zinc bromide (52 mg) were added to a mixed solution of *N,N*-dimethylformamide and water (2.3 mL, 3:1) of 6-[(6-methoxy-2-phenylpyrazolo[1,5-a]pyridin-3-yl)methyl]pyridine-2-carbonitrile (75 mg) at room temperature under an argon atmosphere, and then the mixture was stirred at 80°C for 15 hours. A 1 mol/L hydrochloric acid was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (ethyl acetate:methanol = 4:1) to obtain a title compound as yellow liquid (42 mg).
¹H-NMR (400 MHz, CDCl₃) δ 3.83 (3H, s), 4.45 (2H, s), 6.92 (1H, d, J = 9.7 Hz), 7.36-7.47 (3H, m), 7.68-7.78 (3H, m), 8.02 (1H, s), 8.06-8.16 (2H, m).

### <Example 3-1>

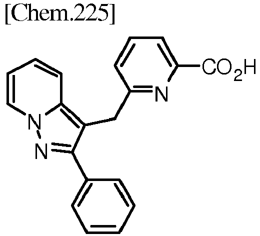

### 6-[(2-phenylpyrazolo[1,5-a]pyridin-3-yl)methyl]pyridine-2-carboxylic acid

A 2 mol/L potassium hydroxide aqueous solution (0.1 mL) was added to a methanol solution (0.3 mL) of methyl 6-[(2-phenylpyrazolo[1,5-a]pyridin-3-yl)methyl]pyridine-2-carboxylate (20 mg) under an argon atmosphere under ice-cooling, and then the mixture was stirred at room temperature for 18 hours. A 1 mol/L hydrochloric acid was added to the reaction solution and the precipitated solid was filtered off to obtain a title compound as a yellow powder (11 mg).
¹H-NMR (400 MHz, DMSO-d₆) δ 4.43 (2H, s), 6.90 (1H, td, J = 6.7, 1.2 Hz), 7.19 (1H, dd, J = 9.7, 6.7 Hz), 7.29 (1H, dd, J = 7.3, 1.8 Hz), 7.39 (1H, dt, J = 7.3, 1.8 Hz), 7.41-7.46 (2H, m), 7.67 (1H, d, J = 8.5 Hz), 7.81 (1H, t, J = 7.3 Hz), 7.82-7.86 (3H, m), 8.69 (1H, d, J = 7.3 Hz).

### <Example 3-2>

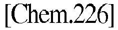

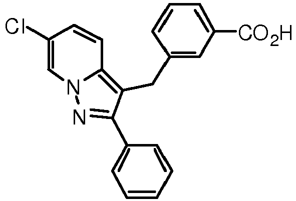

### 3-[(6-Chloro-2-phenylpyrazolo[1,5-a]pyridin-3-yl)methyl]benzoic acid

A 2 mol/L potassium hydroxide aqueous solution (0.3 mL) was added to a methanol solution (0.6 mL) of methyl 3-[(6-chloro-2-phenylpyrazolo[1,5-a]pyridin-3-yl)methyl]benzoate (42 mg) under an argon atmosphere under ice-cooling, and then the mixture was stirred at room temperature for 1 hour. A 1 mol/L hydrochloric acid was added to the reaction solution and the precipitated solid was filtered off to obtain a title compound as a colorless powder (32 mg).
¹H-NMR (400 MHz, DMSO-d₆) δ 4.36 (2H, s), 7.26 (1H, dd, J = 9.1, 1.8 Hz), 7.33-7.46 (5H, m), 7.63-7.68 (4H, m), 7.73 (1H, tt, J = 7.3, 1.2 Hz), 9.04 (1H, d, J = 1.2 Hz), 12.86 (1H, brs).

### <Example 3-3>

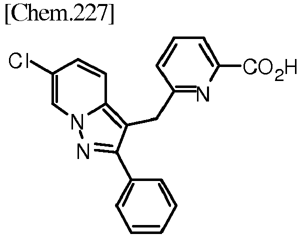

### 6-[(6-Chloro-2-phenylpyrazolo[1,5-a]pyridin-3-yl)methyl]pyridine-2-carboxylic acid

A 2 mol/L potassium hydroxide aqueous solution (0.6 mL) was added to a methanol solution (1.2 mL) of methyl 6-[(6-chloro-2-phenylpyrazolo[1,5-a]pyridin-3-yl)methyl]pyridine-2-carboxylate (90 mg) under an argon atmosphere under ice-cooling, and then the mixture was stirred at room temperature for 6 hours. A 1 mol/L hydrochloric acid was added to the reaction solution and the precipitated solid was filtered off to obtain a title compound as a colorless powder (79 mg).
¹H-NMR (400 MHz, DMSO-d₆) δ 4.44 (2H, s), 7.26 (1H, dd, J = 9.1, 1.8 Hz), 7.31 (1H, dd, J = 7.3, 1.8 Hz), 7.37-7.47 (3H, m), 7.75 (1H, d, J = 9.1 Hz), 7.81-7.86 (4H, m), 9.03 (1H, d, J = 1.8 Hz),13.08 (1H, brs).

### <Example 3-4>

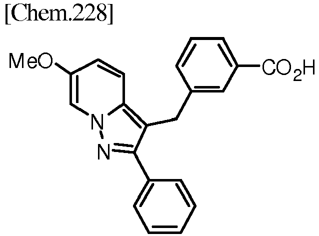

### 3-[(6-Methoxy-2-phenylpyrazolo[1,5-a]pyridin-3-yl)methyl]benzoic acid

A 2 mol/L potassium hydroxide aqueous solution (0.1 mL) was added to a methanol solution (0.2 mL) of methyl 3-[(6-methoxy-2-phenylpyrazolo[1,5-a]pyridin-3-yl)methyl]benzoate (17 mg) under an argon atmosphere under ice-cooling, and then the mixture was stirred at room temperature for 4 hours. A 1 mol/L hydrochloric acid was added to the reaction solution and the precipitated solid was filtered off to obtain a title compound as a colorless powder (15 mg).
¹H-NMR (400 MHz, DMSO-d₆) δ 3.83 (3H, s), 4.32 (2H, s), 7.00 (1H, dd, J = 9.7, 1.8 Hz), 7.33-7.37 (3H, m), 7.41 (2H, t, J = 7.9 Hz), 7.49 (1H, d, J = 9.7 Hz), 7.62-7.66 (3H, m), 7.70-7.74 (1H, m), 8.43 (1H, d, J = 1.8 Hz), 12.80 (1H, brs).

### <Example 3-5>

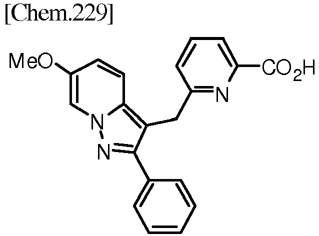

### 6-[(6-Methoxy-2-phenylpyrazolo[1,5-a]pyridin-3-yl)methyl]pyridine-2-carboxylic acid

A 2 mol/L potassium hydroxide aqueous solution (0.1 mL) was added to a methanol solution (0.2 mL) of methyl 6-[(6-methoxy-2-phenylpyrazolo[1,5-a]pyridin-3-yl)methyl]pyridine-2-carboxylate (15 mg) under an argon atmosphere under ice-cooling, and then the mixture was stirred at room temperature for 5 hours. A 1 mol/L hydrochloric acid was added to the reaction solution and the precipitated solid was filtered off to obtain a title compound as a yellow powder (10 mg).
¹H-NMR (400 MHz, DMSO-d₆) δ 3.82 (3H, s), 4.40 (2H, s), 7.00 (1H, dd, J = 9.7, 1.8 Hz), 7.27 (1H, dd, J = 7.3, 1.2 Hz), 7.36 (1H, tt, J = 7.3, 1.2 Hz), 7.42 (2H, t, J = 7.3 Hz), 7.59 (1H, d, J = 9.7 Hz), 7.78-7.85 (4H, m), 8.42 (1H, d, J = 1.8 Hz).

### <Example 3-6>

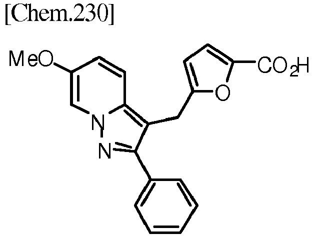

### 5-[(6-Methoxy-2-phenylpyrazolo[1,5-a]pyridin-3-yl)methyl]furan-2-carboxylic acid

A 2 mol/L potassium hydroxide aqueous solution (0.2 mL) was added to a methanol solution (0.3 mL) of methyl 5-[(6-methoxy-2-phenylpyrazolo[1,5-a]pyridin-3-yl)methyl]furan-2-carboxylate (25 mg) under an argon atmosphere under ice-cooling, and then the mixture was stirred at room temperature for 5 hours. A 1 mol/L hydrochloric acid was added to the reaction solution and the precipitated solid was filtered off to obtain a title compound as a colorless powder (19 mg).
¹H-NMR (400 MHz, DMSO-d₆) δ 3.83 (3H, s), 4.29 (2H, s), 6.14 (1H, d, J = 3.6 Hz), 7.02-7.07 (2H, m), 7.36-7.41 (1H, m), 7.46 (2H, t, J = 7.3 Hz), 7.56 (1H, d, J = 9.7 Hz), 7.68-7.72 (2H, m), 8.43 (1H, d, J = 1.8 Hz), 12.87 (1H, brs).

### <Example 3-7>

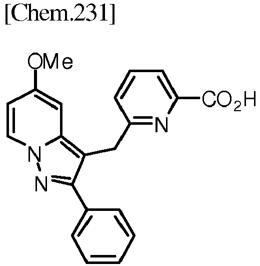

### 6-[(5-Methoxy-2-phenylpyrazolo[1,5-a]pyridin-3-yl)methyl]pyridine-2-carboxylic acid

A 2 mol/L potassium hydroxide aqueous solution (0.6 mL) was added to a methanol solution (1.1 mL) of methyl 6-[(5-methoxy-2-phenylpyrazolo[1,5-a]pyridin-3-yl)methyl]pyridine-2-carboxylate (85 mg) under an argon atmosphere under ice-cooling, and then the mixture was stirred at room temperature for 3 hours. A 1 mol/L hydrochloric acid was added to the reaction solution and the precipitated solid was filtered off to obtain a title compound as a yellow powder (51 mg).
¹H-NMR (400 MHz, DMSO-d₆) δ 3.81 (3H, s), 4.37 (2H, s), 6.57 (1H, dd, J = 7.9, 3.0 Hz), 7.10 (1H, d, J = 3.0 Hz), 7.28 (1H, dd, J = 7.9, 1.2 Hz), 7.34-7.39 (1H, m), 7.42 (2H, t, J = 7.3 Hz), 7.74-7.78 (2H, m), 7.79-7.86 (2H, m), 8.53 (1H, d, J = 7.3 Hz), 13.11 (1H, brs).

### <Example 3-8>

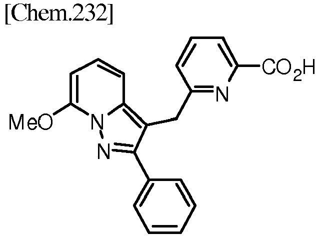

### 6-[(7-Methoxy-2-phenylpyrazolo[1,5-a]pyridin-3-yl)methyl]pyridine-2-carboxylic acid

A 2 mol/L potassium hydroxide aqueous solution (0.6 mL) was added to a methanol solution (1.2 mL) of methyl 6-[(7-methoxy-2-phenylpyrazolo[1,5-a]pyridin-3-yl)methyl]pyridine-2-carboxylate (90 mg) under an argon atmosphere under ice-cooling, and then the mixture was stirred at room temperature for 3 hours. A 1 mol/L hydrochloric acid was added to the reaction solution and the precipitated solid was filtered off to obtain a title compound as a colorless powder (71 mg).
¹H-NMR (400 MHz, DMSO-d₆) δ 4.10 (3H, s), 4.41 (2H, s), 6.38 (1H, dd, J = 7.3, 1.2 Hz), 7.17-7.28 (3H, m), 7.35-7.47 (3H, m), 7.77-7.86 (4H, m).

### <Example 3-9>

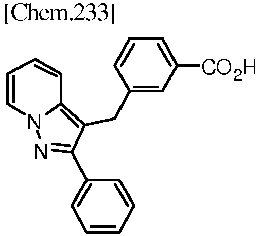

### 3-[(2-Phenylpyrazolo[1,5-a]pyridin-3-yl)methyl]benzoic acid

A 2 mol/L potassium hydroxide aqueous solution (0.1 mL) was added to a methanol solution (0.2 mL) of methyl 3-[(2-phenylpyrazolo[1,5-a]pyridin-3-yl)methyl]benzoate (17 mg) under an argon atmosphere under ice-cooling, and then the mixture was stirred at room temperature for 5 hours. A 1 mol/L hydrochloric acid was added to the reaction solution and the precipitated solid was filtered off to obtain a title compound as a colorless powder (12 mg).
¹H-NMR (400 MHz, DMSO-d₆) δ 4.34 (2H, s), 6.90 (1H, td, J = 6.7, 1.2 Hz), 7.19 (1H, ddd, J = 9.1, 6.7, 1.2 Hz), 7.34-7.37 (2H, m), 7.37-7.39 (1H, m), 7.43 (2H, tt, J = 7.3, 1.2 Hz), 7.58 (1H, dt, J = 9.1, 1.2 Hz), 7.65-7.67 (2H, m), 7.67-7.69 (1H, m), 7.71-7.74 (1H, m), 8.70 (1H, d, J = 7.3 Hz), 12.85 (1H, brs).

### <Example 3-10>

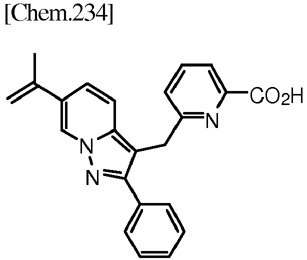

### 6-[[2-Phenyl-6-(propen-2-yl)pyrazolo[1,5-a]pyridin-3-yl]methyl]pyridine-2-carboxylic acid

A 2 mol/L potassium hydroxide aqueous solution (0.2 mL) was added to a methanol solution (0.4 mL) of methyl 6-[[2-phenyl-6-(propen-2-yl)pyrazolo[1,5-a]pyridin-3-yl]methyl]pyridine-2-carboxylate (30 mg) under an argon atmosphere under ice-cooling, and then the mixture was stirred at room temperature for 5 hours. A 1 mol/L hydrochloric acid was added to the reaction solution and the precipitated solid was filtered off to obtain a title compound as a yellow powder (21 mg).
¹H-NMR (400 MHz, DMSO-d₆) δ 2.16 (3H, s), 4.42 (2H, s), 5.17 (1H, s), 5.59 (1H, s), 7.23 (1H, d, J = 7.3 Hz), 7.38-7.45 (3H, m), 7.58-7.63 (2H, m), 7.78 (1H, d, J = 7.3 Hz), 7.79-7.85 (3H, m), 8.71 (1H, s).

### <Example 3-11>

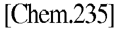

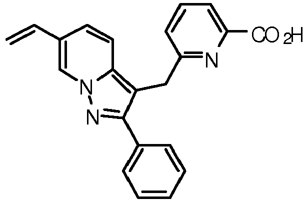

### 6-[(2-Phenyl-6-vinylpyrazolo[1,5-a]pyridin-3-yl)methyl]pyridine-2-carboxylic acid

A 2 mol/L potassium hydroxide aqueous solution (0.2 mL) was added to a methanol solution (0.5 mL) of methyl 6-[(2-phenyl-6-vinylpyrazolo[1,5-a]pyridin-3-yl)methyl]pyridine-2-carboxylate (35 mg) under an argon atmosphere under ice-cooling, and then the mixture was stirred at room temperature for 5 hours. A 1 mol/L hydrochloric acid was added to the reaction solution and the precipitated solid was filtered off to obtain a title compound as a pale yellow powder (28 mg).
¹H-NMR (400 MHz, DMSO-d₆) δ 4.40 (2H, s), 5.31 (1H, d, J = 10.9 Hz), 5.89 (1H, d, J = 17.6 Hz), 6.76 (1H, dd, J = 17.6, 10.9 Hz), 7.17 (1H, d, J = 7.3 Hz), 7.39 (1H, d, J = 7.3 Hz), 7.44 (2H, t, J = 7.3 Hz), 7.49 (1H, dd, J = 9.7, 1.2 Hz), 7.63 (1H, d, J = 9.7 Hz), 7.70-7.78 (2H, m), 7.80-7.84 (2H, m), 8.77 (1H, s).

### <Example 3-12>

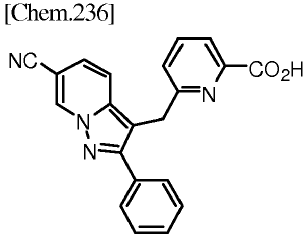

### 6-[(6-Cyano-2-phenylpyrazolo[1,5-a]pyridin-3-yl)methyl]pyridine-2-carboxylic acid

A 2 mol/L potassium hydroxide aqueous solution (0.1 mL) was added to a methanol solution (0.3 mL) of methyl 6-[(6-cyano-2-phenylpyrazolo[1,5-a]pyridin-3-yl)methyl]pyridine-2-carboxylate (20 mg) under an argon atmosphere under ice-cooling, and then the mixture was stirred at room temperature for 2 hours. A 1 mol/L hydrochloric acid was added to the reaction solution and the precipitated solid was filtered off to obtain a title compound as a pale yellow powder (12 mg).
¹H-NMR (400 MHz, DMSO-d₆) δ 4.42 (2H, s), 6.89 (1H, td, J = 6.7, 1.2 Hz), 7.18 (1H, dd, J = 9.7, 6.7 Hz), 7.23-7.30 (4H, m), 7.60 (1H, dd, J = 9.7, 1.2 Hz), 7.63-7.68 (2H, m), 7.71-7.75 (1H, m), 8.69 (1H, d, J = 6.7 Hz).

### <Example 3-13>

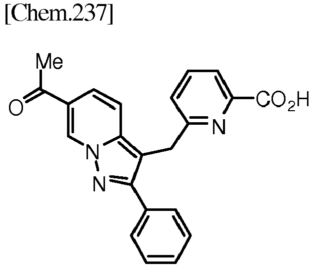

### 6-[(6-Acetyl-2-phenylpyrazolo[1,5-a]pyridin-3-yl)methyl]pyridine-2-carboxylic acid

A 2 mol/L potassium hydroxide aqueous solution (0.1 mL) was added to a methanol solution (0.3 mL) of methyl 6-[(6-acetyl-2-phenylpyrazolo[1,5-a]pyridin-3-yl)methyl]pyridine-2-carboxylate (21 mg) under an argon atmosphere under ice-cooling, and then the mixture was stirred at room temperature for 6 hours. A 1 mol/L hydrochloric acid was added to the reaction solution and the precipitated solid was filtered off to obtain a title compound as a yellow powder (14 mg).
¹H-NMR (400 MHz, DMSO-d₆) δ 2.63 (3H, s), 4.46 (2H, s), 7.32 (1H, dd, J = 7.3, 1.8 Hz), 7.43-7.48 (2H, m), 7.55-7.59 (3H, m), 7.75 (1H, d, J = 9.7 Hz), 7.82-7.84 (2H, m), 7.84-7.89 (2H, m),13.10 (1H, brs).

### <Example 3-14>

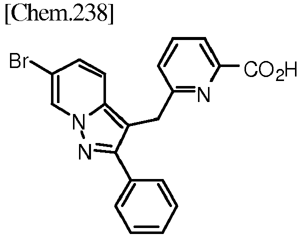

### 6-[(6-Bromo-2-phenylpyrazolo[1,5-a]pyridin-3-yl)methyl]pyridine-2-carboxylic acid

A 2 mol/L potassium hydroxide aqueous solution (0.3 mL) was added to a methanol solution (0.6 mL) of methyl 6-[(6-bromo-2-phenylpyrazolo[1,5-a]pyridin-3-yl)methyl]pyridine-2-carboxylate (50 mg) under an argon atmosphere under ice-cooling, and then the mixture was stirred at room temperature for 2 hours. A 1 mol/L hydrochloric acid was added to the reaction solution and the precipitated solid was filtered off to obtain a title compound as a colorless powder (41 mg).
¹H-NMR (400 MHz, DMSO-d₆) δ 4.43 (2H, s), 7.30 (2H, dd, J = 7.3, 1.8 Hz), 7.32 (2H, dd, J = 9.1, 1.8 Hz), 7.39-7.47 (3H, m), 7.69 (1H, d, J = 9.1 Hz), 7.79-7.86 (4H, m), 9.09 (1H, d, J=1.8Hz), 13.12(1H,brs).

### <Example 3-15>

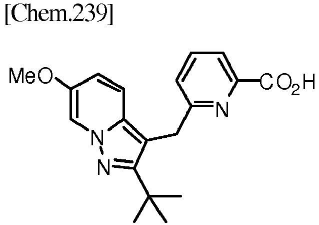

### 6-[(2- tert-Butyl-6-methoxypyrazolo[1,5-a]pyridin-3-yl)methyl]pyridine-2-carboxylic acid

After a KOH aqueous solution (2 mol/L, 0.80 mL) was added to a methanol (1.6 mL) solution of methyl 6-[(2-*tert*-butyl-6-methoxypyrazolo[1,5-a]pyridin-3-yl)methyl]pyridine-2-carboxylate (110 mg), the mixture was stirred at room temperature for 3 hours. A hydrochloric acid (2 mol/L, 0.90 mL) was added to the reaction solution to achieve pH = 1, and then the mixture was extracted with dichloromethane and dried over anhydrous sodium sulfate. The solvent was evaporated to obtain a title compound as red amorphous (105 mg).
¹H-NMR (400 MHz, DMSO- d₆) δ 1.38 (9H, s), 3.85 (3H, s), 4.46 (2H, s), 6.98 (1H, dd, J = 9.7 and 2.4 Hz), 7.13 (1H, d, J = 7.9 Hz), 7.50 (1H, d, J = 9.7 Hz), 7.85 (1H, t, J = 7.9 Hz), 7.90 (1H, d, J = 6.7 Hz), 8.37 (1H, d, J = 1.8 Hz), 13.16 (1H, brs).

### <Example 3-16>

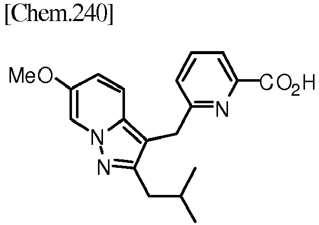

### 6-[(2-Isobutyl-6-methoxypyrazolo[1,5-a]pyridin-3-yl)methyl]pyridine-2-carboxylic acid

After a KOH aqueous solution (2 mol/L, 0.95 mL) was added to a methanol (1.9 mL) solution of methyl 6-[(2-isobutyl-6-methoxypyrazolo[1,5-a]pyridin-3-yl)methyl]pyridine-2-carboxylate (131 mg), the mixture was stirred at room temperature for 3 hours. A hydrochloric acid (2 mol/L, 0.90 mL) was added to the reaction solution to achieve pH = 1, and then the mixture was extracted with dichloromethane and dried over anhydrous sodium sulfate. The solvent was evaporated to obtain a title compound as a red amorphous (120 mg).
¹H-NMR (400 MHz, DMSO-d₆) δ 0.83 (6H, d, J = 6.6 Hz), 1.84-2.00 (1H, m), 2.55 (2H, d, J = 7.3 Hz), 3.77 (3H, s), 4.20 (2H, s), 6.91 (1H, dd, J = 9.7, 1.8 Hz), 7.26 (1H, dd, J = 7.3, 1.8 Hz), 7.52 (1H, d, J = 9.7 Hz), 7.75-7.86 (2H, m), 8.26 (1H, d, J = 1.8 Hz), 13.05 (1H, brs).

### <Example 3-17>

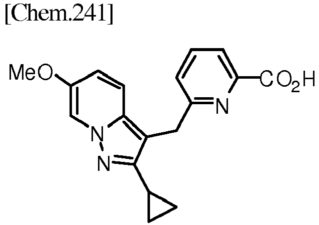

### 6-[(2-Cyclopropyl-6-methoxypyrazolo[1,5-a]pyridin-3-yl)methyl]pyridine-2-carboxylic acid

After a KOH aqueous solution (2 mol/L, 1.4 mL) was added to a methanol (2.7 mL) solution of methyl 6-[(2-cyclopropyl-6-methoxypyrazolo[1,5-a]pyridin-3-yl)methyl]pyridine-2-carboxylate (180 mg), the mixture was stirred at room temperature for 3 hours. A hydrochloric acid (2 mol/L, 1.5 mL) was added to the reaction solution to achieve pH = 1 and the precipitated crystal was filtered off to obtain a title compound as a colorless crystal (135 mg).
¹H-NMR (400 MHz, CDCl₃) δ 0.77-0.92 (4H, m), 2.03-2.12 (1H, m), 3.74 (3H, s), 4.29 (2H, s), 6.90 (1H, dd, J = 9.7, 1.8 Hz), 7.28-7.35 (1H, m), 7.49 (1H, d, J = 9.7 Hz), 7.80-7.87 (2H, m), 8.22 (1H, d, J = 1.8 Hz),13.05 (1H, brs).

### <Example 3-18>

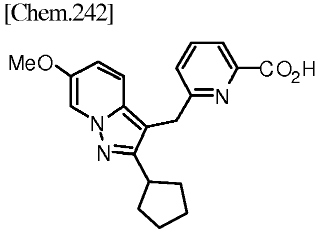

### 6-[(2-Cyclopentyl-6-methoxypyrazolo[1,5-a]pyridin-3-yl)methyl]pyridine-2-carboxylic acid

After a KOH aqueous solution (2 mol/L, 1.2 mL) was added to a methanol (2.3 mL) solution of methyl 6-[(2-cyclopentyl-6-methoxypyrazolo[1,5-a]pyridin-3-yl)methyl]pyridine-2-carboxylate (167 mg), the mixture was stirred at room temperature for 3 hours. A hydrochloric acid (2 mol/L, 1.3 mL) was added to the reaction solution to achieve pH = 1 and the precipitated crystal was filtered off to obtain a title compound as a colorless crystal (136 mg).
¹H-NMR (400 MHz, CDCl₃) δ 1.50-1.63 (2H, m), 1.65-1.78 (4H, m), 1.85-1.95 (2H, m), 3.20-3.40 (1H, m), 3.76 (3H, s), 4.22 (2H, s), 6.90 (1H, dd, J = 9.7, 1.8 Hz), 7.26 (1H, dd, J = 6.7,2.4 Hz), 7.52 (1H, d, J = 9.7 Hz), 7.77-7.85 (2H, m), 8.27 (1H, d, J = 1.2 Hz), 13.07 (1H, brs).

### <Example 3-19>

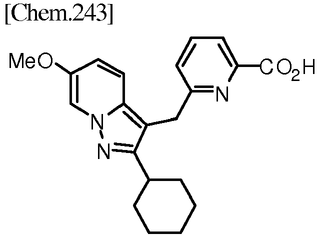

### 6-[(2-Cyclohexyl-6-methoxypyrazolo[1,5-a]pyridin-3-yl)methyl]pyridine-2-carboxylic acid

After a KOH aqueous solution (2 mol/L, 1.3 mL) was added to a methanol (2.6 mL) solution of methyl 6-[(2-cyclohexyl-6-methoxypyrazolo[1,5-a]pyridin-3-yl)methyl]pyridine-2-carboxylate (194 mg), the mixture was stirred at room temperature for 3 hours. A hydrochloric acid (2 mol/L, 1.4 mL) was added to the reaction solution to achieve pH = 1 and the precipitated crystal was filtered off to obtain a title compound as a colorless crystal (170 mg).
¹H-NMR (400 MHz, CDCl₃) δ 1.13-1.37 (3H, m), 1.45-1.57 (2H, m), 1.63-1.75 (5H, m), 2.78-2.90 (1H, m), 3.76 (3H, s), 4.21 (2H, s), 6.90 (1H, dd, J = 9.7, 1.8 Hz), 7.27 (1H, dd, J = 6.7,2.4 Hz), 7.52 (1H, d, J = 9.7 Hz), 7.78-7.85 (2H, m), 8.27 (1H, d, J = 1.8 Hz), 13.03 (1H, brs).

### <Example 3-20>

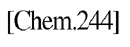

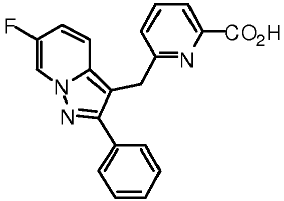

### 6-[(6-Fluoro-2-phenylpyrazolo[1,5-a]pyridin-3-yl)methyl]pyridine-2-carboxylic acid

A 2 mol/L potassium hydroxide aqueous solution (1.0 mL) was added to a methanol solution (1.9 mL) of methyl 6-[(6-fluoro-2-phenylpyrazolo[1,5-a]pyridin-3-yl)methyl]pyridine-2-carboxylate (140 mg) under an argon atmosphere under ice-cooling, and then the mixture was stirred at room temperature for 2 hours. A 1 mol/L hydrochloric acid was added to the reaction solution and the precipitated solid was filtered off to obtain a title compound as a colorless powder (112 mg).
¹H-NMR (400 MHz, DMSO-d₆) δ 4.42 (2H, s), 7.26 (1H, dd, J = 7.3, 1.2 Hz), 7.31 (1H, ddd, J = 9.7, 7.9, 1.8 Hz), 7.36-7.47 (3H, m), 7.74-7.83 (5H, m), 9.01 (1H, dd, J = 4.8, 1.8 Hz).

### <Example 3-21>

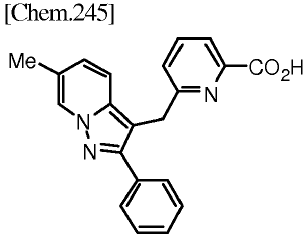

### 6-[(6-Methyl-2-phenylpyrazolo[1,5-a]pyridin-3-yl)methyl]pyridine-2-carboxylic acid

A 2 mol/L potassium hydroxide aqueous solution (0.9 mL) was added to a methanol solution (1.8 mL) of methyl 6-[(6-methyl-2-phenylpyrazolo[1,5-a]pyridin-3-yl)methyl]pyridine-2-carboxylate (126 mg) under an argon atmosphere under ice-cooling, and then the mixture was stirred at room temperature for 4 hours. A 1 mol/L hydrochloric acid was added to the reaction solution and the precipitated solid was filtered off to obtain a title compound as a yellow powder (102 mg).
¹H-NMR (400 MHz, DMSO-d₆) δ 2.29 (3H, s), 4.40 (2H, s), 7.06 (1H, d, J = 9.1 Hz), 7.26 (1H, d, J = 7.3 Hz), 7.37 (1H, d, J = 7.3 Hz), 7.43 (2H, t, J = 7.3 Hz), 7.57 (1H, d, J = 9.1 Hz), 7.77-7.85 (4H, m), 8.52 (1H, s), 13.10 (1H, brs).

### <Example 3-22>

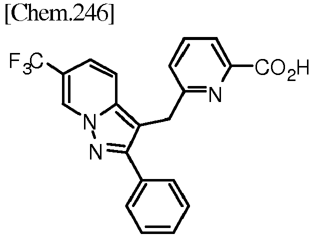

### 6-[[2-Phenyl-6-(trifluoromethyl)pyrazolo[1,5-a]pyridin-3-yl]methyl]pyridine-2-carboxylic acid

A 2 mol/L potassium hydroxide aqueous solution (1.8 mL) was added to a methanol solution (3.6 mL) of methyl 6-[[2-phenyl-6-(trifluoromethyl)pyrazolo[1,5-a]pyridin-3-yl]methyl]pyridine-2-carboxylate (292 mg) under an argon atmosphere under ice-cooling, and then the mixture was stirred at room temperature for 4 hours. A 1 mol/L hydrochloric acid was added to the reaction solution and the precipitated solid was filtered off to obtain a title compound as a colorless powder (211 mg).
¹H-NMR (400 MHz, DMSO-d₆) δ 4.46 (2H, s), 7.29 (1H, dd, J = 7.3, 1.2 Hz), 7.39-7.50 (4H, m), 7.76-7.87 (4H, m), 7.90 (1H, d, J = 9.7 Hz), 9.35 (1H, s).

### <Example 3-23>

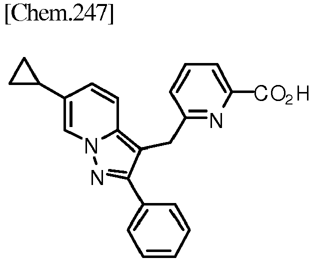

### 6-[(6-Cyclopropyl-2-phenylpyrazolo[1,5-a]pyridin-3-yl)methyl]pyridine-2-carboxylic acid

A 2 mol/L potassium hydroxide aqueous solution (0.5 mL) was added to a methanol solution (1.0 mL) of methyl 6-[(6-cyclopropyl-2-phenylpyrazolo[1,5-a]pyridin-3-yl)methyl]pyridine-2-carboxylate (79 mg) under an argon atmosphere under ice-cooling, and then the mixture was stirred at room temperature for 5 hours. A 1 mol/L hydrochloric acid was added to the reaction solution and the precipitated solid was filtered off to obtain a title compound as a pale yellow powder (59 mg).
¹H-NMR (400 MHz, DMSO-d₆) δ 0.72-0.77 (2H, m), 0.90-0.95 (2H, m), 1.94-2.01 (1H, m), 4.39 (2H, s), 6.93 (1H, dd, J = 9.1, 1.2 Hz), 7.20 (1H, d, J = 7.3 Hz), 7.36 (1H, tt, J = 7.3, 1.2 Hz), 7.43 (2H, tt, J = 7.3, 1.2 Hz), 7.54 (1H, d, J = 9.1 Hz), 7.75 (1H, t, J = 7.3 Hz), 7.78-7.83 (3H, m), 8.52 (1H, s).

### <Example 3-24>

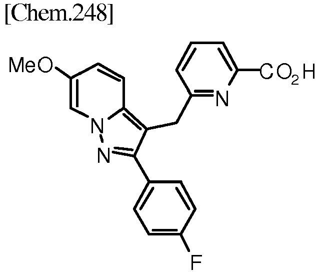

### 6-[[2-(4-Fluorophenyl)-6-methoxypyrazolo[1,5-a]pyridin-3-yl]methyl]pyridine-2-carboxylic acid

Methyl 6-[[2-(4-fluorophenyl)-6-methoxypyrazolo[1,5-a]pyridin-3-yl]methyl]pyridine-2-carboxylate (87.0 mg) was dissolved in a mixed solution of tetrahydrofuran (2.0 mL) and methanol (2.0 mL), then a sodium hydroxide aqueous solution (1.0 mol/L, 1.0 mL) was added under ice-cooling. The mixture was heated up to room temperature, stirred for 2 hours, and then ice-cooled again. Next, hydrochloric acid was slowly added to achieve pH = 1. The organic solvent was evaporated under vacuum and then the produced precipitate was filtered off to obtain a title compound as a white solid (63.5 mg).
¹H-NMR (400 MHz, DMSO-d₆) δ 3.81 (3H, s), 4.38 (2H, s), 7.01 (1H, dd, J = 9.7, 1.8 Hz), 7.25 (2H, t, J = 9.1 Hz), 7.32 (1H, dd, J = 6.7, 1.8 Hz), 7.61 (1H, d, J = 9.7 Hz), 7.79-7.85 (2H, m), 7.87-7.94 (2H, m), 8.41 (1H, d, J = 1.8 Hz),13.11 (1H, s).

### <Example 3-25>

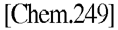

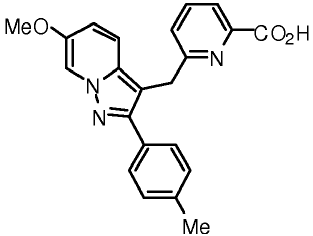

### 6-[[6-Methoxy-2-(4-methylphenyl)pyrazolo[1,5-a]pyridin-3-yl]methyl]pyridine-2-carboxyli c acid

Methyl 6-[[6-methoxy-2-(4-methylphenyl)pyrazolo[1,5-a]pyridin-3-yl]methyl]pyridine-2-carboxylat e (110 mg) was dissolved in a mixed solution of tetrahydrofuran (2.0 mL) and methanol (2.0 mL), then a sodium hydroxide aqueous solution (1.0 mol/L, 1.0 mL) was added under ice-cooling. The mixture was heated up to room temperature, stirred for 8 hours, and then ice-cooled again. Next, hydrochloric acid was slowly added to achieve pH = 1. The organic solvent was evaporated under vacuum and then the produced precipitate was filtered off to obtain a title compound as a pale yellow solid (96.8 mg).
¹H-NMR (400 MHz, DMSO-d₆) δ 2.32 (3H, s), 3.81 (3H, s), 4.39 (2H, s), 6.99 (1H, dd, J = 9.7, 2.4 Hz), 7.22 (2H, d, J = 7.9 Hz), 7.25 (1H, dd, J = 7.3, 1.8 Hz), 7.58 (1H, d, J = 9.7 Hz), 7.69 (2H, d, J = 7.9 Hz), 7.78-7.85 (2H, m), 8.41 (1H, d, J = 1.8 Hz), 13.09 (1H, s).

### <Example 3-26>

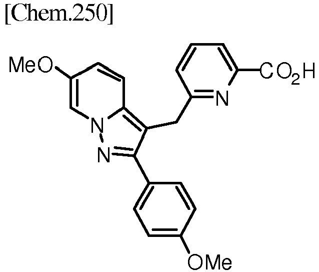

### 6-[[6-Methoxy-2-(4-methoxyphenyl)pyrazolo[1,5-a]pyridin-3-yl]methyl]pyridine-2-carboxy lic acid

Methyl 6-[[6-methoxy-2-(4-methoxyphenyl)pyrazolo[1,5-a]pyridin-3-yl]methyl]pyridine-2-carboxyl ate (75.0 mg) was dissolved in a mixed solution of tetrahydrofuran (2.0 mL) and methanol (2.0 mL), then a sodium hydroxide aqueous solution (1.0 mol/L, 1.0 mL) was added under ice-cooling. The mixture was heated up to room temperature, stirred for 8 hours, and then ice-cooled again. Next, hydrochloric acid was slowly added to achieve pH = 1. The organic solvent was evaporated under vacuum and then the produced precipitate was filtered off to obtain a title compound as a white solid (60.2 mg).
¹H-NMR (400 MHz, DMSO-d₆) δ 3.77 (3H, s), 3.81 (3H, s), 4.38 (2H, s), 6.95-7.01 (3H, m), 7.26 (1H, dd, J = 7.3, 1.2 Hz), 7.57 (1H, d, J = 9.7 Hz), 7.74 (2H, td, J = 9.7, 1.8 Hz), 7.78-7.85 (2H, m), 8.40 (1H, d, J = 1.8 Hz), 13.10 (1H, s).

### <Example 3-27>

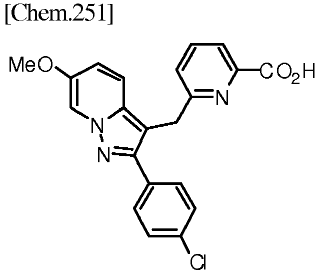

### 6-[[2-(4-Chlorophenyl)-6-methoxypyrazolo[1,5-a]pyridin-3-yl]methyl]pyridine-2-carboxylic acid

Methyl 6-[[2-(4-chlorophenyl)-6-methoxypyrazolo[1,5-a]pyridin-3-yl]methyl]pyridine-2-carboxylat e (85.0 mg) was dissolved in a mixed solution of tetrahydrofuran (2.0 mL) and methanol (2.0 mL), then a sodium hydroxide aqueous solution (1.0 mol/L, 1.0 mL) was added under ice-cooling. The mixture was heated up to room temperature, stirred for 2 hours, and then ice-cooled again. Next, hydrochloric acid was slowly added to achieve pH = 1. The organic solvent was evaporated under vacuum and then the produced precipitate was filtered off to obtain a title compound as a white solid (74.5 mg).
¹H-NMR (400 MHz, DMSO-d₆) δ 3.82 (3H, s), 4.40 (2H, s), 7.02 (1H, dd, J = 9.7, 2.4 Hz), 7.33 (1H, dd, J = 6.7, 1.8 Hz), 7.48 (2H, td, J = 8.5, 1.8 Hz), 7.64 (1H, d, J = 9.7 Hz), 7.79-7.86 (2H, m), 7.91 (2H, dt, J = 8.5,1.8 Hz), 8.42 (1H, d, J = 1.8 Hz), 13.11 (1H, s).

### <Example 3-28>

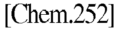

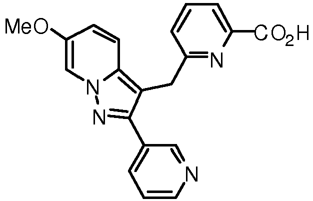

### 6-[[6-Methoxy-2-(pyridin-3-yl)pyrazolo[1,5-a]pyridin-3-yl]methyl]pyridine-2-carboxylic acid

Methyl 6-[[6-methoxy-2-(pyridin-3-yl)pyrazolo[1,5-a]pyridin-3-yl]methyl]pyridine-2-carboxylate (14.5 mg) was dissolved in a mixed solution of tetrahydrofuran (1.0 mL) and methanol (1.0 mL), then a sodium hydroxide aqueous solution (1.0 mol/L, 0.5 mL) was added under ice-cooling. The mixture was heated up to room temperature, stirred for 3 hours, and then ice-cooled again. Next, hydrochloric acid was slowly added to achieve pH = 1. The organic solvent was evaporated under vacuum and then the produced precipitate was filtered off to obtain a title compound as a white solid (7.1 mg).
¹H-NMR (400 MHz, DMSO-d₆) δ 3.83 (3H, s), 4.44 (2H, s), 7.08 (1H, dd, J = 9.7, 1.8 Hz), 7.46-7.52 (1H, m), 7.74 (2H, d, J = 9.7 Hz), 7.83-7.88 (2H, m), 8.46 (1H, d, J = 1.8 Hz), 8.71 (2H, d, J = 6.1 Hz), 9.29 (1H, s).

### <Example 3-29>

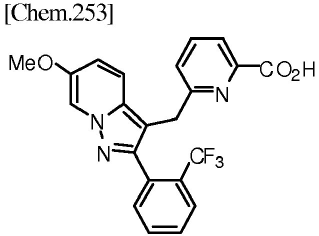

### 6-[[6-Methoxy-2-[2-(trifluoromethyl)phenyl]pyrazolo[1,5-a]pyridin-3-yl]methyl]pyridine-2-carboxylic acid

A 2 mol/L potassium hydroxide aqueous solution (0.3 mL) was added to a methanol solution (0.6 mL) of methyl 6-[[6-methoxy-2-[2-(trifluoromethyl)phenyl]pyrazolo[1,5-a]pyridin-3-yl]methyl]pyridine-2-carboxylate (54 mg) under an argon atmosphere under ice-cooling, and then the mixture was stirred at room temperature for 4 hours. A 1 mol/L hydrochloric acid was added to the reaction solution and the precipitated solid was filtered off to obtain a title compound as a pale yellow powder (36 mg).
¹H-NMR (400 MHz, DMSO-d₆) δ 3.81 (3H, s), 4.07 (2H, s), 7.02 (1H, dd, J = 9.7, 1.8 Hz), 7.12 (1H, dd, J = 7.3, 1.2 Hz), 7.56-7.63 (2H, m), 7.63-7.68 (2H, m), 7.74 (1H, t, J = 7.3 Hz), 7.76-7.79 (1H, m), 7.83 (1H, dd, J = 7.3, 1.8 Hz), 8.35 (1H, d, J = 1.8 Hz), 13.04 (1H, brs).

### <Example 3-30>

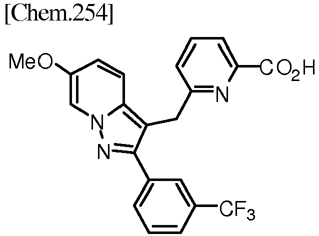

### 6-[[6-Methoxy-2-[3-(trifluoromethyl)phenyl]pyrazolo[1,5-a]pyridin-3-yl]methyl]pyridine-2-carboxylic acid

A 2 mol/L potassium hydroxide aqueous solution (0.09 mL) was added to a methanol solution (0.2 mL) of methyl 6-[[6-methoxy-2-[3-(trifluoromethyl)phenyl]pyrazolo[1,5-a]pyridin-3-yl]methyl]pyridine-2-carboxylate (15 mg) under an argon atmosphere under ice-cooling, and then the mixture was stirred at room temperature for 5 hours. A 1 mol/L hydrochloric acid was added to the reaction solution and the precipitated solid was filtered off to obtain a title compound as a colorless powder (12 mg).
¹H-NMR (400 MHz, DMSO-d₆) δ 3.83 (3H, s), 4.43 (2H, s), 7.05 (1H, dd, J = 9.7, 1.8 Hz), 7.35 (1H, dd, J = 6.7, 1.8 Hz), 7.63-7.73 (3H, m), 7.79-7.85 (2H, m), 8.11 (1H, s), 8.24 (1H, d, J = 7.9 Hz), 8.48 (1H, d, J = 1.8 Hz),13.08 (1H, brs).

### <Example 3-31>

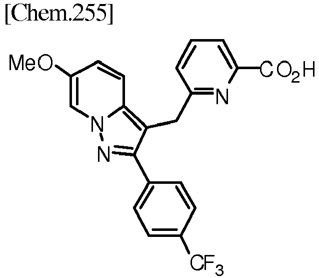

### 6-[[6-Methoxy-2-[4-(trifluoromethyl)phenyl]pyrazolo[1,5-a]pyridin-3-yl]methyl]pyridine-2-carboxylic acid

A 2 mol/L potassium hydroxide aqueous solution (0.7 mL) was added to a methanol solution (1.4 mL) of methyl 6-[[6-methoxy-2-[4-(trifluoromethyl)phenyl]pyrazolo[1,5-a]pyridin-3-yl]methyl]pyridine-2-carboxylate (121 mg) under an argon atmosphere under ice-cooling, and then the mixture was stirred at room temperature for 2 hours. A 1 mol/L hydrochloric acid was added to the reaction solution and the precipitated solid was filtered off to obtain a title compound as a colorless powder (102 mg).
¹H-NMR (400 MHz, DMSO-d₆) δ 3.83 (3H, s), 4.44 (2H, s), 7.04 (1H, dd, J = 9.7, 1.8 Hz), 7.37 (1H, dd, J = 6.7, 2.4 Hz), 7.68 (1H, d, J = 9.7 Hz), 7.78 (2H, d, J = 7.9 Hz), 7.81-7.85 (2H, m), 8.14 (2H, d, J = 7.9 Hz), 8.46 (1H, d, J = 2.4 Hz), 13.15 (1H, brs).

### <Example 3-32>

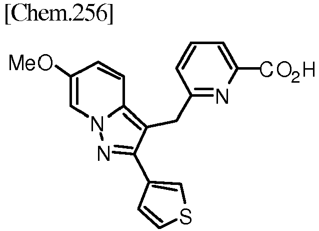

### 6-[[6-Methoxy-2-(thiophen-3-yl)pyrazolo[1,5-a]pyridin-3-yl]methyl]pyridine-2-carboxylic acid

A 2 mol/L potassium hydroxide aqueous solution (0.7 mL) was added to a methanol solution (1.3 mL) of methyl 6-[[6-methoxy-2-(thiophen-3-yl)pyrazolo[1,5-a]pyridin-3-yl]methyl]pyridine-2-carboxylate (101 mg) under an argon atmosphere under ice-cooling, and then the mixture was stirred at room temperature for 2 hours. A 1 mol/L hydrochloric acid was added to the reaction solution and the precipitated solid was filtered off to obtain a title compound as a yellow powder (72 mg).
¹H-NMR (400 MHz, DMSO-d₆) δ 3.81 (3H, s), 4.42 (2H, s), 7.01 (1H, dd, J = 9.7, 2.4 Hz), 7.34 (1H, dd, J = 7.3, 1.2 Hz), 7.57 (1H, dd, J = 5.4, 1.2 Hz), 7.61 (1H, dd, J = 5.4, 2.4 Hz), 7.68 (1H, d, J = 9.7 Hz), 7.81 (1H, d, J = 7.3 Hz), 7.83-7.86 (1H, m), 8.13 (1H, dd, J = 2.4, 1.2 Hz), 8.39 (1H, d, J = 2.4 Hz),13.10 (1H, brs).

### <Example 3-33>

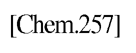

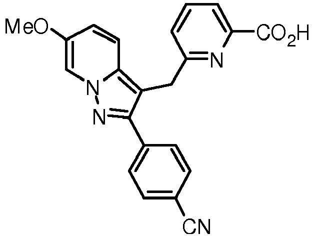

### 6-[[2-(4-Cyanophenyl)-6-methoxypyrazolo[1,5-a]pyridin-3-yl]methyl]pyridine-2-carboxylic acid

A 2 mol/L potassium hydroxide aqueous solution (0.4 mL) was added to a methanol solution (0.5 mL) of methyl 6-[[2-(4-cyanophenyl)-6-methoxypyrazolo[1,5-a]pyridin-3-yl]methyl]pyridine-2-carboxylate (32.0 mg) under an argon atmosphere under ice-cooling, and then the mixture was stirred at room temperature for 2 hours. A 1 mol/L hydrochloric acid was added to the reaction solution and the precipitated solid was filtered off to obtain a title compound as a colorless crystal (27.0 mg).
¹H-NMR (400 MHz, DMSO-d₆) δ 3.83 (3H, s), 4.44 (2H, s), 7.04 (1H, dd, J = 9.7, 1.8 Hz), 7.36 (1H, dd, J = 6.7, 1.8 Hz), 7.68 (1H, d, J = 9.7 Hz), 7.78-7.90 (4H, m), 8.12 (2H, d, J = 7.9 Hz), 8.45 (1H, d, J =1.8 Hz),13.11 (1H, brs).

### <Example 3-34>

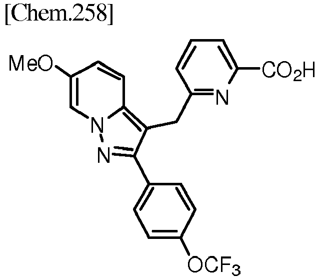

### 6-[[6-Methoxy-2-[4-(trifluoromethoxy)phenyl]pyrazolo[1,5-a]pyridin-3-yl]methyl]pyridine-2-carboxylic acid

A 2 mol/L potassium hydroxide aqueous solution (1.2 mL) was added to a methanol solution (2.3 mL) of methyl 6-[[6-methoxy-2-[4-(trifluoromethoxy)phenyl]pyrazolo[l,5-a]pyridin-3-yl]methyl]pyndine-2-carboxylate (209 mg) under an argon atmosphere under ice-cooling, and then the mixture was stirred at room temperature for 2 hours. A 1 mol/L hydrochloric acid was added to the reaction solution and the precipitated solid was filtered off to obtain a title compound as a colorless crystal (180 mg).
¹H-NMR (400 MHz, DMSO-d₆) δ 3.82 (3H, s), 4.40 (2H, s), 7.02 (1H, dd, J = 9.7, 1.8 Hz), 7.36 (1H, dd, J = 6.7, 1.8 Hz), 7.41 (2H, d, J = 7.9 Hz), 7.65 (1H, d, J = 6.7 Hz), 7.78-7.85 (2H, m), 8.02 (2H, d, J = 7.9 Hz), 8.43 (1H, d, J =1.8 Hz), 13.11 (1H, brs).

### <Example 3-35>

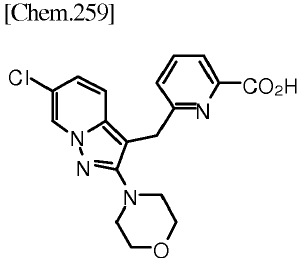

### 6-[[6-Chloro-2-(morpholin-4-yl)pyrazolo[1,5-a]pyridin-3-yl]methyl]pyridine-2-carboxylic acid

A 2 mol/L potassium hydroxide aqueous solution (0.1 mL, 0.2 mmol) was added to a methanol solution (0.4 mL) of methyl 6-[[6-chloro-2-(morpholin-4-yl)pyrazolo[1,5-a]pyridin-3-yl]methyl]pyridine-2-carboxylate (14.0 mg, 0.0362 mmol) under an argon atmosphere under ice-cooling, and then the mixture was stirred at room temperature for 2 hours. A 1 mol/L hydrochloric acid was added to the reaction solution and then the mixture was extracted with dichloromethane : methanol (5:1, v/v) and then dried over anhydrous sodium sulfate. The solvent was evaporated to obtain a title compound as a yellow foam (13.5 mg, 100%).
¹H-NMR (400 MHz, DMSO-d₆) δ 3.17 (4H, t, J = 4.9 Hz), 3.59 (4H, t, J = 4.9 Hz), 4.23 (2H, s), 7.14 (1H, dd, J = 9.7, 1.8 Hz), 7.31 (1H, dd, J = 7.3, 1.8 Hz), 7.51 (1H, d, J = 9.7 Hz), 7.81-7.88 (2H, m), 8.76 (1H, d, J =1.8 Hz).

### <Example 3-36>

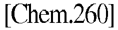

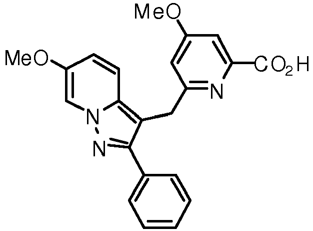

### 4-Methoxy-6-[(6-methoxy-2-phenylpyrazolo[1,5-a]pyridin-3-yl)methyl]pyridine-2-carboxyl ic acid

A tetrahydrofuran-methanol (4 mL, 1:1 v/v) solution of methyl 4-methoxy-6-[(6-methoxy-2-phenylpyrazolo[1,5-a]pyridin-3-yl)methyl]pyridine-2-carboxyl ate (187 mg) was ice-cooled and a sodium hydroxide aqueous solution (1 mol/L, 1.0 mL) was added. The mixture was heated up to room temperature, stirred for 2 hours, and then ice-cooled again. Next, a 1 mol/L hydrochloric acid was slowly added to achieve pH = 1. The organic solvent was evaporated under vacuum and then the produced precipitate was filtered off to obtain a title compound as a colorless solid (173 mg).
¹H-NMR (400 MHz, DMSO-d₆) δ 3.82 (3H, s), 4.34 (2H, s), 6.81 (1H, d, J = 2.4 Hz), 7.01 (1H, dd, J = 9.7, 2.4 Hz), 7.34-7.38 (2H, m), 7.43 (2H, t, J = 7.6 Hz), 7.61 (1H, d, J = 9.7 Hz), 7.84 (2H, d, J = 6.7 Hz), 8.42 (1H, d, J = 2.4 Hz), 13.09 (1H, s).

### <Example 3-37>

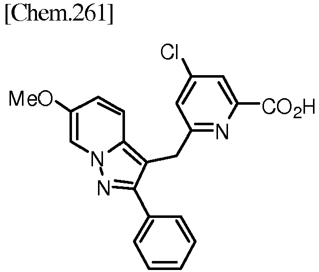

### 4-Chloro-6-[(6-methoxy-2-phenylpyrazolo[1,5-a]pyridin-3-yl)methyl]pyridine-2-carboxylic acid

A tetrahydrofuran-methanol (2 mL, 1:1 v/v) solution of methyl 4-chloro-6-[(6-methoxy-2-phenylpyrazolo[1,5-a]pyridin-3-yl)methyl]pyridine-2-carboxylate (31.7 mg) was ice-cooled and a sodium hydroxide aqueous solution (1 mol/L, 0.5 mL) was added. The mixture was heated up to room temperature, stirred for 2 hours, and then ice-cooled again. Next, a 1 mol/L hydrochloric acid was slowly added to achieve pH = 1. The organic solvent was evaporated under vacuum and then the produced precipitate was filtered off to obtain a title compound as a white solid (30.6 mg).
¹H-NMR (400 MHz, DMSO-d₆) δ 3.82 (3H, s), 4.41 (2H, s), 7.03 (1H, dd, J = 9.7, 1.8 Hz), 7.34-7.45 (4H, m), 7.63 (1H, d, J = 9.7 Hz), 7.79-7.83 (2H, m), 7.85 (1H, d, J =1.8 Hz), 8.43 (1H, d, J = 2.4 Hz),13.49 (1H, s).

### <Example 3-38>

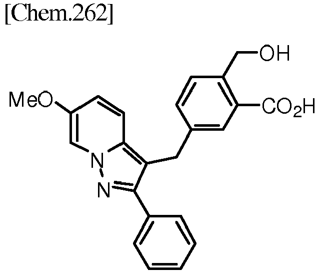

### 2-(Hydroxymethyl)-5-[(6-methoxy-2-phenylpyrazolo[1,5-a]pyridin-3-yl)methyl]benzoic acid

A 2 mol/L potassium hydroxide aqueous solution (0.1 mL) was added to a methanol solution (0.2 mL) of 4-[(6-methoxy-2-phenylpyrazolo[1,5-a]pyridin-3-yl)methyl]-1(3*H*)-isobenzofuranone (15 mg) at 0°C under an argon atmosphere, and then the mixture was stirred at room temperature for 1 hour. A 1 mol/L hydrochloric acid (0.2 mL) was added to the reaction solution and the precipitated solid was filtered off to obtain a title compound as a colorless powder (11 mg).
¹H-NMR (400 MHz, DMSO-d₆) δ 3.82 (3H, s), 4.21 (2H, s), 4.46 (2H, s), 6.98 (1H, dd, J = 9.7, 1.8 Hz), 7.04 (1H, d, J = 7.3 Hz), 7.14-7.21 (1H, m), 7.34 (1H, t, J = 7.3 Hz), 7.38-7.45 (3H, m), 7.50 (1H, d, J =1.8 Hz), 7.64-7.68 (2H, m), 8.42 (1H, d, J =1.8 Hz).

### <Example 3-39>

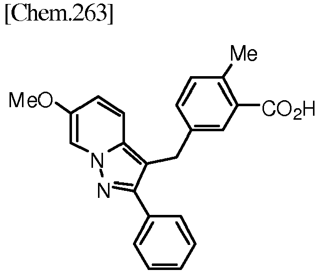

### 5-[(6-Methoxy-2-phenylpyrazolo[1,5-a]pyridin-3-yl)methyl]-2-methylbenzoic acid

A 2 mol/L potassium hydroxide aqueous solution (0.1 mL) was added to a methanol solution (0.3 mL) of methyl 5-[(6-methoxy-2-phenylpyrazolo[1,5-a]pyiidin-3-yl)methyl]-2-methylbenzoate (19 mg) at 0°C under an argon atmosphere, and then the mixture was stirred at room temperature for 2 hours. A 1 mol/L hydrochloric acid (0.2 mL) was added to the reaction solution and the precipitated solid was filtered off to obtain a title compound as a colorless powder (12 mg).
¹H-NMR (400 MHz, DMSO-d₆) δ 2.42 (3H, s), 3.83 (3H, s), 4.24 (2H, s), 6.99 (1H, dd, J = 9.7, 1.8 Hz), 7.13-7.18 (2H, m), 7.32-7.37 (1H, m), 7.41 (2H, t, J = 7.3 Hz), 7.47 (1H, d, J = 9.7 Hz), 7.53 (1H, s), 7.63-7.66 (2H, m), 8.42 (1H, d, J =1.8 Hz), 12.71 (1H, brs).

### <Example 3-40>

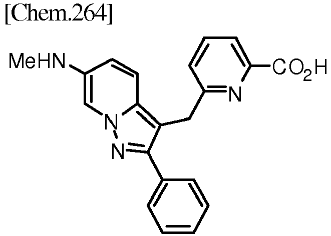

### 6-[[6-(Methylamino)-2-phenylpyrazolo[1,5-a]pyridin-3-yl]methyl]pyridine-2-carboxylic acid

A 2 mol/L potassium hydroxide aqueous solution (0.1 mL) was added to a methanol solution (0.2 mL) of methyl 6-[[6-(methylamino)-2-phenylpyrazolo[1,5-a]pyridin-3-yl]methyl]pyridine-2-carboxylate (18 mg) at 0°C under an argon atmosphere, and then the mixture was stirred at room temperature for 3 hours. A 1 mol/L hydrochloric acid (0.2 mL) was added to the reaction solution and the precipitated solid was filtered off to obtain a title compound as a yellow powder (12 mg).
¹H-NMR (400 MHz, DMSO-d₆) δ 2.97 (3H, s), 4.68 (2H, s), 6.47 (1H, d, J = 7.3 Hz), 6.76 (1H, t, J = 7.3 Hz), 7.32-7.37 (2H, m), 7.41-7.46 (2H, m), 7.60-7.64 (1H, m), 7.96-8.00 (4H, m), 8.25 (1H, d, J = 6.7 Hz).

### <Example 3-41>

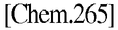

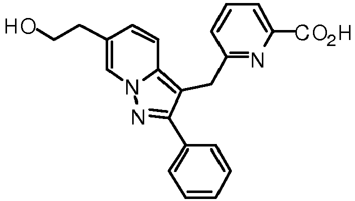

### 6-[[6-(2-Hydroxyethyl)-2-phenylpyrazolo[1,5-a]pyridin-3-yl]methyl]pyridine-2-carboxylic acid

A 2 mol/L potassium hydroxide aqueous solution (0.3 mL) was added to a methanol solution (0.7 mL) of methyl 6-[[6-(2-hydroxyethyl)-2-phenylpyrazolo[1,5-a]pyridin-3-yl]methyl]pyridine-2-carboxylate (50 mg) at 0°C under an argon atmosphere, and then the mixture was stirred at room temperature for 5 hours. A 1 mol/L hydrochloric acid (0.6 mL) was added to the reaction solution and the precipitated solid was filtered off to obtain a title compound as a colorless powder (21 mg).
¹H-NMR (400 MHz, DMSO-d₆) δ 2.73 (2H, t, J = 6.7 Hz), 3.64 (2H, t, J = 6.7 Hz), 7.12 (1H, dd, J = 9.1, 1.2 Hz), 7.27 (1H, dd, J = 7.3, 1.2 Hz), 7.37 (1H, tt, J = 7.3, 1.2 Hz), 7.40-7.45 (2H, m), 7.58 (1H, d, J = 9.1 Hz), 7.78-7.85 (4H, m), 8.51 (1H, s).

### <Example 3-42>

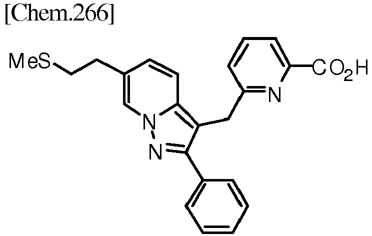

### 6-[[6-[2-(Methylsulfanyl)ethyl]-2-phenylpyrazolo[1,5-a]pyridin-3-yl]methyl]pyridine-2-carb oxylic acid

A 2 mol/L potassium hydroxide aqueous solution (0.5 mL) was added to a methanol solution (1.0 mL) of methyl 6-[[6-[2-(methylsulfanyl)ethyl]-2-phenylpyrazolo[1,5-a]pyridin-3-yl]methyl]pyridine-2-carb oxylate (80 mg) at 0°C under an argon atmosphere, and then the mixture was stirred at room temperature for 1 hour. A 1 mol/L hydrochloric acid (1.0 mL) was added to the reaction solution and the precipitated solid was filtered off to obtain a title compound as a colorless powder (71 mg).
¹H-NMR (400 MHz, DMSO-d₆) δ 2.09 (3H, s), 2.77 (2H, t, J = 7.3 Hz), 2.86 (2H, t, J = 7.3 Hz), 4.39 (2H, s), 7.15 (1H, dd, J = 9.1, 1.2 Hz), 7.22 (1H, d, J = 7.3 Hz), 7.38 (1H, dt, J = 7.3, 1.2 Hz), 7.43 (2H, tt, J = 7.3, 1.2 Hz), 7.59 (1H, d, J = 9.1 Hz), 7.76 (1H, t, J = 7.3 Hz), 7.79-7.84 (3H, m), 8.60 (1H, s).

### <Example 3-43>

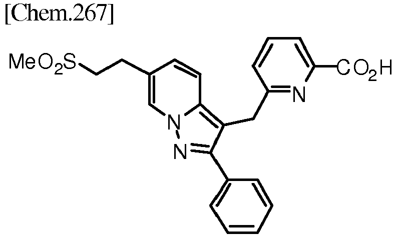

### 6-[[6-[2-(Methylsulfonyl)ethyl]-2-phenylpyrazolo[1,5-a]pyridin-3-yl]methyl]pyridine-2-carb oxylic acid

A 2 mol/L potassium hydroxide aqueous solution (0.03 mL) was added to a methanol solution (0.2 mL) of methyl 6-[[6-[2-(methylsulfonyl)ethyl]-2-phenylpyrazolo[1,5-a]pyridin-3-yl]methyl]pyridine-2-carb oxylate (20 mg) at 0°C under an argon atmosphere, and then the mixture was stirred at room temperature for 1 hour. A 1 mol/L hydrochloric acid (0.06 mL) was added to the reaction solution and the precipitated solid was filtered off to obtain a title compound as a brown powder (15 mg).
¹H-NMR (400 MHz, DMSO-d₆) δ 3.02 (3H, s), 3.03-3.07 (2H, m), 3.47-3.52 (2H, m), 4.32 (2H, s), 6.74 (1H, dd, J = 7.3, 1.2 Hz), 7.16 (1H, dd, J = 9.1, 1.2 Hz), 7.38 (1H, d, J = 7.3 Hz), 7.43 (2H, t, J = 7.3 Hz), 7.48 (1H, d, J = 7.9 Hz), 7.51 (1H, d, J = 9.1 Hz), 7.55 (1H, d, J = 7.9 Hz), 7.75-7.78 (2H, m), 8.66 (1H, s).

### <Example 4-1>

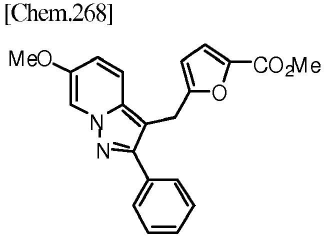

### Methyl 5-[(6-methoxy-2-phenylpyrazolo[1,5-a]pyridin-3-yl)methyl]furan-2-carboxylate

Tetrabutylammonium fluoride (0.1 mL, a 1 mol/L tetrahydrofuran solution) was added to a tetrahydrofuran solution (0.7 mL) of methyl 5-[(6-methoxy-2-phenyl-7-(trimethylsilyl)pyrazolo[1,5-a]pyridin-3-yl)methyl]furan-2-carbo xylate (30 mg) under an argon atmosphere under ice-cooling, and then the mixture was stirred for 1 hour under ice-cooling. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as colorless liquid (26 mg).
¹H-NMR (400 MHz, CDCl₃) δ 3.85 (3H, s), 3.89 (3H, s), 4.25 (2H, s), 5.96 (1H, d, J = 3.6 Hz), 6.94 (1H, dd, J = 9.7, 2.4 Hz), 7.06 (1H, d, J = 3.6 Hz), 7.28 (1H, d, J = 9.7 Hz), 7.35-7.40 (1H, m), 7.41-7.46 (2H, m), 7.63-7.67 (2H, m), 8.10 (1H, d, J = 2.4 Hz).

### <Example 4-2>

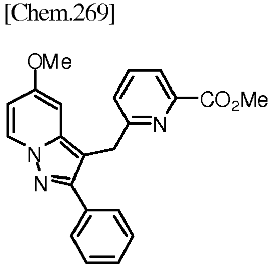

### Methyl 6-[(5-methoxy-2-phenylpyrazolo[1,5-a]pyridin-3-yl)methyl]pyridine-2-carboxylate

Tetrabutylammonium fluoride (0.5 mL, a 1 mol/L tetrahydrofuran solution) was added to a tetrahydrofuran solution (2.7 mL) of methyl 6-[(5-methoxy-2-phenyl-7-(trimethylsilyl)pyrazolo[1,5-a]pyridin-3-yl)methyl]pyridine-2-car boxylate (120 mg) under an argon atmosphere under ice-cooling, and then the mixture was stirred for 1 hour under ice-cooling. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as pale yellow liquid (90 mg).
¹H-NMR (400 MHz, CDCl₃) δ 3.78 (3H, s), 4.03 (3H, s), 4.50 (2H, s), 6.46 (1H, dd, J = 7.9, 3.0 Hz), 6.64 (1H, d, J = 3.0 Hz), 7.14 (1H, d, J = 7.9 Hz), 7.34-7.44 (3H, m), 7.63-7.70 (3H, m), 7.96 (1H, d, J = 7.3 Hz), 8.30 (1H, d, J = 7.3 Hz).

### <Example 4-3>

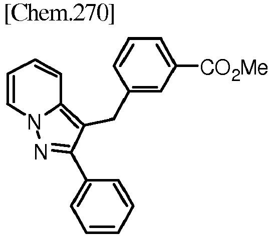

### Methyl 3-[(2-phenylpyrazolo[1,5-a]pyridin-3-yl)methyl]benzoate

Tetrabutylammonium fluoride (0.9 mL) was added to a tetrahydrofuran solution (4.6 mL) of methyl 3-[[2-phenyl-7-(trimethylsilyl)pyrazolo[1,5-a]pyridin-3-yl]methyl]benzoate (190 mg) under an argon atmosphere under ice-cooling, and then the mixture was stirred for 1 hour under ice-cooling. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as yellow liquid (122 mg).
¹H-NMR (400 MHz, CDCl₃) δ 3.88 (3H, s), 4.30 (2H, s), 6.76 (1H, td, J = 6.7, 1.2 Hz), 7.04 (1H, ddd, J = 9.1, 6.7, 1.2 Hz), 7.23 (1H, dt, J = 9.1, 1.2 Hz), 7.32 (2H, dd, J = 4.8, 1.2 Hz), 7.37-7.45 (3H, m), 7.65-7.68 (2H, m), 7.85-7.90 (2H, m), 8.49 (1H, d, J = 6.7 Hz).

### <Example 4-4>

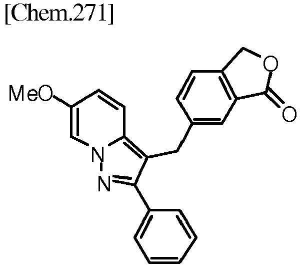

### 4-[(6-Methoxy-2-phenylpyrazolo[1,5-a]pyridin-3-yl)methyl]-1(3H)-isobenzofuranone

Tetrabutylammonium fluoride (0.3 mL, a 1 mol/L tetrahydrofuran solution) was added to a dichloromethane solution (1.7 mL) of 4-[[6-methoxy-2-phenyl-7-(trimethylsilyl)pyrazolo[1,5-a]pyridin-3-yl]methyl]-1 (3*H*)-isoben zofuranone (75 mg) under an argon atmosphere under ice-cooling, and then the mixture was stirred at 0°C for 1 hour. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 3:2) to obtain a title compound as yellow liquid (52 mg).
¹H-NMR (400 MHz, CDCl₃) δ 3.86 (3H, s), 4.34 (2H, s), 5.28 (2H, s), 6.89 (1H, dd, J = 9.7, 1.8 Hz), 7.12 (1H, d, J = 9.7 Hz), 7.37 (2H, d, J = 7.3 Hz), 7.41 (2H, t, J = 7.3 Hz), 7.47 (1H, dd, J = 7.9, 1.2 Hz), 7.60-7.64 (2H, m), 7.70 (1H, d, J =1.2 Hz), 8.15 (1H, d, J =1.8 Hz).

### <Example 4-5>

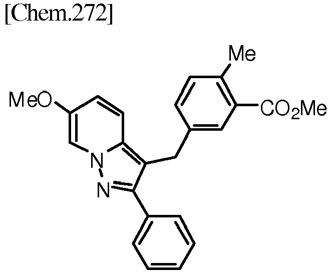

### Methyl 5-[(6-methoxy-2-phenylpyrazolo[1,5-a]pyridin-3-yl)methyl]-2-methylbenzoate

Tetrabutylammonium fluoride (0.1 mL, a 1 mol/L tetrahydrofuran solution) was added to a dichloromethane solution (0.7 mL) of methyl 5-[[6-methoxy-2-phenyl-7-(trimethylsilyl)pyrazolo[1,5-a]pyridin-3-yl]methyl]-2-methylbenz oate (34 mg) at 0°C under an argon atmosphere, and then the mixture was stirred at 0°C for 1 hour. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as yellow liquid (20 mg).
¹H-NMR (400 MHz, CDCl₃) δ 2.54 (3H, s), 3.84 (3H, s), 3.85 (3H, s), 4.22 (2H, s), 6.86 (1H, dd, J = 9.7, 2.4 Hz), 7.09-7.16 (3H, m), 7.37 (1H, d, J = 7.3 Hz), 7.39-7.44 (2H, m), 7.63-7.67 (2H, m), 7.74 (1H, d, J =1.2 Hz), 8.13 (1H, d, J = 2.4 Hz).

### <Example 5-1>

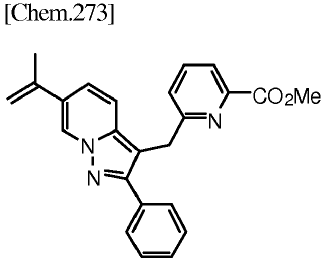

### Methyl 6-[[2-phenyl-6-(propen-2-yl)pyrazolo[1,5-a]pyridin-3-yl]methyl]pyridine-2-carboxylate

Isopropenyl pinacol borate ester (0.03 mL), a 2 mol/L sodium carbonate aqueous solution (0.2 mL), and dichlorobis(triphenylphosphine)palladium (II) (8 mg) were added to a dimethoxyethane solution (0.4 mL) of methyl 6-[(6-bromo-2-phenyl-pyrazolo[1,5-a]pyridin-3-yl)methyl]pyridine-2-carboxylate (50 mg) at room temperature under an argon atmosphere, and then the mixture was stirred at 60°C for 5 hours. Water was added to the reaction solution and the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as yellow liquid (37 mg).
¹H-NMR (400 MHz, CDCl₃) δ2.18 (3H, s), 4.04 (3H, s), 4.57 (2H, s), 5.17 (1H, s), 5.46 (1H, s), 7.11 (1H, d, J = 7.9 Hz), 7.31 (2H, d, J = 1.2 Hz), 7.37-7.45 (3H, m), 7.64 (1H, t, J = 7.9 Hz), 7.69-7.73 (2H, m), 7.96 (1H, d, J = 7.9 Hz), 8.54 (1H, s).

### <Example 5-2>

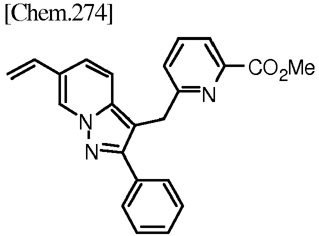

### Methyl 6-[(2-phenyl-6-vinylpyrazolo[1,5-a]pyridin-3-yl)methyl]pyridine-2-carboxylate

A trivinylboroxine-pyridine complex (43 mg), a 2 mol/L sodium carbonate aqueous solution (0.2 mL), and dichlorobis(triphenylphosphine)palladium (II) (8 mg) were added to a dimethoxyethane solution (0.4 mL) of methyl 6-[(6-bromo-2-phenyl-pyrazolo[1,5-a]pyridin-3-yl)methyl]pyridine-2-carboxylate (50 mg) at room temperature under an argon atmosphere, and then the mixture was stirred at 60°C for 7 hours. Water was added to the reaction solution and the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 5:1) to obtain a title compound as yellow liquid (37 mg).
¹H-NMR (400 MHz, CDCl₃) δ 4.04 (3H, s), 4.56 (2H, s), 5.32 (1H, d, J = 10.9 Hz), 5.73 (1H, d, J = 17.6 Hz), 6.67 (1H, dd, J = 17.6, 10.9 Hz), 7.11 (1H, d, J = 7.9 Hz), 7.28-7.45 (5H, m), 7.64 (1H, t, J = 7.9 Hz), 7.69-7.73 (2H, m), 7.96 (1H, d, J = 7.9 Hz), 8.42 (1H, s).

### <Example 5-3>

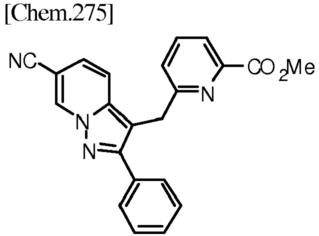

### Methyl 6-[(6-cyano-2-phenylpyrazolo[1,5-a]pyridin-3-yl)methyl]pyridine-2-carboxylate

Zinc cyanide (28 mg), 2-dicyclohexylphosphino-2',6'-dimethoxybiphenyl (10 mg), and dibenzylideneacetonepalladium (7 mg) were added to an *N,N*-limethylformamide-water mixed solution (1.2 mL, 99:1) of methyl 6-[(6-bromo-2-phenyl-pyrazolo[1,5-a]pyridin-3-yl)methyl]pyridine-2-carboxylate (50 mg), at room temperature under an argon atmosphere, and then the mixture was stirred at 60°C for 4 hours. Water was added to the reaction solution and the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as colorless liquid (23 mg).
¹H-NMR (400 MHz, CDCl₃) δ 4.03 (3H, s), 4.54 (2H, s), 7.09 (1H, dd, J = 7.9, 1.2 Hz), 7.14 (1H, dd, J = 9.1, 1.2 Hz), 7.42-7.47 (3H, m), 7.56 (1H, dd, J = 9.1, 1.2 Hz), 7.64-7.68 (1H, m), 7.69-7.73 (3H, m), 7.98 (1H, dd, J = 7.9,1.2 Hz).

### <Example 5-4>

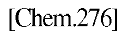

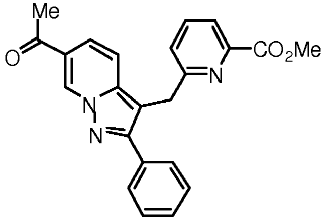

### Methyl 6-[(6-acetyl-2-phenylpyrazolo[1,5-a]pyridin-3-yl)methyl]pyridine-2-carboxylate

*n*-Butylvinylether (0.08 mL), triphenylphosphine (6 mg), triethylamine (0.02 mL), and palladium (II) acetate (3 mg) were added to an acetonitrile solution (0.6 mL) of methyl 6-[(6-bromo-2-phenyl-pyrazolo[1,5-a]pyridin-3-yl)methyl]pyridine-2-carboxylate (50 mg) at room temperature under an argon atmosphere, and then the mixture was stirred at 100°C for 4 hours. The reaction solution was filtered by Celite and extracted with ethyl acetate. The organic layer was washed with saturated saline and then dried over anhydrous magnesium sulfate. The solvent was evaporated, then a hydrogen chloride solution (3 mL, a 4.0 mol/L 1,4-dioxane solution) was added, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as yellow liquid (21 mg).
¹H-NMR (400 MHz, CDCl₃) δ 2.62 (3H, s), 4.04 (3H, s), 4.56 (2H, s), 7.10 (1H, d, J = 7.9 Hz), 7.41-7.48 (4H, m), 7.61-7.66 (2H, m), 7.74 (2H, dd, J = 7.9, 1.8 Hz), 7.97 (1H, d, J = 7.9 Hz),9.14(1H,s).

### <Example 6-1>

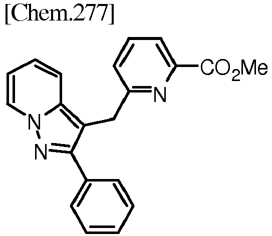

### Methyl 6-[(2-phenylpyrazolo[1,5-a]pyridin-3-yl)methyl]pyridine-2-carboxylate

Triethylsilane (0.3 mL) and trifluoroacetic acid (0.2 mL) were added to a dichloromethane solution (1.4 mL) of methyl 6-[hydroxy(2-phenylpyrazolo[1,5-a]pyridin-3-yl)methyl]pyridine-2-carboxylate (100 mg) at room temperature under an argon atmosphere, and then the mixture was stirred at room temperature for 2 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as yellow liquid (81 mg).
¹H-NMR (400 MHz, CDCl₃) δ 4.04 (3H, s), 4.58 (2H, s), 6.78 (1H, td, J = 6.7, 1.2 Hz), 7.07 (1H, ddd, J = 9.2, 6.7, 1.2 Hz), 7.11 (1H, d, J = 7.9 Hz), 7.35-7.40 (2H, m), 7.42 (2H, tt, J = 6.7, 1.8 Hz), 7.64 (1H, t, J = 7.9 Hz), 7.70-7.73 (2H, m), 7.96 (1H, d, J = 7.9 Hz), 8.48-8.51 (1H, m).

### <Example 6-2>

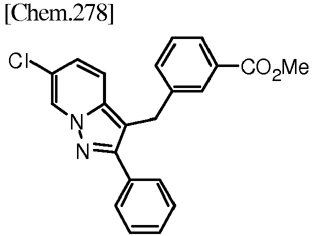

### Methyl 3-[(6-chloro-2-phenylpyrazolo[1,5-a]pyridin-3-yl)methyl]benzoate

Triethylsilane (0.2 mL) and trifluoroacetic acid (0.2 mL) were added to a dichloromethane solution (1.3 mL) of methyl 3-[(6-chloro-2-phenylpyrazolo[1,5-a]pyridin-3-yl)(hydroxy)methyl]benzoate(100 mg) at room temperature under an argon atmosphere, and then the mixture was stirred at room temperature for 2 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as colorless liquid (82 mg).
¹H-NMR (400 MHz, CDCl₃) δ 3.88 (3H, s), 4.28 (2H, s), 7.00 (1H, dd, J = 9.1,1.2 Hz), 7.15 (1H, d, J = 9.1 Hz), 7.28-7.34 (2H, m), 7.38-7.44 (3H, m), 7.64 (2H, dt, J = 7.9, 1.2 Hz), 7.87 (2H, d, J = 8.5 Hz), 8.53 (1H, d, J =1.2 Hz).

### <Example 6-3>

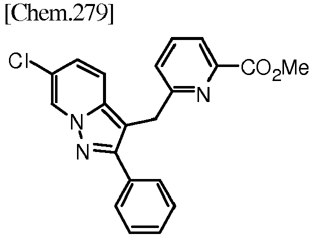

### Methyl 6-[(6-chloro-2-phenylpyrazolo[1,5-a]pyridin-3-yl)methyl]pyridine-2-carboxylate

Triethylsilane (0.2 mL) and trifluoroacetic acid (0.2 mL) were added to a dichloromethane solution (1.3 mL) of methyl 6-[(6-chloro-2-phenylpyrazolo[1,5-a]pyridin-3-yl)(hydroxy)methyl]pyridine-2-carboxylate (100 mg) at room temperature under an argon atmosphere, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as yellow liquid (97 mg).
¹H-NMR (400 MHz, CDCl₃) δ 4.03 (3H, s), 4.54 (2H, s), 7.04 (1H, dd, J = 9.7, 1.8 Hz), 7.09 (1H, d, J = 7.9 Hz), 7.37 (1H, d, J = 9.7 Hz), 7.38-7.46 (3H, m), 7.65 (1H, t, J = 7.9 Hz), 7.68-7.72 (2H, m), 7.96 (1H, d, J = 7.9 Hz), 8.52 (1H, d, J =1.8 Hz).

### <Example 6-4>

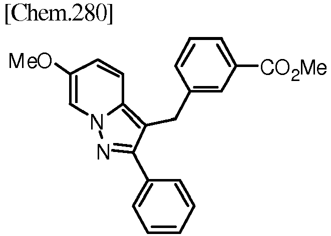

### Methyl 3-[(6-methoxy-2-phenylpyrazolo[1,5-a]pyridin-3-yl)methyl]benzoate

Triethylsilane (0.3 mL) and trifluoroacetic acid (0.3 mL) were added to a dichloromethane solution (1.6 mL) of methyl 3-[hydroxy(6-methoxy-2-phenylpyrazolo[1,5-a]pyridin-3-yl)methyl]benzoate (120 mg) at room temperature under an argon atmosphere, and then the mixture was stirred at room temperature for 2 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as colorless liquid (69 mg).
¹H-NMR (400 MHz, CDCl₃) δ 3.84 (3H, s), 3.88 (3H, s), 4.28 (2H, s), 6.85 (1H, dd, J = 9.7, 2.4 Hz), 7.10 (1H, d, J = 9.7 Hz), 7.30-7.32 (2H, m), 7.35-7.38 (1H, m), 7.41 (2H, tt, J = 7.3, 1.2 Hz), 7.62-7.66 (2H, m), 7.85-7.89 (2H, m), 8.11 (1H, d, J = 2.4 Hz).

### <Example 6-5>

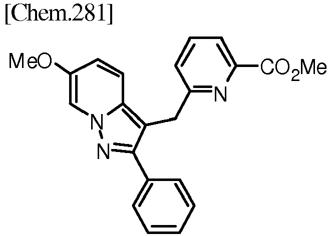

### Methyl 6-[(6-methoxy-2-phenylpyrazolo[1,5-a]pyridin-3-yl)methyl]pyridine-2-carboxylate

Triethylsilane (0.1 mL) and trifluoroacetic acid (0.1 mL) were added to a dichloromethane solution (0.8 mL) of methyl 6-[hydroxy(6-methoxy-2-phenylpyrazolo[1,5-a]pyridin-3-yl)methyl]pyridine-2-carboxylate (60 mg) at room temperature under an argon atmosphere, and then the mixture was stirred at room temperature for 8 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as pale yellow liquid (40 mg).
¹H-NMR (400 MHz, CDCl₃) δ 3.84 (3H, s), 4.04 (3H, s), 4.54 (2H, s), 6.88 (1H, dd, J = 9.7, 2.4 Hz), 7.11 (1H, d, J = 7.9 Hz), 7.26 (1H, d, J = 9.7 Hz), 7.34-7.38 (1H, m), 7.39-7.44 (2H, m), 7.64 (1H, t, J = 7.9 Hz), 7.67-7.71 (2H, m), 7.96 (1H, d, J = 7.9 Hz), 8.10 (1H, d, J = 2.4 Hz).

### <Example 6-6>

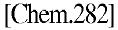

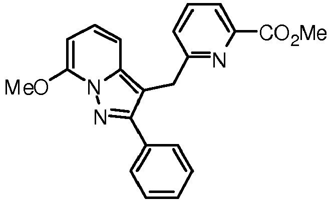

### Methyl 6-[(7-methoxy-2-phenylpyrazolo[1,5-a]pyridin-3-yl)methyl]pyridine-2-carboxylate

Triethylsilane (0.3 mL) and trifluoroacetic acid (0.3 mL) were added to a dichloromethane solution (1.5 mL) of methyl 6-[hydroxy(7-methoxy-2-phenylpyrazolo[1,5-a]pyridin-3-yl)methyl]pyridine-2-carboxylate (120 mg) at room temperature under an argon atmosphere, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 1:1) to obtain a title compound as pale yellow liquid (95 mg).
¹H-NMR (400 MHz, CDCl₃) δ 4.04 (3H, s), 4.18 (3H, s), 4.55 (2H, s), 6.11 (1H, dd, J = 7.3, 1.2 Hz), 7.00 (1H, dd, J = 9.1, 1.2 Hz), 7.06-7.11 (2H, m), 7.33-7.42 (3H, m), 7.62 (1H, t, J = 7.3 Hz), 7.71-7.74 (2H, m), 7.95 (1H, d, J = 7.3 Hz).

### <Example 6-7>

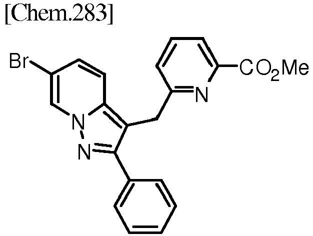

### Methyl 6-[(6-bromo-2-phenylpyrazolo[1,5-a]pyridin-3-yl)methyl]pyridine-2-carboxylate

After trifluoroacetic acid (1.50 mL) and triethylsilane (1.50 mL) were added to a dichloromethane (16 mL) solution of methyl 6-[(6-bromo-2-phenylpyrazolo[1,5-a]pyridin-3-yl)(hydroxy)methyl]pyridine-2-carboxylate (1.39 g), the mixture was stirred at room temperature for 5 hours. A saturated sodium bicarbonate aqueous solution was added to the reaction solution to achieve pH = 8 to 9 and then the mixture was extracted with ethyl acetate. Subsequently, the extract was washed with saturated saline and then dried over anhydrous sodium sulfate. The solvent was evaporated and the residue thus obtained was purified by silica gel column chromatography (*n*-hexane:ethyl acetate = 2:1) to obtain a title compound as a pale yellow oil (1.29 g).
¹H-NMR (400 MHz, CDCl₃) δ 4.04 (3H, s), 4.54 (2H, s), 7.08 (1H, d, J = 7.3 Hz), 7.13 (1H, dd, J = 9.7 and 1.8 Hz), 7.32 (1H, d, J = 9.7 Hz), 7.36-7.47 (3H, m), 7.65 (1H, t, J = 7.9 Hz), 7.67-7.73 (2H, m), 7.96 (1H, d, J = 7.9 Hz), 8.63 (1H, s).

### <Example 6-8>

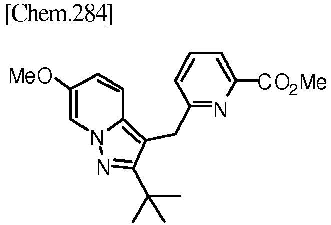

### Methyl 6-[(2-tert-butyl-6-methoxypyrazolo[1,5-a]pyridin-3-yl)methyl]pyridine-2-carboxylate

After trifluoroacetic acid (0.40 mL) and triethylsilane (0.40 mL) were added to a dichloromethane (2.1 mL) solution of methyl 6-[(2-*tert*-butyl-6-methoxypyrazolo[1,5-a]pyridin-3-yl)(hydroxy)methyl]pyridine-2-carboxyl ate (155 mg), the mixture was stirred at room temperature for 5 hours. A saturated sodium bicarbonate aqueous solution was added to the reaction solution to achieve pH = 8 to 9 and then the mixture was extracted with ethyl acetate. Subsequently, the extract was washed with saturated saline and then dried over anhydrous sodium sulfate. The solvent was evaporated and the residue thus obtained was purified by silica gel column chromatography (*n*-hexane:ethyl acetate =1:1) to obtain a title compound as a yellow oil (127 mg).
¹H-NMR (400 MHz, CDCl₃) δ 1.40 (9H, s), 3.80 (3H, s), 4.04 (3H, s), 4.54 (2H, s), 6.80 (1H, dd, J = 9.7 and 1.8 Hz), 6.98 (1H, d, J = 7.9 Hz), 7.04 (1H, d, J = 9.1 Hz), 7.62 (1H, t, J = 7.9 Hz), 7.95 (1H, d, J = 6.7 Hz), 8.03 (1H, d, J =1.8 Hz).

### <Example 6-9>

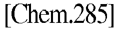

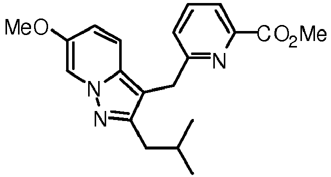

### Methyl 6-[(2-isobutyl-6-methoxypyrazolo[1,5-a]pyridin-3-yl)methyl]pyridine-2-carboxylate

After trifluoroacetic acid (0.47 mL) and triethylsilane (0.47 mL) were added to a dichloromethane (2.6 mL) solution of methyl 6-[(2-isobutyl-6-methoxypyrazolo[1,5-a]pyridin-3-yl)(hydroxy)methyl]pyridine-2-carboxyla te (192 mg), the mixture was stirred at room temperature for 5 hours. A saturated sodium bicarbonate aqueous solution was added to the reaction solution to achieve pH = 8 to 9 and then the mixture was extracted with ethyl acetate. Subsequently, the extract was washed with saturated saline and then dried over anhydrous sodium sulfate. The solvent was evaporated and the residue thus obtained was purified by silica gel column chromatography (*n*-hexane:ethyl acetate =1:1) to obtain a title compound as a yellow oil (177 mg).
¹H-NMR (400 MHz, CDCl₃) δ 0.92 (6H, d, J = 6.6 Hz), 1.95-2.07 (1H, m), 2.62 (2H, d, J = 7.3 Hz), 3.81 (3H, s), 4.04 (3H, s), 4.33 (2H, s), 6.82 (1H, dd, J = 9.7 and 1.8 Hz), 7.06 (1H, d, J = 7.9 Hz), 7.16 (1H, d, J = 9.7 Hz), 7.65 (1H, t, J = 7.9 Hz), 7.95 (1H, d, J = 7.9 Hz), 8.01 (1H,d,J=1.8Hz).

### <Example 6-10>

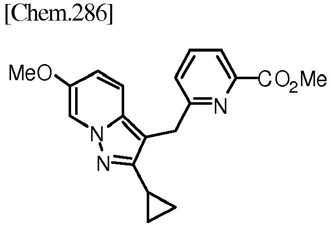

### Methyl 6-[(2-cyclopropyl-6-methoxypyrazolo[1,5-a]pyridin-3-yl)methyl]pyridine-2-carboxylate

After trifluoroacetic acid (0.60 mL) and triethylsilane (0.60 mL) were added to a dichloromethane (3.3 mL) solution of methyl 6-[(2-cyclopropyl-6-methoxypyrazolo[1,5-a]pyridin-3-yl)(hydroxy)methyl]pyridine-2-carbo xylate (232 mg), the mixture was stirred at room temperature for 5 hours. A saturated sodium bicarbonate aqueous solution was added to the reaction solution to achieve pH = 8 to 9 and then the mixture was extracted with ethyl acetate. Subsequently, the extract was washed with saturated saline and then dried over anhydrous sodium sulfate. The solvent was evaporated and the residue thus obtained was purified by silica gel column chromatography (*n*-hexane:ethyl acetate = 1:1) to obtain a title compound as a yellow crystal (200 mg).
¹H-NMR (400 MHz, CDCl₃) δ0.90-1.00 (4H, m),1.88-1.97 (1H, m), 3.78 (3H, s), 4.03 (3H, s), 4.42 (2H, s), 6.82 (1H, dd, J = 9.7, 2.4 Hz), 7.15 (1H, d, J = 7.9 Hz), 7.19 (1H, d, J = 9.7 Hz), 7.67 (1H, t, J = 7.9 Hz), 7.94 (1H, d, J =1.8 Hz), 7.96 (1H, d, J = 7.9 Hz).

### <Example 6-11>

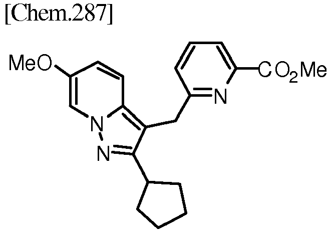

### Methyl 6-[(2-cyclopentyl-6-methoxypyrazolo[1,5-a]pyridin-3-yl)methyl]pyridine-2-carboxylate

After trifluoroacetic acid (0.50 mL) and triethylsilane (0.50 mL) were added to a dichloromethane (2.7 mL) solution of methyl 6-[(2-cyclopentyl-6-methoxypyrazolo[1,5-a]pyridin-3-yl)hydroxymethyl]pyridine-2-carboxy late (204 mg), the mixture was stirred at room temperature for 5 hours. A saturated sodium bicarbonate aqueous solution was added to the reaction solution to achieve pH = 8 to 9 and then the mixture was extracted with ethyl acetate. Subsequently, the extract was washed with saturated saline and then dried over anhydrous sodium sulfate. The solvent was evaporated and the residue thus obtained was purified by silica gel column chromatography (*n*-hexane:ethyl acetate =1:1) to obtain a title compound as a yellow oil (187 mg).
¹H-NMR (400 MHz, CDCl₃) δ 1.58-1.70 (2H, m), 1.77-1.90 (4H, m), 1.95-2.05 (2H, m), 3.15-3.26 (1H, m), 3.80 (3H, s), 4.04 (3H, s), 4.36 (2H, s), 6.82 (1H, dd, J = 9.7,1.8 Hz), 7.07 (1H, d, J = 7.9 Hz), 7.17 (1H, d, J = 9.7 Hz), 7.64 (1H, t, J = 7.9 Hz), 7.95 (1H, d, J = 7.9 Hz), 8.02(1H,d, J=1.8Hz).

### <Example 6-12>

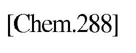

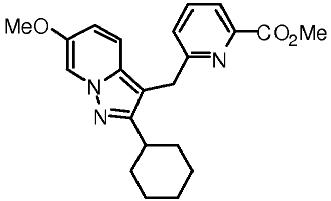

### Methyl 6-[(2-cyclohexyl-6-methoxypyrazolo[1,5-a]pyridin-3-yl)methyl]pyridine-2-carboxylate

After trifluoroacetic acid (0.52 mL) and triethylsilane (0.52 mL) were added to a dichloromethane (2.9 mL) solution of methyl 6-[(2-cyclohexyl-6-methoxypyrazolo[1,5-a]pyridin-3-yl)(hydroxy)methyl]pyridine-2-carbox ylate (227 mg), the mixture was stirred at room temperature for 5 hours. A saturated sodium bicarbonate aqueous solution was added to the reaction solution to achieve pH = 8 to 9 and then the mixture was extracted with ethyl acetate. Subsequently, the extract was washed with saturated saline and then dried over anhydrous sodium sulfate. The solvent was evaporated and the residue thus obtained was purified by silica gel column chromatography (*n*-hexane:ethyl acetate = 1:1) to obtain a title compound as a yellow crystal (215 mg).
¹H-NMR (400 MHz, CDCl₃) δ 1.25-1.40 (3H, m), 1.60-1.75 (3H, m), 1.77-1.87 (4H, m), 2.75-2.84 (1H, m), 3.79 (3H, s), 4.04 (3H, s), 4.35 (2H, s), 6.82 (1H, dd, J = 9.7, 1.8 Hz), 7.06 (1H, d, J = 7.9 Hz), 7.17 (1H, d, J = 9.7 Hz), 7.65 (1H, t, J = 7.9 Hz), 7.95 (1H, d, J = 7.9 Hz), 8.03(1H,d,J=1.8Hz).

### <Example 6-13>

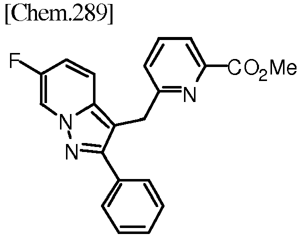

### Methyl 6-[(6-fluoro-2-phenylpyrazolo[1,5-a]pyridin-3-yl)methyl]pyridine-2-carboxylate

Triethylsilane (0.5 mL) and trifluoroacetic acid (0.4 mL) were added to a dichloromethane solution (2.4 mL) of methyl 6-[(6-fluoro-2-phenylpyrazolo[1,5-a]pyndin-3-yl)(hydroxy)methyl]pyndine-2-carboxylate (180 mg) at normal temperature under an argon atmosphere, and then the mixture was stirred at normal temperature for 4 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as a colorless powder (148 mg).
¹H-NMR (400 MHz, CDCl₃) δ 4.04 (3H, s), 4.55 (2H, s), 7.02 (1H, ddd, J = 9.7, 7.9, 1.8 Hz), 7.10 (1H, d, J = 7.9 Hz), 7.38-7.46 (4H, m), 7.65 (1H, t, J = 7.9 Hz), 7.68-7.71 (2H, m), 7.96 (1H, d, J = 7.9 Hz), 8.43 (1H, dd, J = 4.2,2.4 Hz).

### <Example 6-14>

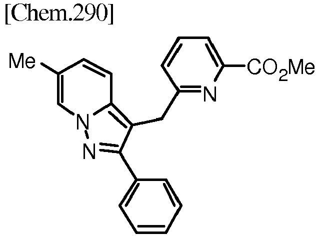

### Methyl 6-[(6-methyl-2-phenylpyrazolo[1,5-a]pyridin-3-yl)methyl]pyridine-2-carboxylate

Triethylsilane (0.5 mL) and trifluoroacetic acid (0.4 mL) were added to a dichloromethane solution (2.5 mL) of methyl 6-[(6-methyl-2-phenylpyrazolo[1,5-a]pyridin-3-yl)(hydroxy)methyl]pyridine-2-carboxylate (185 mg) at normal temperature under an argon atmosphere, and then the mixture was stirred at normal temperature for 16 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as colorless amorphous (126 mg).
¹H-NMR (400 MHz, CDCl₃) δ 2.34 (3H, d, J =1.2 Hz), 4.04 (3H, s), 4.55 (2H, s), 6.92 (1H, dd, J = 9.1, 1.8 Hz), 7.10 (1H, d, J = 7.9 Hz), 7.27 (1H, d, J = 9.1 Hz), 7.33-7.38 (1H, m), 7.38-7.44 (2H, m), 7.63 (1H, t, J = 7.9 Hz), 7.68-7.72 (2H, m), 7.95 (1H, d, J = 7.9 Hz), 8.29 (1H,d,J=1.2Hz).

### <Example 6-15>

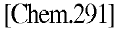

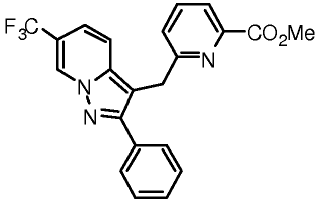

### Methyl 6-[[2-phenyl-6-(trifluoromethyl)pyrazolo[1,5-a]pyridin-3-yl]methyl]pyridine-2-carboxylate

Triethylsilane (0.7 mL) and trifluoroacetic acid (0.7 mL) were added to a dichloromethane solution (3.7 mL) of methyl 6-[[2-phenyl-6-(trifluoromethyl)pyrazolo[1,5-a]pyridin-3-yl] (hydroxy)methyl]pyridine-2-car boxylate (315 mg) at normal temperature under an argon atmosphere, and then the mixture was stirred at normal temperature for 18 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as colorless amorphous (292 mg).
¹H-NMR (400 MHz, CDCl₃) δ 4.04 (3H, s), 4.57 (2H, s), 7.10 (1H, d, J = 7.9 Hz), 7.20 (1H, dd, J = 9.1, 1.2 Hz), 7.41-7.48 (3H, m), 7.55 (1H, d, J = 9.1 Hz), 7.67 (1H, t, J = 7.9 Hz), 7.70-7.74 (2H, m), 7.97 (1H, d, J = 7.9 Hz), 8.82 (1H, d, J =1.2 Hz).

### <Example 6-16>

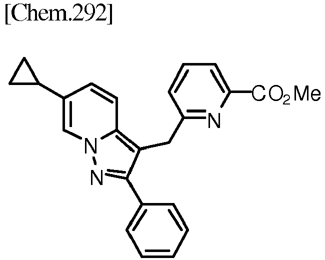

### Methyl 6-[(6-cyclopropyl-2-phenylpyrazolo[1,5-a]pyridin-3-yl)methyl]pyridine-2-carboxylate

Triethylsilane (0.3 mL) and trifluoroacetic acid (0.3 mL) were added to a dichloromethane solution (1.8 mL) of methyl 6-[(6-cyclopropyl-2-phenylpyrazolo[1,5-a]pyridin-3-yl)(hydroxy)methyl]pyridine-2-carboxy late (140 mg) at normal temperature under an argon atmosphere, and then the mixture was stirred at normal temperature for 8 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 4:1) to obtain a title compound as yellow liquid (79 mg).
¹H-NMR (400 MHz, CDCl₃) δ 0.67-0.72 (2H, m), 0.94-1.00 (2H, m), 1.87-1.95 (1H, m), 4.04 (3H, s), 4.55 (2H, s), 6.85 (1H, dd, J = 9.1, 1.2 Hz), 7.10 (1H, d, J = 7.9 Hz), 7.25 (1H, d, J = 7.9 Hz), 7.33-7.38 (1H, m), 7.38-7.44 (2H, m), 7.63 (1H, t, J = 7.9 Hz), 7.68-7.72 (2H, m), 7.95 (1H, d, J = 7.9 Hz), 8.28 (1H, s).

### <Example 6-17>

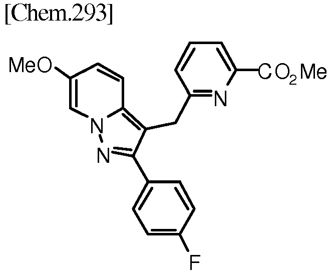

### Methyl 6-[[2-(4-ffuorophenyl)-6-methoxypyrazolo[1,5-a]pyridin-3-yl]methyl]pyridine-2-carboxylate

A dichloromethane (1.3 mL) solution of methyl 6-[[2-(4-fluorophenyl)-6-methoxypyrazolo[1,5-a]pyridin-3-yl] (hydroxy)methyl]pyridine-2-c arboxylate (101 mg) was ice-cooled and triethylsilane (0.238 mL) and trifluoroacetic acid (0.221 mL) were added. The mixture was heated up to room temperature, stirred for 18 hours, and then ice-cooled again. Next, a saturated sodium bicarbonate aqueous solution was slowly added to achieve pH = 9. The organic solvent was evaporated under vacuum and then the residue was purified by silica gel column chromatography (*n*-hexane:ethyl acetate = 5:1 to 1:1) to obtain a title compound as a colorless oil (89.3 mg).
¹H-NMR (400 MHz, CDCl₃) δ3.85 (3H, s), 4.04 (3H, s), 4.50 (2H, s), 6.90 (1H, dd, J = 9.7, 1.8 Hz), 7.06-7.13 (3H, m), 7.29 (1H, d, J = 9.7 Hz), 7.62-7.71 (3H, m), 7.96 (1H, d, J = 7.3 Hz),8.08(1H,d,J=1.8Hz).

### <Example 6-18>

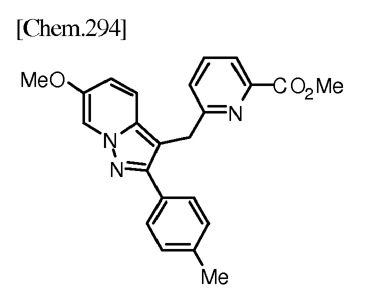

### Methyl 6-[[6-methoxy-2-(4-methylphenyl)pyrazolo[1,5-a]pyridin-3-yl]methyl]pyridine-2-carboxylat e

A dichloromethane (1.7 mL) solution of methyl 6-[hydroxy[6-methoxy-2-(4-methylphenyl)pyrazolo[1,5-a]pyridin-3-yl]methyl]pyridine-2-ca rboxylate (136 mg) was ice-cooled and triethylsilane (0.323 mL) and trifluoroacetic acid (0.300 mL) were added. The mixture was heated up to room temperature, stirred for 18 hours, and then ice-cooled again. Next, a saturated sodium bicarbonate aqueous solution was slowly added to achieve pH = 9. The organic solvent was evaporated under vacuum and then the residue was purified by silica gel column chromatography (*n*-hexane:ethyl acetate = 5:1 to 1:1 ) to obtain a title compound as a pale yellow oil (116 mg).
¹H-NMR (400 MHz, CDCl₃) δ 3.84 (3H, s), 4.04 (3H, s), 4.53 (2H, s), 6.87 (1H, dd, J = 9.7, 1.8 Hz), 7.10 (1H, d, J = 7.9 Hz), 7.23 (3H, t, J = 9.7 Hz), 7.59 (2H, d, J = 7.9 Hz), 7.63 (1H, t, J=7.9 Hz), 7.95 (1H, d, J=7.9 Hz), 8.10 (1H, d, J=1.8 Hz).

### <Example 6-19>

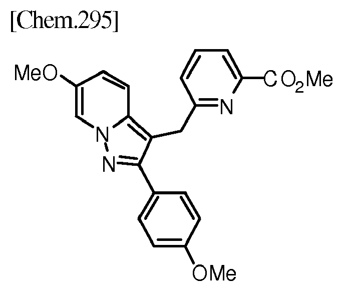

### Methyl 6-[[6-methoxy-2-(4-methoxyphenyl)pyrazolo[1,5-a]pyridin-3-yl]methyl]pyridine-2-carboxyl ate

A dichloromethane (1.2 mL) solution of methyl 6-[hydroxy[6-methoxy-2-(4-methoxyphenyl)pyrazolo[1,5-a]pyridin-3-yl]methyl]pyridine-2-carboxylate (102 mg) was ice-cooled and triethylsilane (0.233 mL) and trifluoroacetic acid (0.216 mL) were added. The mixture was heated up to room temperature, stirred for 18 hours, and then ice-cooled again. Next, a saturated sodium bicarbonate aqueous solution was slowly added to achieve pH = 9. The organic solvent was evaporated under vacuum and then the residue was purified by silica gel column chromatography (*n*-hexane:ethyl acetate = 5:1 to 1:1) to obtain a title compound as a colorless oil (78.5 mg).
¹H-NMR (400 MHz, CDCl₃) δ 3.83 (3H, s), 3.84 (3H, s), 4.04 (3H, s), 4.52 (2H, s), 6.87 (1H, dd, J = 9.7, 1.8 Hz), 6.94 (2H, dt, J = 8.5, 1.8 Hz), 7.11 (1H, d, J = 7.9 Hz), 7.24 (1H, d, J = 9.7 Hz), 7.60-7.66 (3H, m), 7.96 (1H, d, J = 6.7 Hz), 8.09 (1H, d, J =1.8 Hz).

### <Example 6-20>

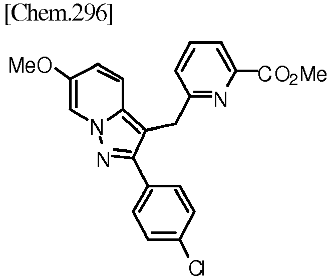

### Methyl 6-[[2-(4-chlorophenyl)-6-methoxypyrazolo[1,5-a]pyridin-3-yl]methyl]pyridine-2-carboxylat e

A dichloromethane (1.3 mL) solution of methyl 6-[[2-(4-chlorophenyl)-6-methoxypyrazolo[1,5-a]pyridin-3-yl](hydroxy)methyl]pyridine-2-c arboxylate (108 mg) was ice-cooled and triethylsilane (0.244 mL) and trifluoroacetic acid (0.227 mL) were added. The mixture was heated up to room temperature, stirred for 18 hours, and then ice-cooled again. Next, a saturated sodium bicarbonate aqueous solution was slowly added to achieve pH = 9. The organic solvent was evaporated under vacuum and then the residue was purified by silica gel column chromatography (*n*-hexane:ethyl acetate = 5:1 to 1:1) to obtain a title compound as a colorless oil (87.2 mg).
¹H-NMR (400 MHz, CDCl₃) δ3.85 (3H, s), 4.04 (3H, s), 4.51 (2H, s), 6.90 (1H, dd, J = 9.7, 1.8 Hz), 7.09 (1H, d, J = 7.9 Hz), 7.28 (1H, d, J = 9.7 Hz), 7.38 (2H, dt, J = 9.1, 2.4 Hz), 7.63-7.68 (3H, m), 7.96 (1H, d, J = 7.9 Hz), 8.08 (1H, d, J =1.8 Hz).

### <Example 6-21>

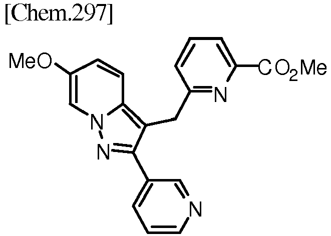

### Methyl 6-[[6-methoxy-2-(pyridin-3-yl)pyrazolo[1,5-a]pyridin-3-yl]methyl]pyridine-2-carboxylate

A dichloromethane (0.2 mL) solution of methyl 6-[hydroxy[6-methoxy-2-(pyridin-3-yl)pyrazolo[1,5-a]pyridin-3-yl]methyl]pyridine-2-carbo xylate (18.6 mg) was ice-cooled and triethylsilane (0.0457 mL) and trifluoroacetic acid (0.00420 mL) were added. The mixture was heated up to room temperature, stirred for 18 hours, and then ice-cooled again. Next, a saturated sodium bicarbonate aqueous solution was slowly added to achieve pH = 9. The organic solvent was evaporated under vacuum and then the residue was purified by silica gel column chromatography (*n*-hexane:ethyl acetate = 1:0 to 1:1) to obtain a title compound as a colorless oil (14.7 mg).
¹H-NMR (400 MHz, CDCl₃) δ 3.86 (3H, s), 4.03 (3H, s), 4.52 (2H, s), 6.93 (1H, dd, J = 9.7, 2.4 Hz), 7.13 (1H, d, J =7.9 Hz), 7.31-7.38 (2H, m), 7.66 (1H, t, J = 7.9 Hz), 7.97 (1H, d, J = 7.9 Hz), 8.05 (1H, td, J =7.9,1.8 Hz), 8.10 (1H, d, J =1.8 Hz), 8.60 (1H, dd, J = 4.8,1.8 Hz), 8.96(1H,d,J=2.4Hz).

### <Example 6-22>

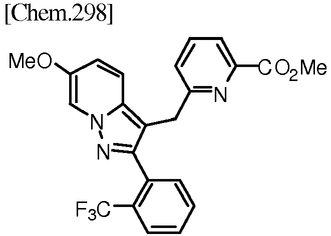

### Methyl 6-[[6-methoxy-2-[2-(triffuoromethyl)phenyl]pyrazolo[1,5-a]pyridin-3-yl]methyl]pyridine-2-carboxylate

Triethylsilane (0.2 mL) and trifluoroacetic acid (0.1 mL) were added to a dichloromethane solution (0.8 mL) of methyl 6-[hydroxy[6-methoxy-2-[2-(triffuoromethyl)phenyl]pyrazolo[1,5-a]pyridin-3-yl]methyl]pyr idine-2-carboxylate (76 mg) at room temperature under an argon atmosphere, and then the mixture was stirred at room temperature for 17 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as colorless liquid (54 mg).
¹H-NMR (400 MHz, CDCl₃) δ 3.83 (3H, s), 4.00 (3H, s), 4.22 (2H, s), 6.89 (1H, dd, J = 9.7, 1.8 Hz), 7.04 (1H, d, J = 7.9 Hz), 7.29 (1H, d, J = 9.7 Hz), 7.41 (1H, dd, J = 7.3, 1.8 Hz), 7.49-7.59 (2H, m), 7.62 (1H, t, J = 7.9 Hz), 7.78 (1H, dd, J = 7.3, 1.8 Hz), 7.91 (1H, d, J = 7.9 Hz),8.05(1H,d,J=1.8Hz).

### <Example 6-23>

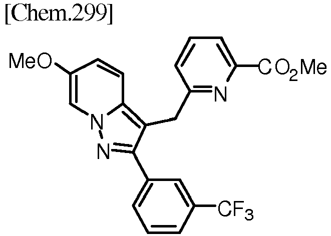

### Methyl 6-[[6-methoxy-2-[3-(trifluoromethyl)phenyl]pyrazolo[1,5-a]pyridin-3-yl]methyl]pyridine-2-carboxylate

Triethylsilane (0.05 mL) and trifluoroacetic acid (0.04 mL) were added to a dichloromethane solution (0.2 mL) of methyl 6-[hydroxy[6-methoxy-2-[3-(tlifluoromethyl)phenyl]pyrazolo[1,5-a]pyridin-3-yl]methyl]pyr idine-2-carboxylate (21 mg) at room temperature under an argon atmosphere, and then the mixture was stirred at room temperature for 15 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as pale yellow amorphous (15 mg).
¹H-NMR (400 MHz, CDCl₃) δ3.85 (3H, s), 4.03 (3H, s), 4.52 (2H, s), 6.92 (1H, dd, J = 9.7, 1.8 Hz), 7.13 (1H, dd, J = 7.9, 1.2 Hz), 7.33 (1H, d, J = 9.7 Hz), 7.52 (1H, t, J = 7.9 Hz), 7.60 (1H, d, J = 7.9 Hz), 7.66 (1H, t, J = 7.9 Hz), 7.88 (1H, d, J = 7.9 Hz), 7.97 (1H, dd, J = 7.9, 1.2 Hz), 8.02 (1H, s), 8.10 (1H, d, J =1.8 Hz).

### <Example 6-24>

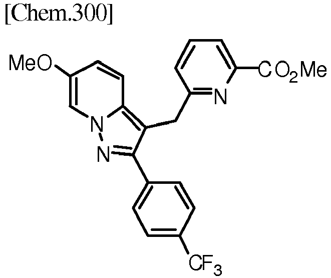

### Methyl 6-[[6-methoxy-2-[4-(trifluoromethyl)phenyl]pyrazolo[1,5-a]pyridin-3-yl]methyl]pyridine-2-carboxylate

Triethylsilane (0.4 mL) and trifluoroacetic acid (0.4 mL) were added to a dichloromethane solution (2.0 mL) of methyl 6-[hydroxy[6-methoxy-2-[4-(trifluoromethyl)phenyl]pyrazolo[1,5-a]pyridin-3-yl]methyl]pyr idine-2-carboxylate (182 mg) at room temperature under an argon atmosphere, and then the mixture was stirred at room temperature for 17 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as a pale yellow powder (138 mg).
¹H-NMR (400 MHz, CDCl₃) δ 3.86 (3H, s), 4.04 (3H, s), 4.53 (2H, s), 6.92 (1H, dd, J = 9.7, 1.8 Hz), 7.11 (1H, d, J = 7.9 Hz), 7.32 (1H, d, J = 9.7 Hz), 7.63-7.68 (3H, m), 7.86 (2H, d, J = 7.9Hz),7.97(1H, d,J=7.9Hz),8.10(1H, d, J=1.8Hz).

### <Example 6-25>

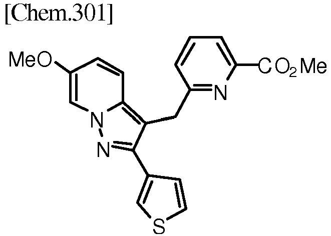

### Methyl 6-[[6-methoxy-2-(thiophen-3-yl)pyrazolo[1,5-a]pyridin-3-yl]methyl]pyridine-2-carboxylate

Triethylsilane (0.3 mL) and trifluoroacetic acid (0.3 mL) were added to a dichloromethane solution (1.6 mL) of methyl 6-[hydroxy[6-methoxy-2-(thiophen-3-yl)pyrazolo[1,5-a]pyridin-3-yl]methyl]pyridine-2-carb oxylate (130 mg) at room temperature under an argon atmosphere, and then the mixture was stirred at room temperature for 15 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as yellow liquid (101 mg).
¹H-NMR (400 MHz, CDCl₃) δ 3.84 (3H, s), 4.05 (3H, s), 4.55 (2H, s), 6.90 (1H, dd, J = 9.7, 1.8 Hz), 7.12 (1H, d, J = 7.9 Hz), 7.28 (1H, d, J = 9.7 Hz), 7.37 (1H, dd, J = 4.8, 3.0 Hz), 7.53 (1H, dd, J = 3.0, 1.2 Hz), 7.61-7.66 (2H, m), 7.96 (1H, d, J = 7.9 Hz), 8.08 (1H, d, J = 1.8 Hz).

### <Example 6-26>

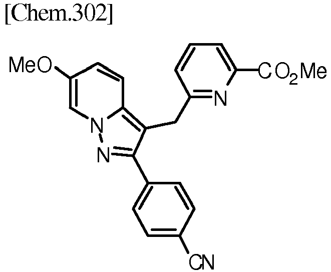

### Methyl 6-[[2-(4-cyanophenyl)-6-methoxypyrazolo[1,5-a]pyridin-3-yl]methyl]pyridine-2-carboxylate

After trifluoroacetic acid (90 µL) and triethylsilane (90 µL) were added to a dichloromethane (1 mL) solution of methyl 6-[[2-(4-cyanophenyl)-6-methoxypyrazolo[1,5-a]pyridin-3-yl](hydroxy)methyl]pyridine-2-c arboxylate (42.0 mg), the mixture was heated and stirred at room temperature for 5 hours. A saturated sodium bicarbonate was added to the reaction solution to achieve pH = 8 to 9 and then the mixture was extracted with ethyl acetate. Subsequently, the extract was washed with saturated saline and then dried over anhydrous sodium sulfate. The solvent was evaporated and the residue thus obtained was purified by silica gel column chromatography (n-hexane:ethyl acetate =1:1) to obtain a title compound as a colorless crystal (39.0 mg).
¹H-NMR (400 MHz, CDCl₃) δ 3.86 (3H, s), 4.04 (3H, s), 4.53 (2H, s), 6.94 (1H, dd, J = 9.7 and 2.4 Hz), 7.10 (1H, d, J = 7.9 Hz), 7.33 (1H, d, J = 9.7 Hz), 7.67 (1H, t, J = 7.9 Hz), 7.68 (2H, d, J = 8.5 Hz), 7.89 (2H, d, J = 8.5 Hz), 7.97 (1H, d, J = 6.6 Hz), 8.08 (1H, d, J = 1.8 Hz).

### <Example 6-27>

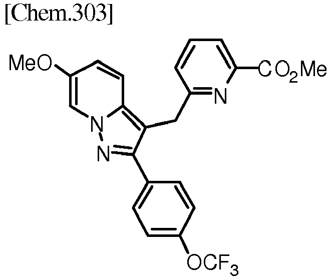

### Methyl 6-[[6-methoxy-2-[4-(trifluoromethoxy)phenyl]pyrazolo[1,5-a]pyridin-3-yl]methyl]pyridine-2-carboxylate

After trifluoroacetic acid (0.60 mL) and triethylsilane (0.60 mL) was added to a dichloromethane (3.4 mL) solution of methyl 6-[hydroxy[6-methoxy-2-[4-(trifluoromethoxy)phenyl]pyrazolo[1,5-a]pyridin-3-yl]methyl]p yridine-2-carboxylate (320 mg), the mixture was heated and stirred at room temperature for 5 hours. A saturated sodium bicarbonate was added to the reaction solution to achieve pH = 8 to 9 and then the mixture was extracted with ethyl acetate. Subsequently, the extract was washed with saturated saline and then dried over anhydrous sodium sulfate. The solvent was evaporated and the residue thus obtained was purified by silica gel column chromatography (n-hexane:ethyl acetate = 1:1) to obtain a title compound as a colorless crystal (229 mg).
¹H-NMR (400 MHz, CDCl₃) δ 3.85 (3H, s), 4.04 (3H, s), 4.51 (2H, s), 6.91 (1H, dd, J = 9.7 and 2.4 Hz), 7.11 (1H, d, J = 7.9 Hz), 7.22-7.32 (3H, m), 7.66 (1H, t, J = 7.9 Hz), 7.75 (2H, d, J = 8.5 Hz), 7.97 (1H, d, J = 7.9 Hz), 8.08 (1H, d, J = 2.4 Hz).

### <Example 6-28>

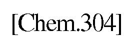

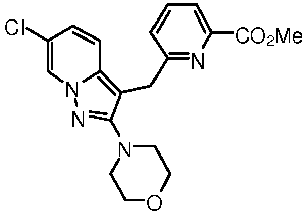

### Methyl 6-[[6-chloro-2-(morpholin-4-yl)pyrazolo[1,5-a]pyridin-3-yl]methyl]pyridine-2-carboxylate

Triethylsilane (0.1 mL, 0.626 mmol) and trifluoroacetic acid (0.1 mL, 1.35 mmol) were added to a dichloromethane solution (0.4 mL) of methyl 6-[[6-chloro-2-(morpholin-4-yl)pyrazolo[1,5-a]pyridin-3-yl] (hydroxy)methyl]pyridine-2-car boxylate (31.0 mg, 0.077 mmol) at room temperature under an argon atmosphere, and then the mixture was stirred at room temperature for 2 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 1:1) to obtain a title compound as a yellow foam (14.6 mg, 49%).
¹H-NMR (400 MHz, CDCl₃) δ 3.23 (4H, t, J = 4.2 Hz), 3.72 (4H, t, J = 4.2 Hz), 4.03 (3H, s), 4.32 (2H, s), 6.97 (1H, d, J = 9.1 Hz), 7.11 (1H, d, J = 9.1 Hz), 7.16 (1H, d, J = 7.9 Hz), 7.70 (1H, t, J = 7.9 Hz), 7.98 (1H, d, J = 7.9 Hz), 8.31 (1H, s).

### <Example 6-29>

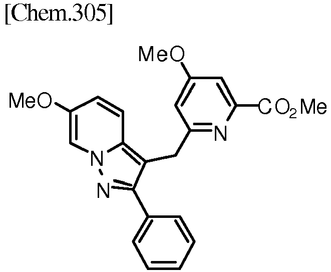

### Methyl 4-methoxy-6-[(6-methoxy-2-phenylpyrazolo[1,5-a]pyridin-3-yl)methyl]pyridine-2-carboxyl ate

Triethylsilane (0.65 mL) and trifluoroacetic acid (0.60 mL) were added to a dichloromethane solution (3.4 mL) of methyl 6-[hydroxy(6-methoxy-2-phenylpyrazolo[1,5-a]pyridin-3-yl)methyl]-4-methoxypyridine-2-c arboxylate (285 mg), and then the mixture was stirred at room temperature for 24 hours. A saturated sodium bicarbonate aqueous solution was slowly added to the reaction solution to achieve pH = 9 and then the mixture was purified by silica gel column chromatography (n-hexane:ethyl acetate =10:1 1 to 1:1) to obtain a title compound as a colorless oil (200 mg).
¹H-NMR (400 MHz, CDCl₃) δ 3.71 (3H, s), 3.85 (3H, s), 4.03 (3H, s), 4.49 (2H, s), 6.57 (1H, d, J = 2.4 Hz), 6.89 (1H, dd, J = 9.7, 1.8 Hz), 7.26 (1H, s), 7.27 (2H, d, J = 9.7 Hz), 7.33-7.43 (3H, m), 7.51 (1H, d, J = 2.4 Hz), 7.69 (2H, td, J = 6.7, 1.8 Hz), 8.10 (1H, d, J =1.8 Hz).

### <Example 6-30>

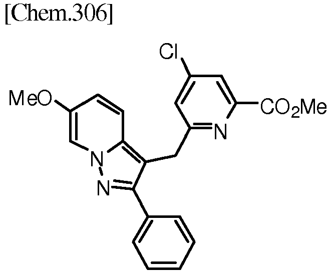

### Methyl 4-chloro-6-[(6-methoxy-2-phenylpyrazolo[1,5-a]pyridin-3-yl)methyl]pyridine-2-carboxylate

Triethylsilane (1.45 mL) and trifluoroacetic acid (1.35 mL) were added to a dichloromethane (7.6 mL) solution of methyl 4-chloro-6-[hydroxy[6-methoxy-2-phenyl-7-(trimethylsilyl)pyrazolo[1,5-a]pyridin-3-yl]met hyl]pyridine-2-carboxylate (752 mg) under an argon atmosphere, and then the mixture was stirred at room temperature for 18 hours. A saturated sodium bicarbonate aqueous solution was added to the reaction solution, and then the mixture was extracted with ethyl acetate. The organic layer was washed with saturated saline and then dried over anhydrous sodium sulfate. The solvent was evaporated under vacuum and then the residue was purified by silica gel column chromatography (n-hexane:ethyl acetate = 10:1 to 2:1) to obtain a title compound as a pale yellow oil (346 mg).
¹H-NMR (400 MHz, CDCl₃) δ 3.86 (3H, s), 4.04 (3H, s), 4.52 (2H, s), 6.92 (1H, dd, J = 9.7, 1.8 Hz), 7.07 (1H, d, J =1.8 Hz), 7.26 (1H, d, J = 9.7 Hz), 7.34-7.45 (3H, m), 7.65-7.69 (2H, m), 7.95 (1H, d, J =1.8 Hz), 8.12 (1H, d, J =1.8 Hz).

### <Example 6-31>

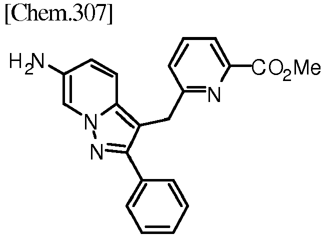

### Methyl 6-[(6-amino-2-phenylpyrazolo[1,5-a]pyridin-3-yl)methyl]pyridine-2-carboxylate

Triethylsilane (0.4 mL) and trifluoroacetic acid (0.4 mL) were added to a dichloromethane solution (2.3 mL) of methyl 6-[[6-[(*tert*-butoxycarbonyl)amino]-2-phenylpyrazolo[1,5-a]pyridin-3-yl](hydroxy)methyl]p yridine-2-carboxylate (220 mg) at room temperature under an argon atmosphere, and then the mixture was stirred at room temperature for 4 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 2:1) to obtain a title compound as a pale yellow amorphous (66 mg).
¹H-NMR (400 MHz, CDCl₃) δ 4.05 (3H, s), 4.73 (2H, d, J = 5.4 Hz), 5.13 (1H, brs), 6.04 (1H, d, J = 7.3 Hz), 6.60 (1H, t, J = 7.3 Hz), 6.83 (1H, s), 7.36 (1H, tt, J = 7.3, 1.2 Hz), 7.46 (2H, t, J = 7.3 Hz), 7.59 (1H, d, J = 7.3 Hz), 7.84 (1H, t, J = 7.9 Hz), 7.95-7.99 (2H, m), 8.00 (1H, d, J = 7.3 Hz), 8.07 (1H, d, J = 7.9 Hz).

### <Example 6-32>

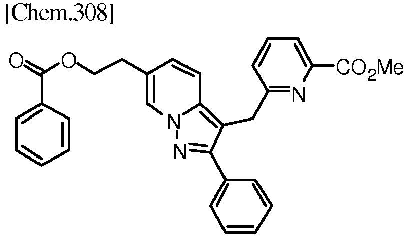

### Methyl 6-[[6-[2-(benzoyloxy)ethyl]-2-phenylpyrazolo[1,5-a]pyridin-3-yl]methyl]pyridine-2-carboxy late

Triethylsilane (2.7 mL) and trifluoroacetic acid (2.5 mL) were added to a dichloromethane solution (14 mL) of methyl 6-[[6-[2-(benzoyloxy)ethyl]-2-phenylpyrazolo[1,5-a]pyridin-3-yl](hydroxy)methyl]pyridine-2-carboxylate (1.4 g) at room temperature under an argon atmosphere, and then the mixture was stirred at room temperature for 7 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 2:1) to obtain a title compound as yellow amorphous (1.25 g).
¹H-NMR (400 MHz, CDCl₃) δ 3.10 (2H, t, J = 6.7 Hz), 4.03 (3H, s), 4.55 (2H, s), 4.57 (2H, t, J = 6.7 Hz), 7.06 (1H, dd, J = 9.1,1.2 Hz), 7.11 (1H, d, J = 7.9 Hz), 7.36 (2H, d, J = 9.1 Hz), 7.38-7.47 (4H, m), 7.56 (1H, tt, J = 7.9, 1.2 Hz), 7.64 (1H, t, J = 7.9 Hz), 7.67-7.71 (2H, m), 7.96 (1H, d, J = 7.9 Hz), 8.00-8.04 (2H, m), 8.47 (1H, s).

### <Example 7-1>

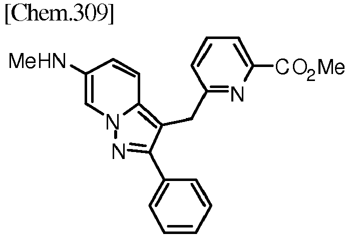

### Methyl 6-[[6-(methylamino)-2-phenylpyrazolo[1,5-a]pyridin-3-yl]methyl]pyridine-2-carboxylate

Methyl iodide (0.07 mL) and cesium carbonate (42 mg) were added to an *N*,*N*-dimethylformamide solution (0.6 mL) of methyl 6-[(6-amino-2-phenylpyrazolo[1,5-a]pyridin-3-yl)methyl]pyridine-2-carboxylate (40 mg) at 0°C under an argon atmosphere, and then the mixture was stirred at room temperature for 23 hours. Water was added to the reaction solution and the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 2:1) to obtain a title compound as yellow liquid (19 mg).
¹H-NMR (400 MHz, CDCl₃) δ 2.99 (3H, s), 4.04 (3H, s), 4.78 (2H, s), 6.42 (1H, d, J = 7.3 Hz), 6.66 (1H, t, J = 7.3 Hz), 6.84 (1H, d, J = 1.2 Hz), 7.31-7.37 (1H, m), 7.42 (2H, t, J = 7.3 Hz), 7.74 (1H, dd, J = 7.9, 1.2 Hz), 7.84-7.90 (2H, m), 7.91 (1H, d, J = 1.2 Hz), 8.09 (1H, d, J = 7.9 Hz), 8.15 (1H, d, J = 7.3 Hz).

### <Example 8-1>

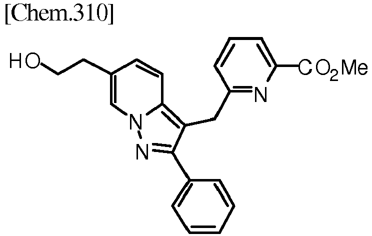

### Methyl 6-[[6-(2-hydroxyethyl)-2-phenylpyrazolo[1,5-a]pyridin-3-yl]methyl]pyridine-2-carboxylate

Sodium methoxide (687 mg) was added to a methanol-tetrahydrofuran mixed solution (25.4 mL, 1:1) of methyl 6-[[6-[2-(benzoyloxy)ethyl]-2-phenylpyrazolo[1,5-a]pyridin-3-yl]methyl]pyridine-2-carboxy late (1.25 g) at room temperature under an argon atmosphere, and then the mixture was stirred at room temperature for 2 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (ethyl acetate) to obtain a title compound as a colorless amorphous (753 mg).
¹H-NMR (400 MHz, CDCl₃) δ 1.50 (1H, t, J = 6.1 Hz), 2.87 (2H, t, J = 6.1 Hz), 3.91 (2H, q, J = 6.1 Hz), 4.04 (3H, s), 4.56 (2H, s), 6.99 (1H, dd, J = 9.1, 1.8 Hz), 7.11 (1H, d, J = 7.9 Hz), 7.33 (1H, d, J = 9.1 Hz), 7.36-7.39 (1H, m), 7.39-7.45 (2H, m), 7.64 (1H, t, J = 7.9 Hz), 7.68-7.73 (2H, m), 7.96 (1H, d, J = 7.9 Hz), 8.38 (1H, s).

### <Example 9-1>

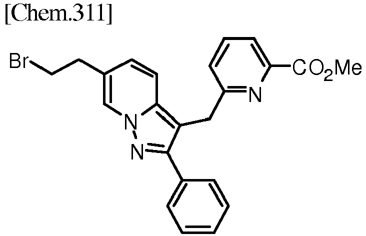

### Methyl 6-[[6-(2-bromoethyl)-2-phenylpyrazolo[1,5-a]pyridin-3-yl]methyl]pyridine-2-carboxylate

Triphenylphosphine (81 mg) and carbon tetrabromide (119 mg) were added to a dichloromethane solution (1.3 mL) of methyl 6-[[6-(2-hydroxyethyl)-2-phenylpyrazolo[1,5-a]pyridin-3-yl]methyl]pyridine-2-carboxylate (100 mg) at room temperature under an argon atmosphere, and then the mixture was stirred at room temperature for 1 hour. The reaction solution was evaporated and then the residue was purified by silica gel column chromatography (n-hexane:ethyl acetate = 3:1) to obtain a title compound as yellow liquid (108 mg).
¹H-NMR (400 MHz, CDCl₃) δ 3.17 (2H, t, J = 7.3 Hz), 3.60 (2H, t, J = 7.3 Hz), 4.04 (3H, s), 4.56 (2H, s), 6.95 (1H, dd, J = 9.1, 1.2 Hz), 7.11 (1H, d, J = 7.9 Hz), 7.35 (1H, d, J = 9.1 Hz), 7.37-7.40 (1H, m), 7.40-7.45 (2H, m), 7.65 (1H, t, J = 7.9 Hz), 7.69-7.73 (2H, m), 7.96 (1H, d, J = 7.9 Hz), 8.38 (1H, s).

### <Example 10-1>

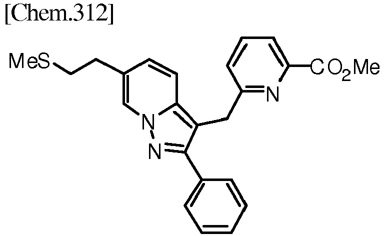

### Methyl 6-[[6-[2-(methylsulfanyl)ethyl]-2-phenylpyrazolo[1,5-a]pyridin-3-yl]methyl]pyridine-2-carb oxylate

Sodium thiomethoxide (20 mg) was added to a methanol solution (1.2 mL) of methyl 6-[[6-(2-bromoethyl)-2-phenylpyrazolo[1,5-a]pyridin-3-yl]methyl]pyridine-2-carboxylate (107 mg) at room temperature under an argon atmosphere, and then the mixture was stirred at 40°C for 3 hours. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (*n*-hexane:ethyl acetate = 2:1) to obtain a title compound as colorless liquid (62 mg).
¹H-NMR (400 MHz, CDCl₃) δ 2.15 (3H, s), 2.79 (2H, t, J = 7.3 Hz), 2.90 (2H, t, J = 7.3 Hz), 4.04 (3H, s), 4.56 (2H, s), 6.96 (1H, dd, J = 9.1, 1.2 Hz), 7.11 (1H, d, J = 7.9 Hz), 7.32 (1H, d, J = 9.1 Hz), 7.35-7.38 (1H, m), 7.39-7.44 (2H, m), 7.63 (1H, t, J = 7.9 Hz), 7.69-7.73 (2H, m), 7.96 (1H, d, J = 7.9 Hz), 8.36 (1H, s).

### <Example 11-1>

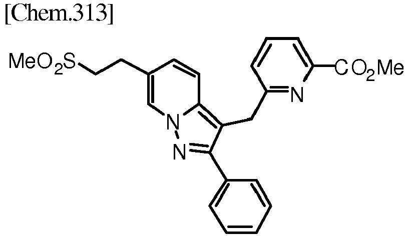

### Methyl 6-[[6-[2-(methylsulfonyl)ethyl]-2-phenylpyrazolo[1,5-a]pyridin-3-yl]methyl]pyridine-2-carb oxylate

3-Chloroperbenzoic acid (50 mg) was added to a dichloromethane solution (1.4 mL) of methyl 6-[[6-[2-(methylsulfanyl)ethyl]-2-phenylpyrazolo[1,5-a]pyridin-3-yl]methyl]pyridine-2-carb oxylate (60 mg) at room temperature under an argon atmosphere, and then the mixture was stirred at room temperature for 23 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (ethyl acetate) to obtain a title compound as yellow liquid (42 mg).
¹H-NMR (400 MHz, CDCl₃) δ 2.94 (3H, s), 3.15-3.22 (2H, m), 3.29-3.35 (2H, m), 4.04 (3H, s), 4.54 (2H, s), 6.95 (1H, d, J = 9.1 Hz), 7.09 (1H, d, J = 7.9 Hz), 7.36-7.45 (4H, m), 7.64 (1H, t, J = 7.9 Hz), 7.70 (2H, d, J = 7.9 Hz), 7.96 (1H, d, J = 7.9 Hz), 8.39 (1H, s).

### <Example 12-1>

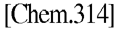

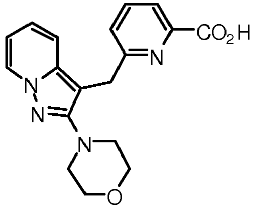

### 6-[[2-(Morpholin-4-yl)pyrazolo[1,5-a]pyridin-3-yl]methyl]pyridine-2-carboxylic acid

A 2 mol/L potassium hydroxide aqueous solution (0.7 mL) was added to a methanol solution (1.4 mL) of methyl 6-[[2-(morpholin-4-yl)pyrazolo[1,5-a]pyridin-3-yl]methyl]pyridine-2-carboxylate (92.0 mg) under an argon atmosphere under ice-cooling, and then the mixture was stirred at room temperature for 2 hours. A 1 mol/L hydrochloric acid was added to the reaction solution and the precipitated crystal was filtered off to obtain a title compound as a colorless crystal (84.7 mg).
¹H-NMR (400 MHz, DMSO-d₆) δ 3.16 (4H, t, J = 4.8 Hz), 3.60 (4H, t, J = 4.8 Hz), 4.22 (2H, s), 6.66 (1H, t, J = 7.3 Hz), 7.08 (1H, t, J = 7.3 Hz), 7.29 (1H, d, J = 7.3 Hz), 7.42 (1H, d, J = 7.3 Hz), 7.78-7.89 (2H, m), 8.43 (1H, d J = 7.3 Hz), 13.10 (1H, brs).

The following Examples 12-2 to 12-56 were obtained by using corresponding esters in the same manner as Example 12-1.

### <Example 12-2>

### 6-[[6-Methyl-2-(morpholin-4-yl)pyrazolo[1,5-a]pyridin-3-yl]methyl]pyridine-2-carboxylic acid

¹H-NMR (400 MHz, DMSO-d₆) δ 2.21 (3H, s), 3.14 (4H, t, J = 4.8 Hz), 3.60 (4H, t, J = 4.8 Hz), 4.19 (2H, s), 6.94 (1H, d, J = 9.2 Hz), 7.28 (1H, d, J = 7.3 Hz), 7.34 (1H, d, J = 9.2 Hz), 7.78-7.89 (2H, m), 8.27 (1H, s), 13.05 (1H, brs).

### <Example 12-3>

### 6-[[6-Bromo-2-(morpholin-4-yl)pyrazolo[1,5-a]pyridin-3-yl]methyl]pyridine-2-carboxylic acid

¹H-NMR (400 MHz, DMSO-d₆) δ 3.17 (4H, t, J = 4.8 Hz), 3.59 (4H, t, J = 4.8 Hz), 4.22 (2H, s), 7.20 (1H, dd, J = 9.2, 1.8 Hz), 7.29 (1H, d, J = 7.3 Hz), 7.45 (1H, d, J = 9.2 Hz), 7.78-7.88 (2H, m), 8.81 (1H, s), 13.09 (1H, brs).

### <Example 12-4>

6-[[6-Iodo-2-(morpholin-4-yl)pyrazolo[1,5-a]pyridin-3-yl]methyl]pyridine-2-carboxylic acid ¹H-NMR (400 MHz, DMSO-d₆) δ 3.16 (4H, t, J = 4.8 Hz), 3.58 (4H, t, J = 4.8 Hz), 4.21 (2H, s), 7.22-7.36 (3H, m), 7.79-7.90 (2H, m), 8.79 (1H, s), 13.08 (1H, s).

### <Example 12-5>

### 6-[[2-(Morpholin-4-yl)-6-(trifluoromethyl)pyrazolo[1,5-a]pyridin-3-yl]methyl]pyridine-2-car boxylic acid

¹H-NMR (400 MHz, DMSO-d₆) δ 3.23 (4H, t, J = 4.8 Hz), 3.59 (4H, t, J = 4.8 Hz), 4.28 (2H, s), 7.25-7.37 (2H, m), 7.65 (1H, d, J = 9.8 Hz), 7.80-7.92 (2H, m), 9.05 (1H, s), 13.09 (1H, brs).

### <Example 12-6>

### 6-[[6-Methoxy-2-(morpholin-4-yl)pyrazolo[1,5-a]pyridin-3-yl]methyl]pyridine-2-carboxylic acid

¹H-NMR (400 MHz, DMSO-d₆) δ 3.00-3.20 (4H, m), 3.50-3.70 (4H, m), 3.77 (3H, s), 4.10-4.80 (2H, m), 6.91 (1H, d, J = 9.2 Hz), 7.00-7.50 (2H, m), 7.75-7.90 (1H, m), 7.90-8.15 (1H, m), 8.20-8.35 (1H, m), 13.04 (1H, brs).

### <Example 12-7>

### 2-[(6-Chloro-2-phenylpyrazolo[1,5-a]pyridin-3-yl)methyl]thiazole-4-carboxylic acid

¹H-NMR (400 MHz, DMSO-d₆) δ 4.66 (2H, s), 7.35 (1H, dd, J=9.7, 1.8Hz), 7.39-7.49 (3H, m), 7.71-7.74 (2H, m), 7.82 (1H, d, J=9.7 Hz), 8.22 (1H, s), 9.09 (1H, s), 12.95 (1H, s).

### <Example 12-8>

### 6-[(7-Chloro-2-phenylpyrazolo[1,5-a]pyridine)methyl]pyridine-2-carboxylic acid

¹H-NMR (400 MHz, DMSO-d₆) δ 4.46 (2H, s), 7.21-7.25 (2H, m), 7.32 (1H, dd, J=7.3, 1.8 Hz), 7.40-7.48 (3H, m), 7.75 (1H, dd, J=7.3, 2.4 Hz), 7.79-7.87 (4H, m), 13.12 (1H, brs).

### <Example 12-9>

### 3-[(6-Methoxy-2-phenylpyrazolo[1,5-a]pyridin-3-yl)methyl]-5-methylbenzoic acid

¹H-NMR (400 MHz, DMSO-d₆) δ 2.25 (3H, s), 3.83 (3H, s), 4.26 (2H, s), 7.00 (1H, dd, J = 9.7, 2.4 Hz), 7.15 (1H, s), 7.35 (1H, d, J = 7.3 Hz), 7.38-7.44 (3H, m), 7.46 (1H, d, J = 9.7 Hz), 7.53 (1H, s), 7.64 (2H, d, J = 7.3 Hz), 8.43 (1H, d, J = 2.4 Hz), 12.85 (1H, brs).

### <Example 12-10>

### 3-(Hydroxymethyl)-5-[(6-methoxy-2-phenylpyrazolo[1,5-a]pyridin-3-yl)methyl]benzoic acid

¹H-NMR (400 MHz, DMSO-d₆) δ 3.83 (3H, s), 4.30 (2H, s), 4.45 (2H, d, J = 6.1 Hz), 5.22 (1H, t, J = 6.1 Hz), 7.00 (1H, dd, J = 9.7, 2.4 Hz), 7.31 (1H, s), 7.35 (1H, d, J = 7.3 Hz), 7.41 (2H, t, J = 7.3 Hz), 7.46 (1H, d, J = 9.7 Hz), 7.51 (1H, s), 7.64 (2H, d, J = 7.3 Hz), 7.70 (1H, s), 8.43 (1H, d, J = 2.4 Hz), 12.80 (1H, s).

### <Example 12-11>

### 3-[(6-Methoxy-2-phenylpyrazolo[1,5-a]pyridin-3-yl)methyl]-5-[(methylthio)methyl]benzoic acid

¹H-NMR (400 MHz, DMSO-d₆) δ 2.49 (3H, s), 3.64 (2H, s), 3.83 (3H, s), 4.29 (2H, s), 7.00 (1H, dd, J = 9.7, 2.4 Hz), 7.24 (1H, s), 7.34 (1H, d, J = 7.3 Hz), 7.40 (2H, t, J = 7.3 Hz), 7.47-7.52 (2H, m), 7.62-7.66 (3H, m), 8.43 (1H, d, J = 2.4 Hz).

### <Example 12-12>

### 3-[(6-Methoxy-2-phenylpyrazolo[1,5-a]pyridin-3-yl)methyl]-5-[(methylsulfinyl)methyl]ben zoic acid

¹H-NMR (400 MHz, DMSO-d₆) δ 2.37 (3H, s), 3.83 (3H, s), 3.92 (1H, d, J = 12.7 Hz), 4.13 (1H, d, J = 12.7 Hz), 4.32 (2H, s), 7.00 (1H, dd, J = 9.7,2.4 Hz), 7.32 (1H, s), 7.34 (1H, d, J = 7.3 Hz), 7.40 (2H, t, J = 7.3 Hz), 7.51 (1H, d, J = 9.7 Hz), 7.60-7.63 (2H, m), 7.64 (1H, d, J = 1.8 Hz), 7.69 (1H, s), 8.43 (1H, d, J = 2.4 Hz), 12.94 (1H, s).

### <Example 12-13>

### 3-[(6-Methoxy-2-phenylpyrazolo[1,5-a]pyridin-3-yl)methyl]-5-[(methylsulfonyl)methyl]ben zoic acid

¹H-NMR (400 MHz, DMSO-d₆) δ 2.81 (3H, s), 3.83 (3H, s), 4.33 (2H, s), 4.50 (2H, s), 7.00 (1H, dd, J = 9.7, 2.4 Hz), 7.34 (1H, d, J = 7.3 Hz), 7.40 (2H, t, J = 7.3 Hz), 7.43 (1H, s), 7.51 (1H, d, J = 9.7 Hz), 7.62 (1H, s), 7.64 (2H, d, J = 7.3 Hz), 7.80 (1H, s), 8.44 (1H, d, J = 2.4 Hz), 13.01 (1H, brs).

### <Example 12-14>

### 3-Amino-5-[(6-methoxy-2-phenylpyrazolo[1,5-a]pyridin-3-yl)methyl]benzoic acid

¹H-NMR (400 MHz, DMSO-d₆) δ 3.83 (3H, s), 4.16 (2H, s), 6.55 (1H, s), 6.97-7.02 (2H, m), 7.04 (1H, s), 7.32-7.37 (1H, m), 7.39-7.45 (3H, m), 7.65 (2H, d, J = 7.3 Hz), 8.43 (1H, d, J = 1.8 Hz).

### <Example 12-15>

### 3-[(6-Methoxy-2-phenylpyrazolo[1,5-a]pyridin-3-yl)methyl]-5-nitrobenzoic acid

¹H-NMR (400 MHz, DMSO-d₆) δ 3.83 (3H, s), 4.49 (2H, s), 7.04 (1H, dd, J = 9.7, 2.4 Hz), 7.35 (1H, d, J = 7.3 Hz), 7.41 (2H, t, J = 7.3 Hz), 7.60 (1H, d, J = 9.7 Hz), 7.62-7.66 (2H, m), 8.00 (1H, t, J = 1.8 Hz), 8.12 (1H, t, J = 1.8 Hz), 8.39 (1H, t, J = 1.8 Hz), 8.45 (1H, d, J = 2.4 Hz), 13.70 (1H, brs).

### <Example 12-16>

### 6-[(2,6-diphenylpyrazolo[1,5-a]pyridin-3-yl)methyl]pyridine-2-carboxylic acid

¹H-NMR (400 MHz, DMSO-d₆) δ 4.44 (2H, s), 7.31 (1H, d, J = 7.3 Hz), 7.37-7.42 (3H, m), 7.43-7.49 (3H, m), 7.57 (1H, dd, J = 9.1, 1.8 Hz), 7.64-7.67 (2H, m), 7.71-7.76 (3H, m), 7.86 (2H, d, J = 7.3 Hz), 8.22 (1H, s).

### <Example 12-17>

### 6-[[6-(Methylthio)-2-phenylpyrazolo[1,5-a]pyridin-3-yl]methyl]pyridine-2-carboxylic acid

¹H-NMR (400 MHz, DMSO-d₆) δ 2.49 (3H, s), 4.38 (2H, s), 7.14 (1H, dd, J = 9.1, 1.8 Hz), 7.25 (1H, dd, J = 7.3, 1.2 Hz), 7.35 (1H, d, J = 7.3 Hz), 7.39 (2H, t, J = 7.3 Hz), 7.60 (1H, d, J = 9.1 Hz), 7.74-7.82 (4H, m), 8.56 (1H, s).

### <Example 12-18>

### 6-[[6-(Methylsulfinyl)-2-phenylpyrazolo[1,5-a]pyridin-3-yl]methyl]pyridine-2-carboxylic acid

¹H-NMR (400 MHz, DMSO-d₆) δ 2.87 (3H, s), 4.46 (2H, s), 7.32 (1H, dd, J = 7.3, 1.8 Hz), 7.40-7.51 (4H, m), 7.79-7.87 (4H, m), 7.90 (1H, d, J = 9.7 Hz), 8.96 (1H, s), 13.13 (1H, brs).

### <Example 12-19>

### 6-[[6-(Methylsulfonyl)-2-phenylpyrazolo[1,5-a]pyridin-3-yl]methyl]pyridine-2-carboxylic acid

¹H-NMR (400 MHz, DMSO-d₆) δ 3.34 (3H, s), 4.47 (2H, s), 7.33 (1H, dd, J = 6.7, 1.8 Hz), 7.43-7.50 (3H, m), 7.55 (1H, dd, J = 9.1, 1.8 Hz), 7.79-7.88 (4H, m), 7.93 (1H, d, J = 9.1 Hz), 9.18 (1H, s).

### <Example 12-20>

### 6-[(6-Chloro-2-cyclopropylpyrazolo[1,5-a]pyridin-3-yl)methyl]pyridine-2-carboxylic acid

¹H NMR (400 MHz, CDCl₃) δ 0.95-1.01 (4H, m), 1.92-1.94 (1H, m), 4.35 (2H, s), 7.02 (1H, dd, J = 9.2, 1.8 Hz), 7.23 (1H, d, J = 9.2 Hz), 7.36 (1H, d, J = 7.3 Hz), 7.84 (1H, dd, J = 7.9, 7.3 Hz), 8.07 (1H, d, J = 7.9 Hz), 8.36 (1H, d, J = 1.8 Hz).

### <Example 12-21>

### 6-[(6-Chloro-2-cyclopentylpyrazolo[1,5-a]pyridin-3-yl)methyl]pyridine-2-carboxylic acid

¹H NMR (400 MHz, CDCl₃) δ 1.60-1.70 (2H, m), 1.79-1.87 (4H, m), 1.94-2.01 (2H, m), 3.16-3.24 (1H, m), 4.29 (2H, s), 7.02 (1H, dd, J = 9.2, 1.8 Hz), 7.22 (1H, J = 9.2 Hz), 7.28 (1H, d, J = 7.9 Hz), 7.82 (1H, dd, J = 7.9, 7.3 Hz), 8.07 (1H, d, J = 7.3 Hz), 8.44 (1H, d, J = 1.8 Hz).

### <Example 12-22>

### 6-[(6-Chloro-2-cyclohexylpyrazolo[1,5-a]pyridin-3-yl)methyl]pyridine-2-carboxylic acid

¹H NMR (400 MHz, CDCl₃) δ 1.30-1.37 (3H, m), 1.65-1.80 (7H, m), 2.78 (1H, tt, J = 12.1, 3.6 Hz), 4.29 (2H, s), 7.02 (1H, dd, J = 9.7, 1.8 Hz), 7.21 (1H, d, J = 9.7 Hz), 7.28 (1H, d, J = 7.9 Hz), 7.82 (1H, dd, J = 7.9,7.3 Hz), 8.07 (1H, d, J = 7.3 Hz), 8.45 (1H, d, J = 1.8 Hz).

### <Example 12-23>

### 6-[(2-tert-Butyl-6-chloropyrazolo[1,5-a]pyridin-3-yl)methyl]pyridine-2-carboxylic acid

¹H NMR (400 MHz, CDCl₃) δ 1.42 (9H, s), 4.49 (2H, s), 7.03 (1H, dd, J = 9.7, 1.8 Hz), 7.16 (1H, d, J = 9.7 Hz), 7.23 (1H, d, J = 7.3 Hz), 7.83 (1H, dd, J = 7.9, 7.3 Hz), 8.09 (1H, d, J = 7.9 Hz), 8.49 (1H, s).

### <Example 12-24>

### 6-[[6-Chloro-2-(2-methylpropyl)pyrazolo[1,5-a]pyridin-3-yl]methyl]pyridine-2-carboxylic acid

¹H NMR (400 MHz, CDCl₃) δ 0.92 (6H, d, J = 6.7 Hz), 1.97-2.07 (1H, m), 2.63 (2H, d, J = 7.3 Hz), 4.27 (2H, s), 7.03 (1H, dd, J = 9.1, 1.8 Hz), 7.21 (1H, d, J = 9.1 Hz), 7.28 (1H, d, J = 7.9 Hz), 7.82 (1H, dd, J = 7.9,7.3 Hz), 8.07 (1H, d, J = 7.3 Hz), 8.44 (1H, s).

### <Example 12-25>

### 6-[[6-Chloro-2-(pyrrolidin-1-yl)pyrazolo[1,5-a]pyridin-3-yl]methyl]pyridine-2-carboxylic acid

¹H NMR (400 MHz, CDCl₃) δ 1.92-1.96 (4H, m), 3.47-3.48 (4H, m), 4.34 (2H, s), 6.98 (1H, dd, J = 9.7, 1.8 Hz), 7.06 (1H, d, J = 9.7 Hz), 7.35 (1H, d, J = 7.9 Hz), 7.83 (1H, dd, J = 7.9, 7.3 Hz), 8.06 (1H, d, J = 7.3 Hz), 8.30 (1H, d, J = 1.8 Hz).

### <Example 12-26>

### 6-[[6-Chloro-2-(piperidin-1-yl)pyrazolo[1,5-a]pyridin-3-yl]methyl]pyridine-2-carboxylic acid

¹H NMR (400 MHz, CDCl₃) δ 1.53-1.62 (6H, m), 3.17-3.18 (4H, m), 4.25 (2H, s), 6.98 (1H, dd, J = 9.7, 1.8 Hz), 7.07 (1H, d, J = 9.7 Hz), 7.38 (1H, d, J = 7.9 Hz), 7.83 (1H, t, J = 7.3 Hz), 8.07 (1H, d, J = 7.3 Hz), 8.31 (1H, d, J = 1.8 Hz).

### <Example 12-27>

### 6-[[6-Chloro-2-(hexahydro-1H-azepin-1-yl)pyrazolo[1,5-a]pyridin-3-yl]methyl]pyridine-2-c arboxylic acid

¹H NMR (400 MHz, CDCl₃) δ 1.57-1.60 (4H, m), 1.73-1.79 (4H, m), 3.48-3.50 (4H, m), 4.29 (2H, s), 6.99 (1H, dd, J = 9.8, 1.8 Hz), 7.06 (1H, d, J = 9.8 Hz), 7.34 (1H, d, J = 7.9 Hz), 7.84 (1H, dd, J = 7.9, 7.3 Hz), 8.08 (1H, d, J = 7.3 Hz), 8.29 (1H, d, J = 1.8 Hz).

### <Example 12-28>

### 6-[[6-Chloro-2-(dimethylamino)pyrazolo[1,5-a]pyridin-3-yl]methyl]pyridine-2-carboxylic acid

¹H NMR (400 MHz, CDCl₃) δ 2.92 (6H, s), 4.30 (2H, s), 6.99 (1H, dd, J = 9.7, 1.8 Hz), 7.07 (1H, d, J = 9.7 Hz), 7.36 (1H, d, J = 7.9 Hz), 7.83 (1H, dd, J = 7.9,7.3 Hz), 8.06 (1H, d, J = 7.3Hz), 8.31 (1H, d, J = 1.8 Hz).

### <Example 12-29>

### 6-[[6-Chloro-2-(thiomorpholin-4-yl)pyrazolo[1,5-a]pyridin-3-yl]methyl]pyridine-2-carboxyli c acid

¹H NMR (400 MHz, CDCl₃) δ 2.66-2.68 (4H, m), 3.51-3.53 (4H, m), 4.22 (2H, s), 7.01 (1H, dd, J = 9.7, 1.8 Hz), 7.09 (1H, d, J = 9.7 Hz), 7.35 (1H, d, J = 7.9 Hz), 7.84 (1H, dd, J = 7.9, 7.3 Hz), 8.08 (1H, d, J = 7.3 Hz), 8.32 (1H, d, J = 1.8 Hz).

### <Example 12-30>

### 6-[[6-chloro-2-(thiophen-3-yl)pyrazolo[1,5-a]pyridin-3-yl]methyl]pyridine-2-carboxylic acid

¹H-NMR (400 MHz, DMSO-d₆) δ 4.46 (2H, s), 7.26 (1H, dd, J = 9.7, 1.8 Hz), 7.37 (1H, dd, J = 7.3, 1.2 Hz), 7.58 (1H, dd, J = 4.8, 1.2 Hz), 7.63 (1H, dd, J = 4.8,2.4 Hz), 7.80- 7.86 (3H, m), 8.22 (1H, dd, J = 2.4,1.2 Hz), 8.99 (1H, s).

### <Example 12-31>

### 6-[[6-Chloro-2-(thiophen-2-yl)pyrazolo[1,5-a]pyridin-3-yl]methyl]pyridine-2-carboxylic acid

¹H-NMR (400 MHz, DMSO-d₆) δ 4.51 (2H, s), 7.11 (1H, t, J = 4.2 Hz), 7.27 (1H, d, J = 4.2 Hz), 7.30 (1H, s), 7.60 (1H, d, J = 4.8 Hz), 7.62 (1H, d, J = 3.6 Hz), 7.80- 7.86 (3H, m), 9.03 (1H, s), 13.08 (1H, brs).

### <Example 12-32>

### 6-[[6-Chloro-2-(furan-2-yl)pyrazolo[1,5-a]pyridin-3-yl]methyl]pyridine-2-carboxylic acid

¹H-NMR (400 MHz, DMSO-d₆) δ 4.52 (2H, s), 6.11 (1H, dd, J = 3.0, 1.8 Hz), 6.95 (1H, d, J = 3.0 Hz), 7.27 (1H, d, J = 1.8 Hz), 7.29 (1H, d, J = 1.8 Hz), 7.79- 7.85 (4H, m), 9.00 (1H, d, J = 1.2 Hz).

### <Example 12-33>

### 6-[[6-Chloro-2-(furan-3-yl)pyrazolo[1,5-a]pyridin-3-yl]methyl]pyridine-2-carboxylic acid

¹H-NMR (400 MHz, DMSO-d₆) δ 4.40 (2H, s), 6.92 (1H, s), 7.27 (1H, dd, J = 9.4, 1.8 Hz), 7.40 (1H, dd, J = 6.7, 1.8 Hz), 7.77 (1H, s), 7.82-7.86 (3H, m), 8.45 (1H, s), 8.97 (1H, s), 13.16 (1H, brs).

### <Example 12-34>

### 6-[(6-Chloro-2-methoxypyrazolo[1,5-a]pyridin-3-yl)methyl]pyridine-2-carboxylic acid

¹H NMR (400 MHz, CDCl₃) δ 4.05 (3H, s), 4.15 (2H, s), 7.04 (1H, dd, J = 9.6, 1.9 Hz), 7.15 (1H, d, J = 9.6 Hz), 7.43 (1H, d, J = 7.6 Hz), 7.83 (1H, t, J = 7.6 Hz), 8.05 (1H d, J = 7.6 Hz), 8.28 (1H, s).

### <Example 12-35>

### 6-[[6-Chloro-2-(2-propyloxy)pyrazolo[1,5-a]pyridin-3-yl]methyl]pyridine-2-carboxylic acid

¹H-NMR (400 MHz, DMSO-d₆) δ 1.27 (6H, d, J = 6.1 Hz), 4.08 (2H, s), 4.90-5.00 (1H, m), 7.15 (1H, dd, J = 9.7, 1.8 Hz), 7.26-7.33 (1H, m), 7.47 (1H, d, J = 9.7 Hz), 7.80-7.88 (2H, m), 8.76 (1H, d, J = 1.8 Hz), 13.04 (1H, brs).

### <Example 12-36>

### 6-[[6-Chloro-2-(1-methylcyclopropyl)pyrazolo[1,5-a]pyridin-3-yl]methyl]pyridine-2-carbox ylic acid

¹H-NMR (400 MHz, DMSO-d₆) δ 0.63 (2H, dd, J = 6.1, 4.2 Hz), 0.86 (2H, dd, J = 6.1, 4.2 Hz), 1.28 (3H, s), 4.35 (2H, s), 7.14 (1H, dd, J = 9.7, 1.8 Hz), 7.27 (1H, dd, J = 7.3, 1.8 Hz), 7.62 (1H, d, J = 9.7 Hz), 7.78-7.87 (2H, m), 8.83 (1H, s), 13.09 (1H, brs).

### <Example 12-37>

### 6-[[6-Chloro-2-(4-fluorophenyl)pyrazolo[1,5-a]pyridin-3-yl]methyl]pyridine-2-carboxylic acid

¹H-NMR (400 MHz, CDCl₃) δ 4.44 (2H, s), 7.10-7.14 (4H, m), 7.31 (2H, t, J = 9.1 Hz), 7.62 (1H, dd, J = 8.5, 5.4 Hz), 7.81 (1H, t, J = 7.9, Hz), 8.06 (1H, d, J = 7.9, Hz), 8.53 (1H, s).

### <Example 12-38>

### 6-[[6-Chloro-2-(3-fluorophenyl)pyrazolo[1,5-a]pyridin-3-yl]methyl]pyridine-2-carboxylic acid

¹H-NMR (400 MHz, DMSO-d₆) δ 4.44 (2H, s), 7.24 (1H, td, J = 8.6, 2.6 Hz), 7.38 (1H, dd, J = 6.1, 3.0 Hz), 7.48 (1H, q, J = 7.5 Hz), 7.70 (1H, d, J = 8.6 Hz), 7.73 (1H, d, J = 9.8 Hz), 7.82 (3H, m), 9.04 (1H, s).

### <Example 12-39>

### 6-[[6-Chloro-2-(2-fluorophenyl)pyrazolo[1,5-a]pyridin-3-yl]methyl]pyridine-2-carboxylic acid

¹H-NMR (400 MHz, DMSO-d₆) δ 4.24 (2H, s), 7.18 (1H, d, J = 6.7 Hz), 7.27 (2H, dd, J = 9.7, 1.8 Hz), 7.31 (1H, dd, J = 7.9, 5.4 Hz), 7.46-7.52 (1H, m), 7.63 (1H, d, J = 7.9, 1.8 Hz), 7.72-7.80 (3H, m), 9.05 (1H, s).

### <Example 12-40>

### 6-[[6-Chloro-2-(4-methoxyphenyl)pyrazolo[1,5-a]pyridin-3-yl]methyl]pyridine-2-carboxylic acid

¹H-NMR (400 MHz, DMSO-d₆) δ 3.78 (3H, s), 4.41 (2H, s), 7.00 (2H, d, J = 9.1 Hz), 7.24(2H, dd, J = 9.7, 1.8 Hz), 7.30 (1H, dd, J = 7.3, 1.8 Hz), 7.72 (1H, d, J = 9.7 Hz), 7.76 (2H, d, J = 8.5 Hz), 7.81-7.86 (2H, m), 9.00 (1H, s), 13.09 (1H, brs).

### <Example 12-41>

### 6-[[6-Chloro-2-(3-methoxyphenyl)pyrazolo[1,5-a]pyridin-3-yl]methyl]pyridine-2-carboxylic acid

¹H-NMR (400 MHz, DMSO-d₆) δ 3.72 (3H, s), 4.44 (2H, s), 6.95 (2H, d, J = 9.1 Hz), 7.26 (1H, dd, J = 9.7, 1.8 Hz), 7.30-7.39 (4H, m), 7.76 (1H, d, J = 9.1 Hz), 7.79-7.83 (2H, m), 9.05 (1H, s).

### <Example 12-42>

### 6-[[6-Chloro-2-(2-methoxyphenyl)pyrazolo[1,5-a]pyridin-3-yl]methyl]pyridine-2-carboxylic acid

¹H-NMR (400 MHz, DMSO-d₆) δ 3.55 (3H, s), 4.15 (2H, s), 6.99 (1H, t, J = 7.6 Hz), 7.05 (1H, d, J = 1.8 Hz), 7.08 (1H, s), 7.22 (1H, dd, J = 9.7, 1.8 Hz), 7.34 (1H, dd, J =7.6, 1.8 Hz), 7.40 (1H, td, J = 7.6, 1.8 Hz), 7.62 (1H, d, J = 9.7 Hz),7.73 (1H, t, J = 7.6 Hz), 7.79 (1H, d, J = 7.6 Hz), 8.98 (1H, d, J = 1.8 Hz).

### <Example 12-43>

### 6-[[6-Chloro-2-(4-methylphenyl)pyrazolo[1,5-a]pyridin-3-yl]methyl]pyridine-2-carboxylic acid

¹H-NMR (400 MHz, CDCl₃) δ 2.40 (3H, s), 4.46 (2H, s), 7.09 (2H, d, J = 9.1 Hz), 7.25 (2H, d, J = 9.1 Hz), 7.30 (2H, d, J = 9.1 Hz), 7.53 (2H, d, J = 7.9 Hz), 7.79 (1H, t, J = 7.9 Hz), 8.05 (1H, d, J = 7.9 Hz), 8.54 (1H, s).

### <Example 12-44>

### 6-[[6-Chloro-2-(3-methylphenyl)pyrazolo[1,5-a]pyridin-3-yl]methyl]pyridine-2-carboxylic acid

¹H-NMR (400 MHz, CDCl₃) δ 2.39 (3H, s), 4.46 (2H, s), 7.10 (1H, d, J = 9.7 Hz), 7.22 (1H, d, J = 7.3 Hz), 7.28-7.34 (3H, m), 7.40 (1H, d, J = 7.3 Hz), 7.47 (1H, s), 7.80 (1H, t, J = 7.9 Hz), 8.05 (1H, d, J = 7.3 Hz), 8.54 (1H, s).

### <Example 12-45>

### 6-[[6-Chloro-2-(2-methylphenyl)pyrazolo[1,5-a]pyridin-3-yl]methyl]pyridine-2-carboxylic acid

¹H-NMR (400 MHz, CDCl₃) δ 2.15 (3H, s), 4.19 (2H, s), 7.11-7.33 (6H, m), 7.37 (1H, d, J = 9.1 Hz), 7.74 (1H, t, J = 7.9 Hz), 8.00 (1H, d, J = 7.9 Hz), 8.51 (1H, d, J = 1.2 Hz).

### <Example 12-46>

### 6-[[6-Chloro-2-[4-(trifluoromethyl)phenyl]pyrazolo[1,5-a]pyridin-3-yl]methyl]pyridine-2-ca rboxylic acid

¹H-NMR (400 MHz, CDCl₃) δ 4.48 (2H, s), 7.14 (1H, d, J = 9.7 Hz), 7.29 (1H, d, J = 9.1 Hz), 7.35 (1H, d, J = 9.7 Hz), 7.71 (2H, d, J = 7.9 Hz), 7.78 (2H, d, J = 7.9 Hz), 7.83 (1H, t, J = 7.9 Hz), 8.08 (1H, d, J = 7.9 Hz), 8.56 (1H, s).

### <Example 12-47>

### 6-[[6-Chloro-2-[3-(trifluoromethyl)phenyl]pyrazolo[1,5-a]pyridin-3-yl]methyl]pyridine-2-ca rboxylic acid

¹H-NMR (400 MHz, CDCl₃) δ 4.46 (2H, s), 7.16 (1H, dd, J = 9.7, 1.5 Hz), 7.31 (1H, d, J = 7.9 Hz), 7.40 (1H, d, J = 9.7 Hz), 7.56 (1H, t, J = 7.9 Hz), 7.66 (1H, d, J = 7.9 Hz), 7.83 (2H, t, J = 7.9 Hz), 7.89 (1H, s), 8.07 (1H, d, J = 7.3 Hz), 8.56 (1H, s).

### <Example 12-48>

### 6-[[6-Chloro-2-[2-(trifluoromethyl)phenyl]pyrazolo[1,5-a]pyridin-3-yl]methyl]pyridine-2-ca rboxylic acid

¹H-NMR (400 MHz, CDCl₃) δ 4.18 (2H, s), 7.14 (1H, dd, J = 9.7, 1.5 Hz), 7.18 (1H, d, J = 7.9 Hz), 7.33 (1H, d, J = 9.7 Hz), 7.56 (2H, t, J = 4.5 Hz), 7.75 (1H, t, J = 7.3 Hz), 7.80 (1H, t, J = 4.5 Hz), 8.01 (1H, d, J = 7.9 Hz), 8.52 (1H, s).

### <Example 12-49>

### 6-[[6-Chloro-2-(4-chlorophenyl)pyrazolo[1,5-a]pyridin-3-yl]methyl]pyridine-2-carboxylic acid

¹H-NMR (400 MHz, CDCl₃) δ 4.45 (2H, s), 7.12 (1H, dd, J = 9.4, 1.5 Hz), 7.31 (2H, t, J = 9.4 Hz), 7.42 (2H, d, J = 8.5 Hz), 7.59 (2H, d, J = 8.5 Hz), 7.82 (1H, t, J = 7.9 Hz), 8.07 (1H, d, J = 7.9 Hz), 8.54 (1H, s).

### <Example 12-50>

### 6-[[6-Chloro-2-(3-chlorophenyl)pyrazolo[1,5-a]pyridin-3-yl]methyl]pyridine-2-carboxylic acid

¹H-NMR (400 MHz, CDCl₃) δ 4.46 (2H, s), 7.13 (1H, dd, J = 9.4, 1.5 Hz), 7.30 (2H, d J = 7.9 Hz), 7.35-7.37 (3H, m), 7.51-7.53 (1H, m), 7.66 (1H, s), 7.82 (1H, t, J = 7.9 Hz), 8.07 (1H, d, J = 7.3 Hz), 8.54 (1H, s).

### <Example 12-51>

### 6-[[6-Chloro-2-(2-chlorophenyl)pyrazolo[1,5-a]pyridin-3-yl]methyl]pyridine-2-carboxylic acid

¹H-NMR (400 MHz, CDCl₃) δ 4.26 (2H, s), 7.13 (1H, dd, J = 9.1, 1.8 Hz), 7.31-7.40 (4H, m), 7.48 (1H, d, J = 7.9 Hz), 7.74 (1H, t, J = 7.9Hz), 7.98 (1H, t, J = 7.3 Hz), 8.53 (1H, s).

### <Example 12-52>

### 6-[[2-(2-Fluorophenyl)-6-methoxypyrazolo[1,5-a]pyridin-3-yl]methyl]pyridine-2-carboxylic acid

¹H NMR (400 MHz, DMSO-d₆) δ 3.81 (3H, s), 4.21 (2H, s), 7.01 (1H, dd, J = 9.7, 1.8 Hz), 7.16 (1H, d, J = 7.3 Hz), 7.24-7.32 (2H, m), 7.42-7.49 (1H, m), 7.55 (1H, d, J = 9.7 Hz), 7.63 (1H, td, J = 7.3, 1.8 Hz), 7.73-7.80 (2H, m), 8.42 (1H, d, J = 1.8 Hz), 13.04 (1H, s).

### <Example 12-53>

### 6-[[2-(3-Fluorophenyl)-6-methoxypyrazolo[1,5-a]pyridin-3-yl]methyl]pyridine-2-carboxylic acid

¹H NMR (400 MHz, DMSO-d₆) δ 3.82 (3H, s), 4.42 (2H, s), 7.03 (1H, dd, J = 9.7, 1.8 Hz), 7.19 (1H, td, J = 8.5, 2.4 Hz), 7.35 (1H, dd, J = 6.7, 2.4 Hz), 7.46 (1H, td, J = 7.9, 6.7 Hz), 7.65 (1H, d, J = 9.7 Hz), 7.67-7.73 (2H, m), 7.80-7.85 (2H, m), 8.43 (1H, d, J = 1.8 Hz), 13.10 (1H, s).

### <Example 12-54>

### 4-(3-Hydroxypropyl)-6-[(6-methoxy-2-phenylpyrazolo[1,5-a]pyridin-3-yl)methyl]pyridine-2 -carboxylic acid

¹H NMR (400 MHz, DMSO-d₆) δ 1.55-1.62 (2H, m), 2.57 (2H, t, J = 7.6 Hz), 3.30 (2H, t, J = 6.4 Hz), 3.82 (3H, s), 4.36 (2H, s), 7.00 (1H, dd, J = 9.7, 1.8 Hz), 7.14 (1H, s), 7.35 (1H, t, J = 7.3 Hz), 7.42 (2H, t, J = 7.6 Hz), 7.59 (1H, d, J = 9.7 Hz), 7.69 (1H, s), 7.81 (2H, d, J = 7.3 Hz), 8.41 (1H, d, J = 1.8 Hz), 12.99 (1H, s).

### <Example 12-55>

### 6-[(6-Methoxy-2-phenylpyrazolo[1,5-a]pyridin-3-yl)methyl]-4-[3-(methylsulfonyl)propyl]p yridine-2-carboxylic acid

¹H NMR (400 MHz, DMSO-d₆) δ 1.84-1.92 (2H, m), 2.67 (2H, t, J = 7.6 Hz), 2.91 (3H, s), 3.00 (2H, t, J = 8.2 Hz), 3.82 (3H, s), 4.37 (2H, s), 6.99 (1H, dd, J = 9.7, 1.8 Hz), 7.18 (1H, d, J = 1.2 Hz), 7.35 (1H, tt, J = 7.3, 1.2 Hz), 7.40-7.44 (2H, m), 7.59 (1H, d, J = 9.7 Hz), 7.73 (1H, d, J = 1.2 Hz), 7.81 (2H, dd, J = 8.5, 1.2 Hz), 8.42 (1H, d, J = 1.8 Hz), 13.05 (1H, s).

### <Example 12-56>

### 6-[(6-Methoxy-2-phenylpyrazolo[1,5-a]pyridin-3-yl)methyl]pyrazine-2-carboxylic acid

¹H NMR (400 MHz, DMSO-d₆) δ 3.82 (3H, s), 4.48 (2H, s), 7.03 (1H, dd, J = 9.7, 2.4 Hz), 7.33-7.39 (1H, m), 7.42 (2H, t, J = 7.3 Hz), 7.64 (1H, d, J = 9.7 Hz), 7.79 (2H, dd, J = 6.7, 1.8 Hz), 8.43 (1H, d, J = 1.8 Hz), 8.63 (1H, s), 8.96 (1H, s), 13.65 (1H, s).

### <Example 12-57>

### 6-[[6-Methoxy-2-(1-methylcyclopropyl)pyrazolo[1,5-a]pyridin-3-yl]methyl]pyridine-2-carb oxylic acid

¹H NMR (400 MHz, DMSO-d₆) δ 0.60 (2H, dd, J = 6.1, 4.2 Hz), 0.85 (2H, dd, J = 6.1, 4.2 Hz), 1.27 (3H, s), 3.76 (2H, s), 4.32 (2H, s), 6.89 (1H, dd, J = 9.8, 1.8 Hz), 7.22 (1H, dd, J = 7.3, 1.8 Hz), 7.45 (1H, d, J = 9.8 Hz), 7.77-7.88 (2H, m), 8.23 (1H, s), 13.09 (1H, brs).

### <Example 12-58>

### 6-[(6-Chloro-5-methyl-2-phenylpyrazolo[1,5-a]pyridin-3-yl)methyl]pyridine-2-carboxylic acid

¹H NMR (400 MHz, DMSO-d₆) δ 2.36 (3H, s), 4.39 (2H, s), 7.29 (1H, d, J = 7.3 Hz), 7.35-7.48 (3H, m), 7.76-7.87 (5H, m), 9.01 (1H, s).

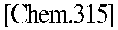

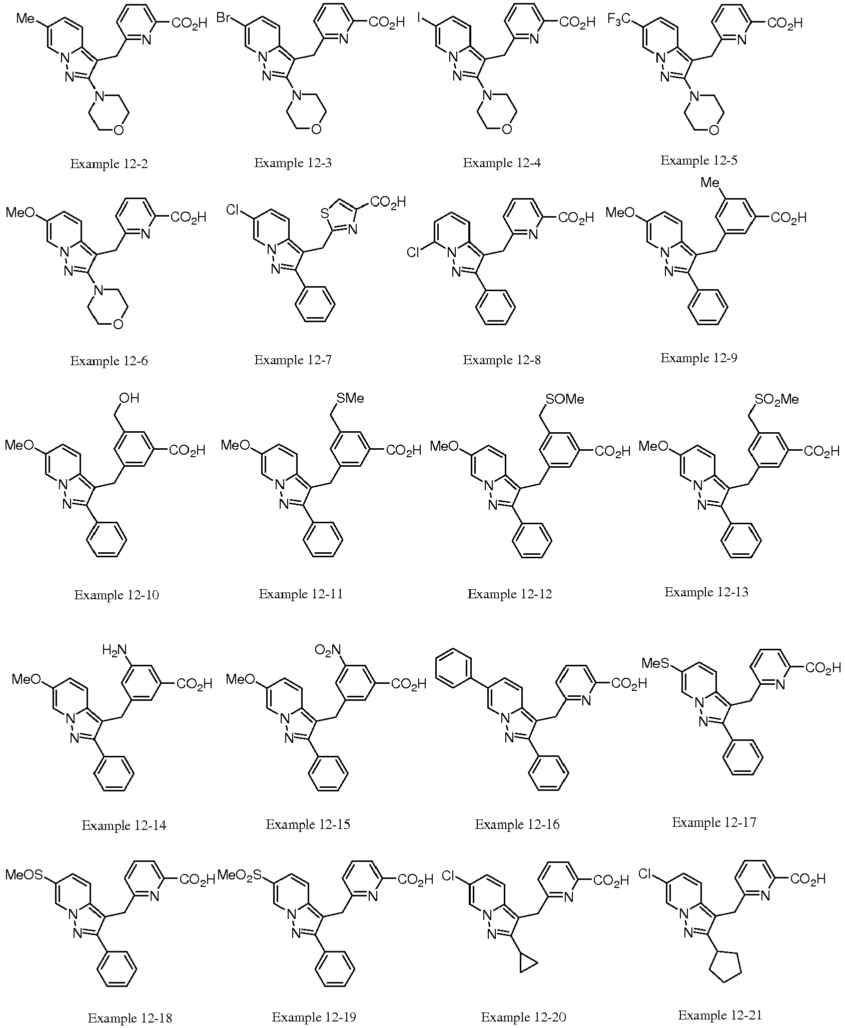

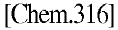

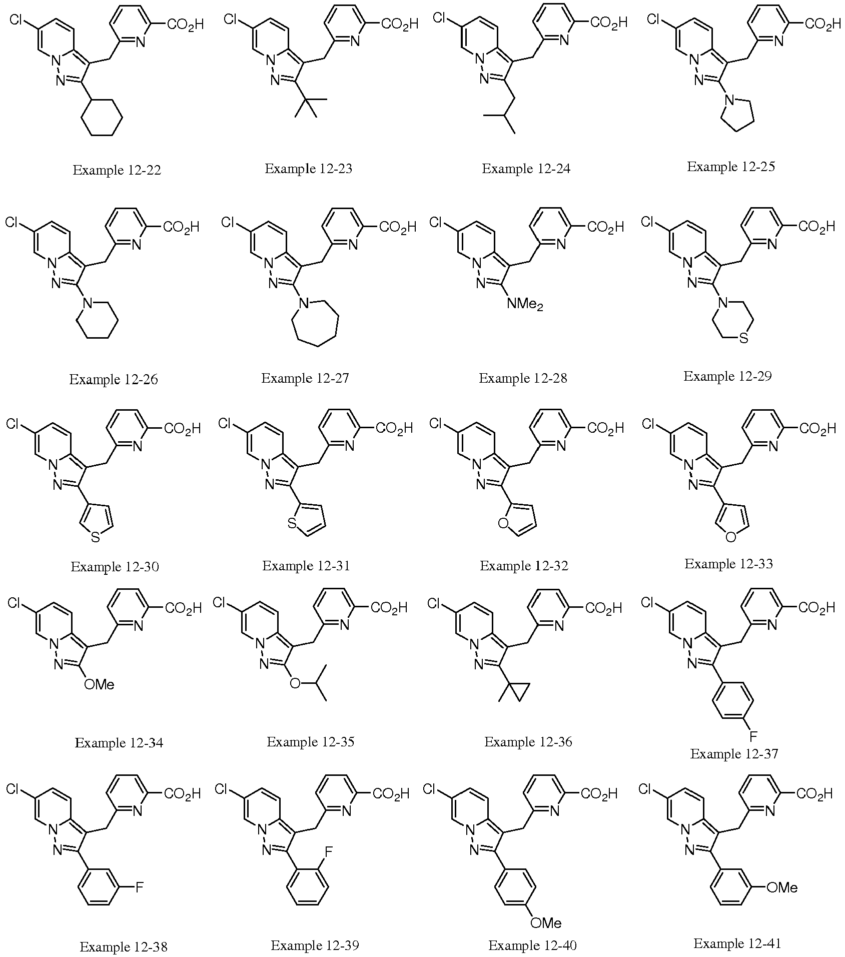

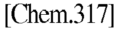

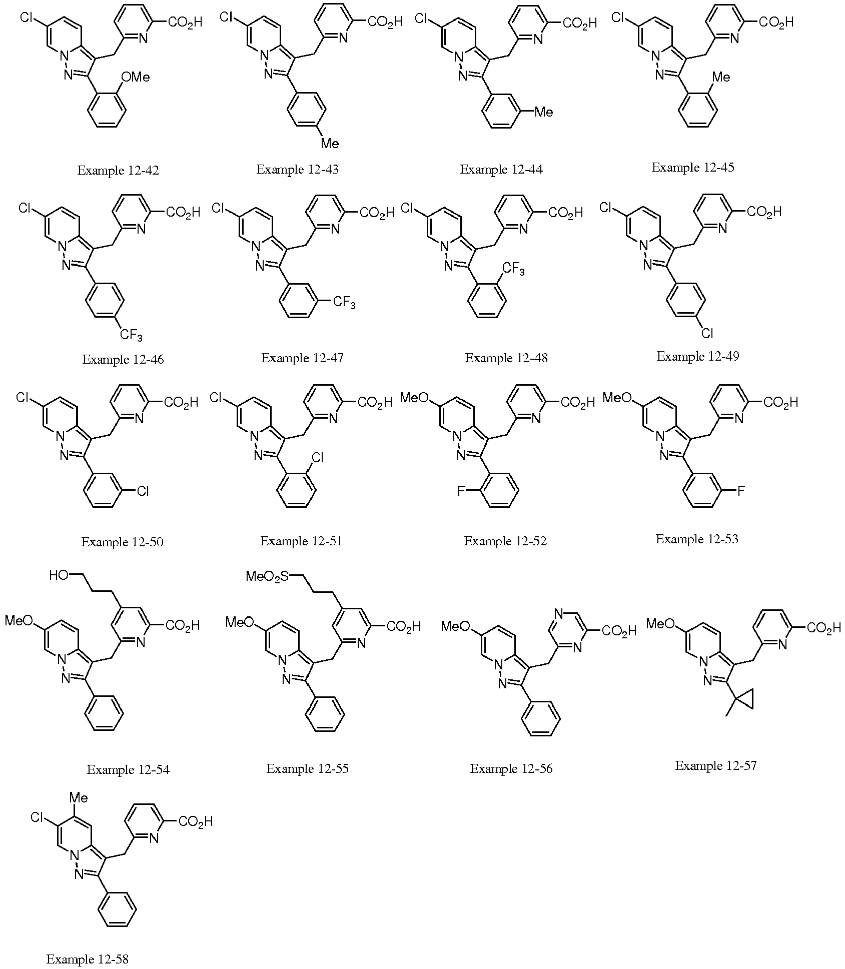

### <Example 13-1>

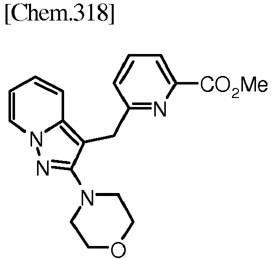

### Methyl 6-[[2-(morpholin-4-yl)pyrazolo[1,5-a]pyridin-3-yl]methyl]pyridine-2-carboxylate

Trifluoroacetic acid (75 µL) and triethylsilane (0.22 mL) were serially added to a dichloromethane (4.9 mL) solution of 2-(morpholin-4-yl)pyrazolo[1,5-a]pyridine (100 mg) and methyl 6-formylpyridine-2-carboxylate (122 mg) at room temperature, and then the mixture was stirred at room temperature for 5 hours. A saturated sodium bicarbonate aqueous solution was added to the reaction solution (pH = 7 to 8) under ice-cooling and the mixture was extracted with ethyl acetate and dried over anhydrous sodium sulfate. The solvent was evaporated and the residue thus obtained was purified by silica gel chromatography (n-hexane:ethyl acetate = 1:1) to obtain a title compound as a pale yellow crystal (170 mg).
¹H-NMR (400 MHz, CDCl₃) δ 3.24 (4H, t, J = 4.8 Hz), 3.72 (4H, t, J = 4.8 Hz), 4.03 (3H, s), 4.35 (2H, s), 6.59 (1H, t, J = 7.3 Hz), 7.00 (1H, t, J = 7.3 Hz), 7.15 (1H, d, J = 6.7 Hz), 7.19 (1H, d, J = 7.9 Hz), 7.69 (1H, t, J = 7.9 Hz), 7.98 (1H, d, J =. 7.9 Hz), 8.26 (1H, d, J = 7.3 Hz).

The following Examples 13-2 to 13-31 were obtained by using corresponding pyrazolopyridines and aldehydes in the same manner as Example 13-1.

### <Example 13-2>

### Methyl

6-[[6-methyl-2-(morpholin-4-yl)pyrazolo[1,5-a]pyridin-3-yl]methyl]pyridine-2-carboxylate ¹H-NMR (400 MHz, CDCl₃) δ 2.27 (3H, s), 3.22 (4H, t, J = 4.8 Hz), 3.72 (4H, t, J = 4.8 Hz), 4.03 (3H, s), 4.33 (2H, s), 6.86 (1H, d, J = 9.2 Hz), 7.05 (1H, d, J = 9.2 Hz), 7.18 (1H, d, J = 7.9 Hz), 7.68 (1H, t, J = 7.9 Hz), 7.97 (1H, d, J =. 7.9 Hz), 8.08 (1H, s).

### <Example 13-3>

### Methyl

6-[[6-bromo-2-(morpholin-4-yl)pyrazolo[1,5-a]pyridin-3-yl]methyl]pyridine-2-carboxylate ¹H-NMR (400 MHz, CDCl₃) δ 3.23 (4H, t, J = 4.8 Hz), 3.72 (4H, t, J = 4.8 Hz), 4.03 (3H, s), 4.32 (2H, s), 7.07 (2H, s), 7.16 (1H, d, J = 7.9 Hz), 7.70 (1H, t, J = 7.9 Hz), 7.98 (1H, d, J =. 7.9 Hz), 8.91 (1H, s).

### <Example 13-4>

### Methyl

6-[[6-iodo-2-(morpholin-4-yl)pyrazolo[1,5-a]pyridin-3-yl]methyl]pyridine-2-carboxylate ¹H-NMR (400 MHz, CDCl₃) δ 3.23 (4H, t, J = 4.8 Hz), 3.71 (4H, t, J = 4.8 Hz), 4.03 (3H, s), 4.31 (2H, s), 6.96 (1H, d, J = 9.2 Hz), 7.15 (1H, d, J = 9.2 Hz), 7.15 (1H, d, J = 7.9 Hz), 7.69 (1H, t, J = 7.9 Hz), 7.98 (1H, d, J =. 7.9 Hz), 8.53 (1H, s).

### <Example 13-5>

### Methyl 6-[[2-(morpholin-4-yl)-6-(trifluoromethyl)pyrazolo[1,5-a]pyridin-3-yl]methyl]pyridine-2-car boxylate

¹H-NMR (400 MHz, CDCl₃) δ 3.27 (4H, t, J = 4.8 Hz), 3.71 (4H, t, J = 4.8 Hz), 4.03 (3H, s), 4.35 (2H, s), 7.12 (1H, d, J = 9.7 Hz), 7.17 (1H, d, J = 7.9 Hz), 7.25 (1H, d, J = 9.7 Hz), 7.71 (1H, t, J =. 7.9 Hz), 8.00 (1H, d, J =. 7.9 Hz), 8.60 (1H, s).

### <Example 13-6>

### Methyl 6-[[6-methoxy-2-(morpholin-4-yl)pyrazolo[1,5-a]pyridin-3-yl]methyl]pyridine-2-carboxylat e

¹H-NMR (400 MHz, CDCl₃) δ 3.20 (4H, t, J = 4.9 Hz), 3.73 (4H, t, J = 4.9 Hz), 3.79 (3H, s), 4.03 (3H, s), 4.32 (2H, s), 6.83 (1H, dd, J = 9.8, 1.8 Hz), 7.06 (1H, d, J = 7.9 Hz), 7.19 (1H, d, J = 7.9 Hz), 7.68 (1H, d, J = 7.9 Hz), 7.92 (1H, d, J =. 1.8 Hz), 7.97 (1H, d, J =. 7.9 Hz).

### <Example 13-7>

### Ethyl 2-[(6-chloro-2-phenylpyrazolo[1,5-a]pyridin-3-yl)methyl]thiazole-4-carboxylate

¹H-NMR (400 MHz, CDCl₃) δ 1.42 (3H, t, J=7.0 Hz), 4.44 (2H, q, J=7.0 Hz), 4.63 (2H, s), 7.12 (1H, dd, J=9.1, 1.8 Hz), 7.36-7.48 (4H, m), 7.68 (2H, dd, J=8.2, 1.5 Hz), 8.01 (1H, s), 8.55 (1H, d, J=1.2 Hz).

### <Example 13-8>

### Methyl 6-[(7-chloro-2-phenylpyrazolo[1,5-a]pyridine-2-yl)methyl]pyridine-2-carboxylate

¹H-NMR (CDCl₃, 400 MHz) δ 4.04 (3H, s), 4.57 (2H, s), 6.93 (1H, dd, J= 7.3, 1.8 Hz), 7.03-7.10 (2H, m), 7.37-7.45 (4H, m), 7.64 (1H, t, J= 7.6 Hz), 7.73-7.76 (2H, m), 7.96 (1H, d, J=7.3 Hz).

### <Example 13-9>

### Methyl 6-[[6-chloro-2-(hexahydro-1H-azepin-1-yl)pyrazolo[1,5-a]pyridin-3-yl]methyl]pyridine-2-ca rboxylate

¹H NMR (CDCl₃, 400 MHz) δ 1.59-1.62 (4H, m), 1.77-1.80 (4H, m), 3.51-3.52 (4H, m), 4.09 (3H, s), 4.42 (2H, s), 6.97 (1H, dd, J = 9.1, 1.8 Hz), 7.06 (1H, d, J = 9.1 Hz), 7.18 (1H, d, J = 7.9 Hz), 7.73 (1H, dd, J = 7.9,7.3 Hz), 8.02 (1H, d, J = 7.3 Hz), 8.32 (1H, d, J = 1.8 Hz).

### <Example 13-10>

### Methyl 6-[[6-chloro-2-(dimethylamino)pyrazolo[1,5-a]pyridin-3-yl]methyl]pyridine-2-carboxylate

¹H NMR (CDCl₃, 400 MHz) δ 2.90 (6H, s), 4.03 (3H, s), 4.37 (2H, s), 6.93 (1H, dd, J = 9.7, 1.8 Hz), 7.04 (1H, d, J = 9.7 Hz), 7.16 (1H, d, J = 7.9 Hz), 7.68 (1H, dd, J = 7.9, 7.9 Hz), 7.97 (1H, d, J = 7.9 Hz), 8.29 (1H, d, J = 1.8 Hz).

### <Example 13-11>

### Methyl 6-[[6-chloro-2-(thiomorpholin-4-yl)pyrazolo[1,5-a]pyridin-3-yl]methyl]pyridine-2-carboxyla te

¹H NMR (CDCl₃, 400 MHz) δ 2.63-2.66 (4H, m), 3.52-3.54 (4H, m), 4.03 (3H, s), 4.28 (2H, s), 6.96 (1H, dd, J = 9.1, 1.8 Hz), 7.12 (1H, d, J = 9.1 Hz), 7.15 (1H, d, J = 7.9 Hz), 7.69 (1H, dd, J = 7.9, 7.3 Hz), 7.98 (1H, d, J = 7.3 Hz), 8.30 (1H, d, J = 1.8 Hz).

### <Example 13-12>

### Methyl 6-[[6-chloro-2-(thiphen-3-yl)pyrazolo[1,5-a]pyridin-3-yl]methyl]pyridine-2-carboxylate

¹H-NMR(CDCl₃, 400 MHz) δ 4.04 (3H, s), 4.61 (2H, s), 7.15 (1H, dd, J = 9.7, 1.8 Hz), 7.18 (1H, d, J = 7.9 Hz), 7.41-7.45 (2H, m), 7.46 (1H, dd, J = 5.1, 1.5 Hz), 7.65 (1H, dd, J = 2.4, 1.2 Hz), 7.80 (1H, t, J = 7.9 Hz), 8.05 (1H, d, J = 7.3 Hz), 8.67 (1H, d, J = 1.2 Hz).

### <Example 13-13>

### Methyl 6-[[6-chloro-2-(thiophen-2-yl)pyrazolo[1,5-a]pyridin-3-yl]methyl]pyridine-2-carboxylate

¹H-NMR(CDCl₃, 400 MHz) δ 4.04 (3H, s), 4.60 (2H, s), 7.05- 7.09 (1H, m), 7.12 (1H, d, J = 7.3 Hz), 7.18 (1H, d, J = 7.9 Hz), 7.36-7.39 (3H, m), 7.67 (1H, t, J = 7.9 Hz), 7.98 (1H, d, J = 7.9 Hz), 8.53 (1H, s).

### <Example 13-14>

### Methyl 6-[[6-chloro-2-(furan-2-yl)pyrazolo[1,5-a]pyridin-3-yl]methyl]pyridine-2-carboxylate

¹H-NMR(CDCl₃, 400 MHz) δ 4.04 (3H, s), 4.63 (2H, s), 6.50 (1H, dd, J = 3.0,1.2 Hz), 6.82 (1H, d, J = 3.0 Hz), 7.05 (1H, dd, J = 9.7, 1.8 Hz), 7.15 (1H, d, J = 7.9 Hz), 7.44 (1H, d, J = 9.7 Hz), 7.52 (1H, d, J = 1.2 Hz), 7.64 (1H, t, J = 7.9 Hz), 7.95 (1H, d, J = 7.9 Hz), 8.48 (1H, d, J = 1.2 Hz).

### <Example 13-15>

### Methyl 6-[[6-chloro-2-(1-methylcyclopropyl)pyrazolo[1,5-a]pyridin-3-yl]methyl]pyridine-2-carboxy late

¹H-NMR (400 MHz, CDCl₃) δ 0.74 (2H, dd, J = 6.1, 4.2 Hz), 0.96 (2H, dd, J = 6.1, 4.2 Hz), 1.38 (3H, s), 4.04 (3H, s), 4.49 (2H, s), 6.96 (1H, dd, J = 9.1, 1.2), 7.08 (1H, d, J = 8.9 (2H, s), 7.25 (1H, d, J = 9.1 Hz), 7.68 (1H, t, J = 7.9 Hz), 7.97 (1H, d, J =. 7.9 Hz), 8.40 (1H, d, J = 1.2).

### <Example 13-16>

### Methyl 6-[[6-chloro-2-(4-fluorophenyl)pyrazolo[1,5-a]pyridin-3-yl]methyl]pyridine-2-carboxylate

¹H-NMR (400 MHz, CDCl₃) δ 4.04 (3H, s), 4.50 (2H, s), 7.05-7.14 (4H, m), 7.38 (1H, d, J = 10.4 Hz), 7.65-7.71 (3H, m), 7.97 (1H, d, J = 7.3, Hz), 8.51 (1H, s).

### <Example 13-17>

### Methyl 6-[[6-chloro-2-(3-fluorophenyl)pyrazolo[1,5-a]pyridine]methyl-3-yl]pyridine-2-carboxylate

¹H-NMR (400 MHz, CDCl₃) δ 4.04 (3H, s), 4.52 (2H, s), 7.05-7.11 (3H, m), 7.36-7.42 (2H, m), 7.47-7.53 (2H, m), 7.68 (1H, t, J = 7.3 Hz), 7.98 (1H, d, J = 7.3, Hz), 8.52 (1H, s).

### <Example 13-18>

### Methyl 6-[[6-chloro-2-(2-fluorophenyl)pyrazolo[1,5-a]pyridin-3-yl]methyl]pyridine-2-carboxylate

¹H-NMR (400 MHz, CDCl₃) δ 4.02 (3H, s), 4.37 (2H, s), 7.04 (1H, dd, J = 9.8, 1.8 Hz), 7.11 (1H, d, J = 7.9 Hz), 7.17 (1H, dt, J = 8.5, 1.2 Hz), 7.24 (1H, dd, J = 7.3, 1.2 Hz), 7.40-7.45 (2H, m), 7.56 (1H, td, J = 7.3,1.8 Hz), 7.64 (1H, t, J = 7.9 Hz), 7.94 (1H, d, J = 7.3 Hz), 8.52 (1H, s).

### <Example 13-19>

### Methyl 6-[[6-chloro-2-(4-methoxyphenyl)pyrazolo[1,5-a]pyridin-3-yl]methyl]pyridine-2-carboxylat e

¹H-NMR (400 MHz, CDCl₃) δ 3.84 (3H, s), 4.04 (3H, s), 4.52 (2H, s), 6.96 (1H, d, J = 9.1 Hz), 7.03 (1H, dd, J = 9.7, 1.8 Hz), 7.09 (1H, d, J = 7.9 Hz), 7.34 (1H, d, J = 9.7 Hz), 7.46-7.67 (3H, m), 7.96 (1H, d, J = 7.9 Hz), 8.51 (1H, s).

### <Example 13-20>

### Methyl 6-[[6-chloro-2-(3-methoxyphenyl)pyrazolo[1,5-a]pyridin-3-yl]methyl]pyridine-2-carboxylat e

¹H-NMR (400 MHz, CDCl₃) δ 3.80 (3H, s), 4.03 (3H, s), 4.54 (2H, s), 6.94 (1H, d, J = 9.1 Hz), 7.04 (1H, d, J = 9.7 Hz), 7.10 (1H, d, J = 7.9 Hz), 7.26 (1H, d, J =7.6 Hz), 7.34 (1H, t, J =7.9 Hz), 7.38 (1H, d, J =9.7 Hz), 7.66 (1H, t, J =7.6 Hz), 7.96 (1H, d, J =7.9 Hz), 8.53 (1H, s).

### <Example 13-21>

### Methyl 6-[[6-chloro-2-(2-methoxyphenyl)pyrazolo[1,5-a]pyridin-3-yl]methyl]pyridine-2-carboxylat e

¹H-NMR (400 MHz, CDCl₃) δ 3.62 (3H, s), 4.02 (3H, s), 4.32 (2H, s), 6.95 (1H, d, J = 7.9 Hz), 6.99-7.09 (3H, m), 7.30 (1H, t, J = 10.3 Hz), 7.39 (1H, t, J = 7.9 Hz), 7.44 (1H, d, J =7.9 Hz), 7.61 (1H, t, J =7.9 Hz), 7.92 (1H, d, J =7.9 Hz), 8.51 (1H, s).

### <Example 13-22>

### Methyl 6-[[6-chloro-2-(4-methylphenyl)pyrazolo[1,5-a]pyridin-3-yl]methyl]pyridine-2-carboxylate

¹H-NMR (400 MHz, CDCl₃) δ 2.39 (3H, s), 4.04 (3H, s), 4.53 (2H, s), 7.03 (1H, dd, J = 9.7, 1.8 Hz), 7.08 (1H, t, J = 7.9 Hz), 7.24 (2H, d, J = 7.9 Hz), 7.35 (1H, d, J = 9.7 Hz), 7.59 (2H, d, J = 7.9 Hz), 7.65 (1H, t, J =7.9 Hz), 7.96 (1H, d, J =7.9 Hz), 8.51 (1H, s).

### <Example 13-23>

### Methyl 6-[[6-chloro-2-(3-methylphenyl)pyrazolo[1,5-a]pyridin-3-yl]methyl]pyridine-2-carboxylate

¹H-NMR (400 MHz, CDCl₃) δ 2.39 (3H, s), 4.04 (3H, s), 4.54 (2H, s), 7.04 (1H, dd, J = 9.7, 1.8 Hz), 7.09 (1H, d, J = 7.9 Hz), 7.20 (1H, d, J = 7.9 Hz), 7.31 (1H, t, J = 7.6 Hz), 7.37 (1H, t, J = 9.7 Hz), 7.46(1H, d, J =7.6 Hz), 7.54 (1H, s), 7.65 (1H, t, J =7.9 Hz), 7.96 (1H, d, J =7.9 Hz), 8.52 (1H, s).

### <Example 13-24>

### Methyl 6-[[6-chloro-2-(2-methylphenyl)pyrazolo[1,5-a]pyridin-3-yl]methyl]pyridine-2-carboxylate

¹H-NMR (400 MHz, CDCl₃) δ 2.22 (3H, s), 4.01 (3H, s), 4.24 (2H, s), 6.99 (1H, d, J = 7.9 Hz), 7.05 (1H, dd, J = 9.7, 1.5 Hz), 7.21-7.34 (4H, m), 7.48 (1H, d, J = 9.7 Hz), 7.62 (1H, t, J = 7.9 Hz), 7.91 (1H, d, J =7.9 Hz), 8.48 (1H, s).

### <Example 13-25>

### Methyl 6-[[6-chloro-2-[4-(trifluoromethyl)phenyl]pyrazolo[1,5-a]pyridin-3-yl]methyl]pyridine-2-car boxylate

¹H-NMR (400 MHz, CDCl₃) δ 4.03 (3H, s), 4.53 (2H, s), 6.99 (1H, d, J = 7.9 Hz), 7.08-7.10 (2H, m), 7.43 (1H, d, J = 9.7 Hz), 7.66-7.70 (3H, m),7.87 (2H, d J = 8.5 Hz), 7.97 (1H, d, J =7.3 Hz), 8.53 (1H, d, J =1.2 Hz).

### <Example 13-26>

### Methyl 6-[[6-chloro-2-[3-(trifluoromethyl)phenyl]pyrazolo[1,5-a]pyridin-3-yl]methyl]pyridine-2-car boxylate

¹H-NMR (400 MHz, CDCl₃) δ 4.03 (3H, s), 4.51 (2H, s), 7.09 (1H, dd, J = 9.7, 1.5 Hz), 7.12 (1H, d, J = 7.9 Hz), 7.45 (1H, d, J = 9.7 Hz), 7.55 (1H, t, J = 7.9 Hz), 7.64 (1H, d, J = 7.9 Hz), 7.68 (2H, t, J = 7.9 Hz), 7.90 (1H, d, J =7.3 Hz), 7.97 (1H, d, J =7.3 Hz), 8.01 (1H, s), 8.53 (1H, s).

### <Example 13-27>

### Methyl 6-[[6-chloro-2-[2-(trifluoromethyl)phenyl]pyrazolo[1,5-a]pyridin-3-yl]methyl]pyridine-2-car boxylate

¹H-NMR (400 MHz, CDCl₃) δ 4.00 (3H, s), 4.22 (2H, s), 7.02 (1H, d, J = 7.9 Hz), 7.07 (1H, dd, J = 9.7, 1.8 Hz), 7.40 (1H, d, J = 7.9 Hz), 7.44 (1H, d, J = 9.7 Hz), 7.56-7.58 (2H, m), 7.63 (1H, t, J = 7.9 Hz), 7.80 (1H, dd, J = 7.9, 1.8 Hz), 7.92 (1H, d, J =7.9 Hz), 8.49 (1H, s).

### <Example 13-28>

### Methyl 6-[[6-chloro-2-(4-chlorophenyl)pyrazolo[1,5-a]pyridin-3-yl]methyl]pyridine-2-carboxylate

¹H-NMR (400 MHz, CDCl₃) δ 4.04 (3H, s), 4.51 (2H, s), 7.07 (1H, dd, J = 9.7, 1.8 Hz), 7.08 (1H, d, J = 7.9 Hz), 7.40 (2H, d, J = 8.6 Hz), 7.40 (1H, d, J = 9.7 Hz),7.64-7.68 (3H, m),7.97 (1H, dJ = 7.3 Hz), 8.51 (1H, s).

### <Example 13-29>

### Methyl 6-[[6-chloro-2-(3-chlorophenyl)pyrazolo[1,5-a]pyridin-3-yl]methyl]pyridine-2-carboxylate

¹H-NMR (400 MHz, CDCl₃) δ 4.03 (3H, s), 4.51 (2H, s), 7.07 (1H, dd, J = 9.7, 1.8 Hz), 7.10 (1H, d, J = 7.9 Hz), 7.36-7.37 (1H, m), 7.41 (1H, d, J = 9.7 Hz), 7.56-7.59 (1H, m), 7.68 (1H, t, J = 7.9 Hz), 7.77 (1H, s), 7.97 (1H, d, J = 7.9 Hz), 8.51 (1H, d, J = 1.8 Hz).

### <Example 13-30>

### Methyl 6-[[6-chloro-2-(2-chlorophenyl)pyrazolo[1,5-a]pyridin-3-yl]methyl]pyridine-2-carboxylate

¹H-NMR (400 MHz, CDCl₃) δ 4.01 (3H, s), 4.29 (2H, s), 7.06 (2H, dd, J = 9.7, 1.2 Hz), 7.33-7.36 (2H, m), 7.41 (1H, td, J = 6.7, 2.4 Hz), 7.45-7.49 (2H, m), 7.62 (1H, t, J = 7.6 Hz), 7.91 (1H, d, J = 7.3 Hz), 8.50 (1H, d, J = 1.2 Hz).

### <Example 13-31>

### Methyl 6-[[6-methoxy-2-(1-methylcyclopropyl)pyrazolo[1,5-a]pyridin-3-yl]methyl]pyridine-2-carbo xylate

¹H-NMR (400 MHz, CDCl₃) δ 0.71 (2H, dd, J = 6.1, 4.2 Hz), 0.94 (2H, dd, J = 6.1, 4.2 Hz), 1.37 (3H, s), 3.79 (3H, s), 4.04 (3H, s), 4.49 (2H, s), 6.80 (1H, dd, J = 9.7,2.4 Hz), 7.07 (1H, d, J = 7.9 Hz), 7.13 (1H, d, J = 9.7 Hz), 7.66 (1H, t, J = 7.9 Hz), 7.96 (1H, d, J =. 7.9 Hz), 7.99 (1H, d, J = 2.4 Hz).

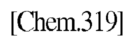

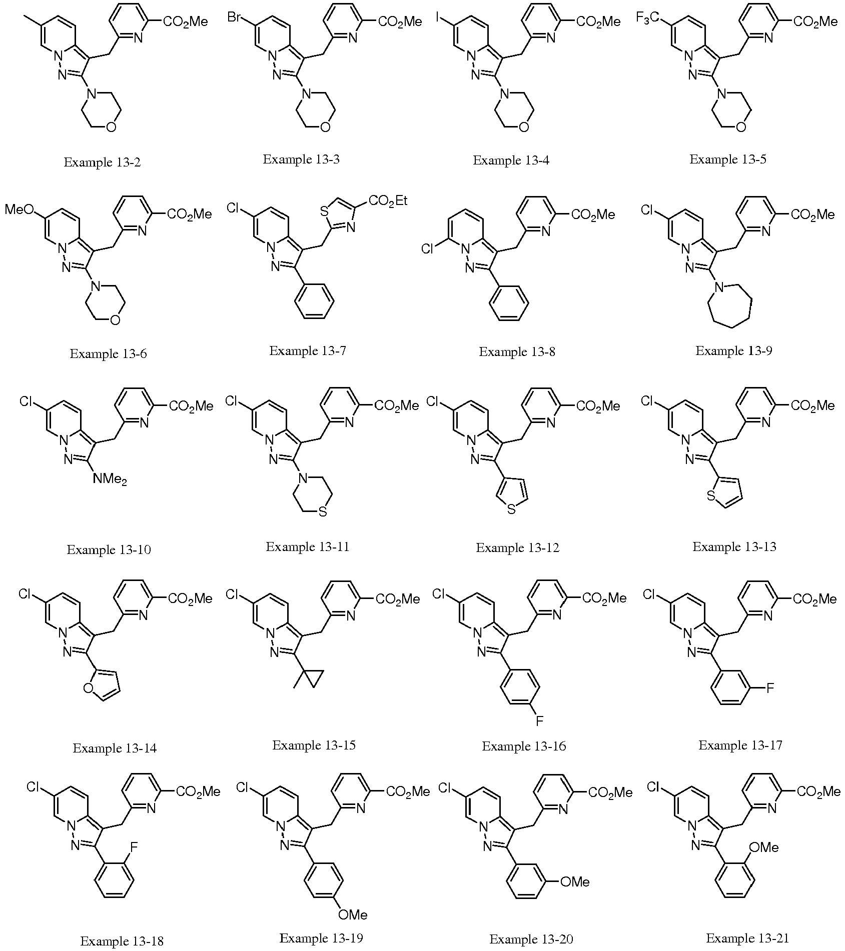

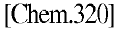

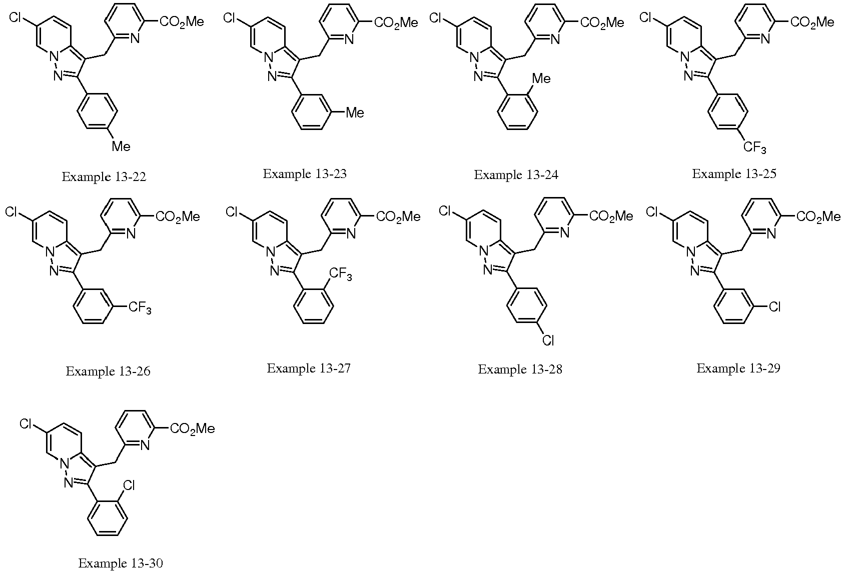

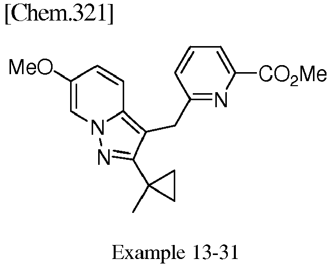

### <Example 14-1>

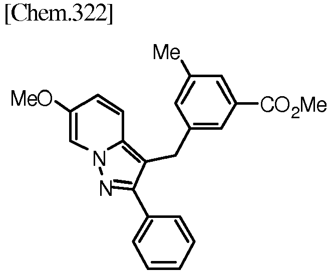

### Methyl 3-[(6-methoxy-2-phenylpyrazolo[1,5-a]pyridin-3-yl)methyl-5-methylbenzoate

Tetrabutylammonium fluoride (0.4 mL, a 1 mol/L tetrahydrofuran solution) was added to a tetrahydrofuran solution (2.0 mL) of methyl 3-[[6-methoxy-2-phenyl-7-(trimethylsilyl)pyrazolo[1,5-a]pyridin-3-yl]methyl]-5-methylbenz oate (92 mg) under an argon atmosphere under ice-cooling, and then the mixture was stirred for 1 hour under ice-cooling. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 4:1) to obtain a title compound as yellow liquid (59 mg).
¹H-NMR (400 MHz, CDCl₃) δ 2.30 (3H, s), 3.85 (3H, s), 3.87 (3H, s), 4.24 (2H, s), 6.85 (1H, dd, J = 9.7, 2.4 Hz), 7.10 (1H, d, J = 7.3 Hz), 7.11 (1H, s), 7.36 (1H, d, J = 7.3 Hz), 7.42 (2H, t, J = 7.3 Hz), 7.63-7.67 (2H, m), 7.67-7.70 (2H, m), 8.12 (1H, d, J = 1.8 Hz).

### <Example 14-2>

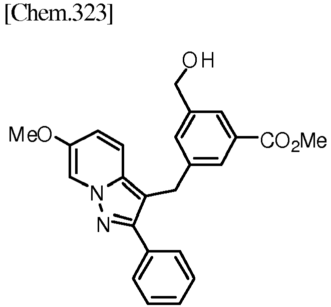

### Methyl 3-(hydroxymethyl)-5-[(6-methoxy-2-phenylpyrazolo[1,5-a]pyridin-3-yl)methyl]benzoate

Tetrabutylammonium fluoride (1.6 mL, a 1 mol/L tetrahydrofuran solution) was added to a tetrahydrofuran solution (5.2 mL) of methyl 3-[[tert-butyldimethylsilyl)oxy]methyl]-5-[[6-methoxy-2-phenyl-7-(trimethylsilyl)pyrazolo[ 1,5-a]pyridin-3-yl]methyl]benzoate (309 mg) under an argon atmosphere under ice-cooling, and then the mixture was stirred for 1 hour under ice-cooling. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 3:2) to obtain a title compound as yellow amorphous (205 mg).
¹H-NMR (400 MHz, CDCl₃) δ 3.85 (3H, s), 3.88 (3H, s), 4.28 (2H, s), 4.65 (2H, s), 6.86 (1H, dd, J = 9.7, 2.4 Hz), 7.11 (1H, d, J = 9.7 Hz), 7.30 (1H, s), 7.36 (1H, d, J = 7.3 Hz), 7.41 (2H, t, J = 7.3 Hz), 7.64 (2H, d, J = 7.3 Hz), 7.80 (1H, s), 7.87 (1H, s), 8.11 (1H, d, J = 2.4 Hz).

### <Example 14-3>

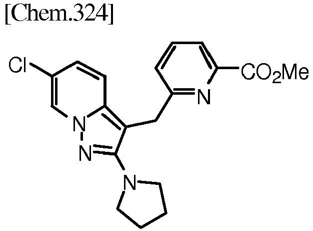

### Methyl 6-[[6-chloro-2-(pyrrolidin-1-yl)pyrazolo[1,5-a]pyridin-3-yl]methyl]pyridine-2-carboxylate

Methyl 6-[[6-chloro-2-(pyrrolidin-1-yl)-7-(trimethylsilyl)pyrazolo[1,5-a]pyridin-3-yl]methyl]pyridin e-2-carboxylate (210 mg) was dissolved in tetrahydrofuran (2.35 mL), tetrabutylammonium fluoride (0.95 mL, a 1 mol/L tetrahydrofuran solution) was added under ice-cooling, and then the mixture was stirred at the same temperature for 1 hour. A saturated ammonium chloride aqueous solution was added to the reaction solution, and then the mixture was extracted with ethyl acetate. The organic layer was washed with saturated saline and dried over anhydrous sodium sulfate. The desiccant was filtered off, the filtrate was evaporated under vacuum, and the residue thus obtained was purified by silica gel column chromatography (n-hexane:ethyl acetate = 2:1) to obtain a title compound as a yellow solid (136 mg).
¹H-NMR (400 MHz, CDCl₃) δ 1.87-1.90 (4H, m), 3.42-3.46 (4H, m), 4.03 (3H, s), 4.42 (2H, s), 6.92 (1H, dd, J = 9.1, 1.8 Hz), 7.01 (1H, d, J = 9.1 Hz), 7.14 (1H, d, J = 7.9 Hz), 7.67 (1H, dd, J = 7.9, 7.3 Hz), 7.96 (1H, d, J = 7.3 Hz), 8.30 (1H, d, J = 1.8 Hz).

### <Example 14-4>

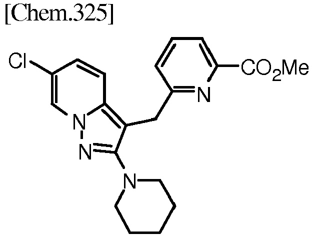

### Methyl 6-[[6-chloro-2-(piperidin-1-yl)pyrazolo[1,5-a]pyridin-3-yl]methyl]pyridine-2-carboxylate

Methyl 6-[[6-chloro-2-(piperidin-1-yl)-7-(trimethylsilyl)pyrazolo[1,5-a]pyridin-3-yl]methyl]pyridine -2-carboxylate (300 mg) was dissolved in tetrahydrofuran (3.3 mL), tetrabutylammonium fluoride (1.90 mL, a 1 mol/L tetrahydrofuran solution) was added under ice-cooling, and then the mixture was stirred at the same temperature for 1 hour. A saturated ammonium chloride aqueous solution was added to the reaction solution, and then the reaction solution was extracted with ethyl acetate. The organic layer was washed with saturated saline and dried over anhydrous sodium sulfate. The desiccant was filtered off, the filtrate was evaporated under vacuum, and the residue thus obtained was purified by silica gel column chromatography (*n*-hexane:ethyl acetate = 2:1) to obtain a title compound as a yellow oil (200 mg).
¹H-NMR (400 MHz, CDCl₃) δ 1.52-1.6 (6H, m), 3.17-3.18 (4H, m), 4.03 (3H, s), 4.34 (2H, s), 6.92 (1H, dd, J = 9.7, 1.8 Hz), 7.05 (1H, d, J = 9.7 Hz), 7.18 (1H, d, J = 7.9 Hz), 7.68 (1H, t, J = 7.3 Hz), 7.97 (1H, d, J = 7.3 Hz), 8.30 (1H, d, J = 1.8 Hz).

### <Example 14-5>

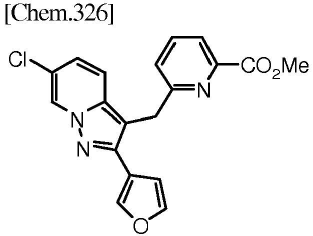

### Methyl 6-[[6-chloro-2-(furan-3-yl)pyrazolo[1,5-a]pyridin-3-yl]methyl]pyridine-2-carboxylate

Tetrabutylammonium fluoride (123 µL, a 1 mol/L tetrahydrofuran solution) was added to a tetrahydrofuran (0.6 mL) solution of methyl 6-[[6-chloro-2-(furan-3-yl)-7-(trimethylsilyl)pyrazolo[1,5-a]pyridin-3-yl]methyl]pyridine-2-c arboxylate (27.0 mg) at 0°C, and then the mixture was stirred at 0°C for 30 minutes. After the reaction was completed, a saturated ammonium chloride aqueous solution (10 mL) was added and then the mixture was extracted with ethyl acetate (20 mL) and washed with a saturated ammonium chloride aqueous solution (10 mL) and saturated saline (10 mL). The organic layer was dried over anhydrous sodium sulfate, the solvent was evaporated under vacuum, and then the residue thus obtained was purified by silica gel column chromatography (*n*-hexane:ethyl acetate = 10:0 to 6:4) to obtain a title compound as a colorless oil (20 mg).
¹H-NMR (400 MHz, CDCl₃) δ 4.04 (3H, s), 4.48 (2H, s), 6.85 (1H, d, J = 1.8 Hz), 7.05 (1H, dd, J = 9.7. 1.8 Hz), 7.08 (1H, d, J = 7.9 Hz), 7.37 (1H, d, J = 9.7 Hz), 7.48 (1H, s), 7.65 (1H, t, J = 7.6 Hz), 7.85 (1H, s), 7.97 (1H, d, J = 7.9 Hz), 8.49 (1H, s).

### <Example 14-6>

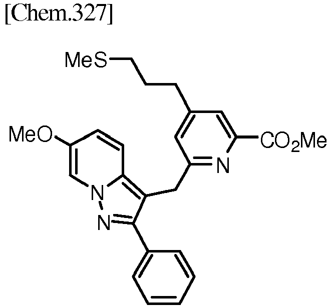

### Methyl 6-[(6-methoxy-2-phenylpyrazolo[1,5-a]pyridin-3-yl)methyl]-4-[3-(methylthio)propyl]pyridi ne-2-carboxylate

Tetrabutylammonium fluoride (0.113 mL, a 1 mol/L tetrahydrofuran solution) was added to a tetrahydrofuran (0.6 mL) solution of methyl 6-[[6-methoxy-2-phenyl-7-(trimethylsilyl)pyrazolo[1,5-a]pyridin-3-yl]methyl]-4-[3-(methylt hio)propyl]pyridine-2-carboxylate (30.0 mg) under ice-cooling, and then the mixture was stirred for 2 hours. Water was slowly added to the reaction solution and then the mixture was extracted with ethyl acetate. The organic layer was washed with saturated saline and then dried over anhydrous sodium sulfate to obtain a title compound as a pale brown oil (23.2 mg).
¹H NMR (400 MHz, CDCl₃) δ 1.71-1.79 (2H, m), 2.00 (3H, s), 2.35 (2H, t, J = 7.3 Hz), 2.60 (2H, t, J = 7.6 Hz), 3.85 (3H, s), 4.03 (3H, s), 4.52 (2H, s), 6.88 (1H, dd, J = 9.7, 1.8 Hz), 6.91 (1H, d, J =1.2 Hz), 7.24 (1H, d, J = 8.5 Hz), 7.33-7.43 (3H, m), 7.68-7.71 (2H, m), 7.80 (1H, d, J = 1.2 Hz), 8.11 (1H, d, J = 1.8 Hz).

### <Example 15-1>

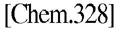

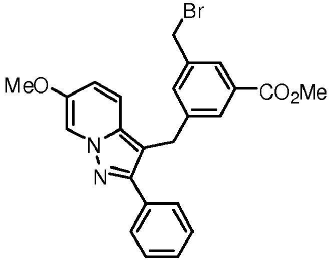

### Methyl 3-(bromomethyl)-5-[(6-methoxy-2-phenylpyrazolo[1,5-a]pyridin-3-yl)methyl]benzoate

Triphenylphosphine (136 mg) and carbon tetrabromide (202 mg) were added to a dichloromethane solution (2.2 mL) of methyl 3-(hydroxymethyl)-5-[(6-methoxy-2-phenylpyrazolo[1,5-a]pyridin-3-yl)methyl]benzoate (175 mg) at room temperature under an argon atmosphere, and then the mixture was stirred at room temperature for 1 hour. The reaction solution was evaporated and then the residue was purified by silica gel column chromatography (*n*-hexane:ethyl acetate = 2:1) to obtain a title compound as a pale yellow oil (203 mg).
¹H-NMR (400 MHz, CDCl₃) δ 3.85 (3H, s), 3.88 (3H, s), 4.27 (2H, s), 4.41 (2H, s), 6.87 (1H, dd, J = 9.7, 1.8 Hz), 7.11 (1H, d, J = 9.7 Hz), 7.32 (1H, s), 7.37 (1H, d, J = 7.3 Hz), 7.42 (2H, t, J = 7.3 Hz), 7.62 (2H, d, J = 7.3 Hz), 7.79 (1H, s), 7.90 (1H, s), 8.11 (1H, d, J = 1.8 Hz).

### <Example 16-1>

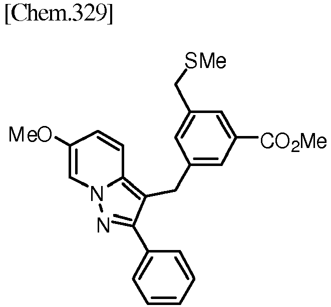

### Methyl 3-[(6-methoxy-2-phenylpyrazolo[1,5-a]pyridin-3-yl)methyl]-5-[(methylthio)methyl]benzoat e

Sodium thiomethoxide (37 mg) was added to a methanol solution (2.2 mL) of methyl 3-(bromomethyl)-5-[(6-methoxy-2-phenylpyrazolo[1,5-a]pyridin-3-yl)methyl]benzoate (203 mg) at room temperature under an argon atmosphere, and then the mixture was stirred at room temperature for 1 hour. A saturated ammonium chloride aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 2:1) to obtain a title compound as a pale yellow oil (156 mg).
¹H-NMR (400 MHz, CDCl₃) δ 1.90 (3H, s), 3.60 (2H, s), 3.85 (3H, s), 3.87 (3H, s), 4.27 (2H, s), 6.86 (1H, dd, J = 9.7, 2.4 Hz), 7.11 (1H, d, J = 9.7 Hz), 7.25 (1H, s), 7.36 (1H, d, J = 7.3 Hz), 7.41 (2H, t, J = 7.3 Hz), 7.64 (2H, d, J = 7.3 Hz), 7.75 (1H, s), 7.80 (1H, s), 8.11 (1H, d, J = 2.4 Hz).

### <Example 17-1>

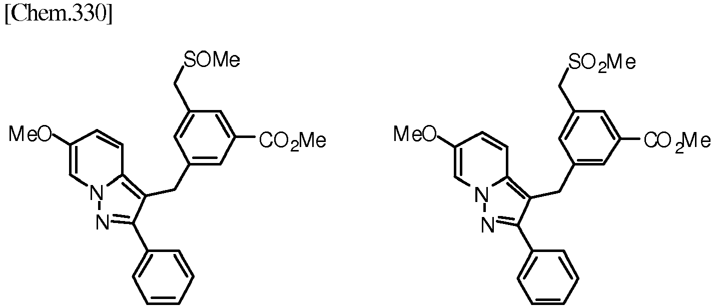

### Methyl 3-[(6-methoxy-2-phenylpyrazolo[1,5-a]pyridin-3-yl)methyl]-5-[(methylsulfinyl)methyl]benz oate

### Methyl 3-[(6-methoxy-2-phenylpyrazolo[1,5-a]pyridin-3-yl)methyl]-5-[(methylsulfonyl)methyl]ben zoate

*m*-Chloroperbenzoic acid (100 mg) was added to a dichloromethane solution (2.9 mL) of methyl 3-[(6-methoxy-2-phenylpyrazolo[1,5-a]pyridin-3-yl)methyl]-5-[(methylthio)methyl]benzoat e (125 mg)at room temperature under an argon atmosphere, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (ethyl acetate) to obtain title compounds as a pale yellow oil (61 mg) and yellow liquid (56 mg), respectively.

### Methyl 3-[(6-methoxy-2-phenylpyrazolo[1,5-a]pyridin-3-yl)methyl]-5-[(methylsulfinyl)methyl]benz oate

¹H-NMR (400 MHz, CDCl₃) δ 2.36 (3H, s), 3.85 (3H, s), 3.87-3.95 (2H, m), 3.88 (3H, s), 4.30 (2H, s), 6.88 (1H, dd, J = 9.7, 1.8 Hz), 7.14 (1H, d, J = 9.7 Hz), 7.22 (1H, s), 7.35 (1H, d, J = 7.3 Hz), 7.40 (2H, t, J = 7.3 Hz), 7.63 (2H, d, J = 7.3 Hz), 7.78 (1H, s), 7.85 (1H, s), 8.11 (1H, d, J = 1.8 Hz).

### Methyl 3-[(6-methoxy-2-phenylpyrazolo[1,5-a]pyridin-3-yl)methyl]-5-[(methylsulfonyl)methyl]ben zoate

¹H-NMR (400 MHz, CDCl₃) δ 2.65 (3H, s), 3.85 (3H, s), 3.88 (3H, s), 4.17 (2H, s), 4.31 (2H, s), 6.89 (1H, dd, J = 9.7, 1.8 Hz), 7.15 (1H, d, J = 9.7 Hz), 7.33-7.37 (2H, m), 7.41 (2H, t, J = 7.3 Hz), 7.63 (2H, d, J = 7.3 Hz), 7.89 (2H, s), 8.11 (1H, s).

### <Example 17-2>

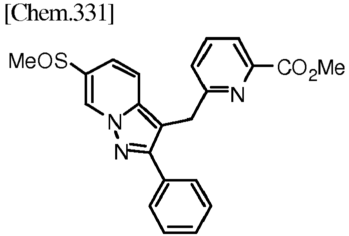

### Methyl 6-[[6-(methylsulfinyl)-2-phenylpyrazolo[1,5-a]pyridin-3-yl]methyl]pyridine-2-carboxylate

*m*-Chloroperbenzoic acid (30 mg) was added to a dichloromethane solution (1.7 mL) of methyl 6-[[6-(methylthio)-2-phenylpyrazolo[1,5-a]pyridin-3-yl]methyl]pyridine-2-carboxylate (68 mg) under an argon atmosphere under ice-cooling, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (ethyl acetate) to obtain a title compound as yellow liquid (68 mg).
¹H-NMR (400 MHz, CDCl₃) δ 2.84 (3H, s), 4.04 (3H, s), 4.57 (2H, s), 7.11 (1H, d, J = 7.9 Hz), 7.23 (1H, dd, J = 9.1, 1.8 Hz), 7.39-7.48 (3H, m), 7.60 (1H, d, J = 9.1 Hz), 7.67 (1H, t, J = 7.9 Hz), 7.73 (2H, dd, J = 7.9, 1.8 Hz), 7.97 (1H, d, J = 7.9 Hz), 8.82 (1H, d, J = 1.8 Hz).

### <Example 17-3>

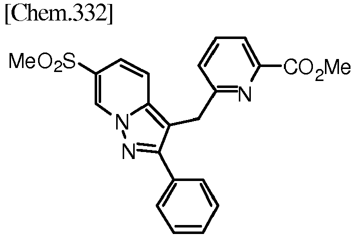

### Methyl 6-[[6-(methylsulfonyl)-2-phenylpyrazolo[1,5-a]pyridin-3-yl]methyl]pyridine-2-carboxylate

*m*-Chloroperbenzoic acid (60 mg) was added to a dichloromethane solution (1.7 mL) of methyl 6-[[6-(methylthio)-2-phenylpyrazolo[1,5-a]pyridin-3-yl]methyl]pyridine-2-carboxylate (68 mg) under an argon atmosphere under ice-cooling, and then the mixture was stirred at room temperature for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (ethyl acetate) to obtain a title compound as pale yellow liquid (47 mg).
¹H-NMR (400 MHz, CDCl₃) δ 3.14 (3H, s), 4.04 (3H, s), 4.57 (2H, s), 7.10 (1H, d, J = 7.9 Hz), 7.40 (1H, dd, J = 9.1, 1.8 Hz), 7.43-7.50 (3H, m), 7.62 (1H, dd, J = 9.1, 1.2 Hz), 7.68 (1H, t, J = 7.9 Hz), 7.72-7.75 (2H, m), 7.98 (1H, d, J = 7.9 Hz), 9.14 (1H, d, J = 1.8 Hz).

### <Example 17-4>

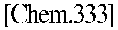

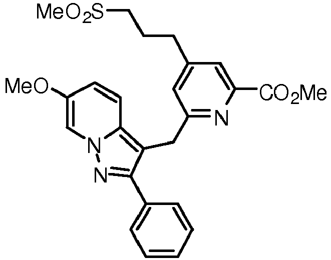

### Methyl 6-[(6-methoxy-2-phenylpyrazolo[1,5-a]pyridin-3-yl)methyl]-4-[3-(methylsulfonyl)propyl]py ridine-2-carboxylate

A dichloromethane (0.5 mL) solution of methyl 6-[[6-methoxy-2-phenylpyrazolo[1,5-a]pyridin-3-yl]methyl]-4-[3-(methylthio)propyl]pyridi ne-2-carboxylate (23.0 mg) was ice-cooled and m-chloroperbenzoic acid (24.5 mg) was added. The mixture was heated up to room temperature and stirred for 23 hours. The reaction mixed solution was purified by silica gel column chromatography (n-hexane:ethyl acetate = 5:1 to 1:4) to obtain a title compound as a colorless oil (13.1 mg).
¹H NMR (400 MHz, CDCl₃) δ 2.02-2.07 (2H, m), 2.67 (2H, t, J = 7.9 Hz), 2.80 (3H, s), 2.83 (2H, t, J = 7.9 Hz), 3.85 (3H, s), 4.04 (3H, s), 4.54 (2H, s), 6.88-6.93 (2H, m), 7.27 (1H, d, J = 9.7 Hz), 7.35 (1H, t, J = 7.3 Hz), 7.41 (2H, t, J = 7.3 Hz), 7.70 (2H, d, J = 6.7 Hz), 7.80 (1H, s), 8.11 (1H, d, J = 1.8 Hz).

### <Example 18-1>

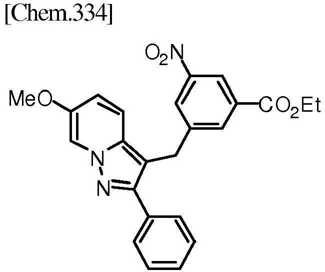

### Ethyl 3-[(6-methoxy-2-phenylpyrazolo[1,5-a]pyridin-3-yl)methyl]-5-nitrobenzoate

Triethylsilane (0.2 mL) and trifluoroacetic acid (0.1 mL) were added to a dichloromethane solution (0.8 mL) of ethyl 3-[hydroxy(6-methoxy-2-phenylpyrazolo[1,5-a]pyridin-3-yl)methyl]-5-nitrobenzoate (74 mg) at room temperature under an argon atmosphere, and then the mixture was stirred at room temperature for 16 hours. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 3:1) to obtain a title compound as yellow liquid (60 mg).
¹H-NMR (400 MHz, CDCl₃) δ 1.39 (3H, t, J = 7.3 Hz), 3.86 (3H, s), 4.37 (2H, s), 4.39 (2H, q, J = 7.3 Hz), 6.92 (1H, dd, J = 9.7, 1.8 Hz), 7.13 (1H, d, J = 9.7 Hz), 7.34-7.45 (3H, m), 7.60 (2H, d, J = 7.3 Hz), 8.09-8.14 (2H, m), 8.16 (1H, s), 8.66 (1H, s).

The following Examples 18-2 to 18-10 were obtained by using corresponding hydroxy derivatives in the same manner as Example 18-1.

### <Example 18-2>

### Methyl 6-[[6-(methylthio)-2-phenylpyrazolo[1,5-a]pyridin-3-yl]methyl]pyridine-2-carboxylate

¹H-NMR (400 MHz, CDCl₃) δ 2.50 (3H, s), 4.04 (3H, s), 4.55 (2H, s), 7.06 (1H, dd, J = 9.1, 1.8 Hz), 7.10 (1H, d, J = 7.9 Hz), 7.32 (1H, d, J = 9.1 Hz), 7.37 (1H, t, J = 7.3 Hz), 7.42 (2H, t, J = 7.3 Hz), 7.65 (1H, t, J = 7.9 Hz), 7.70 (2H, d, J = 7.3 Hz), 7.96 (1H, d, J = 7.9 Hz), 8.42 (1H, d, J = 1.8 Hz).

### <Example 18-3>

### Methyl 6-[(6-chloro-2-cyclopropylpyrazolo[1,5-a]pyridin-3-yl)methyl]pyridine-2-carboxylate

¹H NMR (400 MHz, CDCl₃) δ 0.90-1.00 (4H, m), 1.91-1.98 (1H, m), 4.03 (3H, s), 4.42 (2H, s), 6.97 (1H, dd, J = 9.5, 1.8 Hz), 7.14 (1H, d, J = 7.9 Hz), 7.28 (1H, d, J = 9.5 Hz), 7.68 (1H, dd, J = 7.9, 7.3 Hz), 7.97 (1H, d, J = 7.3 Hz), 8.34 (1H, d, J = 1.8 Hz).

### <Example 18-4>

### Methyl 6-[(6-chloro-2-cyclopentylpyrazolo[1,5-a]pyridin-3-yl)methyl]pyridine-2-carboxylate

¹H NMR (400 MHz, CDCl₃) δ 1.64-1.68 (2H, m), 1.79-1.88 (4H, m), 1.97-2.03 (2H, m), 3.19-3.27 (1H, m), 4.04 (3H, s), 4.36 (2H, s), 7.06 (1H, dd, J = 9.8, 1.8 Hz), 7.06 (1H, d, J = 7.9 Hz), 7.26 (1H, d, J = 9.8 Hz), 7.66 (1H, dd, J = 7.9, 7.3 Hz), 7.96 (1H, d, J = 7.3 Hz), 8.43 (1H, d, J = 1.8 Hz).

### <Example 18-5>

### Methyl 6-[(6-chloro-2-cyclohexylpyrazolo[1,5-a]pyridin-3-yl)methyl]pyridine-2-carboxylate

¹H NMR (400 MHz, CDCl₃) δ 1.27-1.39 (3H, m), 1.62-1.71 (3H, m), 1.81-1.83 (4H, m), 2.82 (1H, tt, J = 12.2, 3.3 Hz), 4.04 (3H, s), 4.36 (2H, s), 6.97 (1H, dd, J = 9.2, 1.8 Hz), 7.05 (1H, d, J = 7.3 Hz), 7.26 (1H, d, J = 9.2 Hz), 7.66 (1H, dd, J = 7.9, 7.3 Hz), 7.69 (1H, d, J = 7.9 Hz), 8.43 (1H, d, J = 1.8 Hz).

### <Example 18-6>

### Methyl 6-[(2-tert-butyl-6-chloropyrazolo[1,5-a]pyridin-3-yl)methyl]pyridine-2-carboxylate

¹H NMR (400 MHz, CDCl₃) δ 1.43 (9H, s), 4.08 (3H, s), 4.58 (2H, s), 7.98 (1H, dd, J = 9.7, 1.8 Hz), 7.00 (1H, d, J = 7.9 Hz), 7.16 (1H, d, J = 9.7 Hz), 7.67 (1H, dd, J = 7.9, 7.3 Hz), 8.00 (1H, d, J = 7.3 Hz), 8.48 (1H, d, J = 1.8 Hz).

### <Example 18-7>

### Methyl 6-[[6-chloro-2-(2-methylpropyl)pyrazolo[1,5-a]pyridin-3-yl]methyl]pyridine-2-carboxylate

¹H NMR (400 MHz, CDCl₃) δ 0.97 (6H, d, J = 6.7 Hz), 1.97-2.08 (1H, m), 2.64 (2H, d, J = 7.9 Hz), 4.03 (3H, s), 4.34 (2H, s), 6.98 (1H, dd, J = 9.1, 1.8 Hz), 7.05 (1H, d, J = 7.9 Hz), 7.27 (1H, d, J = 9.1 Hz), 7.66 (1H, dd, J = 7.9, 7.3 Hz), 7.97 (1H, d, J = 7.3 Hz), 8.41 (1H, d, J = 1.8 Hz).

### <Example 18-8>

### Methyl 6-[[2-(2-fluorophenyl)-6-methoxypyrazolo[1,5-a]pyridin-3-yl]methyl]pyridine-2-carboxylate

¹H NMR (400 MHz, CDCl₃) δ 3.83 (3H, s), 4.02 (3H, s), 4.37 (2H, s), 6.87 (1H, dd, J = 9.5, 2.1 Hz), 7.12-7.29 (4H, m), 7.35-7.41 (1H, m), 7.57 (1H, td, J = 7.6, 1.8 Hz), 7.63 (1H, t, J = 7.6 Hz), 7.93 (1H, d, J = 6.7 Hz), 8.09 (1H, d, J =1.2 Hz).

### <Example 18-9>

### Methyl 6-[[2-(3-fluorophenyl)-6-methoxypyrazolo[1,5-a]pyridin-3-yl]methyl]pyridine-2-carboxylate

¹H NMR (400 MHz, CDCl₃) δ 3.85 (3H, s), 4.04 (3H, s), 4.53 (2H, s), 6.90 (1H, dd, J = 9.7, 2.4 Hz), 7.05 (1H, tdd, J = 8.5, 2.4, 1.2 Hz), 7.12 (1H, d, J = 7.9 Hz), 7.29 (1H, d, J = 9.7 Hz), 7.36 (1H, td, J = 8.5, 6.1 Hz), 7.47 (1H, dd, J = 6.1, 1.2 Hz), 7.49-7.52 (1H, m), 7.66 (1H, t, J = 7.9 Hz), 7.97 (1H, d, J = 7.3 Hz), 8.09 (1H, d, J = 1.8 Hz).

### <Example 18-10>

### Methyl 6-[(6-chloro-5-methyl-2-phenylpyrazolo[1,5-a]pyridin-3-yl)methyl]pyridine-2-carboxylate

¹H NMR (400 MHz, CDCl₃) δ 2.37 (3H, s), 4.04 (3H, s), 4.51 (2H, s), 7.09 (1H, d, J = 7.9 Hz), 7.28 (1H, s), 7.35-7.45 (3H, m), 7.63-7.70 (3H, m), 7.97 (1H, d, J = 7.9 Hz), 8.53 (1H, s).

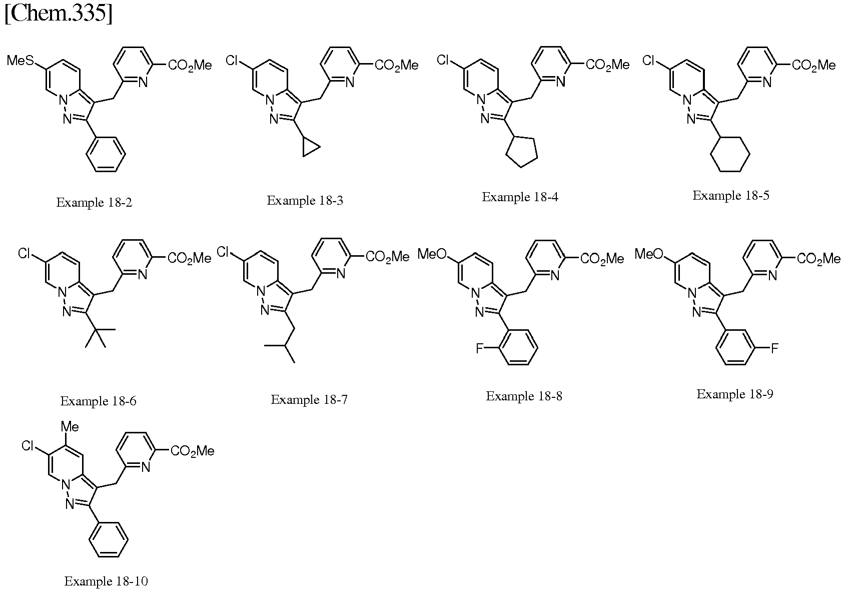

### <Example 19-1>

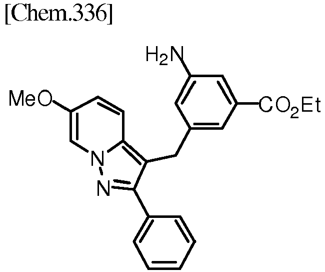

### Ethyl 3-amino-5-[(6-methoxy-2-phenylpyrazolo[1,5-a]pyridin-3-yl)methyl]benzoate

Iron powder (26 mg) was added to an acetic acid-ethanol mixed solution (1.0 mL, 10:9) of ethyl 3-[(6-methoxy-2-phenylpyrazolo[1,5-a]pyridin-3-yl)methyl]-5-nitrobenzoate (40 mg) at room temperature under an argon atmosphere, and then the mixture was stirred at 80°C for 1 hour. A saturated sodium bicarbonate aqueous solution was added to the reaction solution, and then the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 2:1) to obtain a title compound as colorless liquid (29 mg).
¹H-NMR (400 MHz, CDCl₃) δ 1.36 (3H, t, J = 7.3 Hz), 3.67 (2H, brs), 3.85 (3H, s), 4.19 (2H, s), 4.33 (2H, q, J = 7.3 Hz), 6.56 (1H, s), 6.86 (1H, dd, J = 9.7, 1.8 Hz), 7.14 (1H, d, J = 9.7 Hz), 7.18 (1H, s), 7.34 (1H, s), 7.37 (1H, d, J = 7.3 Hz), 7.42 (2H, t, J = 7.3 Hz), 7.67 (2H, d, J = 7.3 Hz), 8.11 (1H, d, J = 1.8 Hz).

### <Example 20-1>

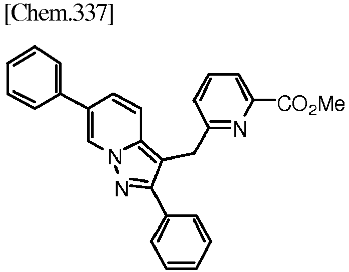

### Methyl 6-[(2,6-(diphenylpyrazolo[1,5-a]pyridin-3-yl)methyl]pyridine-2-carboxylate

Phenylboronic acid (22 mg), a 2 mol/L sodium carbonate aqueous solution (0.2 mL), and dichlorobis(triphenylphosphine)palladium (II) (8 mg) were added to a dimethoxyethane solution (0.4 mL) of methyl 6-[(6-bromo-2-phenylpyrazolo[1,5-a]pyridin-3-yl)methyl]pyridine-2-carboxylate (50 mg) at room temperature under an argon atmosphere, and then the mixture was stirred at 60°C for 2 hours. Water was added to the reaction solution and the mixture was extracted with ethyl acetate. The organic layer was washed with saturated saline and then dried over anhydrous magnesium sulfate. The solvent was evaporated and then the residue was purified by silica gel column chromatography (n-hexane:ethyl acetate = 4:1) to obtain a title compound as yellow liquid (38 mg).
¹H-NMR (400 MHz, CDCl₃) δ 4.05 (3H, s), 4.60 (2H, s), 6.83 (1H, d, J = 7.9 Hz), 7.16 (1H, d, J = 7.9 Hz), 7.23 (2H, d, J = 7.3 Hz), 7.34-7.39 (2H, m), 7.45-7.49 (4H, m), 7.58-7.61 (4H, m), 7.97 (1H, d, J = 7.9 Hz), 8.72 (1H, s).

### <Example 21-1>

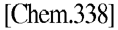

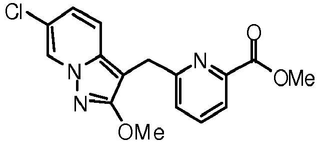

### Methyl 6-[(6-chloro-2-methoxypyrazolo[1,5-a]pyridin-3-yl)methyl]pyridine-2-carboxylate

Potassium carbonate (33 mg) and iodomethane (0.029 mL) were added to an *N,N*-dimethylformamide solution (0.785 mL) of methyl 6-[(6-chloro-2-hydroxypyrazolo[1,5-a]pyridin-3-yl)methyl]pyridine-2-carboxylate (50 mg), and then the mixture was stirred at room temperature for 2 hours. A saturated ammonium chloride aqueous solution was added to the reaction solution to achieve about pH 7 and the mixture was extracted twice with ethyl acetate. The combined ethyl acetate layer was washed with saturated saline and dried over anhydrous sodium sulfate, and then the solvent was evaporated under vacuum. The residue thus obtained was purified by column chromatography (n-hexane:ethyl acetate = 88:12 to 0:100) to obtain a title compound as a colorless solid (46 mg).
¹H NMR (400 MHz, CDCl₃) δ 4.02 (3H, s), 4.05 (3H, s), 4.22 (2H, s), 6.97 (1H, dd, J = 9.6, 1.9 Hz), 7.21-7.26 (2H, m), 7.69 (1H, t, J = 7.6 Hz), 7.96 (1H, d, J = 7.6 Hz), 8.26 (1H, d, J = 1.9 Hz).

### <Example 21-2>

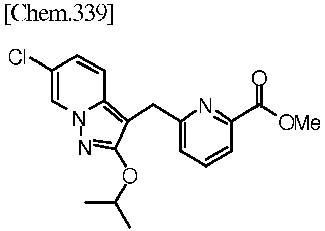

### Methyl 6-[[6-chloro-2-(2-propyloxy)pyrazolo[1,5-a]pyridin-3-yl]methyl]pyridine-2-carboxylate

After potassium carbonate (82.0 mg) and 2-iodopropane (43 µL) were added to an *N,N*-dimethylformamide solution (1.5 mL) of methyl 6-[[5-chloro-2-hydroxypyrazolo[1,5-a]pyridin-3-yl]methyl]pyridine-2-carboxylate (260 mg) under an argon atmosphere, and then the mixture was stirred at room temperature for 2 hours. The reaction solution was diluted with ethyl acetate, then washed with water and saturated saline, and then dried over anhydrous sodium sulfate. The solvent was evaporated and the residue thus obtained was purified by silica gel chromatography (n-hexane:ethyl acetate = 2: 1) to obtain a title compound as a pale yellow oil (106 mg).
¹H-NMR (400 MHz, CDCl₃) δ 1.37 (6H, d, J = 6.1 Hz), 4.02 (3H, s), 4.21 (2H, s), 5.00-5.12 (1H, m), 6.94 (1H, dd, J = 9.2, 1.8 Hz), 7.21 (1H, d, J = 9.2 Hz), 7.25 (1H, d, J = 7.9 Hz), 7.68 (1H, t, J = 7.9 Hz), 7.94 (1H, d, J = 7.9 Hz), 8.24 (1H, d, J = 1.8Hz).

### <Example 22-1>

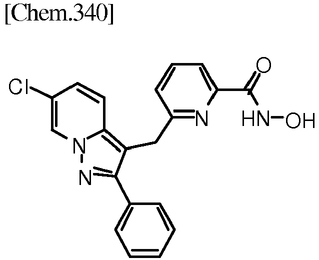

### 6-[(6-Chloro-2-phenylpyrazolo[1,5-a]pyridin-3-yl)methyl]-N-hydroxypyridine-2-carboxami de

Lithium hydroxide monohydrate (267 mg) was added to a methanol solution (1.30 mL) of hydroxylamine hydrochloride (221 mg), and then the mixture was stirred at room temperature for 30 minutes. A methanol solution (1.29 mL) of methyl 6-[(6-chloro-2-phenylpyrazolo[1,5-a]pyridin-3-yl)methyl]pyridine-2-carboxylate (120 mg) was added, and then the mixture was stirred at room temperature for 8 hours. A 1 mol/L hydrochloric acid was added to the reaction solution to achieve the range between pH 1 and pH 2 and then the mixture was extracted twice with ethyl acetate. The combined ethyl acetate layer was washed with water and saturated saline and dried over anhydrous sodium sulfate, and then the solvent was evaporated under vacuum. The residue thus obtained was purified with triturate (diisopropyl ether:ethyl acetate = 1:1) to obtain a title compound as a colorless solid (90 mg).
¹H-NMR (400 MHz, DMSO- d₆ δ 4.44 (2H, s), 7.16 (1H, d, J = 7.3 Hz), 7.26 (1H, dd, J = 9.8, 1.8 Hz), 7.40-7.47 (3H, m), 7.72-7.82 (5H, m), 9.02 (1H, s), 9.13 (1H, s), 10.98 (1H, s).

### <Example 23-1>

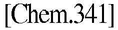

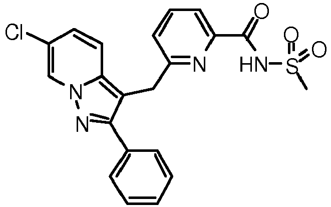

### 6-[(6-Chloro-2-phenylpyrazolo[1,5-a]pyridin-3-yl)methyl]-N-(methylsulfonyl)pyridine-2-car boxamide

Methanesulfonamide (131 mg), 4-(dimethylamino)pyridine (40 mg), and 1-ethyl-3-(3-dimethylaminopropyl)carbodiimide (EDCI) (63 mg) were added to an N,N-dimethylformamide solution (1.37 mL) of 6-[(6-chloro-2-phenylpyrazolo[1,5-a]pyridin-3-yl)methyl]pyridine-2-carboxylic acid (100 mg) under ice-cooling, and then the mixture was stirred at room temperature for 2 hours. A 1 mol/L hydrochloric acid was added to the reaction solution to achieve about pH 4 and the mixture was extracted twice with ethyl acetate. The combined ethyl acetate layer was washed with saturated saline and dried over anhydrous sodium sulfate, and then the solvent was evaporated under vacuum. The residue thus obtained was purified with triturate (diisopropyl ether:ethyl acetate =1:3) to obtain a title compound as a colorless solid (44 mg).
¹H-NMR (400 MHz, DMSO- d₆) δ 3.37 (3H, s), 4.51 (2H, s), 7.28 (1H, dd, J = 9.2, 1.8 Hz), 7.35-7.42 (2H, m), 7.46 (2H, t, J = 7.3 Hz), 7.76-7.79 (2H, m), 7.83-7.91 (3H, m), 9.04 (1H, d, J = 1.8 Hz), 11.26 (1H, s).

### <Example 23-2>

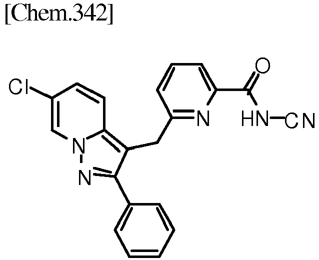

### 6-[(6-Chloro-2-phenylpylazolo[1,5-a]pyridin-3-yl)methyl]-N-cyanopyridine-2-carboxamide

Cyanamide (58 mg), 4-(dimethylamino)pyridine (40 mg), and 1-ethyl-3-(3-dimethylaminopropyl)carbodiimide (EDCI) (63 mg) were added to an N,N-dimethylformamide solution (1.37 mL) of 6-[(6-chloro-2-phenylpyrazolo[1,5-a]pyridin-3-yl)methyl]pyridine-2-carboxylic acid (100 mg) under ice-cooling, and then the mixture was stirred at room temperature for 2 hours. A 1 mol/L hydrochloric acid was added to the reaction solution to achieve the range between pH 2 and pH 3 and then the mixture was extracted twice with ethyl acetate. The combined ethyl acetate layer was washed with saturated saline and dried over anhydrous sodium sulfate, and then the solvent was evaporated under vacuum. The residue thus obtained was purified by column chromatography (ethyl acetate:methanol = 96:4 to 66:34) and with triturate (diisopropyl ether:ethyl acetate = 1:1) to obtain a title compound as a yellow amorphous (33 mg).
¹H-NMR (400 MHz, DMSO-d₆) δ 4.37 (2H, s), 7.01 (1H, d, J = 7.6 Hz), 7.23 (1H, dd, J = 9.4, 1.7 Hz), 7.40 (1H, t, J = 7.3 Hz), 7.43-7.47 (3H, m), 7.61 (1H, s), 7.74 (1H, s), 7.81 (2H, s), 9.04 (1H, s).

### <Example 24-1>

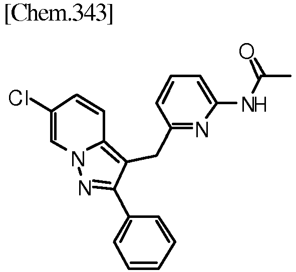

### [6-[(6-chloro-2-phenylpyrazolo[1,5-a]pyridin-3-yl)methyl]pyridin-2-yl]acetamide

Acetic anhydride (0.057 mL) was added to a tetrahydrofuran solution (1.49 mL) of 3-(6-aminopyridin-2-yl)methyl-6-chloro-2-phenylpyrazolo[1,5-a]pyridine (100 mg), and then the mixture was stirred at room temperature for 23 hours. Water was added to the reaction solution and the mixture was extracted twice with ethyl acetate. The combined ethyl acetate layer was washed with saturated sodium bicarbonate aqueous solution and saturated saline and dried over anhydrous sodium sulfate, and then the solvent was evaporated under vacuum. The residue thus obtained was purified with triturate (diisopropyl ether:ethyl acetate = 3:1) to obtain a title compound as a colorless solid (98 mg).
¹H-NMR (400 MHz, CDCl₃) δ 2.06 (3H, s), 4.30 (2H, s), 6.74 (1H, d, J = 7.6 Hz), 7.25 (1H, dd, J = 9.6, 1.9 Hz), 7.37-7.41 (1H, m), 7.43-7.47 (2H, m), 7.59-7.63 (2H, m), 7.70-7.72 (2H, m), 7.88 (1H, d, J = 8.4 Hz), 9.04-9.04 (1H, m), 10.37 (1H, s).

### <Example 25-1>

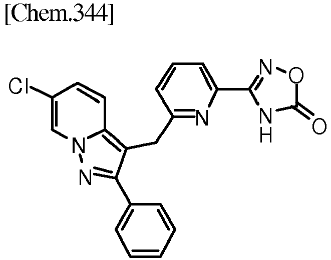

### 3-[6-[(6-Chloro-2-phenylpyrazolo[1,5-a]pyridin-3-yl)methyl]pyridin-2-yl]-1,2,4-oxadiazole-5(4H)-one

Pyridine (0.028 mL) and ethyl chloroformate (0.030 mL) were added to an *N,N*-dimethylformamide solution (1.33 mL) of 6-[(6-chloro-2-phenylpyrazolo[1,5-a]pyridin-3-yl)methyl]-*N*-hydroxypyridine-2-carboxamid ine (100 mg) under ice-cooling, and then the mixture was stirred at the same temperature for 1.5 hours. Water was added to the reaction solution and the mixture was extracted twice with ethyl acetate. The combined ethyl acetate layer was washed with water and saturated saline and dried over anhydrous sodium sulfate, and then the solvent was evaporated under vacuum. Xylene (1.33 mL) and DBU (0.040 mL) were added to the residue thus obtained, and then the mixture was stirred at 80°C for 2 hours. A 1 mol/L hydrochloric acid was added to the reaction solution to achieve the range between pH 1 and pH 2 and the mixture was extracted twice with ethyl acetate. The combined ethyl acetate layer was washed with water and saturated saline and dried over anhydrous sodium sulfate, and then the solvent was evaporated under vacuum. The residue thus obtained was purified with triturate (diisopropyl ether:ethyl acetate = 2:1) to obtain a title compound as a colorless solid (75 mg).
¹H-NMR (400 MHz, DMSO-d₆) δ 4.48 (2H, s), 7.19 (1H, d, J = 8.6 Hz), 7.27 (1H, dd, J =9.2, 1.8 Hz), 7.37-7.46 (3H, m), 7.72-7.78 (4H, m), 7.84 (1H, t, J = 7.6 Hz), 9.05 (1H, d, J = 1.8 Hz).

### <Example 26-1>

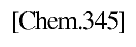

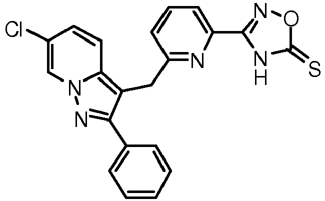

### 3-[6-[(6-Chloro-2-phenylpyrazolo[1,5-a]pyridin-3-yl)methyl]pyridin-2-yl]-1,2,4-oxadiazole-5(4H)-thione

Thiocarbonyldiimidazole (21 mg) and DBU (0.047 mL) were added to an acetonitrile solution (0.397 mL) of 6-[(6-chloro-2-phenylpyrazolo[1,5-a]pyridin-3-yl)methyl]-*N*-hydroxypyridine-2-carboxamid ine (30 mg), and then the mixture was stirred at room temperature for 30 minutes. A 1 mol/L hydrochloric acid was added to the reaction solution to achieve the range between pH 1 and pH 2 and then the mixture was extracted twice with ethyl acetate. The combined ethyl acetate layer was washed with saturated saline and dried over anhydrous sodium sulfate, and then the solvent was evaporated under vacuum. The residue thus obtained was triturated (diisopropyl ether:ethyl acetate = 1:1) to obtain a title compound as a colorless solid (30 mg).
¹H-NMR (400 MHz, DMSO-d₆) δ 4.48 (2H, s), 7.23 (1H, d, J = 6.7 Hz), 7.27 (1H, dd, J = 9.8, 1.8 Hz), 7.37-7.46 (3H, m), 7.75-7.88 (5H, m), 9.05 (1H, d, J = 1.2 Hz).

### <Example 27-1>

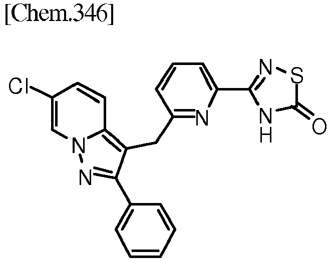

### 3-[6-[(6-Chloro-2-phenylpyrazolo[1,5-a]pyridin-3-yl)methyl]pyridin-2-yl]-1,2,4-thiadiazole-5(4H)-one

Thiocarbonyldiimidazole (35 mg) was added to a tetrahydrofuran solution (0.660 mL) of 6-[(6-chloro-2-phenylpylazolo[1,5-a]pyridin-3-yl)methyl]-N-hydroxypyridine-2-carboxamid ine (50 mg), and then the mixture was stirred at room temperature for 1 hour. Water was added to the reaction solution and the mixture was extracted twice with ethyl acetate. The combined ethyl acetate layer was washed with water and saturated saline and dried over anhydrous sodium sulfate and then the solvent was evaporated under vacuum. Tetrahydrofuran (1.32 mL) and a boron trifluoride-ethyl ether complex (0.050 mL) were added to the residue thus obtained, and then the mixture was stirred at room temperature for 22 hours. A 1 mol/L hydrochloric acid was added to the reaction solution to achieve the range between pH 2 and pH 3 and the mixture was extracted twice with ethyl acetate. The combined ethyl acetate layer was washed with water and saturated saline and dried over anhydrous sodium sulfate and then the solvent was evaporated under vacuum. The residue thus obtained was purified with triturate (diisopropyl ether:ethyl acetate = 1:2) to obtain a title compound as a pale yellow solid (31 mg).
¹H-NMR (400 MHz, DMSO-d₆) δ 4.49 (2H, s), 7.10 (1H, d, J = 7.9 Hz), 7.27 (1H, dd, J = 9.8, 1.8 Hz), 7.37-7.47 (3H, m), 7.75-7.82 (4H, m), 7.89 (1H, d, J = 6.7 Hz), 9.04 (1H, d, J = 1.8 Hz).

### <Example 28-1>

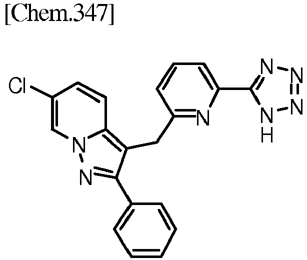

### 6-Chloro-2-phenyl-3-[[6-(1H-tetrazol-5-yl)pyridin-2-yl]methyl]pylazolo[1,5-a]pyridine

Zinc bromide (69 mg) and sodium azide (30 mg) were added to an *N,N*-dimethylformamide-aqueous solution (3:1, 2.90 mL) of 6-[(6-chloro-2-phenylpyrazolo[1,5-a]pyridin-3-yl)methyl]pyridine-2-carbonitrile (100 mg), and then the mixture was stirred at 80°C for 22 hours. A 1 mol/L hydrochloric acid was added to the reaction solution to achieve the range between pH 1 and pH 2 and then the mixture was extracted twice with ethyl acetate. The combined ethyl acetate layer was washed with water and saturated saline and dried over anhydrous sodium sulfate and then the solvent was evaporated under vacuum. The residue thus obtained was purified with triturate (diisopropyl ether:ethyl acetate = 3:1) to obtain a title compound as a colorless solid (79 mg).
¹H-NMR (400 MHz, DMSO-d₆) δ 4.55 (2H, s), 7.16 (1H, d, J = 7.6 Hz), 7.29 (1H, dd, J = 9.4, 1.7 Hz), 7.39 (1H, t, J = 7.3 Hz), 7.44 (2H, t, J = 7.3 Hz), 7.74 (2H, d, J = 6.9 Hz), 7.78 (1H, d, J = 9.6 Hz), 7.89 (1H, t, J = 7.8 Hz), 8.03 (1H, d, J = 6.9 Hz), 9.08-9.09 (1H, m).

### <Example 29-1>

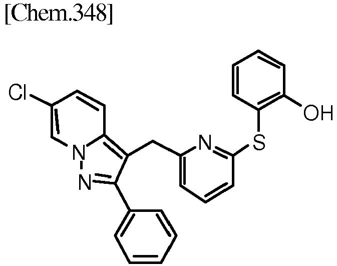

### 2-[6-[(6-Chloro-2-phenylpyrazolo[1,5-a]pyridin-3-yl)methyl]-2-pyridylthio]phenol

Trifluoroacetic acid (0.729 g) was added to a dichloromethane (2.5 mL) solution of 6-chloro-3-[hydroxy[6-[2-(methoxymethoxy)phenylthio]pyridin-2-yl]methyl]-2-phenylpyra zolo[1,5-a]pyridine (0.269 g), and then the mixture was stirred at room temperature for 3 hours. Subsequently, triethylsilane (0.372 g) was added, the mixture was stirred at the same temperature for 2 hours and then at 40°C for 30 minutes. The reaction solution was cooled down to room temperature and then a saturated sodium bicarbonate aqueous solution and ethyl acetate were added to the reaction solution to separate it. The organic layer was washed with saturated saline and dried over anhydrous sodium sulfate, and then the solvent was evaporated under vacuum. The residue thus obtained was washed with ethyl acetate to obtain a title compound as a colorless crystal (0.147 g).
¹H-NMR (400 MHz, CDCl₃) δ 4.36 (2H, s), 6.72 (1H, d, J = 8.6 Hz), 6.87-6.93 (1H, m), 6.97 (1H, dd, J = 8.6, 1.8 Hz), 7.05 (2H, d, J = 8.6 Hz), 7.24-7.29 (1H, m), 7.35-7.43 (5H, m), 7.49 (1H, dd, J = 8.6,1.8 Hz), 7.60-7.65 (2H, m), 8.50 (1H, d, J = 1.8 Hz), 9.73 (1H, s).

### <Example 29-2>

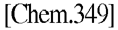

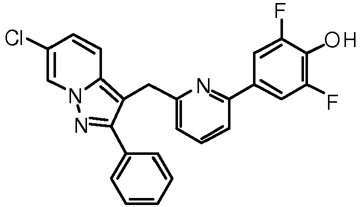

### 4-[6-[(6-Chloro-2-phenylpyrazolo[1,5-a]pyridin-3-yl)methyl]-2-pyridyl]-2,6-difluorophenol

Trifluoroacetic acid (1.08 g) was added to a dichloromethane (3 mL) solution of 6-chloro-3-[hydroxy[6-[3,5-difluoro-4-(methoxymethoxy)phenyl]pyridin-2-yl]methyl]-2-ph enylpyrazolo[1,5-a]pyridine (0.479 g), and then the mixture was stirred at room temperature for 3 hours. Subsequently, triethylsilane (0.550 g) was added, then the mixture was stirred at the same temperature for 16 hours and then at 40°C for 3 hours. The reaction solution was cooled down to room temperature and then a saturated sodium bicarbonate aqueous solution and ethyl acetate were added to the reaction solution to separate it. The organic layer was washed with saturated saline and dried over anhydrous sodium sulfate, and then the solvent was evaporated under vacuum. The residue thus obtained was washed with ethyl acetate to obtain a title compound as a colorless crystal (0.340 g).
¹H-NMR (400 MHz, DMSO-d₆) δ 4.42 (2H, s), 7.17 (1H, dd, J = 8.6, 1.8 Hz), 7.28 (1H, dd, J = 8.6, 1.8 Hz), 7.39-7.50 (3H, m), 7.65-7.75 (4H, m), 7.83 (1H, d, J = 8.6 Hz), 7.87-7.93 (2H, m), 9.02 (1H, s), 10.50 (1H, br).

### <Example 30-1>

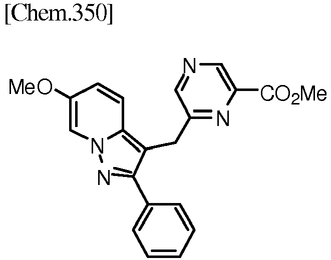

### Methyl 6-[(6-methoxy-2-phenylpyrazolo[1,5-a]pyridin-3-yl)methyl]pyrazine-2-carboxylate

Molybdenum hexacarbonyl (110 mg), palladium acetate (6.2 mg), (±)-2,2'-bis(diphenylphosphino)-1,1'-binaphthyl (17.4 mg), and cesium carbonate (90.9 mg) were added to a mixed solution of methanol (1.4 mL) and toluene (1.4 mL) of 3-[(6-chloropyrazin-2-yl)methyl]-6-methoxy-2-phenylpyrazolo[1,5-a]pyridine (97.8 mg) under an argon atmosphere, and then the mixture was stirred at 60°C for 3 hours. The reaction solution was purified by silica gel column chromatography (*n*-hexane:ethyl acetate = 10:1 1 to 2:1) to obtain a title compound as a colorless oil (23.0 mg).
¹H NMR (400 MHz, CDCl₃) δ 3.84 (3H, s), 4.05 (3H, s), 4.54 (2H, s), 6.93 (1H, dd, J = 9.7, 2.4 Hz), 7.33 (1H, d, J = 9.7 Hz), 7.37 (1H, tt, J = 7.3, 1.2 Hz), 7.42-7.45 (2H, m), 7.68 (2H, dd, J = 7.9, 1.2 Hz), 8.10 (1H, d, J = 2.4 Hz), 8.40 (1H, s), 9.10 (1H, s).

Next, results supporting usefulness as to the compound of the present invention will be shown with reference to Test Examples.

### <Test Example 1>

### Test for confirming EP₁ receptor antagonistic effect

### (1) Preparation of rat EP₁ expression vector

The first PCR was performed with Rat Kidney BD Marathon-Ready cDNA (Nippon Becton Dickinson Co., Ltd.) as a template using a forward primer represented by SEQ ID NO: 1 and a reverse primer represented by SEQ ID NO: 2 and using KOD-Plus-Ver 2.0 (Toyobo Co., Ltd.). The second PCR was further performed in a similar manner with this amplification product as a template using a forward primer represented by SEQ ID NO: 3 and a reverse primer represented by SEQ ID NO: 4. The amplification product obtained by the second PCR was incorporated into a vector (pcDNA3.1 D/V5-His-TOPO (registered trademark), Invitrogen Corporation). This vector with the incorporated amplification product was introduced to E. coli (One Shot TOP10 Competent Cell, Invitrogen Corporation) by a conventional method to perform transformation. This transformed E. coli was cultured for 1 day in an LB agar medium. After the culture, colonies were selected and cultured in an LB liquid medium containing 50 µg/mL of ampicillin. After the culture, the vector was purified using QIAprep Spin Miniprep Kit (Qiagen Corporation). The base sequence (SEQ ID NO: 5) of the inserted site in this vector was compared with the base sequence (Ptger1) of rat EP₁ registered under Accession No. NM_013100 in a heretofore known database (NCBI) and consequently was completely identical except for 1 base. Moreover, an amino acid sequence translated from this base sequence was completely identical to the amino acid sequence of the rat EP₁ receptor registered under Accession No. NP_037232 in NCBI. Thus, it was confirmed that the cloned gene sequence was the base sequence of the rat EP₁ receptor and the obtained amino acid sequence was the rat EP₁ receptor. The pcDNA3.1 D/V5-His-TOPO (registered trademark) in which the nucleic acid represented by SEQ ID NO: 5 was inserted was used as a rat EP₁ expression vector.

### (2) Preparation of rat EP₁ receptor-expressing cell

### (2-1) COS-1 cell culture

COS-1 cells (Dainippon Sumitomo Pharma Co., Ltd.) were cultured at 37°C in an incubator under 5% CO₂ gas conditions using a D-MEM liquid medium (containing high glucose and L-glutamine, Invitrogen Corporation) supplemented with a penicillin-streptomycin solution (Invitrogen Corporation, final concentration: 100 U/mL as benzylpenicillin; 100 µg/mL as streptomycin) as antibiotics, MEM non-essential amino acids (Invitrogen Corporation, final concentration: 0.1 mM), and fetal bovine serum (Moregate Biotech, final concentration: 10%), until reaching confluence.

### (2-2) Subculture of COS-1 cell

The cells that reached confluence were dissociated with 0.05% trypsin/0.53 mM EDTA·4Na (Invitrogen Corporation) and resuspended in the liquid medium. The resuspended cells were diluted with the liquid medium so that the spread ratio became 1:4 to 1:8, and were cultured.

### (2-3) Preparation of cell for rat EP₁ expression vector introduction

The cells that reached confluence were dissociated with 0.05% trypsin/0.53 mM EDTA·4Na and resuspended in a D-MEM liquid medium (containing high glucose and L-glutamine, Invitrogen Corporation) supplemented with MEM non-essential amino acids (final concentration: 0.1 mM) and fetal bovine serum (final concentration: 10%). This resuspended cell suspension was prepared with a liquid medium so as to become the number of cells 5 x 10⁴ cells/100 µL of the liquid medium/well in each well of a poly-D-lysine-coated 96-well microplate (BD BioCoat (registered trademark), Nippon Becton Dickinson Co., Ltd.), and this cell preparation was dispensed at 100 µL to each well and seeded. After the seeding, the cells were cultured at 37°C in an incubator under 5% CO₂ gas conditions. The introduction of the rat EP₁ expression vector was performed by procedures shown below at the point in time when the cells for introduction of this rat EP₁ expression vector adhered (approximately 2 hours after the seeding).

### (2-4) Rat EP₁ expression vector introduction

Lipofectamine 2000 (Invitrogen Corporation) was used for the introduction of the rat EP₁ expression vector. The rat EP₁ expression vector was diluted with OPTI-MEM (registered trademark) I Reduced-Serum Medium (Invitrogen Corporation) so as to become 200 ng/25 µL/well. At the same time, Lipofectamine 2000 (Invitrogen Corporation) was diluted with OPTI-MEM (registered trademark) I Reduced-Serum Medium (Invitrogen Corporation) so as to become 0.5 µL/25 µL/well and incubated at room temperature for 5 minutes. After the incubation for 5 minutes, for the complex formation of rat EP₁ expression vector/Lipofectamine 2000, the diluted rat EP₁ expression vector and the diluted Lipofectamine 2000 were mixed and incubated at room temperature for 30 minutes. After the incubation for 30 minutes, the complex of rat EP₁ expression vector/Lipofectamine 2000 was dispensed at 50 µL/well to the cells for rat EP₁ expression vector introduction. The cells to which this complex of rat EP₁ expression vector/Lipofectamine 2000 was dispensed were cultured at 37°C for 24 hours in an incubator under 5% CO₂ gas conditions. After the culture for 24 hours, the cells were used as rat EP₁ receptor-expressing cells in the measurement of intracellular calcium concentrations.

### (3) Study on effect of suppressing in rise in intracellular calcium concentration

The suppressing effect of each test compound on a prostaglandin E₂-induced rise in intracellular calcium concentration was studied by a method shown below using the rat EP₁ receptor-expressing cells.

### Method:

A 10 mM dimethylsulfoxide solution of each test compound was diluted with an assay buffer (20 mM HEPES/Hank's Balanced Salt Solution (HBSS), pH 7.2).

The rat EP₁ receptor-expressing cells were washed with an assay buffer. Pluronic F-127 (Invitrogen Corporation) was mixed at a final concentration of 0.0004% with a fluorescent calcium indicator (Fluo 4-AM (Dojindo Laboratories)), and then, an assay buffer was added to prepare a 4 µmol/L Fluo 4-AM solution. 100 µL of this solution was added to each well and incubated at 37°C for 90 minutes in an incubator. Then, the whole of a cell supernatant was aspirated, and 100 µL of an assay buffer containing 2.5 mM probenecid was added to each well and incubated for 15 minutes in an incubator, followed by measurement of intracellular calcium concentrations.

The intracellular calcium concentrations were measured as fluorescent signals using FlexStation (registered trademark) (manufactured by Molecular Devices, LLC.). After 20 seconds from the start of fluorescent signal reading, 50 µL of each above-described test compound (final concentration: 0.1 nM to 10 µM) diluted with an assay buffer was added to each well, and fluorescent signals were measured for 60 seconds. Then, 50 µL of a prostaglandin E₂ buffer solution was added (final concentration: 10 nM) to each well, and fluorescent signals were measured for 60 seconds.

A concentration that exhibited 50% inhibition was defined as an IC₅₀ value from the dose-response curve of the test compound wherein a fluorescent signal obtained at the time of addition of prostaglandin E₂ when an assay buffer was added instead of the test compound in the method shown above was defmed as 100% and a signal obtained when any of the test compound and prostaglandin E₂ were not added was defmed as 0%. The obtained IC₅₀ value of each test compound is shown in the following Table 1:

**[Table 1]**

| Test compound | IC₅₀(nM) | Test compound | IC₅₀(nM) |
|---|---|---|---|
| Example 2-1 | 56 | Example 3-32 | 43 |
| Example 3-2 | 29 | Example 12-7 | 110 |
| Example 3-3 | 8 | Example 12-17 | 87 |
| Example 3-4 | 11 | Example 12-23 | 48 |
| Example 3-5 | 6 | Example 12-26 | 79 |
| Example 3-6 | 60 | Example 12-36 | 160 |
| Example 3-11 | 21 | Example 12-38 | 39 |
| Example 3-14 | 11 | Example 12-53 | 2.9 |
| Example 3-15 | 39 | Example 23-1 | 62 |
| Example 3-18 | 170 | Example 25-1 | 46 |
| Example 3-21 | 19 | Example 26-1 | 70 |
| Example 3-22 | 19 | Example 28-1 | 2.5 |
| Example 3-24 | 16 | | |

From Table 1, it is obvious that the compound (I) of the present invention or the salt thereof exhibits excellent EP₁ receptor antagonistic activity.

### <Test Example 2>

### Suppressing effect on 17-phenyl trinor prostaglandin E2 (17-PTP)-induced bladder contraction

### (1) Preparation of experimental animal

A 10 w/v% urethane solution was subcutaneously administered (s. c.) at a dose of 1.5 g/kg to a Wistar-type male rat to thereby induce anesthesia. Approximately 40 minutes after the urethane administration, the rat was confirmed to be fully anesthetized, hair in the back, the thighs on both sides, the abdomen, and the neck was shaved, and fixation in a face-down position was performed. After midline incision of the dorsal skin, thoracodorsal muscles on both sides were incised along the spine, and the muscular layer was opened with a retractor to expose the thoracic spine. While the ninth thoracic vertebra was pulled cranially, a small hole was made with bone forceps, and from it, the spinal cord was cut using a soldering iron. At this time, in the case where bleeding was heavy, a gelatin sponge for hemostasis was put into the cut portion. The thoracodorsal incision layer was closed using a surgical adhesive, and then, the rat was fixed in a face-up position. The skin of the left thigh was incised to expose and decorticate the femoral artery, and then, an arterial catheter filled with heparin of 200 heparin units/mL was inserted 15 mm. The left femoral incision layer was closed using a surgical adhesive. The skin of the right thigh was incised to expose and decorticate the femoral artery, which was then ligated. In the case of intravenously administering an agent, the femoral vein on the right side was exposed and decorticated, and then, a venous catheter was inserted. The right femoral incision layer was closed using a surgical adhesive. Midline incision was made in the abdomen to expose ureters on both sides, which were then ligated, and then cut on the renal side. Two Michel forceps were hanged on the top of the bladder at an interval of approximately 3 mm, between which incision was made, and from it, a bladder catheter was inserted 5 mm to perform a purse-string suture. The abdominal incision layer was sutured, and then, the penis was exposed and ligated. Midline incision was made in the neck to expose the trachea where incision was then made and ligated on the peripheral side, and then the end of an Eppendorf tip was placed under the trachea so that the trachea was slightly lifted to thereby secure an airway.

### (2) Experimental method

The rat was placed in a face-up position on a heating pad for small animals, and a rectal temperature probe was inserted from the anus to keep the body temperature at 37.5°C. The body temperature was confirmed to become 37.5°C, then 0.1 mL of a physiological saline solution was injected into the bladder, and intravesical pressure was measured. After approximately 30 minutes, the intravesical pressure was confirmed to be stabilized, and 17-PTP was administered in 5 seconds through the arterial catheter. A test compound was intravenously administered (i. v.) (1 mg/kg) via the venous catheter 5 minutes before 17-PTP administration as to an individual for which it was judged that bladder contraction was stably obtained by intraarterially administering (i. a.) 17-PTP at 30-minute intervals. After the test compound administration, 17-PTP was i. a. administered 9 times (5 minutes, 35 minutes, 65 minutes, 95 minutes, 125 minutes, 155 minutes, 185 minutes, 215 minutes, and 245 minutes later after the test compound administration), and change in bladder contraction pressure was observed. Incidentally, the number of 17-PTP i. a. administration was appropriately changed depending on the strength and sustention of the effect of the test compound.

### (3) Analysis

A value in which a 30-second average intravesical pressure immediately before the 17-PTP administration was subtracted from a 30-second average intravesical pressure around the maximum bladder contraction pressure after the 17-PTP administration was defined as the amount of change (ΔmmHg) in intravesical pressure by 17-PTP. The amount of change in intravesical pressure after the test compound administration relative to an average value of the amounts of changes in intravesical pressure by 17-PTP two times before the test compound administration was indicated by %. The amount of change in intravesical pressure most decreased until 95 minutes after the test compound administration was defined as the effect of the test compound. The obtained amount of change in intravesical pressure of the test compound is shown in the following Table 2.

**[Table 2]**

| Test compound | Amount of change in intravesical pressure(%) |
|---|---|
| Example 3-3 | 53 |

From Table 2, it is obvious that the compound (I) of the present invention or the salt thereof exhibits an excellent suppressing effect on bladder contraction.

### Industrial Applicability

The compound of the present invention has a strong EP₁ receptor antagonistic effect and as such, is useful as a therapeutic agent or a prophylactic agent for diseases or symptoms attributed to the activation of the EP₁ receptor by the stimulating effect of PGE₂. Among others, it is useful as a therapeutic agent for lower urinary tract symptoms (LUTS), particularly, overactive bladder syndrome (OABs), and the like, or a prophylactic agent therefor.

### Sequence Listing Free Text

<SEQ ID NO: 1>
   SEQ ID NO: 1 is the sequence of a forward primer (5' primer) used for amplifying a DNA of SEQ ID NO: 5.
<SEQ ID NO: 2>
   SEQ ID NO: 2 is the sequence of a reverse primer (3' primer) used for amplifying the DNA of SEQ ID NO: 5.
<SEQ ID NO: 3>
   SEQ ID NO: 3 is the sequence of a forward primer (5' primer) used for amplifying the DNA of SEQ ID NO: 5.
<SEQ ID NO: 4>
   SEQ ID NO: 4 is the sequence of a reverse primer (3' primer) used for amplifying the DNA of SEQ ID NO: 5.
<SEQ ID NO: 5>
   SEQ ID NO: 5 is a DNA sequence for expressing a rat EP1 receptor, which was amplified using the primers of SEQ ID NO: 1, SEQ ID NO: 2, SEQ ID NO: 3, and SEQ ID NO: 4.

## Claims

1. A pyrazolopyridine derivative represented by the following formula (I) or a pharmacologically acceptable salt thereof: 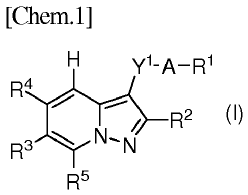 wherein A is a group selected from the group consisting of the following a) to h): 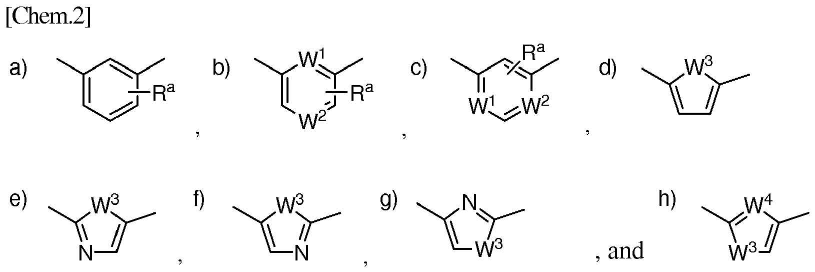 or a group i) constituted together with R¹: 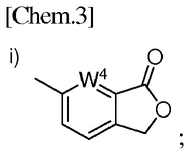 R^{a} is a group selected from the group consisting of the following j) to q):
j) a hydrogen atom,
k) a halogen atom,
l) a hydroxy group,
m) a cyano group,
n) a nitro group,
o) an amino group unsubstituted or substituted by one or two C₁₋₆ alkyl groups,
p) a C₁₋₆ alkyl group unsubstituted or substituted by one or two groups independently selected from the group consisting of a halogen atom, a C₁₋₆ alkoxy group, a C₁₋₆ alkylsulfanyl group, a C₁₋₆ alkylsulfinyl group, a C₁₋₆ alkylsulfonyl group, a hydroxy group, and a cyano group, and
q) a C₁₋₆ alkoxy group unsubstituted or substituted by one or two groups independently selected from the group consisting of a halogen atom, a C₁₋₆ alkoxy group, a C₁₋₆ alkylsulfanyl group, a C₁₋₆ alkylsulfonyl group, a hydroxy group, and a cyano group;
one of W¹ and W² is a nitrogen atom, and the other is -CH= or a nitrogen atom;
W³ is an oxygen atom or a sulfur atom;
W⁴ is -CH= or a nitrogen atom;
Y¹ is C₁₋₆ alkylene;
R¹ is a group selected from the group consisting of the following r) to x):
r) -C(=O)-OZ¹,
s) -C(=O)-NHSO₂Z²,
t) -C(=O)-NHOH,
u) -C(=O)-NHCN,
v) -NH-C(=O)-Z³,
w) an acidic 5-membered heterocyclic group, and
x) a 6-membered ring group substituted by a phenolic hydroxy group;
Z¹ is a hydrogen atom, a C₁₋₆ alkyl group, or a C₇₋₁₀ aralkyl group;
Z² and Z³ are independently a group selected from the group consisting of the following aa) to ee):
aa) a C₁₋₆ alkyl group,
bb) a halo-C₁₋₆ alkyl group,
cc) a C₃₋₆ cycloalkyl group,
dd) an aryl group unsubstituted or in which a ring is substituted by one to five groups independently selected from the group consisting of a halogen atom, a C₁₋₆ alkyl group, a halo-C₁₋₆ alkyl group, and a C₁₋₆ alkoxy group, and
ee) a heterocyclic group;
R² is a group selected from the group consisting of the following A) to H):
A) a branched C₃₋₆ alkyl group,
B) a C₃₋₆ cycloalkyl group unsubstituted or in which a ring is substituted by one C₁₋₆ alkyl group,
C) a phenyl group unsubstituted or in which a ring is substituted by one to five groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, and a cyano group,
D) a 6-membered aromatic heterocyclic group unsubstituted or in which a ring is substituted by one to four groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, and a cyano group,
E) a 5-membered aromatic heterocyclic group unsubstituted or in which a ring is substituted by one to four groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, and a cyano group,
F) an amino group substituted by one or two C₁₋₆ alkyl groups,
G) a 4- to 8-membered cyclic amino group unsubstituted or in which a ring is substituted by one to three groups independently selected from the group consisting of a C₁₋₆ alkyl group and a halogen atom, and
H) a C₁₋₆ alkoxy group;
R³ is a group selected from the group consisting of the following I) to W):
I) a hydrogen atom,
J) a halogen atom,
K) a hydroxy group,
L) a cyano group,
M) a nitro group,
N) a C₃₋₆ cycloalkyl group,
O) a C₂₋₆ alkenyl group,
P) a C₁₋₇ alkanoyl group,
Q) a C₁₋₆ alkylsulfanyl group,
R) a C₁₋₆ alkylsulfinyl group,
S) a C₁₋₆ alkylsulfonyl group,
T) an amino group unsubstituted or substituted by one or two C₁₋₆ alkyl groups,
U) a C₁₋₆ alkyl group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, a benzoyloxy group, and a cyano group,
V) a C₁₋₆ alkoxy group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, and a cyano group, and
W) a phenyl group unsubstituted or in which a ring is substituted by one to five groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, and a cyano group; and
R⁴ and R⁵ each independently represent a hydrogen atom, a halogen atom, a C₁₋₆ alkyl group, or a C₁₋₆ alkoxy group.

2. The pyrazolopyridine derivative or the pharmacologically acceptable salt thereof according to claim 1, wherein in the above formula (I), A is a group selected from the group consisting of the following a), b), d), and h): 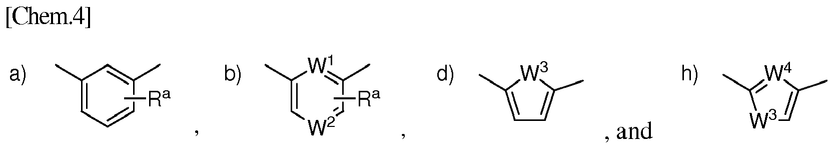 wherein, R^{a}, W¹, W², W³, and W⁴ have the same meanings as above.

3. The pyrazolopyridine derivative or the pharmacologically acceptable salt thereof according to claim 2, wherein in the above formula (I), A is a group selected from the group consisting of the following a), b1), and d1):

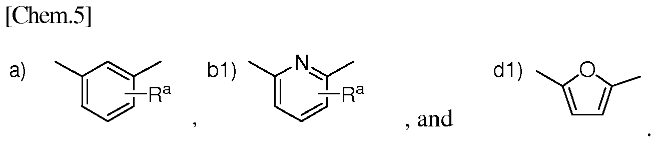

4. The pyrazolopyridine derivative or the pharmacologically acceptable salt thereof according to claim 3, wherein in the above formula (I), Y¹ is methylene.

5. The pyrazolopyridine derivative or the pharmacologically acceptable salt thereof according to claim 4, wherein in the above formula (I), R⁴ and R⁵ are each independently a hydrogen atom, a halogen atom, or a C₁₋₆ alkoxy group.

6. The pyrazolopyridine derivative or the pharmacologically acceptable salt thereof according to claim 5, wherein in the above formula (I), Z¹ is a hydrogen atom or a C₁₋₆ alkyl group, and Z² is a C₁₋₆ alkyl group.

7. The pyrazolopyridine derivative or the pharmacologically acceptable salt thereof according to claim 6, wherein in the above formula (I), R² is a group selected from the group consisting of the following A, B), C1), D1), E1), and G):
A) a branched C₃₋₆ alkyl group,
B) a C₃₋₆ cycloalkyl group unsubstituted or in which a ring is substituted by one C₁₋₆ alkyl group,
C1) a phenyl group unsubstituted or in which a ring is substituted by one to five groups independently selected from the group consisting of a halogen atom, a C₁₋₆ alkyl group, a halo-C₁₋₆ alkyl group, a C₁₋₆ alkoxy group, a halo-C₁₋₆ alkoxy group, and a cyano group,
D1) a 6-membered aromatic heterocyclic group,
E1) a 5-membered aromatic heterocyclic group, and
G) a 4- to 8-membered cyclic amino group unsubstituted or in which a ring is substituted by one to three groups independently selected from the group consisting of a C₁₋₆ alkyl group and a halogen atom.

8. The pyrazolopyridine derivative or the pharmacologically acceptable salt thereof according to claim 7, wherein in the above formula (I), R² is a group selected from the group consisting of the following A), B), C1), D2), E2), and G1):
A) a branched C₃₋₆ alkyl group,
B) a C₃₋₆ cycloalkyl group unsubstituted or in which a ring is substituted by one C₁₋₆ alkyl group,
C1) a phenyl group unsubstituted or in which a ring is substituted by one to five groups independently selected from the group consisting of a halogen atom, a C₁₋₆ alkyl group, a halo-C₁₋₆ alkyl group, a C₁₋₆ alkoxy group, a halo-C₁₋₆ alkoxy group, and a cyano group,
D2) a pyridyl group,
E2) a thienyl group, and
G1) a morpholinyl group.

9. The pyrazolopyridine derivative or the pharmacologically acceptable salt thereof according to claim 7, wherein in the above formula (I), R³ is a group selected from the group consisting of the following I) to L), N), O) to Q), and T) to W):
I) a hydrogen atom,
J) a halogen atom,
K) a hydroxy group,
L) a cyano group,
N) a C₃₋₆ cycloalkyl group,
O) a C₂₋₆ alkenyl group,
P) a C₁₋₇ alkanoyl group,
Q) a C₁₋₆ alkylsulfanyl group,
T) an amino group unsubstituted or substituted by one or two C₁₋₆ alkyl groups,
U) a C₁₋₆ alkyl group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, a benzoyloxy group, and a cyano group,
V) a C₁₋₆ alkoxy group unsubstituted or substituted by one to three groups independently selected from the group consisting of a halogen atom, a C₁₋₆ alkoxy group, a halo-C₁₋₆ alkoxy group, a C₁₋₆ alkylsulfanyl group, a C₁₋₇ alkanoyl group, a C₁₋₆ alkylsulfonyl group, a hydroxy group, and a cyano group, and
W) a phenyl group unsubstituted or in which a ring is substituted by one to five groups independently selected from the group consisting of a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, and a cyano group.

10. The pyrazolopyridine derivative or the pharmacologically acceptable salt thereof according to claim 9, wherein in the above formula (I), R³ is a hydrogen atom, a halogen atom, a cyano group, a C₃₋₆ cycloalkyl group, a C₂₋₆ alkenyl group, a C₁₋₇ alkanoyl group, an amino group, a methylamino group, a halo-C₁₋₆ alkyl group, a C₁₋₆ alkylsulfanyl-C₁₋₆ alkyl group, a C₁₋₆ alkylsulfonyl-C₁₋₆ alkyl group, a hydroxy-C₁₋₆ alkyl group, a benzoyloxy-C₁₋₆ alkyl group, a C₁₋₆ alkyl group, a C₁₋₆ alkoxy group, or a phenyl group.

11. The pyrazolopyridine derivative or the pharmacologically acceptable salt thereof according to claim 9, wherein in the above formula (I), R^{a} is a group selected from the group consisting of the following j), k), o), p1), and q1):
j) a hydrogen atom,
k) a halogen atom,
o) an amino group unsubstituted or substituted by one or two C₁₋₆ alkyl groups,
p1) a C₁₋₆ alkyl group unsubstituted or substituted by one or two groups independently selected from the group consisting of a C₁₋₆ alkoxy group, a C₁₋₆ alkylsulfonyl group, and a hydroxy group, and
q1) a C₁₋₆ alkoxy group unsubstituted or substituted by one or two groups independently selected from the group consisting of a C₁₋₆ alkoxy group, a C₁₋₆ alkylsulfonyl group, and a hydroxy group.

12. The pyrazolopyridine derivative or the pharmacologically acceptable salt thereof according to claim 11, wherein in the above formula (I), R^{a} is a hydrogen atom, a halogen atom, a C₁₋₆ alkyl group, a hydroxy-C₁₋₆ alkyl group, or a C₁₋₆ alkoxy group.

13. The pyrazolopyridine derivative or the pharmacologically acceptable salt thereof according to claim 1, wherein the compound represented by the above formula (I) is
3-[(6-chloro-2-phenylpyrazolo[1,5-a]pyridin-3-yl)methyl]benzoic acid,
6-[(6-chloro-2-phenylpyrazolo[1,5-a]pyridin-3-yl)methyl]pyridine-2-carboxylic acid,
3-[(6-methoxy-2-phenylpyrazolo[1,5-a]pyridin-3-yl)methyl]benzoic acid,
6-[(6-methoxy-2-phenylpyrazolo[1,5-a]pyridin-3-yl)methyl]pyridine-2-carboxylic acid,
6-[(2-phenyl-6-vinylpyrazolo[1,5-a]pyridin-3-yl)methyl]pyridine-2-carboxylic acid,
6-[(2-*tert*-butyl-6-methoxypyrazolo[1,5-a]pyridin-3-yl)methyl]pyridine-2-carboxylic acid,
6-[[2-phenyl-6-(trifluoromethyl)pyrazolo[1,5-a]pyridin-3-yl]methyl]pyridine-2-carboxylic acid,
6-[(6-methyl-2-phenylpyrazolo[1,5-a]pyridin-3-yl)methyl]pyridine-2-carboxylic acid,
6-[[2-(3-fluorophenyl)-6-methoxypyrazolo[1,5-a]pyridin-3-yl]methyl]pyridine-2-carboxylic acid,
6-[[6-chloro-2-(3-fluorophenyl)pyrazolo[1,5-a]pyridin-3-yl]methyl]pyridine-2-carboxylic acid,
6-methoxy-2-phenyl-3-[[6-(1*H*-tetrazol-5-yl)pyridin-2-yl]methyl]pylazolo[1,5-a]pyridine,
6-chloro-2-phenyl-3-[[6-(1*H*-tetrazol-5-yl)pyridin-2-yl]methyl]pyrazolo[1,5-a]pyridine,
5-[(6-methoxy-2-phenylpyrazolo[1,5-a]pyridin-3-yl)methyl]furan-2-carboxylic acid,
6-[(6-bromo-2-phenylpyrazolo[1,5-a]pyridin-3-yl)methyl]pyridine-2-carboxylic acid,
6-[(2-cyclopentyl-6-methoxypyrazolo[1,5-a]pyridin-3-yl)methyl]pyridine-2-carboxylic acid,
6-[[2-(4-fluorophenyl)-6-methoxypyrazolo[1,5-a]pyridin-3-yl]methyl]pyridine-2-carboxylic acid,
6-[[6-methoxy-2-(thiophen-3-yl)pyrazolo[1,5-a]pyridin-3-yl]methyl]pyridine-2-carboxylic acid,
2-[(6-chloro-2-phenylpyrazolo[1,5-a]pyridin-3-yl)methyl]thiazole-4-carboxylic acid,
6-[[6-(methylthio)-2-phenylpyrazolo[1,5-a]pyridin-3-yl]methyl]pyridine-2-carboxylic acid,
6-[(2-*tert*-butyl-6-chloropyrazolo[1,5-a]pyridin-3-yl)methyl]pyridine-2-carboxylic acid,
6-[[6-chloro-2-(piperidin-1-yl)pyrazolo[1,5-a]pyridin-3-yl]methyl]pyridine-2-carboxylic acid,
6-[[6-chloro-2-(1-methylcyclopropyl)pyrazolo[1,5-a]pyridin-3-yl]methyl]pyridine-2-carboxy lic acid,
6-[(6-chloro-2-phenylpyrazolo[1,5-a]pyridin-3-yl)methyl]-*N*-(methylsulfonyl)pyridine-2-car boxamide,
3-[6-[(6-chloro-2-phenylpyrazolo[1,5-a]pyridin-3-yl)methyl]pyridin-2-yl]-1,2,4-oxadiazol-5( 4*H*)-one, or
3-[6-[(6-chloro-2-phenylpyrazolo[1,5-a]pyridin-3-yl)methyl]pyridin-2-yl]-1,2,4-oxadiazol-5( 4*H*)-thione.

14. A pharmaceutical composition comprising the pyrazolopyridine derivative or the pharmacologically acceptable salt thereof according to any one of claims 1 to 13 as an active ingredient.

15. An EP₁ receptor antagonist comprising the pyrazolopyridine derivative or the pharmacologically acceptable salt thereof according to any one of claims 1 to 13 as an active ingredient.

16. A therapeutic or prophylactic agent for lower urinary tract symptoms comprising the pyrazolopyridine derivative or the pharmacologically acceptable salt thereof according to any one of claims 1 to 13 as an active ingredient.

17. A method for treating or preventing lower urinary tract symptoms, comprising administering a pharmaceutical composition comprising the pyrazolopyridine derivative or the pharmacologically acceptable salt thereof according to any one of claims 1 to 13 as an active ingredient to a patient.

18. Use of the pyrazolopyridine derivative or the pharmacologically acceptable salt thereof according to any one of claims 1 to 13 for the manufacture of a pharmaceutical composition for prevention or treatment of lower urinary tract symptoms.

19. The pyrazolopyridine derivative or the pharmacologically acceptable salt thereof according to any one of claims 1 to 13 for use in prevention or treatment of lower urinary tract symptoms.
